(19) Europäisches Patentamt
European Patent Office
Office européen des brevets

(11) EP 3 733 670 A1

(12) EUROPEAN PATENT APPLICATION
published in accordance with Art. 153(4) EPC

(43) Date of publication:
**04.11.2020 Bulletin 2020/45**

(21) Application number: **18894394.8**

(22) Date of filing: **26.12.2018**

(51) Int Cl.:
***C07D 487/04*** *(2006.01)* ***C07D 493/04*** *(2006.01)*
***C07D 495/04*** *(2006.01)* ***C07D 498/04*** *(2006.01)*
***C07D 513/00*** *(2006.01)* ***A61P 25/18*** *(2006.01)*

(86) International application number:
**PCT/CN2018/123751**

(87) International publication number:
**WO 2019/129025 (04.07.2019 Gazette 2019/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.12.2017 CN 201711435683**

(71) Applicant: **Mediconns (Shanghai) Biopharmaceutical Co., Ltd**
**Shanghai 200031 (CN)**

(72) Inventors:
- **DANG, Zhu**
  **Shanghai 200031 (CN)**
- **CAI, Christine Jue**
  **Shanghai 200031 (CN)**
- **LUO, Zhen**
  **Shanghai 200031 (CN)**
- **WANG, Liugang**
  **Shanghai 200031 (CN)**
- **BAO, Dan**
  **Shanghai 200031 (CN)**

(74) Representative: **Dehns**
**St. Bride's House**
**10 Salisbury Square**
**London EC4Y 8JD (GB)**

# (54) TETRAHYDROPYRROLE COMPOUND, PREPARATION METHOD THEREFOR, PHARMACEUTICAL COMPOSITION CONTAINING SAME, AND USE THEREOF

(57) The present invention discloses a tetrahydropyrrole compound, a preparation method therefor, a pharmaceutical composition containing the same, and a use thereof. The tetrahydropyrrole compound of the present invention is represented by general formula (I). The tetrahydropyrrole compound of the present invention has better inhibitory effects on the positive symptoms of schizophrenia, and the potency thereof is equivalent to or slightly stronger than that of the positive drug olanzapine. In addition, the compound of the present invention has dual inhibitory effects on D2 receptors and DAT receptors, and is effective for treating schizophrenia and improving negative symptoms and cognitive functions, while also reducing vertebral side effects and prolactin secretion.

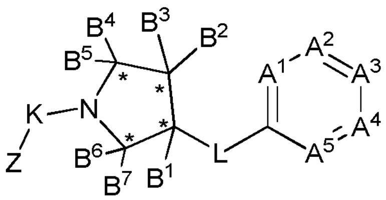

(I)

EP 3 733 670 A1

## Description

The present application claims the priority of Chinese patent application CN 201711435683.3 filed on December 26, 2017. The aforementioned Chinese patent application is incorporated into the present application by reference in its entirety.

## Technical Field

The present invention relates to a tetrahydropyrrole compound, a preparation method therefor, a pharmaceutical composition containing the same, and a use thereof.

## Background Arts

Schizophrenic disorder or schizophrenia is a very serious mental disease, which is characterized by lack of connection with reality, hallucinations, delusions and abnormal thinking, and obvious damage to social function. Schizophrenic disorder is a worldwide public health problem, and has a global total prevalence rate of about 0.8%-1%.

The peak age of schizophrenic disorder is 18-25 years old for men and 26-45 years old for women. However, it is not uncommon for children or adolescents and patients with onset in their later years. Different patients have different severity of symptoms and clinical manifestations. Schizophrenic disorder can be classified into three types: positive symptoms, negative symptoms, and cognitive deficits.

Positive symptoms are characterized by hallucinations and delusions, agitation, paranoia, thinking disorders and behavioral abnormalities; negative symptoms are characterized by emotional retardation, silence, lack of interest, lack of pleasure and loneliness; cognitive deficits are characterized by inability to concentrate, severe memory decline and inability to act according to plan.

A patient with schizophrenic disorder can have one or all of the above symptoms, which are often more serious and obviously affect the patient's work, interpersonal communication and even personal life. The general purpose of treating schizophrenic disorder is to reduce symptoms, avoid recurrence, restore functional defects and improve rehabilitation as much as possible.

At present, there are many hypotheses about the pathogenesis of schizophrenic disorder, among which the hypothesis of brain dopaminergic nervous system hyperfunction is the traditional hypothesis of schizophrenic disorder, and it is believed that the pathogenesis may be related to dopamine dysfunction in the brain. Dopamine (DA) is a catecholamine neurotransmitter, and its biological activity is mediated by G protein coupled receptor (GPCR). 5 dopamine receptor subtypes $D_1$-$D_5$ have been found in human. Dopamine transporter (DAT) is a glycoprotein located in the presynaptic membrane of dopamine neurons. Reuptake of dopamine from synaptic space into the presynaptic membrane is the main way to terminate the physiological effect of dopamine.

Dopamine has several pathways in the brain, of which the mesolimbic pathway and the nigtostriatal pathway are related to mental, emotional, emotive and other behaviors. The third pathway is the hypophyseal - infundibular pathway, which is responsible for the endocrine function of the anterior pituitary. The fourth pathway is the nigro-striatal pathway, which belongs to the extrapyramidal system and coordinates movement.

When the dopamine receptors in the mesolimbic pathway are inhibited, then the effect of anti-schizophrenia positive symptoms can be produced; when the dopamine receptors in the nigro-striatal pathway are inhibited, then side effects in the extrapyramidal system are produced; blocking the dopamine receptors in cerebral cortex system will produce negative symptoms; blocking the dopamine pathway in the hypophyseal - infundibular pathway will lead to endocrine changes.

The first generation of anti-schizophrenia drugs are also called typical antipsychotic drugs which mainly include selective dopamine D2 receptor inhibitors, but are often accompanied by serious side effects in the extrapyramidal system. The second generation of anti-schizophrenia drugs are also called atypical antipsychotic drugs which mainly include serotonin 5-HT2A/5-HT2C receptor blockers and dopamine D2 receptor inhibitors, have therapeutic effects on the positive symptoms of schizophrenia similar to those of the first generation of anti-schizophrenia drugs, but have obviously smaller side effects in the extrapyramidal system.

At present, the first and second generation of therapeutic drugs for schizophrenia used clinically have good therapeutic effects on the positive symptoms of schizophrenia, and can reduce or eliminate symptoms such as delusions, hallucinations and thinking disorders. After the acute symptoms are eliminated, maintaining the use of antipsychotic drugs can reduce the possibility of recurrence. However, almost all clinical drugs have no significant therapeutic effects on the negative symptoms of schizophrenia, cognitive impairment and memory impairment, which leads to a decrease in the quality of life of patients.

Kulagowski et al. (J. Med. Chem. 1996, 39, 1941-1942) reported the activity of a piperidine compound L741626 with 4-phenyl and 4-hydroxyl substitutions as a dopamine D2 receptor antagonist, but no DAT inhibitory activity was

reported. Sikazwe et al. (Bioorg. Med. Chem. 17 (2009) 1716-1723) reported a tetrahydropyrrole compound 4 with 3-phenyl and 3-hydroxyl substitutions, which showed moderate intensity of D4 receptor antagonism, but substantially no antagonism to D2 receptor. The structures of the compound L741626 and the compound 4 are as follows:

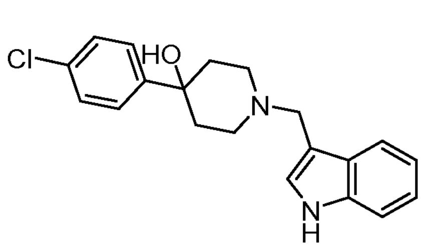

**L741626**

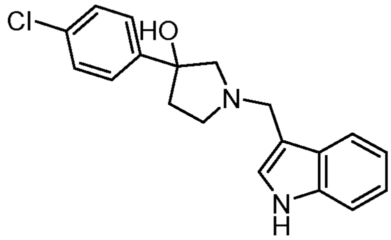

**Compound 4**

J. Med. Chem. 1996, 39, 1941-1942 Bioorg. Med. Chem. 17 (2009) 1716-1723

At present, no other compounds with similar structures have been reported as dual antagonists or inhibitors of D2 receptor and DAT receptor.

## Content of the Present Invention

The present invention provide a tetrahydropyrrole compound, a preparation method therefor, a pharmaceutical composition containing the same, and a use thereof. The tetrahydropyrrole compound of the present invention has better inhibitory effects on the positive symptoms of schizophrenia, and the potency thereof is equivalent to or slightly stronger than that of the positive drug olanzapine. In addition, the compound of the present invention has dual inhibitory effects on D2 receptors and DAT receptors, and is effective for treating schizophrenia and improving negative symptoms and cognitive functions, while also reducing vertebral side effects and prolactin secretion.

The present invention provides a tetrahydropyrrole compound represented by general formula (I), enantiomer, diastereomer, isotope compound, pharmaceutically acceptable prodrug, pharmaceutically acceptable ester or pharmaceutically acceptable salt thereof:

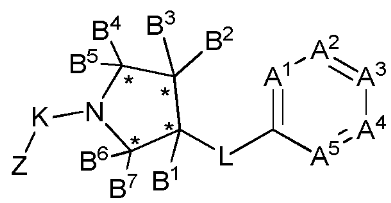

(I)

wherein:

$A^1$ is C-$R^1$ or N;

$A^2$ is C-$R^{1a}$ or N;

$A^3$ is C-$R^{1b}$ or N;

$A^4$ is C-$R^{1c}$ or N;

$A^5$ is C-$R^{1d}$ or N;

no more than 3 nitrogen atoms are present in $A^1$, $A^2$, $A^3$, $A^4$ and $A^5$;

$R^1$, $R^{1a}$, $R^{1b}$, $R^{1c}$ and $R^{1d}$ are each independently a hydrogen atom, halogen, cyano, nitro, substituted or unsubstituted $C_1$-$C_4$alkyl, substituted or unsubstituted $C_1$-$C_4$alkoxy, substituted or unsubstituted $C_3$-$C_8$cycloalkyl, substituted or

unsubstituted $C_6$-$C_{14}$aryl, substituted or unsubstituted $C_2$-$C_{10}$heteroaryl,

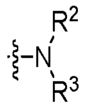 , 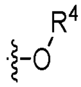 , 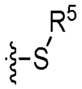 , 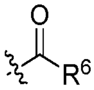 ,

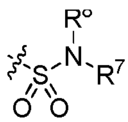 , 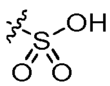 , 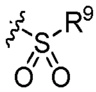 or 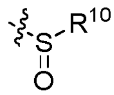 ,

or, adjacent $R^1$ and $R^{1a}$; or $R^{1a}$ and $R^{1b}$; or $R^{1b}$ and $R^{1c}$; or $R^{1c}$ and $R^{1d}$ and the atoms attached thereto together form substituted or unsubstituted $C_3$-$C_8$cycloalkyl, substituted or unsubstituted $C_2$-$C_8$heterocyclyl, substituted or unsubstituted $C_6$-$C_{14}$aryl,or substituted or unsubstituted $C_2$-$C_{10}$heteroaryl; the heteroatoms in the $C_2$-$C_8$heterocyclyl are selected from N, O and S, the number of the heteroatoms is 1-3, and when the number of the heteroatoms is 2 or 3, then the heteroatoms can be the same or different; the $C_2$-$C_8$heterocyclylis a saturated $C_2$-$C_8$heterocyclylor an unsaturated $C_2$-$C_8$heterocyclyl, the ring atoms are selected from two, three or four of C, N, O and S, and when the ring atom is C or S, then the C or S can be formed with oxygen into

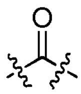 or 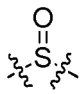 ;

$R^2$ and $R^3$ are each independently a hydrogen atom, hydroxyl, amino, substituted or unsubstituted $C_1$-$C_4$alkyl, substituted or unsubstituted $C_1$-$C_4$alkoxy, substituted or unsubstituted $C_3$-$C_8$cycloalkyl, substituted or unsubstituted $C_6$-$C_{14}$aryl, substituted or unsubstituted $C_2$-$C_{10}$heteroaryl,

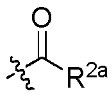 , 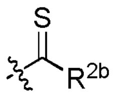 or 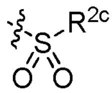 ;

$R^{2a}$ and $R^{2b}$ are each independently a hydrogen atom, substituted or unsubstituted $C_1$-$C_4$alkyl, substituted or unsubstituted $C_1$-$C_4$alkoxy, substituted or unsubstituted $C_3$-Cscycloalkyl, substituted or unsubstituted $C_6$-$C_{14}$aryl, substituted or unsubstituted $C_2$-$C_{10}$heteroaryl, hydroxyl or

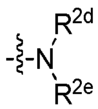 ;

$R^{2c}$ is substituted or unsubstituted $C_1$-$C_4$alkyl, substituted or unsubstituted $C_1$-$C_4$alkoxy, substituted or unsubstituted $C_3$-$C_8$cycloalkyl, substituted or unsubstituted $C_6$-$C_{14}$aryl, or substituted or unsubstituted $C_2$-$C_{10}$heteroaryl;

$R^{2d}$ and $R^{2e}$ are each independently a hydrogen atom, substituted or unsubstituted $C_1$-$C_4$alkyl, substituted or unsubstituted $C_1$-$C_4$alkoxy, substituted or unsubstituted $C_3$-Cscycloalkyl, substituted or unsubstituted $C_6$-$C_{14}$aryl, or substituted or unsubstituted $C_2$-$C_{10}$heteroaryl;

$R^4$ and $R^5$ are each independently a hydrogen atom, substituted or unsubstituted $C_1$-$C_4$alkyl, substituted or unsubstituted $C_1$-$C_4$alkoxy, substituted or unsubstituted $C_3$-Cscycloalkyl, substituted or unsubstituted $C_6$-$C_{14}$aryl, substituted or unsubstituted $C_2$-$C_{10}$heteroaryl,

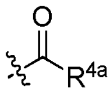 or 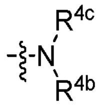 ;

$R^{4a}$ is a hydrogen atom, substituted or unsubstituted $C_1$-$C_4$alkyl, substituted or unsubstituted $C_2$-$C_4$alkenyl, substituted or unsubstituted $C_2$-$C_4$alkynyl, substituted or unsubstituted $C_1$-$C_4$alkoxy, substituted or unsubstituted $C_3$-$C_8$cycloalkyl, substituted or unsubstituted $C_6$-$C_{14}$aryl, substituted or unsubstituted $C_2$-$C_{10}$heteroaryl or

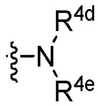 ;

$R^{4b}$, $R^{4c}$, $R^{4d}$ and $R^{4e}$ are each independently a hydrogen atom, substituted or unsubstituted $C_1$-$C_4$alkyl, substituted or unsubstituted $C_3$-$C_8$cycloalkyl, substituted or unsubstituted $C_6$-$C_{14}$aryl, or substituted or unsubstituted $C_2$-$C_{10}$heteroaryl;

$R^6$ is a hydrogen atom, substituted or unsubstituted $C_1$-$C_4$alkyl, substituted or unsubstituted $C_3$-$C_8$cycloalkyl, substituted or unsubstituted $C_6$-$C_{14}$aryl, substituted or unsubstituted $C_2$-$C_{10}$heteroaryl,

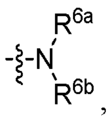 , 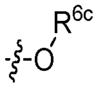 or 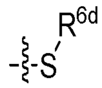 ;

$R^{6a}$, $R^{6b}$, $R^7$ and $R^8$ are each independently a hydrogen atom, amino, hydroxyl, substituted or unsubstituted $C_1$-$C_4$alkyl, substituted or unsubstituted $C_1$-$C_4$alkoxy, substituted or unsubstituted $C_3$-$C_8$cycloalkyl, substituted or unsubstituted $C_6$-$C_{14}$aryl, or substituted or unsubstituted $C_2$-$C_{10}$heteroaryl;

$R^{6c}$ and $R^{6d}$ are each independently a hydrogen atom, amino, substituted or unsubstituted $C_1$-$C_4$alkyl, substituted or unsubstituted $C_1$-$C_4$alkoxy, substituted or unsubstituted $C_3$-$C_8$cycloalkyl, substituted or unsubstituted $C_6$-$C_{14}$aryl, or substituted or unsubstituted $C_2$-$C_{10}$heteroaryl;

$R^9$ and $R^{10}$ are each independently substituted or unsubstituted $C_1$-$C_4$alkyl, substituted or unsubstituted $C_3$-$C_8$cycloalkyl, substituted or unsubstituted $C_6$-$C_{14}$aryl, or substituted or unsubstituted $C_2$-$C_{10}$heteroaryl;

$B^1$ is a hydrogen atom, cyano, halogen, sulfydryl, carboxyl, amino, substituted or unsubstituted $C_1$-$C_4$alkyl, or substituted or unsubstituted $C_3$-$C_8$cycloalkyl;

$B^2$, $B^3$, $B^4$, $B^5$, $B^6$ and $B^7$ are each independently a hydrogen atom, hydroxyl, substituted or unsubstituted $C_1$-$C_4$alkoxy, cyano, halogen, sulfydryl, carboxyl, amino, substituted or unsubstituted $C_1$-$C_4$alkyl, or substituted or unsubstituted $C_3$-$C_8$cycloalkyl;

L and K are each independently $C_1$-$C_4$alkylene, direct bond, $C_2$-$C_4$alkenylene,

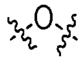 ,

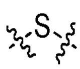 , 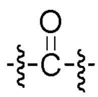 , 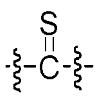 or 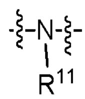 ;

$R^{11}$ is a hydrogen atom, hydroxyl, substituted or unsubstituted $C_1$-$C_4$alkyl, substituted or unsubstituted $C_3$-$C_8$cycloalkyl, substituted or unsubstituted $C_6$-$C_{14}$aryl, substituted or unsubstituted $C_2$-$C_{10}$heteroaryl or

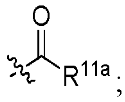

$R^{11a}$ is a hydrogen atom, substituted or unsubstituted $C_1$-$C_4$alkyl, substituted or unsubstituted $C_3$-$C_8$cycloalkyl, substituted or unsubstituted $C_6$-$C_{14}$aryl, or substituted or unsubstituted $C_2$-$C_{10}$heteroaryl;

Z is substituted or unsubstituted $C_2$-$C_{10}$heteroaryl;

the substituents in the substituted $C_1$-$C_4$alkyl ($R^1$, $R^2$, $R^3$, $R^{2a}$, $R^{2b}$, $R^{2c}$ $R^{2d}$ $R^{2e}$ $R^4$, $R^5$, $R^{4a}$, $R^{4b}$, $R^{4c}$, $R^{4d}$, $R^{4e}$, $R^6$, $R^{6a}$, $R^{6b}$, $R^7$, $R^8$, $R^{6c}$, $R^{6d}$, $R^9$, $R^{10}$, $B^1$, $B^2$, $B^3$, $R^{11}$ or $R^{11a}$), the substituted $C_1$-$C_4$alkoxy ($R^{2a}$ $R^{2b}$, $R^{2c}$, $R^{2d}$, $R^{2e}$, $R^4$, $R^5$, $R^{4a}$ $R^{6a}$, $R^{6b}$ $R^7$, $R^8$, $R^{6c}$, $R^{6d}$, $B^2$ or $B^3$), the substituted $C_3$-$C_8$cycloalkyl ($R^1$, $R^2$, $R^3$, $R^{2a}$, $R^{2b}$, $R^{2c}$, $R^{2d}$, $R^{2e}$, $R^4$, $R^5$, $R^{4a}$, $R^{4b}$, $R^{4c}$, $R^{4d}$, $R^{4e}$, $R^6$, $R^{6a}$, $R^{6b}$, $R^7$, $R^8$, $R^{6c}$, $R^{6d}$, $R^9$, $R^{10}$, $B^1$, $B^2$, $B^3$, $R^{11}$ or $R^{11a}$), the $C_6$-$C_{14}$aryl ($R^1$, $R^2$, $R^3$, $R^{2a}$, $R^{2b}$, $R^{2c}$ $R^{2d}$, $R^{2e}$, $R^4$, $R^5$, $R^{4a}$ , $R^{4b}$ $R^{4c}$, $R^{4d}$, $R^{4e}$, $R^6$, $R^{6a}$, $R^{6b}$ $R^7$, $R^8$, $R^{6c}$, $R^{6d}$, $R^9$, $R^{10}$, $R^{11}$ or $R^{11a}$), the $C_2$-$C_{10}$heteroaryl ($R^1$, $R^2$, $R^3$, $R^{2a}$, $R^{2b}$, $R^{2c}$, $R^{2d}$, $R^{2e}$, $R^4$, $R^5$, $R^{4a}$, $R^{4b}$, $R^{4c}$, $R^{4d}$, $R^{4e}$, $R^6$, $R^{6a}$, $R^{6b}$, $R^7$, $R^8$, $R^{6c}$, $R^{6d}$, $R^9$, $R^{10}$, $R^{11}$, $R^{11a}$ or Z), the substituted $C_2$-$C_4$alkenyl ($R^{4a}$), the substituted $C_2$-$C_4$alkynyl ($R^{4a}$) and the substituted $C_2$-$C_8$heterocyclyl are each independently one or more of $C_1$-$C_4$alkyl, $C_1$-$C_4$alkyl substituted with halogen and/or hydroxyl, halogen, hydroxyl, amino, cyano, nitro, sulfydryl and carboxyl; when the substituents are plural, then the substituents can be the same or different;

the heteroatoms in the $C_2$-$C_{10}$heteroaryl are selected from O, N and S, the number of the heteroatoms is 1-3, and the heteroatoms can be the same or different;

carbon labeled with * refers to S-configuration chiral carbon, R-configuration chiral carbon or achiral carbon.

In a preferred embodiment of the invention, when the substituent is $C_1$-$C_4$alkyl, then the $C_1$-$C_4$alkyl is preferably methyl, ethyl, n-propyl, isopropyl, n-butyl, isobutyl or tert-butyl.

In a preferred embodiment of the invention, when the substituent is $C_1$-$C_4$alkyl substituted with halogen and/or hydroxyl, then one or more hydrogen in the $C_1$-$C_4$alkyl in the $C_1$-$C_4$alkyl substituted with halogen and/or hydroxyl are preferably substituted with halogen and/or hydroxyl. The $C_1$-$C_4$alkyl substituted with halogen and/or hydroxyl is preferably

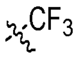 , 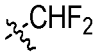 or 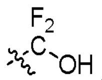 .

In a preferred embodiment of the invention, when the substituent is halogen, then the halogen is preferably F, Cl, Br or I.

In a preferred embodiment of the invention, the substituents in the substituted $C_1$-$C_4$alkyl (in $R^1$, $R^{1a}$, $R^{1b}$, $R^{1c}$, $R^{1d}$, $R^2$, $R^3$, $R^{2a}$, $R^{2b}$, $R^{2c}$, $R^{2d}$, $R^{2e}$, $R^4$, $R^5$, $R^{4a}$, $R^{4b}$, $R^{4c}$, $R^{4d}$, $R^{4e}$, $R^6$, $R^{6a}$, $R^{6b}$, $R^7$, $R^8$, $R^{6c}$, $R^{6d}$, $R^9$, $R^{10}$, $B^1$, $B^2$, $B^3$, $R^{11}$ and $R^{11a}$), the substituted $C_1$-$C_4$alkoxy (in $R^2$, $R^3$, $R^{2a}$, $R^{2b}$, $R^{2c}$, $R^{2d}$, $R^{2e}$, $R^4$, $R^5$, $R^{4a}$, $R^{6a}$, $R^{6b}$, $R^7$, $R^8$, $R^{6c}$, $R^{6d}$, $B^2$ and $B^3$), the substituted $C_3$-$C_8$cycloalkyl (in $R^1$, $R^{1a}$, $R^{1b}$, $R^{1c}$, $R^{1d}$, $R^2$, $R^3$, $R^{2a}$, $R^{2b}$, $R^{2c}$, $R^{2d}$, $R^{2e}$, $R^4$, $R^5$, $R^{4a}$, $R^{4b}$, $R^{4c}$, $R^{4d}$, $R^{4e}$, $R^6$, $R^{6a}$, $R^{6b}$, $R^7$, $R^8$, $R^{6c}$, $R^{6d}$, $R^9$, $R^{10}$, $B^1$, $B^2$, $B^3$, $R^{11}$ and $R^{11a}$), the $C_6$-$C_{14}$aryl (in $R^1$, $R^{1a}$, $R^{1b}$, $R^{1c}$, $R^{1a}$, $R^2$, $R^3$, $R^{2a}$, $R^{2b}$, $R^{2c}$, $R^{2d}$, $R^{2e}$, $R^4$, $R^5$, $R^{4a}$, $R^{4b}$, $R^{4c}$, $R^{4d}$, $R^{4e}$, $R^6$, $R^{6a}$, $R^{6b}$, $R^7$, $R^8$, $R^{6c}$, $R^{6d}$ $R^9$, $R^{10}$, $R^{11}$, $R^{11a}$ and Z), the $C_2$-$C_{10}$heteroaryl (in $R^1$, $R^{1a}$, $R^{1b}$, $R^{1c}$, $R^{1d}$, $R^2$, $R^3$, $R^{2a}$, $R^{2b}$, $R^{2c}$, $R^{2d}$, $R^{2e}$, $R^4$, $R^5$, $R^{4a}$, $R^{4b}$, $R^{4c}$, $R^{4d}$, $R^{4e}$, $R^6$, $R^{6a}$, $R^{6b}$, $R^7$, $R^8$, $R^{6c}$, $R^{6d}$, $R^9$, $R^{10}$, $R^{11}$, $R^{11a}$ and Z), the substituted $C_2$-$C_4$alkenyl ($R^{4a}$), the substituted $C_2$-$C_4$alkynyl ($R^{4a}$) and the substituted $C_2$-$C_8$heterocyclyl are each independently one or more of halogen, hydroxyl, amino, cyano and sulfydryl.

In a preferred embodiment of the invention, the substituents in the substituted $C_1$-$C_4$alkyl (in $R^1$, $R^2$, $R^3$, $R^{2a}$, $R^{2b}$, $R^{2c}$, $R^{2d}$, $R^{2e}$, $R^4$, $R^5$, $R^{4a}$ $R^{4b}$, $R^{4c}$, $R^{4d}$, $R^{4e}$, $R^6$, $R^{6a}$, $R^{6b}$, $R^7$, $R^8$, $R^{6c}$, $R^{6d}$, $R^9$, $R^{10}$, $B^1$, $B^2$, $B^3$, $R^{11}$ and $R^{11a}$), the substituted $C_1$-$C_4$alkoxy (in $R^{2a}$, $R^{2b}$, $R^{2c}$, $R^{2d}$, $R^{2e}$, $R^4$, $R^5$, $R^{4a}$, $R^{6a}$, $R^{6b}$, $R^7$, $R^8$, $R^{6c}$, $R^{6d}$, $B^2$ and $B^3$), the substituted $C_3$-$C_8$cycloalkyl (in $R^1$, $R^2$, $R^3$, $R^{2a}$, $R^{2b}$, $R^{2c}$, $R^{2d}$, $R^{2e}$, $R^4$, $R^5$, $R^{4a}$ $R^{4b}$, $R^{4c}$, $R^{4d}$, $R^{4e}$, $R^6$, $R^{6a}$, $R^{6b}$, $R^7$, $R^8$, $R^{6c}$, $R^{6d}$, $R^9$, $R^{10}$, $B^1$, $B^2$, $B^3$, $R^{11}$ and $R^{11a}$), the $C_6$-$C_{14}$aryl (in $R^1$, $R^2$, $R^3$, $R^{2a}$, $R^{2b}$, $R^{2c}$, $R^{2d}$, $R^{2e}$, $R^4$, $R^5$, $R^{4a}$, $R^{4b}$, $R^{4c}$, $R^{4d}$, $R^{4e}$, $R^6$, $R^{6a}$, $R^{6b}$, $R^7$, $R^8$, $R^{6c}$, $R^{6d}$, $R^9$, $R^{10}$, $R^{11}$ and $R^{11a}$), the $C_2$-$C_{10}$heteroaryl (in $R^1$, $R^2$, $R^3$, $R^{2a}$, $R^{2b}$, $R^{2c}$, $R^{2d}$, $R^{2e}$, $R^4$, $R^5$, $R^{4a}$, $R^{4b}$, $R^{4c}$, $R^{4d}$, $R^{4e}$, $R^6$, $R^{6a}$, $R^{6b}$, $R^7$, $R^8$, $R^{6c}$, $R^{6d}$, $R^9$, $R^{10}$, $R^{11}$, $R^{11a}$ and Z), the substituted $C_2$-$C_4$alkenyl ($R^{4a}$), the substituted $C_2$-$C_4$alkynyl ($R^{4a}$) and the substituted $C_2$-$C_8$heterocyclylare each independently one or more of $C_1$-$C_4$alkyl, $C_3$-$C_8$cycloalkyl, halogen, hydroxyl, amino, cyano and sulfydryl.

In $R^1$, $R^{10}$, $R^{1b}$, $R^{1c}$, $R^{1d}$, $B^1$, $B^2$, $B^3$, $B^4$, $B^5$, $B^6$ and $B^7$, the halogen is preferably F, Cl, Br or I.

In $R^1$, $R^{1a}$, $R^{1b}$, $R^{1c}$, $R^{10}$, $R^2$, $R^3$, $R^{2a}$, $R^{2b}$, $R^{2c}$, $R^{2d}$, $R^{2e}$, $R^4$, $R^5$, $R^{4a}$, $R^{4b}$, $R^{4c}$, $R^{4d}$, $R^{4e}$, $R^6$, $R^{6a}$, $R^{6b}$, $R^7$, $R^8$, $R^{6c}$, $R^{6d}$, $R^9$, $R^{10}$, $B^1$, $B^2$, $B^3$, $R^{11}$ and $R^{11a}$, the $C_1$-$C_4$alkyl in the substituted or unsubstituted $C_1$-$C_4$alkyl is preferably methyl, ethyl, n-propyl, isopropyl, n-butyl, isobutyl or tert-butyl; the substituents in the substituted $C_1$-$C_4$alkyl are preferably one or more of halogen, hydroxyl and $C_3$-$C_8$cycloalkyl;the substituted $C_1$-$C_4$alkyl is preferably

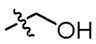, 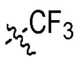, 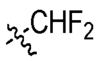, 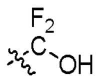, 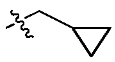, 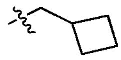

or

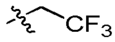.

In $R^2$, $R^3$, $R^{2a}$, $R^{2b}$, $R^{2c}$, $R^{2d}$, $R^{2e}$, $R^4$, $R^5$, $R^{4a}$, $R^{6a}$, $R^{6b}$, $R^7$, $R^8$, $R^{6c}$, $R^{6d}$, $B^2$ and $B^3$, the $C_1$-$C_4$alkoxy in the substituted or unsubstituted $C_1$-$C_4$alkoxy is preferably methoxy, ethoxy, n-propoxy, isopropoxy, n-butoxy, isobutoxy, or tert-butoxy.

In $R^1$, $R^{1a}$, $R^{1b}$, $R^{1c}$, $R^{1d}$, $R^2$, $R^3$, $R^{2a}$, $R^{2b}$, $R^{2c}$, $R^{2d}$, $R^{2e}$, $R^4$, $R^5$, $R^{4a}$, $R^{4b}$, $R^{4c}$, $R^{4d}$, $R^{4e}$, $R^6$, $R^{6a}$, $R^{6b}$, $R^7$ , $R^8$, $R^{6c}$, $R^{6d}$, $R^9$, $R^{10}$, $B^1$, $B^2$, $B^3$, $R^{11}$ and $R^{11a}$, the $C_3$-Cscycloalkyl in the substituted or unsubstituted $C_3$-$C_8$cycloalkyl is preferably cyclopropyl, cyclobutyl, cyclopentyl, cyclohexyl, cycloheptyl or cyclooctyl. In $R^1$, $R^{1a}$, $R^{1b}$, $R^{1c}$, $R^{1d}$, $R^2$, $R^3$, $R^{2a}$, $R^{2b}$, $R^{2c}$, $R^{2d}$, $R^{2e}$, $R^4$, $R^5$, $R^{4a}$, $R^{4b}$, $R^{4c}$, $R^{4d}$, $R^{4e}$, $R^6$, $R^{6a}$ $R^{6b}$, $R^7$, $R^8$, $R^{6c}$, $R^{6d}$, $R^9$, $R^{10}$, $R^{11}$ and $R^{11a}$, the $C_6$-$C_{14}$aryl in the substituted or unsubstituted $C_6$-$C_{14}$aryl is preferably phenyl, naphthyl, anthracyl or phenanthryl.

In $R^1$, $R^{1a}$, $R^{1b}$, $R^{1c}$, $R^{10}$, $R^2$, $R^3$, $R^{2a}$, $R^{2b}$, $R^{2c}$, $R^{2d}$, $R^{2e}$, $R^4$, $R^5$, $R^{4a}$, $R^{4b}$, $R^{4c}$, $R^{4d}$, $R^{4e}$, $R^6$, $R^{6a}$, $R^{6b}$, $R^7$, $R^8$, $R^{6c}$, $R^{6d}$, $R^9$, $R^{10}$, $R^{11}$, $R^{11a}$ and Z, the $C_2$-$C_{10}$heteroaryl in the substituted or unsubstituted $C_2$-$C_{10}$heteroaryl is preferably $C_2$-$C_8$heteroaryl, the $C_2$-$C_8$heteroaryl preferably has 1-2 heteroatoms selected from O, N and S, for example, pyridyl (for example

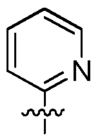, 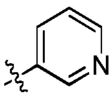 or 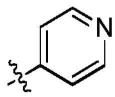

), furanyl (for example

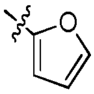

), thienyl (for example

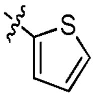

), thiazolyl (for example

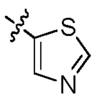, 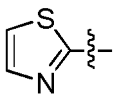 or 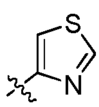

), isothiazolyl (

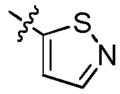, 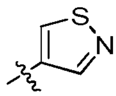 or 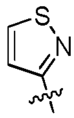

), oxazolyl (for example

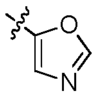,

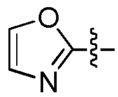 or 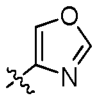

), isoxazolyl (for example

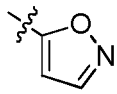, 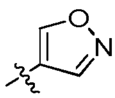 or 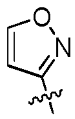

), pyrrolyl (for example

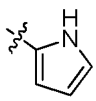

), imidazolyl (for example

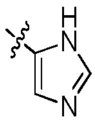

), pyrazolyl (for example

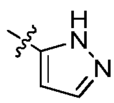, 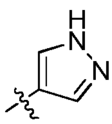 or 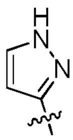

), indolyl (for example

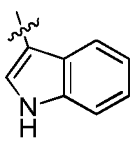, 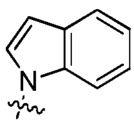,

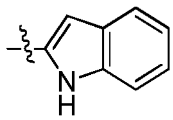, 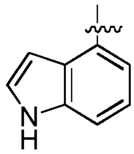, 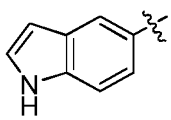, 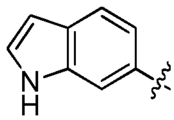 or 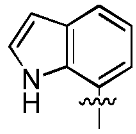

), 4-azaindolyl (for example

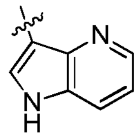

), 5-azaindolyl (for example

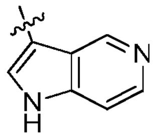

), 6-azaindolyl (for example

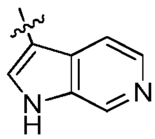

), 7-azaindolyl (for example

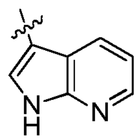

), quinolinyl, isoquinolinyl, quinazolinyl, quinoxalinyl, cinnolinyl, 1,5-naphthyridinyl, 1,6-naphthyridinyl, 1,7-naphthyridinyl, 1,8-naphthyridinyl, purinyl, indazolyl, benzimidazolyl, benzothienyl (for example

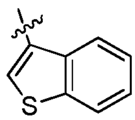

), benzofuranyl (for example

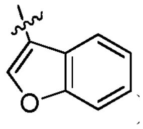

), benzotriazolyl, benzopyrazolyl (for example

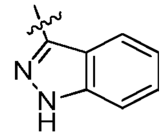

), benzoxazolyl, benzisoxazolyl (for example

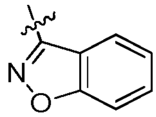

), benzothiazolyl or benzisothiazolyl; The substituents in the substituted $C_2$-$C_{10}$heteroaryl are preferably one or more of halogen and $C_1$-$C_4$alkyl; the substituted $C_2$-$C_{10}$heteroaryl is preferably

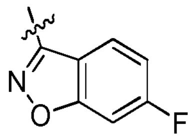

,

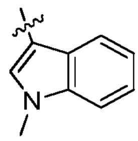

or

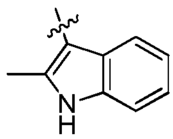

.

When adjacent $R^1$ and $R^{1a}$; or $R^{1a}$ and $R^{1b}$; or $R^{1b}$ and $R^{1c}$; or $R^{1c}$ and $R^{1d}$ and the atoms attached thereto together form substituted or unsubstituted $C_3$-$C_8$cycloalkyl, substituted or unsubstituted $C_6$-$C_{14}$aryl,or substituted or unsubstituted $C_2$-$C_{10}$heteroaryl, then the $C_3$-$C_8$cycloalkyl, the $C_6$-$C_{14}$aryl,or the $C_2$-$C_{10}$heteroaryl are defined as previously described.

In a preferred embodiment of the invention, when adjacent $R^1$ and $R^{1a}$; or $R^{1a}$ and $R^{1b}$; or $R^{1b}$ and $R^{1c}$; or $R^{1c}$ and $R^{1d}$ and the atoms attached thereto together form substituted or unsubstituted $C_2$-$C_8$heterocyclyl and the atom in the ring is C or S, then C can form

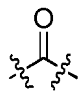

with oxygen, or S can form

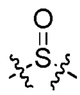

or

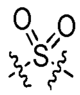

with oxygen.

In a preferred embodiment of the invention, when adjacent $R^1$ and $R^{1a}$; or $R^{1a}$ and $R^{1b}$; or $R^{1b}$ and $R^{1c}$; or $R^{1c}$ and $R^{1d}$ and the atoms attached thereto together form substituted or unsubstituted $C_2$-$C_8$heterocyclyl, then the $C_2$-$C_8$heterocyclylis preferably $C_2$-$C_6$heterocyclyl. The $C_2$-$C_6$ preferably have heteroatoms selected from N, O and S, and the number of the heteroatoms is 2-4, preferably 2-3.

In a preferred embodiment of the invention, when adjacent $R^1$ and $R^{1a}$; or $R^{1a}$ and $R^{1b}$; or $R^{1b}$ and $R^{1c}$; or $R^{1c}$ and $R^{1d}$ and the atoms attached thereto together form substituted or unsubstituted $C_2$-$C_8$heterocyclyl, then the $C_2$-$C_8$heterocyclyl is preferably

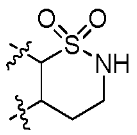, 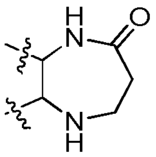, 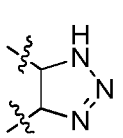, 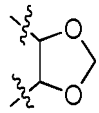, 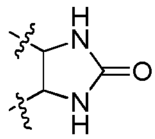, 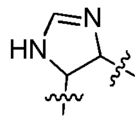, 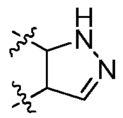,

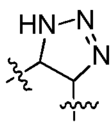, 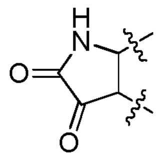 or 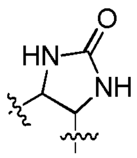.

In a preferred embodiment of the present invention, no more than 1 or 2 of $A^1$, $A^2$, $A^3$, $A^4$ and $A^5$ in the tetrahydropyrrole compound represented by general formula (I) are N.

In another preferred embodiment of the invention,

$A^1$ is C-$R^1$; $A^2$ is C-$R^{1a}$; $A^3$ is C-$R^{1b}$ or N; $A^4$ is C-$R^{1c}$ or N; and $A^5$ is C-$R^{1d}$ or N;

or, $A^1$ is CH; $A^2$ is CH; $A^3$ is C-$R^{1b}$ or N; $A^4$ is C-$R^{1c}$ or N; and $A^5$ is C-$R^{1d}$ or N;

or $A^1$ is C-$R^1$; $A^2$ is C-$R^{1a}$; $A^3$ is C-$R^{1b}$; $A^4$ is C-$R^{1c}$ and $A^5$ is C-$R^{1d}$;

or $A^1$ is C-$R^1$; $A^2$ is CH; $A^3$ is CH; $A^4$ is C-$R^{1c}$ and $A^5$ is CH;

or $A^1$ is CH; $A^2$ is C-$R^{1a}$; $A^3$ is CH; $A^4$ is C-$R^{1c}$ and $A^5$ is CH;

or $A^1$ is CH; $A^2$ is CH; $A^3$ is C-$R^{1b}$; $A^4$ is C-$R^{1c}$ and $A^5$ is CH;

or $A^1$ is CH; $A^2$ is CH; $A^3$ is CH; $A^4$ is C-$R^{1c}$ and $A^5$C-$R^{1d}$; or $R^{1c}$, $R^{1d}$ and the C attached thereto together form substituted or unsubstituted $C_2$-$C_8$heterocyclyl;

or $A^1$ is CH; $A^2$ is CH; $A^3$ is CH; $A^4$ is C-$R^{1c}$ and $A^5$ is CH.

In a preferred embodiment of the invention, in the tetrahydropyrrole compound represented by general formula (I), $R^1$, $R^{1a}$, $R^{1b}$, $R^{1c}$ and $R^{1d}$ are each independently a hydrogen atom, halogen, cyano, nitro, substituted or unsubstituted $C_1$-$C_4$alkyl,

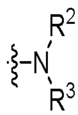, 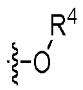, 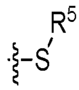, 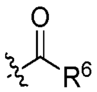, 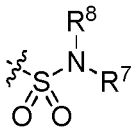, 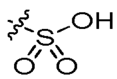, 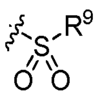, 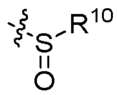,

or adjacent $R^1$ and $R^{1a}$; or $R^{1a}$ and $R^{1b}$; or $R^{1b}$ and $R^{1c}$; or $R^{1c}$ and $R^{1d}$ and the atoms attached thereto together form substituted or unsubstituted $C_3$-$C_8$cycloalkyl, substituted or unsubstituted $C_2$-$C_8$heterocyclyl, substituted or unsubstituted $C_6$-$C_{14}$aryl, or substituted or unsubstituted $C_2$-$C_{10}$heteroaryl; preferably, $R^1$, $R^{1a}$, $R^{1b}$, $R^{1c}$ and $R^{1d}$ are each independently a hydrogen atom, halogen, cyano, nitro, substituted or unsubstituted $C_1$-$C_4$alkyl,

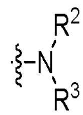, 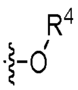, 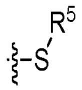, 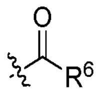, 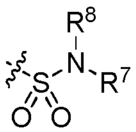, 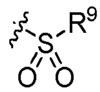, 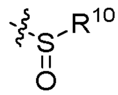,

or, adjacent $R^1$ and $R^{1a}$; or $R^{1a}$ and $R^{1b}$; or $R^{1b}$ and $R^{1c}$; or $R^{1c}$ and $R^{1d}$ and the atoms attached thereto together form substituted or unsubstituted $C_3$-$C_8$cycloalkyl, substituted or unsubstituted $C_2$-$C_8$heterocyclyl, substituted or unsubstituted $C_6$-$C_{14}$aryl, or substituted or unsubstituted $C_2$-$C_{10}$heteroaryl; further preferably, $R^1$, $R^{1a}$, $R^{1b}$, $R^{1c}$ and $R^{1d}$ are each independently a hydrogen atom, halogen, substituted or unsubstituted $C_1$-$C_4$alkyl,

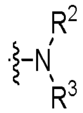, 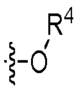, 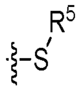, 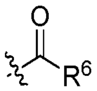, 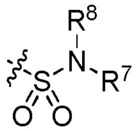, 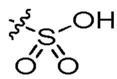, 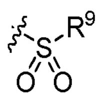 or 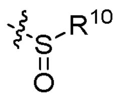,

or adjacent $R^1$ and $R^{1a}$; or $R^{1a}$ and $R^{1b}$; $R^{1c}$ and $R^{1d}$ and the atoms attached thereto together form $C_2$-$C_8$heterocyclyl. In a preferred embodiment of the invention, in the tetrahydropyrrole compound represented by general formula (I), $R^2$ and $R^3$ are each independently a hydrogen atom, hydroxyl, amino, substituted or unsubstituted $C_1$-$C_4$alkyl, substituted or unsubstituted $C_1$-$C_4$alkoxy, substituted or unsubstituted $C_3$-Cscycloalkyl,

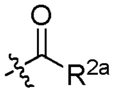, 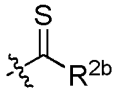 or 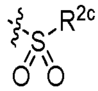 ,

preferably $R^2$ and $R^3$ are each independently a hydrogen atom, hydroxyl, amino, substituted or unsubstituted $C_1$-$C_4$alkyl, $C_1$-$C_4$alkoxy, substituted or unsubstituted $C_3$-$C_8$cycloalkyl,

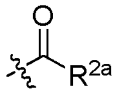

or

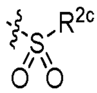 ;

more preferably one of $R^2$ and $R^3$ is hydrogen, the other is substituted or unsubstituted $C_1$-$C_4$alkyl,

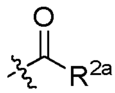 or 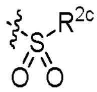 ;

or $R^2$ and $R^3$ are both substituted or unsubstituted $C_1$-$C_4$alkyl; one of $R^2$ and $R^3$ is hydrogen, the other is substituted or unsubstituted $C_1$-$C_4$alkyl,

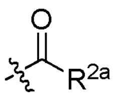 or 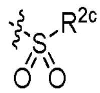 ,

or $R^2$ and $R^3$ are both $C_1$-$C_4$alkyl. In a preferred embodiment of the invention, in the tetrahydropyrrole compound represented by general formula (I), $R^{2a}$ and $R^{2b}$ are each independently a hydrogen atom, substituted or unsubstituted $C_1$-$C_4$alkyl, substituted or unsubstituted $C_1$-$C_4$alkoxy, substituted or unsubstituted $C_3$-$C_8$cycloalkyl or

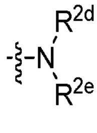 .

In a preferred embodiment of the invention, in the tetrahydropyrrole compound represented by general formula (I), $R^{2a}$ is a hydrogen atom, or substituted or unsubstituted $C_1$-$C_4$alkyl, preferably $R^{2a}$ is $C_1$-$C_4$alkyl.

In a preferred embodiment of the invention, in the tetrahydropyrrole compound represented by general formula (I), $R^{2c}$ is substituted or unsubstituted $C_1$-$C_4$alkyl, substituted or unsubstituted $C_3$-$C_8$cycloalkyl, or substituted or unsubstituted $C_2$-$C_{10}$heteroaryl; preferably, $R^{2c}$ is substituted or unsubstituted $C_1$-$C_4$alkyl, $C_3$-Cscycloalkyl or $C_2$-$C_{10}$heteroaryl.

In a preferred embodiment of the invention, in $R^{2c}$, the substituents in the substituted $C_1$-$C_4$alkyl are preferably selected from one or more of halogen and $C_3$-Cscycloalkyl. In a preferred embodiment of the invention, in the tetrahydropyrrole compound represented by general formula (I), $R^{2d}$ and $R^{2e}$ are independently a hydrogen atom, substituted or unsubstituted $C_1$-$C_4$alkyl, or substituted or unsubstituted $C_3$-$C_8$cycloalkyl.

In a preferred embodiment of the invention, in the tetrahydropyrrole compound represented by general formula (I), R4 and $R^5$ are each independently a hydrogen atom, substituted or unsubstituted $C_1$-$C_4$alkyl, substituted or unsubstituted $C_3$-$C_8$cycloalkyl,

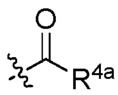 or 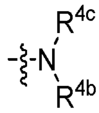

(such as amino); preferably, $R^4$ is a hydrogen atom or

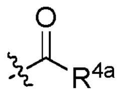;

$R^5$ is a hydrogen atom. In a preferred embodiment of the invention, in the tetrahydropyrrole compound represented by general formula (I), $R^{4a}$ is a hydrogen atom, substituted or unsubstituted $C_1$-$C_4$alkyl, substituted or unsubstituted $C_3$-$C_8$cycloalkyl or

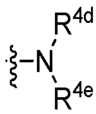,

preferably a hydrogen atom, substituted or unsubstituted $C_1$-$C_4$alkyl, or substituted or unsubstituted $C_3$-$C_8$cycloalkyl, further preferably a hydrogen atom or $C_1$-$C_4$alkyl.

In a preferred embodiment of the invention, in the tetrahydropyrrole compound represented by general formula (I), $R^{4b}$, $R^{4c}$, $R^{4d}$ and $R^{4e}$ are each independently a hydrogen atom, substituted or unsubstituted $C_1$-$C_4$alkyl, or substituted or unsubstituted $C_3$-$C_8$cycloalkyl.

In a preferred embodiment of the invention, in the tetrahydropyrrole compound represented by general formula (I), $R^4$ can also be

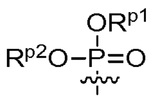,

wherein $R^{p1}$ and $R^{p2}$ are independently a substituted or unsubstituted $C_1$-$C_4$alkyl, preferably are independently $C_1$-$C_4$alkyl.

In a preferred embodiment of the invention, in the tetrahydropyrrole compound represented by general formula (I), $R^6$ is a hydrogen atom, substituted or unsubstituted $C_3$-$C_8$cycloalkyl,

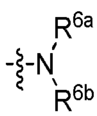, 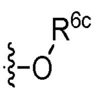 or 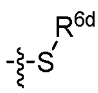;

preferably

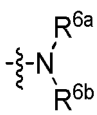

or

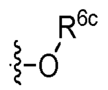

In a preferred embodiment of the invention, in the tetrahydropyrrole compound represented by general formula (I), $R^{6a}$ and $R^{6b}$ are each independently a hydrogen atom, amino, hydroxyl, substituted or unsubstituted $C_1$-$C_4$alkyl, substituted or unsubstituted $C_1$-$C_4$alkoxy, or substituted or unsubstituted $C_3$-$C_8$cycloalkyl, more preferably are each independently a hydrogen atom, amino, hydroxyl, substituted or unsubstituted $C_1$-$C_4$alkyl, or substituted or unsubstituted $C_3$-$C_8$cycloalkyl, further preferably are each independently a hydrogen atom, substituted or unsubstituted $C_1$-$C_4$alkyl, or substituted or unsubstituted $C_3$-$C_8$cycloalkyl; still further preferably are independently a hydrogen atom or $C_1$-$C_4$alkyl.

In a preferred embodiment of the invention, in the tetrahydropyrrole compound represented by general formula (I), $R^{6c}$ and $R^{6d}$ are each independently a hydrogen atom, substituted or unsubstituted $C_1$-$C_4$alkyl, or substituted or unsubstituted $C_3$-$C_8$cycloalkyl, preferably $R^{6c}$ and $R^{6d}$ is H.

In a preferred embodiment of the invention, in the tetrahydropyrrole compound represented by general formula (I), $R^7$ and $R^8$ are each independently a hydrogen atom, amino, hydroxyl, substituted or unsubstituted $C_1$-$C_4$alkyl, or substituted or unsubstituted $C_3$-$C_8$cycloalkyl, preferably are each independently a hydrogen atom, substituted or unsubstituted $C_1$-$C_4$alkyl, or substituted or unsubstituted $C_3$-$C_8$cycloalkyl, more preferably are independently a hydrogen atom or $C_1$-$C_4$alkyl.

In a preferred embodiment of the invention, in the tetrahydropyrrole compound represented by general formula (I), $R^9$ and $R^{10}$ are each independently a substituted or unsubstituted $C_1$-$C_4$alkyl, substituted or unsubstituted $C_3$-$C_8$cycloalkyl or substituted or unsubstituted $C_2$-$C_{10}$heteroaryl.

In a preferred embodiment of the invention, in the tetrahydropyrrole compound represented by general formula (I), $R^9$ is substituted or unsubstituted $C_1$-$C_4$alkyl.

In a preferred embodiment of the invention, in the tetrahydropyrrole compound represented by general formula (I), $R^{10}$ is $C_1$-$C_4$alkyl.

In a preferred embodiment of the invention, $R^1$, $R^{1a}$, $R^{1b}$, $R^{1c}$ and $R^{1d}$ are each independently a hydrogen atom, halogen, substituted or unsubstituted $C_1$-$C_4$alkyl,

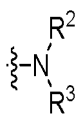, 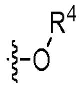, 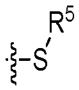, 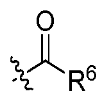, 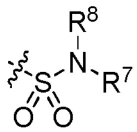, 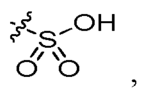, 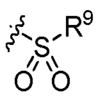 or 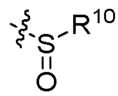,

wherein:

in $R^1$, $R^{1a}$, $R^{1b}$, $R^{1c}$ and $R^{1d}$, the substituents in the substituted $C_1$-$C_4$alkyl are selected from one or more of hydroxyl and halogen;

one of $R^2$ and $R^3$ is hydrogen, the other is substituted or unsubstituted $C_1$-$C_4$alkyl,

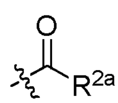 or 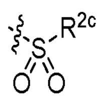,

or $R^2$ and $R^3$ are both $C_1$-$C_4$alkyl; $R^{2a}$ is $C_1$-$C_4$alkyl; $R^{2c}$ is substituted or unsubstituted $C_1$-$C_4$alkyl, $C_3$-$C_8$cycloalkyl or $C_2$-$C_{10}$heteroaryl, in $R^{2c}$, the substituents in the substituted $C_1$-$C_4$alkyl are selected from one or more of halogen and $C_3$-$C_8$cycloalkyl;

$R^4$ is a hydrogen atom,

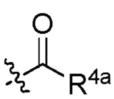 or 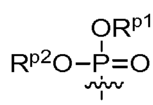;

$R^{4a}$ is a hydrogen atom or $C_1$-$C_4$alkyl; $R^{p1}$ and $R^{p2}$ are independently $C_1$-$C_4$alkyl;

$R^5$ is a hydrogen atom;

$R^6$ is

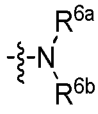 or 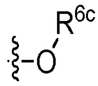 ;

$R^{6a}$ and $R^{6b}$ are a hydrogen atom or $C_1$-$C_4$alkyl; $R^6$ is H; $R^7$ and $R^8$ are each independently a hydrogen atom or $C_1$-$C_4$alkyl; $R^9$ is substituted or unsubstituted $C_1$-$C_4$alkyl; and $R^{10}$ is $C_1$-$C_4$alkyl.

In a preferred embodiment of the invention, $R^1$, $R^{1a}$, $R^{1b}$, $R^{1c}$ and $R^{1d}$ are each independently H,

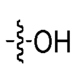, 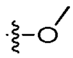, Cl, F, 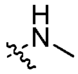, 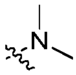, 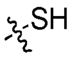, 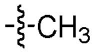, 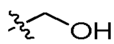,

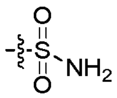, 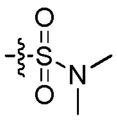, 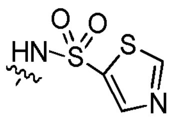, 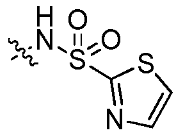, 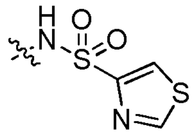, 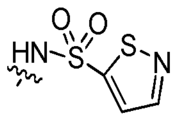,

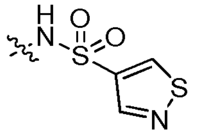, 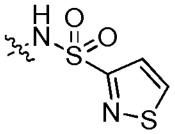, 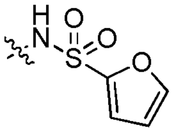, 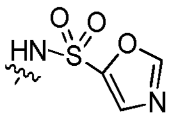, 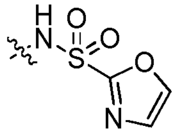,

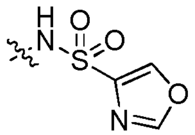, 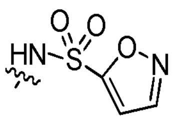, 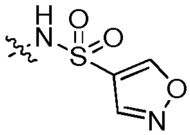, 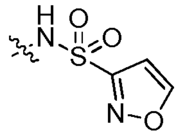, 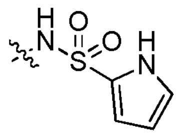,

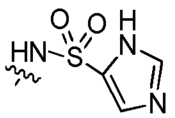, 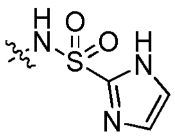, 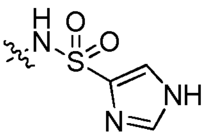, 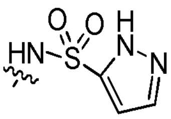, 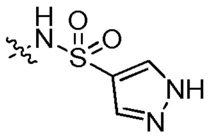,

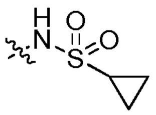, 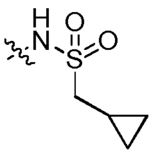, 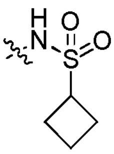, 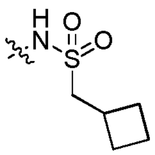, 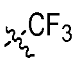, 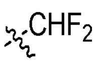, 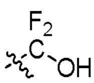,

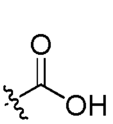, 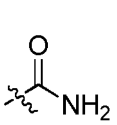, 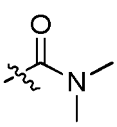, , , , ,

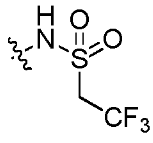, 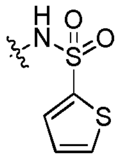, 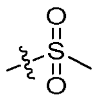, 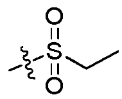, 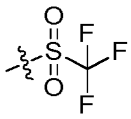, 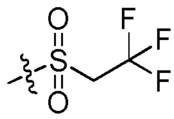, 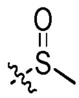,

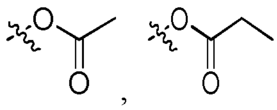 or 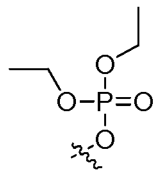.

In a preferred embodiment of the invention,

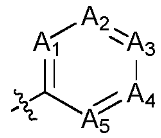

is preferably

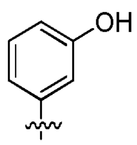, 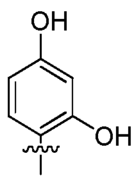, 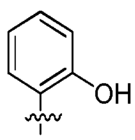, 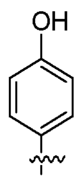, 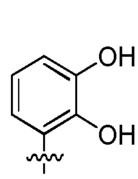, 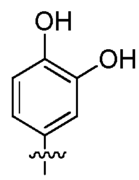, 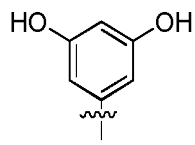,

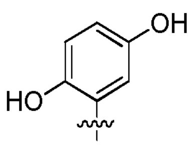, 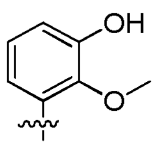, 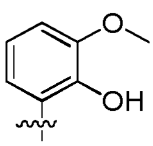, 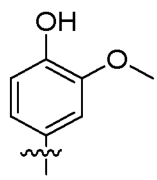, 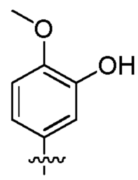, 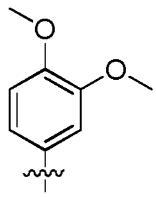,

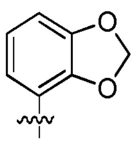, 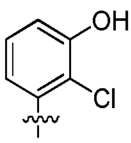, 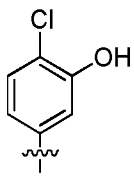, 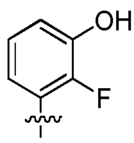, 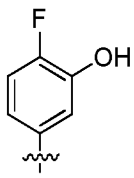, 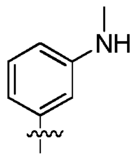, 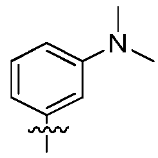,

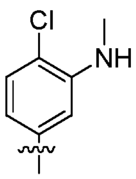, 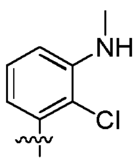, 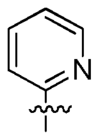, 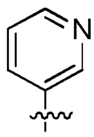, 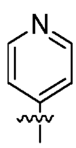, 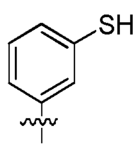, 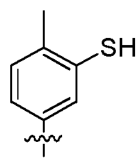,

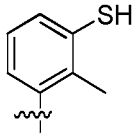, 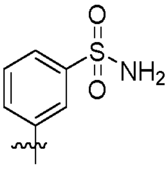, 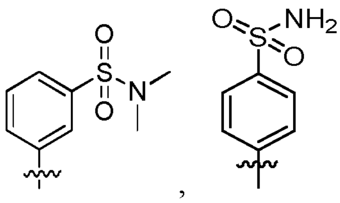, 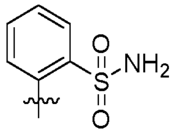, 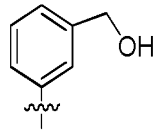,

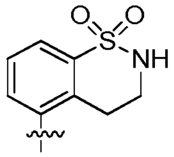, 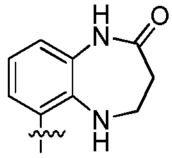, 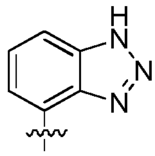, 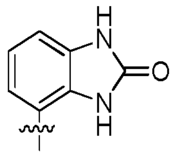, 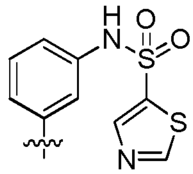,

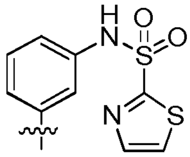, 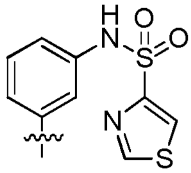, 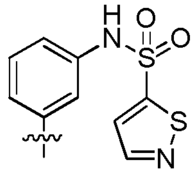, 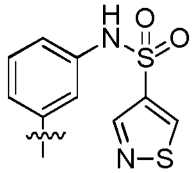, 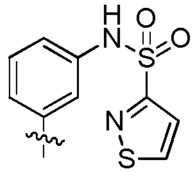,

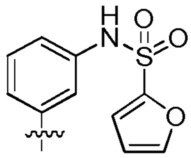, 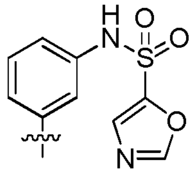, 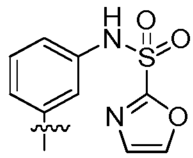, 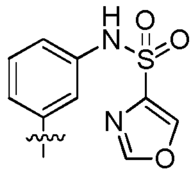, 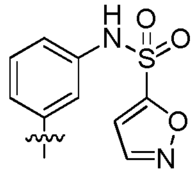,

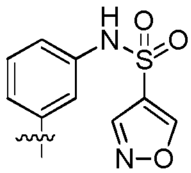, 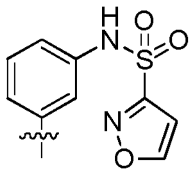, 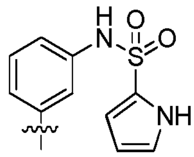, 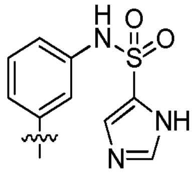, 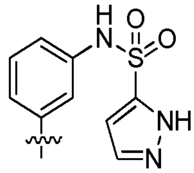,

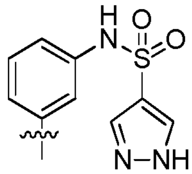, 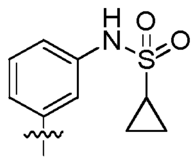, 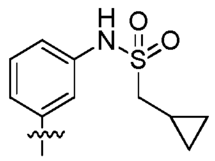, 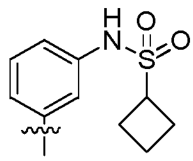,

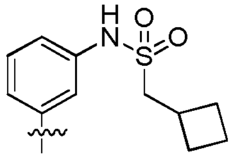, 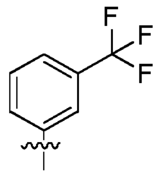, 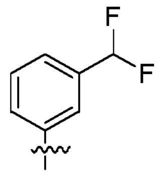, 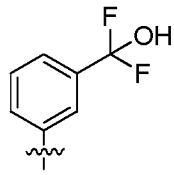, 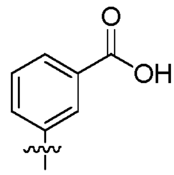,

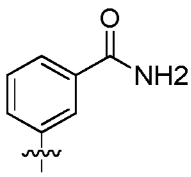, 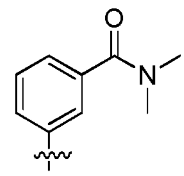, 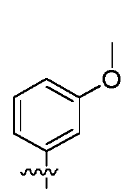, 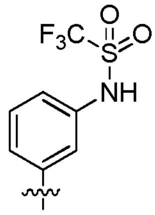, 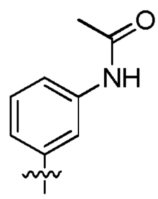, 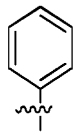,

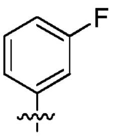 , 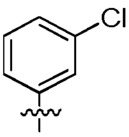 , 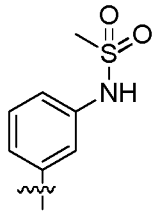 , 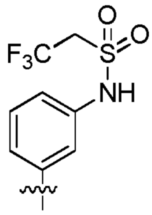 , 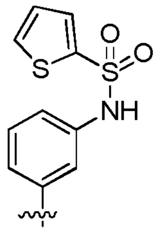 , 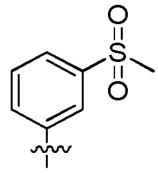 ,

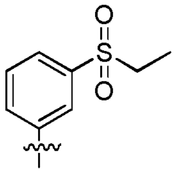 , 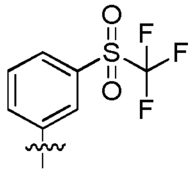 , 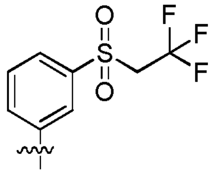 , 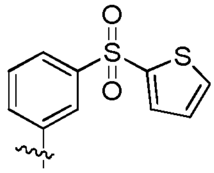 , 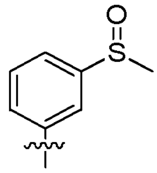 ,

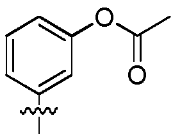 , 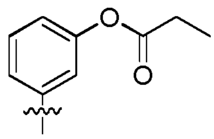 , 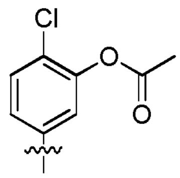 or 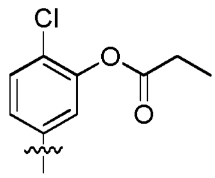 .

In a preferred embodiment of the invention,

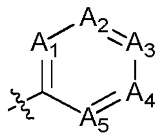

is further preferably

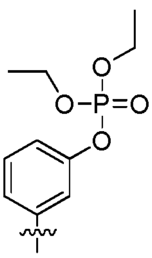 , 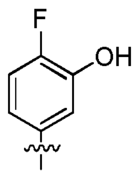 , 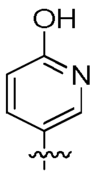 , 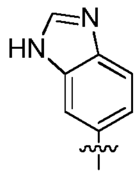 , 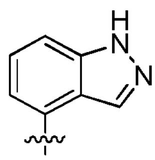 , 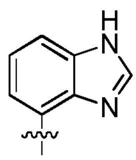 , 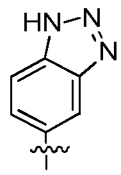 ,

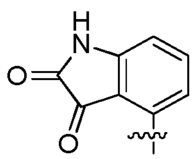 , 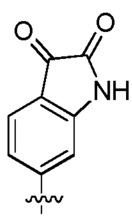 or 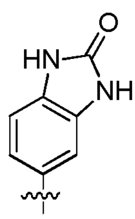 .

In a preferred embodiment of the invention, in the tetrahydropyrrole compound represented by general formula (I), $B^1$ is a hydrogen atom, cyano, halogen, sulfydryl, amino, or substituted or unsubstituted $C_1$-$C_4$alkyl, preferably a hydrogen atom, cyano, halogen, or substituted or unsubstituted $C_1$-$C_4$alkyl; Preferably $B^1$ is a hydrogen atom.

In a preferred embodiment of the invention, in the tetrahydropyrrole compound represented by general formula (I), $B^2$, $B^3$, $B^4$, $B^5$, $B^6$ and $B^7$ are each independently a hydrogen atom, hydroxyl, $C_1$-$C_4$alkoxy, cyano, halogen, sulfydryl, carboxyl, amino, or substituted or unsubstituted $C_1$-$C_4$alkyl; preferably $B^2$, $B^3$, $B^4$, $B^5$, $B^6$ and $B^7$ are hydrogen atoms.

In a preferred embodiment of the invention, in the tetrahydropyrrole compound represented by general formula (I), $B^1$, $B^2$, $B^3$, $B^4$, $B^5$, $B^6$ and $B^7$ are all H.

In a preferred embodiment of the invention, in the tetrahydropyrrole compound represented by general formula (I), L and K are each independently $C_1$-$C_4$alkylene, direct bond,

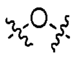, 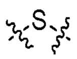, 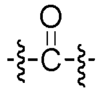 or 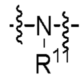,

preferably

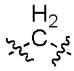,

direct bond,

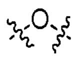, 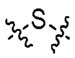, 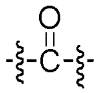 or 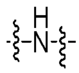.

In a preferred embodiment of the invention, in the tetrahydropyrrole compound represented by general formula (I), L is a direct bond.

In a preferred embodiment of the invention, in the tetrahydropyrrole compound represented by general formula (I), K is

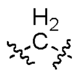.

In a preferred embodiment of the invention, in the tetrahydropyrrole compound represented by general formula (I), $R^{11}$ is a hydrogen atom, hydroxyl, substituted or unsubstituted $C_1$-$C_4$alkyl or

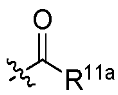.

In a preferred embodiment of the invention, in the tetrahydropyrrole compound represented by general formula (I), $R^{11a}$ is a hydrogen atom, substituted or unsubstituted $C_1$-$C_4$alkyl, or substituted or unsubstituted $C_3$-$C_8$cycloalkyl.

In a preferred embodiment of the invention, in the tetrahydropyrrole compound represented by general formula (I), Z is substituted or unsubstituted $C_2$-$C_{10}$heteroaryl containing at least one nitrogen atom, preferably substituted or unsubstituted $C_6$-$C_8$heteroaryl containing at least one nitrogen atom; more preferably substituted or unsubstituted $C_6$-$C_8$heteroaryl with 1 or 2 heteroatoms selected from N, O and S; the $C_6$-$C_8$heteroaryl is preferably a heteroaryl with two fused rings, more preferably a heteroaryl with a heteroaromatic ring fused to an aromatic ring.

In a preferred embodiment of the invention, in the tetrahydropyrrole compound represented by general formula (I), in Z, the substituents in the substituted $C_2$-$C_{10}$heteroaryl are selected from one or more of halogen and $C_1$-$C_4$alkyl.

In a preferred embodiment of the invention, in the tetrahydropyrrole compound represented by general formula (I), Z is preferably

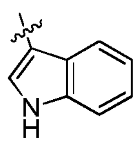, 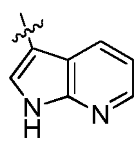,

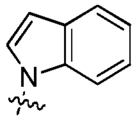, 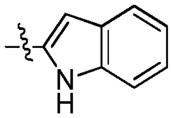, 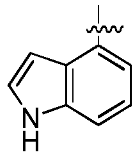, 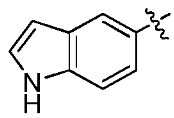, 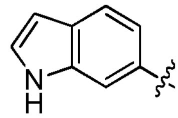, 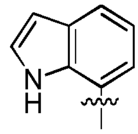,

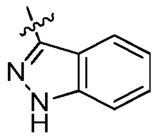, 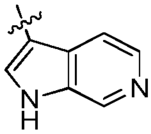, 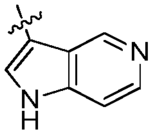, 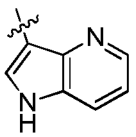, 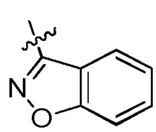, 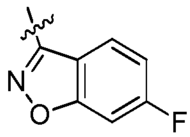,

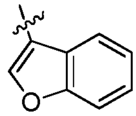, 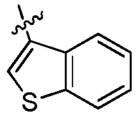, 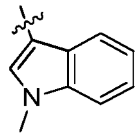 or 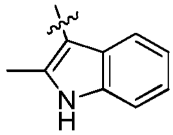.

In a preferred embodiment of the invention, in the tetrahydropyrrole compound represented by general formula (I),

$A^1$ is C-$R^1$; $A^2$ is C-$R^{1a}$; $A^3$ is C-$R^{1b}$ or N; $A^4$ is C-$R^{1c}$ or N; and $A^5$ is C-$R^{1d}$ or N;
$R^1$, $R^{1a}$, $R^{1b}$, $R^{1c}$ and $R^{1d}$ are each independently a hydrogen atom, halogen, cyano, nitro, substituted or unsubstituted $C_1$-$C_4$alkyl,

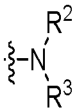, 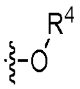, 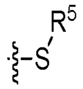, 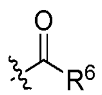, 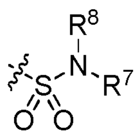,

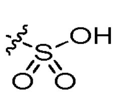, 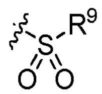, 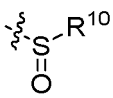,

or, adjacent $R^1$ and $R^{1a}$; or $R^{1a}$ and $R^{1b}$; or $R^{1b}$ and $R^{1c}$; or $R^{1c}$ and $R^{1d}$ and the atoms attached thereto together form substituted or unsubstituted $C_3$-$C_8$cycloalkyl, substituted or unsubstituted $C_2$-$C_8$heterocyclyl, substituted or unsubstituted $C_6$-$C_{14}$aryl, or substituted or unsubstituted $C_2$-$C_{10}$heteroaryl;
$R^2$ and $R^3$ are each independently a hydrogen atom, hydroxyl, amino, substituted or unsubstituted $C_1$-$C_4$alkyl, substituted or unsubstituted $C_1$-$C_4$alkoxy, substituted or unsubstituted $C_3$-$C_8$cycloalkyl,

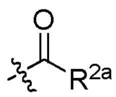, 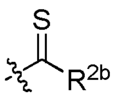 or 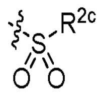;

$R^{2a}$ and $R^{2b}$ are each independently a hydrogen atom, substituted or unsubstituted $C_1$-$C_4$alkyl, substituted or unsubstituted $C_1$-$C_4$alkoxy, substituted or unsubstituted $C_3$-Cscycloalkyl or

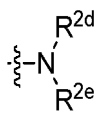;

$R^{2a}$ is a hydrogen atom, or substituted or unsubstituted $C_1$-$C_4$alkyl;
$R^{2c}$ is substituted or unsubstituted $C_1$-$C_4$alkyl, substituted or unsubstituted $C_3$-Cscycloalkyl, or substituted or unsubstituted $C_2$-$C_{10}$heteroaryl;

$R^{2d}$ and $R^{2e}$ are independently a hydrogen atom, substituted or unsubstituted $C_1$-$C_4$alkyl, or substituted or unsubstituted $C_3$-$C_8$cycloalkyl;
$R^4$ and $R^5$ are each independently a hydrogen atom, substituted or unsubstituted $C_1$-$C_4$alkyl, substituted or unsubstituted $C_3$-$C_8$cycloalkyl,

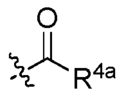 or 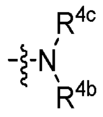

(such as amino);
$R^{4a}$ is a hydrogen atom, substituted or unsubstituted $C_1$-$C_4$alkyl, substituted or unsubstituted $C_3$-$C_8$cycloalkyl or

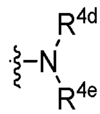 ;

$R^{4b}$, $R^{4c}$, $R^{4d}$ and $R^{4e}$ are each independently a hydrogen atom, substituted or unsubstituted $C_1$-$C_4$alkyl, or substituted or unsubstituted $C_3$-$C_8$cycloalkyl;
$R^6$ is a hydrogen atom, substituted or unsubstituted $C_1$-$C_4$alkyl, substituted or unsubstituted $C_3$-$C_8$cycloalkyl,

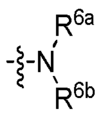 , 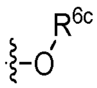 or 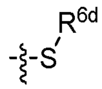 ;

$R^{6a}$ and $R^{6b}$ are each independently a hydrogen atom, amino, hydroxyl, substituted or unsubstituted $C_1$-$C_4$alkyl, substituted or unsubstituted $C_1$-$C_4$alkoxy, or substituted or unsubstituted $C_3$-$C_8$cycloalkyl;
$R^{6c}$ and $R^{6d}$ are each independently a hydrogen atom, substituted or unsubstituted $C_1$-$C_4$alkyl, or substituted or unsubstituted $C_3$-$C_8$cycloalkyl;
$R^7$ and $R^8$ are each independently a hydrogen atom, amino, hydroxyl, substituted or unsubstituted $C_1$-$C_4$alkyl, or substituted or unsubstituted $C_3$-$C_8$cycloalkyl;
$R^9$ and $R^{10}$ are each independently substituted or unsubstituted $C_1$-$C_4$alkyl, substituted or unsubstituted $C_3$-$C_8$cycloalkyl or substituted or unsubstituted $C_2$-$C_{10}$heteroaryl;
$B^1$ is a hydrogen atom, cyano, halogen, sulfydryl, amino, or substituted or unsubstituted $C_1$-$C_4$alkyl;
$B^2$, $B^3$, $B^4$, $B^5$, $B^6$ and $B^7$ are each independently a hydrogen atom, hydroxyl, $C_1$-$C_4$alkoxy, cyano, halogen, sulfydryl, carboxyl, amino, or substituted or unsubstituted $C_1$-$C_4$alkyl;
L and K are each independently $C_1$-$C_4$alkylene, direct bond,

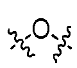 , 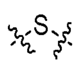 , 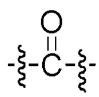

or

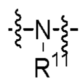 ,

preferably

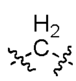 ,

direct bond,

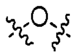, 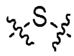, 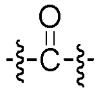 or 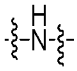;

$R^{11}$ is a hydrogen atom, hydroxyl, substituted or unsubstituted $C_1$-$C_4$alkyl or

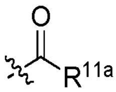;

$R^{11a}$ is a hydrogen atom, substituted or unsubstituted $C_1$-$C_4$alkyl, or substituted or unsubstituted $C_3$-$C_8$cycloalkyl; and Z is substituted or unsubstituted $C_2$-$C_{10}$heteroaryl containing at least one nitrogen atom.

In a preferred embodiment of the invention, in the tetrahydropyrrole compound represented by general formula (I),
$A^1$ is CH; $A^2$ is CH; $A^3$ is C-$R^{1b}$ or N; $A^4$ is C-$R^{1c}$ or N; and $A^5$ is C-$R^{1d}$ or N;
or $A^1$ is C-$R^{1a}$; $A^2$ is C-$R^{1a}$; $A^3$ is C-$R^{1b}$; $A^4$ is C-$R^{1c}$ and $A^5$ is C-$R^{1d}$;
$R^{1a}$ and $R^{1b}$; or $R^{1b}$ and $R^{1c}$; or $R^{1c}$ and $R^{1d}$ and the atoms attached thereto together form substituted or unsubstituted $C_3$-$C_8$cycloalkyl, substituted or unsubstituted $C_2$-$C_8$heterocyclyl, substituted or unsubstituted $C_6$-$C_{14}$aryl, or substituted or unsubstituted $C_2$-$C_{10}$heteroaryl; preferably, $R^1$, $R^{1a}$, $R^{1b}$, $R^{1c}$ and $R^{1d}$ are each independently a hydrogen atom, halogen, cyano, nitro, substituted or unsubstituted $C_1$-$C_4$alkyl, $C_1$-$C_4$alkoxy,

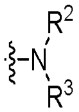, 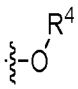, 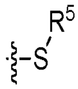, 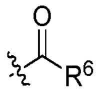, 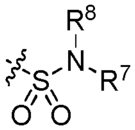, 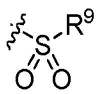, 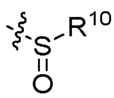;

or, adjacent $R^1$ and $R^{1a}$; or $R^{1a}$ and $R^{1b}$; or $R^{1b}$ and $R^{1c}$; or $R^{1c}$ and $R^{1d}$ and the atoms attached thereto together form substituted or unsubstituted $C_3$-$C_8$cycloalkyl, substituted or unsubstituted $C_2$-$C_8$heterocyclyl, substituted or unsubstituted $C_6$-$C_{14}$aryl, or substituted or unsubstituted $C_2$-$C_{10}$heteroaryl;
$R^2$ and $R^3$ are each independently a hydrogen atom, hydroxyl, amino, substituted or unsubstituted $C_1$-$C_4$alkyl, $C_1$-$C_4$alkoxy, substituted or unsubstituted $C_3$-$C_8$cycloalkyl,

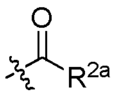 or 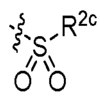;

$R^{2a}$ is a hydrogen atom, or substituted or unsubstituted $C_1$-$C_4$alkyl;
$R^{2c}$ is substituted or unsubstituted $C_1$-$C_4$alkyl, substituted or unsubstituted $C_3$-Cscycloalkyl, or substituted or unsubstituted $C_2$-$C_{10}$heteroaryl;
$R^4$ is a hydrogen atom or

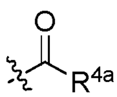;

$R^5$ is a hydrogen atom;
$R^6$ is a hydrogen atom, substituted or unsubstituted $C_1$-$C_4$alkyl, substituted or unsubstituted $C_3$-$C_8$cycloalkyl,

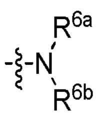, 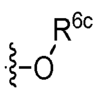 or 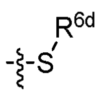;

$R^{6a}$ and $R^{6b}$ are each independently a hydrogen atom, amino, hydroxyl, substituted or unsubstituted $C_1$-$C_4$alkyl, or substituted or unsubstituted $C_3$-$C_8$cycloalkyl;

$R^{6c}$ and $R^{6d}$ are each independently a hydrogen atom, substituted or unsubstituted $C_1$-$C_4$alkyl, or substituted or unsubstituted $C_3$-$C_8$cycloalkyl;

$R^7$ and $R^8$ are each independently a hydrogen atom, substituted or unsubstituted $C_1$-$C_4$alkyl, or substituted or unsubstituted $C_3$-$C_8$cycloalkyl;

$R^9$ and $R^{10}$ are each independently substituted or unsubstituted $C_1$-$C_4$alkyl, substituted or unsubstituted $C_3$-$C_8$cycloalkyl or substituted or unsubstituted $C_2$-$C_{10}$heteroaryl;

$B^1$, $B^2$, $B^3$, $B^4$, $B^5$, $B^6$ and $B^7$ are hydrogen atoms;

L and K are each independently

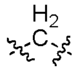,

direct bond,

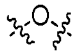, 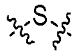, 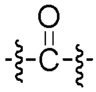 or 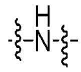;

and

Z is substituted or unsubstituted $C_2$-$C_{10}$heteroaryl containing at least one nitrogen atom; the substituents in the substituted $C_2$-$C_{10}$heteroaryl are selected from one or more of halogen and $C_1$-$C_4$alkyl.

In a preferred embodiment of the invention, in the tetrahydropyrrole compound represented by general formula (I),

$A^1$ is C-$R^1$; $A^2$ is CH; $A^3$ is CH; $A^4$ is C-$R^{1c}$ and $A^5$ is CH;

or $A^1$ is CH; $A^2$ is C-$R^{1a}$; $A^3$ is CH; $A^4$ is C-$R^{1c}$ and $A^5$ is CH;

or $A^1$ is CH; $A^2$ is CH; $A^3$ is C-$R^{1b}$; $A^4$ is C-$R^{1c}$ and $A^5$ is CH;

or $A^1$ is CH; $A^2$ is CH; $A^3$ is CH; $A^4$ is C-$R^{1c}$ and $A^5$C-$R^{1d}$; or $R^{1c}$, $R^{1d}$ and the C attached thereto together form substituted or unsubstituted $C_2$-$C_8$heterocyclyl;

or $A^1$ is CH; $A^2$ is CH; $A^3$ is CH; $A^4$ is C-$R^{1c}$ and $A^5$ is CH;

$R^1$, $R^{1a}$, $R^{1b}$, $R^{1c}$ and $R^{1d}$ are each independently a hydrogen atom, halogen, cyano, nitro, substituted or unsubstituted $C_1$-$C_4$alkyl, $C_1$-$C_4$alkoxy,

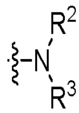, 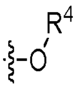, 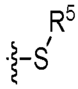, 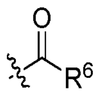,

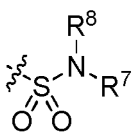, 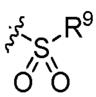, 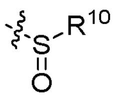;

one of $R^2$ and $R^3$ is hydrogen, the other is substituted or unsubstituted $C_1$-$C_4$alkyl,

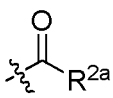 or 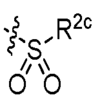;

or $R^2$ and $R^3$ are both substituted or unsubstituted $C_1$-$C_4$alkyl;

$R^{2a}$ is a hydrogen atom, or substituted or unsubstituted $C_1$-$C_4$alkyl;

$R^{2c}$ is substituted or unsubstituted $C_1$-$C_4$alkyl, substituted or unsubstituted $C_3$-Cscycloalkyl, or substituted or unsubstituted $C_2$-$C_{10}$heteroaryl;

$R^4$ and $R^5$ are hydrogen atoms;
$R^6$ is a hydrogen atom, substituted or unsubstituted $C_1$-$C_4$alkyl, substituted or unsubstituted $C_3$-$C_8$cycloalkyl,

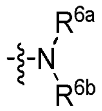, 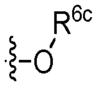 or 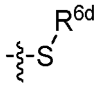 ;

$R^{6a}$ and $R^{6b}$ are each independently a hydrogen atom, substituted or unsubstituted $C_1$-$C_4$alkyl, or substituted or unsubstituted $C_3$-$C_8$cycloalkyl;
$R^{6c}$ and $R^{6d}$ are each independently a hydrogen atom, substituted or unsubstituted $C_1$-$C_4$alkyl, or substituted or unsubstituted $C_3$-$C_8$cycloalkyl;
$R^7$ and $R^8$ are each independently a hydrogen atom, substituted or unsubstituted $C_1$-$C_4$alkyl, or substituted or unsubstituted $C_3$-$C_8$cycloalkyl;
$R^9$ and $R^{10}$ are each independently substituted or unsubstituted $C_1$-$C_4$alkyl, substituted or unsubstituted $C_3$-$C_8$cycloalkyl or substituted or unsubstituted $C_2$-$C_{10}$heteroaryl;
$B^1$ is a hydrogen atom;
$B^2$, $B^3$, $B^4$, $B^5$, $B^6$ and $B^7$ are hydrogen atoms;
L is a direct bond;
K is

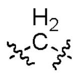;

and Z is substituted or unsubstituted $C_2$-$C_{10}$heteroaryl containing at least one nitrogen atom; the substituents in the substituted $C_2$-$C_{10}$heteroaryl are selected from one or more of halogen and $C_1$-$C_4$alkyl.

In a preferred embodiment of the invention, in the tetrahydropyrrole compound represented by general formula (I),

$A^1$ is C-$R^1$; $A^2$ is CH; $A^3$ is CH; $A^4$ is C-$R^{1c}$ and $A^5$ is CH;
or $A^1$ is CH; $A^2$ is C-$R^{1a}$; $A^3$ is CH; $A^4$ is C-$R^{1c}$ and $A^5$ is CH;
or $A^1$ is CH; $A^2$ is CH; $A^3$ is C-$R^{1b}$; $A^4$ is C-$R^{1c}$ and $A^5$ is CH;
or $A^1$ is CH; $A^2$ is CH; $A^3$ is CH; $A^4$ is C-$R^{1c}$ and $A^5$C-$R^{1d}$; or $R^{1c}$, $R^{1d}$ and the C attached thereto together form substituted or unsubstituted $C_2$-$C_8$heterocyclyl;
or $A^1$ is CH; $A^2$ is CH; $A^3$ is CH; $A^4$ is C-$R^{1c}$ and $A^5$ is CH;
$R^1$, $R^{1a}$, $R^{1b}$, $R^{1c}$ and $R^{1d}$ are each independently a hydrogen atom, halogen, substituted or unsubstituted $C_1$-$C_4$alkyl,

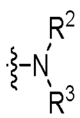, 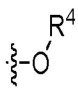, 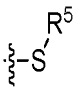, 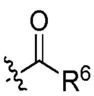, 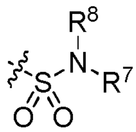, 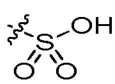,

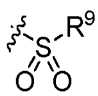 or 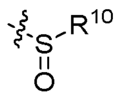 ,

wherein:

in $R^1$, $R^{1a}$, $R^{1b}$, $R^{1c}$ and $R^{1d}$, the substituents in the substituted $C_1$-$C_4$alkyl are selected from one or more of hydroxyl and halogen;
one of $R^2$ and $R^3$ is hydrogen, the other is substituted or unsubstituted $C_1$-$C_4$alkyl,

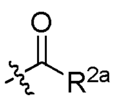 or 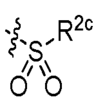 ,

or $R^2$ and $R^3$ are both $C_1$-$C_4$alkyl; $R^{2a}$ is $C_1$-$C_4$alkyl; $R^{2c}$ is substituted or unsubstituted $C_1$-$C_4$alkyl, $C_3$-$C_8$cycloalkyl or $C_2$-$C_{10}$heteroaryl, in $R^{2c}$, the substituents in the substituted $C_1$-$C_4$alkyl are selected from one or more of halogen and $C_3$-$C_8$cycloalkyl;
$R^4$ is a hydrogen atom,

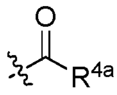 or 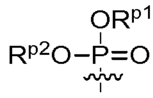 ;

$R^{4a}$ is a hydrogen atom or $C_1$-$C_4$alkyl; $R^{p1}$ and $R^{p2}$ are independently $C_1$-$C_4$alkyl;
$R^5$ is a hydrogen atom;
$R^6$ is

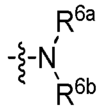 or 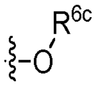 ;

$R^{6a}$ and $R^{6b}$ are a hydrogen atom or $C_1$-$C_4$alkyl; $R^6$ is H;
$R^7$ and $R^8$ are each independently a hydrogen atom or $C_1$-$C_4$alkyl;
$R^9$ is substituted or unsubstituted $C_1$-$C_4$alkyl;
$R^{10}$ is $C_1$-$C_4$alkyl;
$B^1$, $B^2$, $B^3$, $B^4$, $B^5$, $B^6$ and $B^7$ are hydrogen atoms;
L is a direct bond;
K is

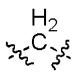 ;

and Z is substituted or unsubstituted $C_2$-$C_{10}$heteroaryl containing at least one nitrogen atom; the substituents in the substituted $C_2$-$C_{10}$heteroaryl are selected from one or more of halogen and $C_1$-$C_4$alkyl.

In a preferred embodiment of the invention, in the tetrahydropyrrole compound represented by general formula (I),
$A^1$ is CH; $A^2$ is C-$R^{1a}$; $A^3$ is CH; $A^4$ is CH and $A^5$ is CH;
$R^{1a}$ is hydroxyl or

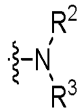 ;

one of $R^2$ and $R^3$ is hydrogen, the other is

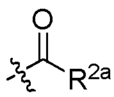 or 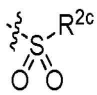 ,

$R^{2a}$ is $C_1$-$C_4$alkyl; $R^{2c}$ is substituted or unsubstituted $C_1$-$C_4$alkyl or $C_2$-$C_{10}$heteroaryl, in $R^{2c}$, the substituents in the substituted $C_1$-$C_4$alkyl are substituted with one or more of halogens;
$B^1$, $B^2$, $B^3$, $B^4$, $B^5$, $B^6$ and $B^7$ are hydrogen atoms;
L is a direct bond;
K is

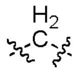;

and Z is $C_2$-$C_{10}$heteroaryl containing at least one nitrogen atom; the substituents in the substituted $C_2$-$C_{10}$heteroaryl are selected from one or more of halogen and $C_1$-$C_4$alkyl.

In a preferred embodiment of the invention, in the tetrahydropyrrole compound represented by general formula (I),

$A^1$ is C-$R^1$; $A^2$ is C-$R^{1a}$; $A^3$ is C-$R^{1b}$; $A^4$ is C-$R^{1c}$ and $A^5$ is C-$R^{1d}$;

$R^1$, $R^{1a}$, $R^{1b}$, $R^{1c}$ and $R^{1d}$ are each independently a hydrogen atom, halogen or

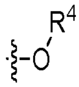,

or adjacent $R^1$ and $R^{1a}$; or $R^{1a}$ and $R^{1b}$; or $R^{1b}$ and $R^{1c}$; or $R^{1c}$ and $R^{1d}$ and the atoms attached thereto together form substituted or unsubstituted $C_2$-$C_8$heterocyclyl;

$R^4$ is a hydrogen atom or

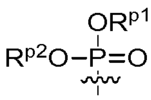,

wherein $R^{p1}$ and $R^{p2}$ are independently $C_1$-$C_4$alkyl;

$B^1$, $B^2$, $B^3$, $B^4$, $B^5$, $B^6$ and $B^7$ are hydrogen atoms;

L is a direct bond;

K is

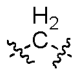

and Z is $C_2$-$C_{10}$heteroaryl containing at least one nitrogen atom; the substituents in the substituted $C_2$-$C_{10}$heteroaryl are selected from one or more of halogen and $C_1$-$C_4$alkyl.

In some preferred embodiments of the invention, the tetrahydropyrrole compound represented by general formula (I), the enantiomer, diastereomer, isotope compound, pharmaceutically acceptable prodrug, pharmaceutically acceptable estersor pharmaceutically acceptable salt thereof is any of the following compounds:

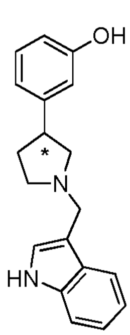
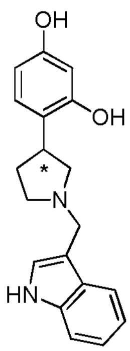
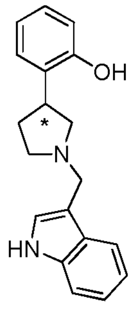
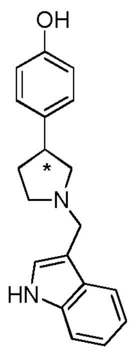
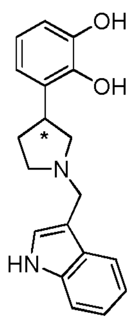
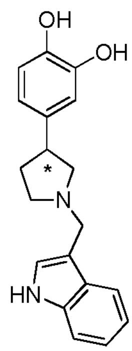

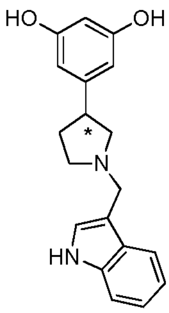 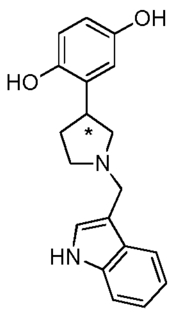 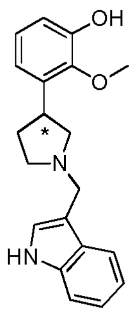 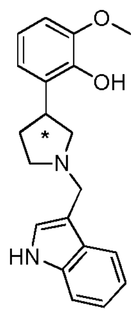 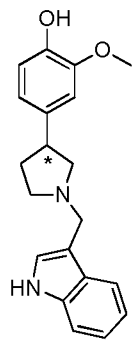 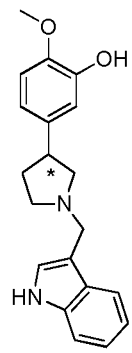

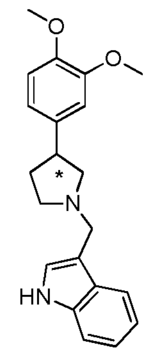 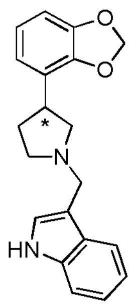 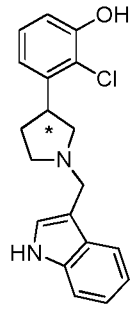 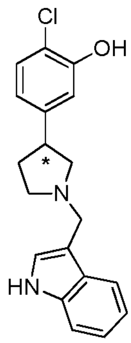 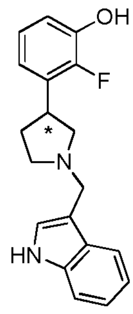 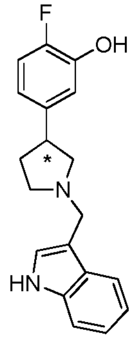

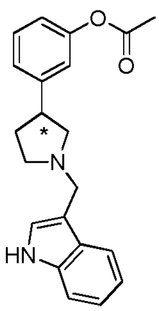 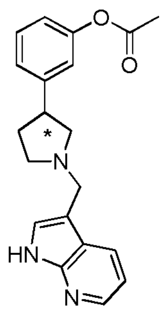 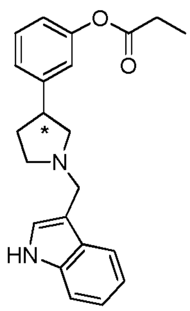 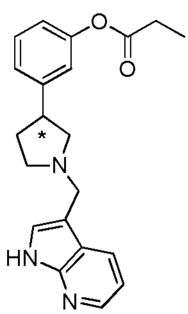 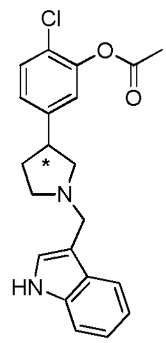 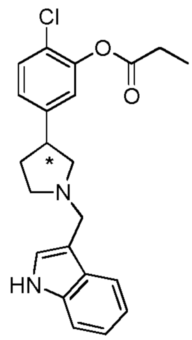

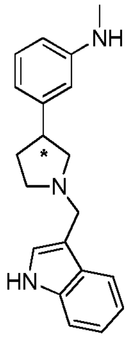 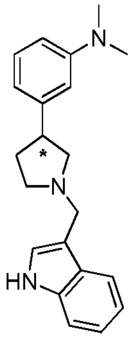 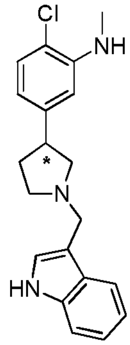 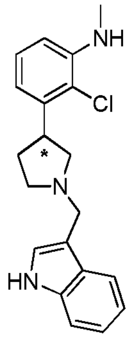 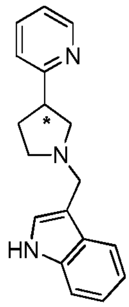 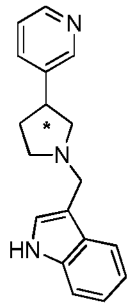

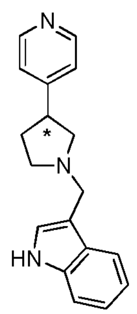
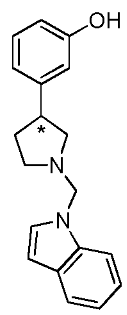
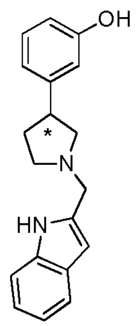
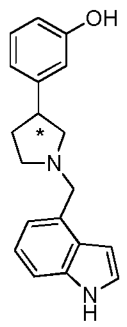
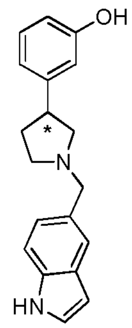
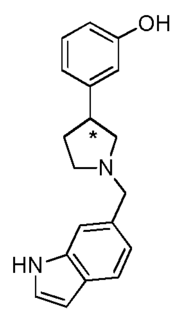

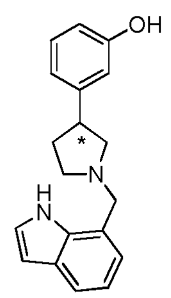
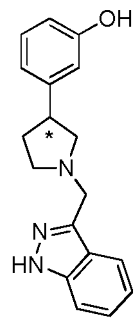
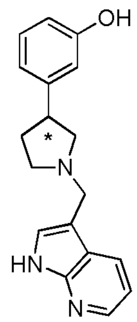
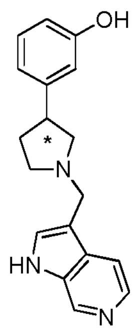
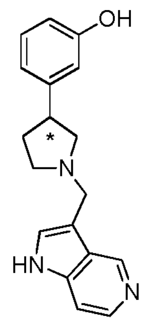
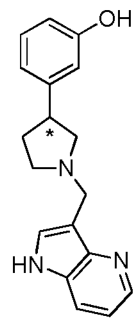

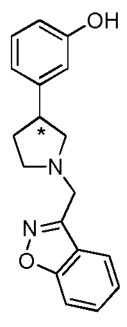
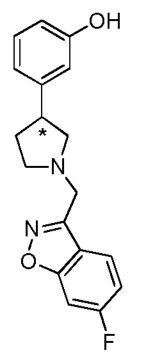
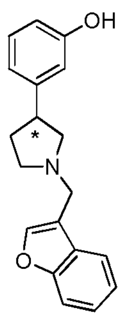
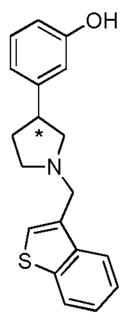
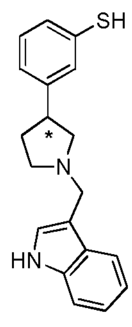
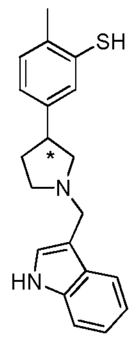

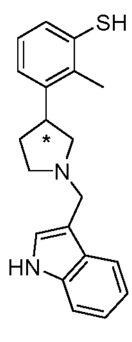
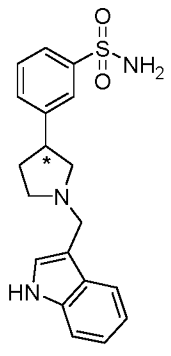
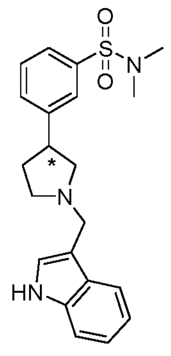
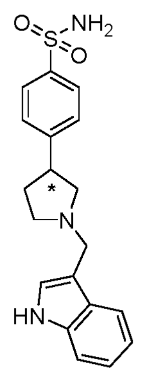
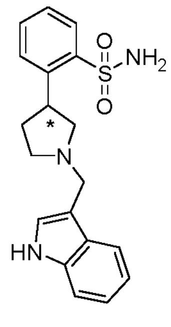
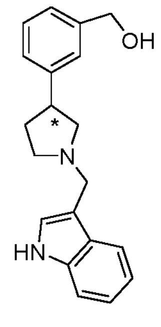

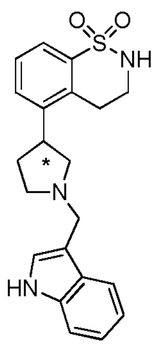
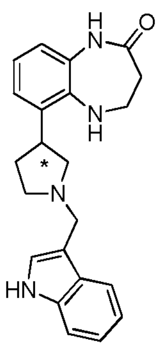
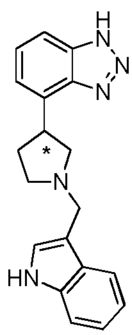
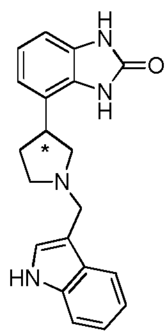
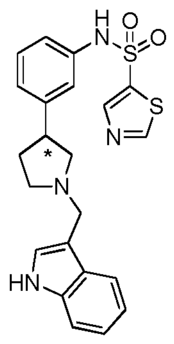
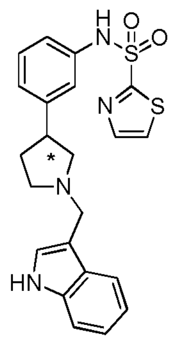

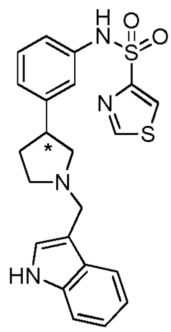
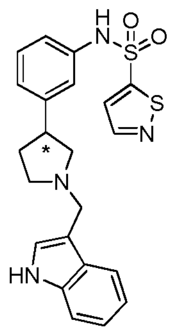
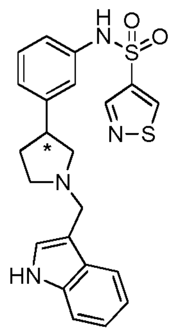
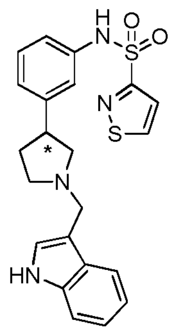
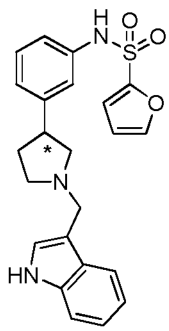
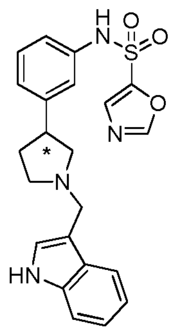

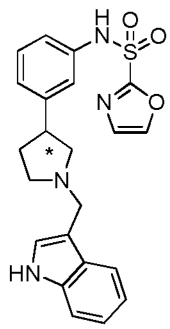
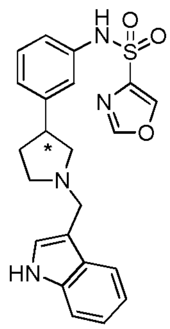
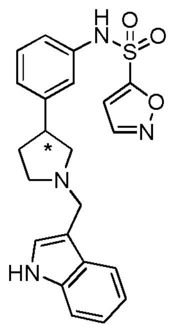
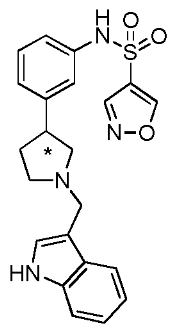
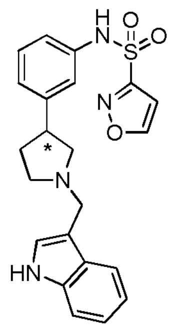
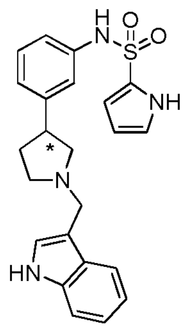

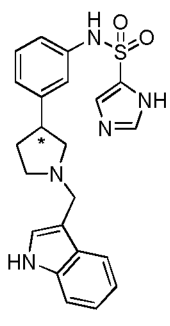
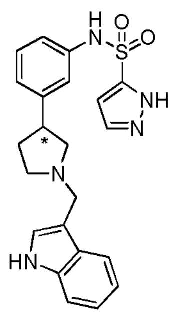
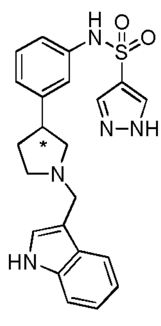
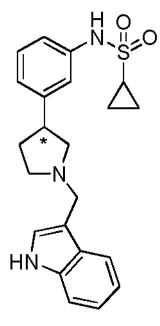
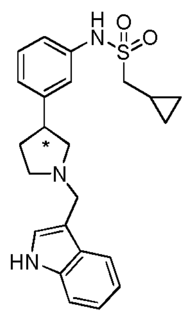
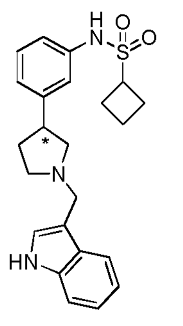

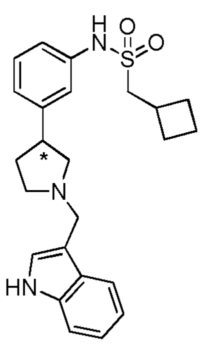
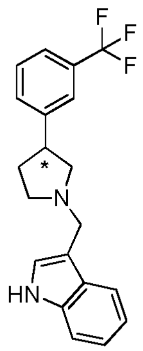
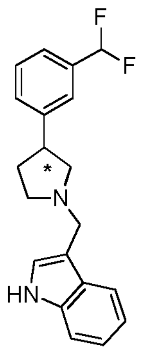
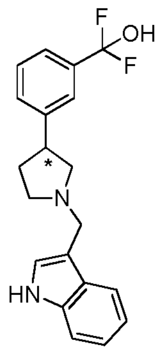
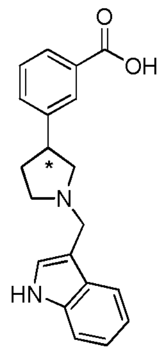
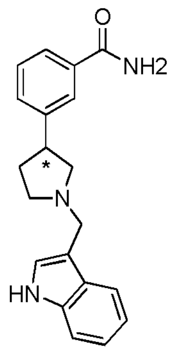

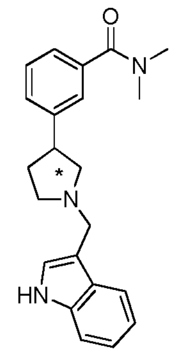
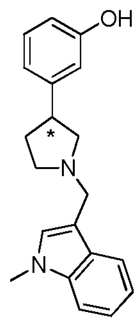
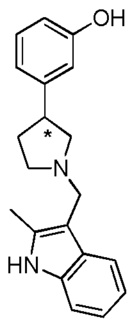
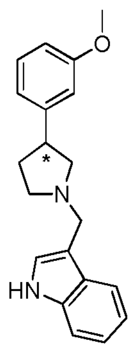
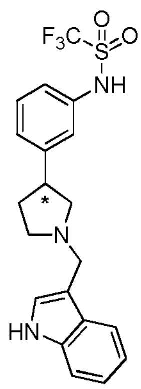
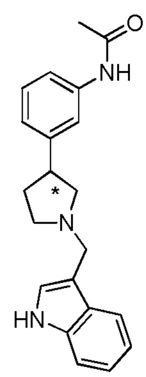

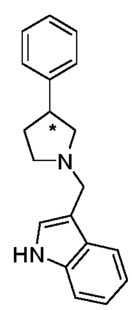
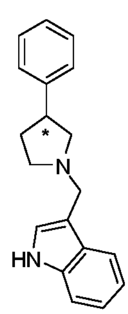
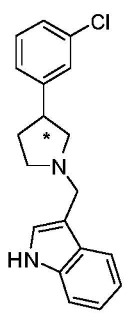
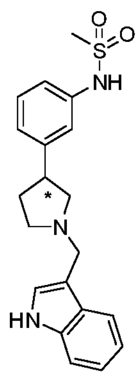
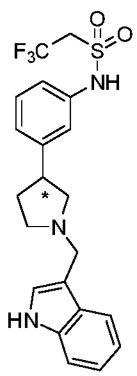
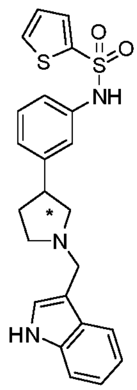

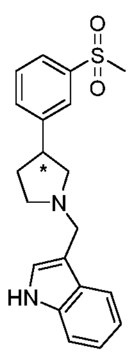
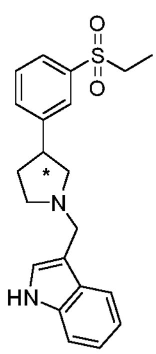
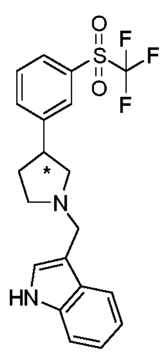
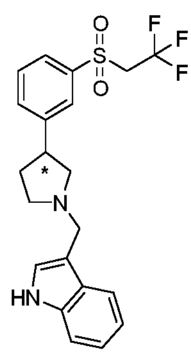
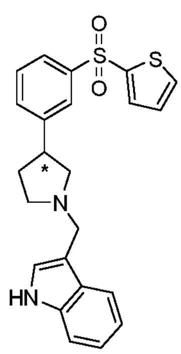
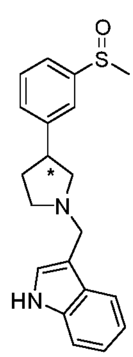

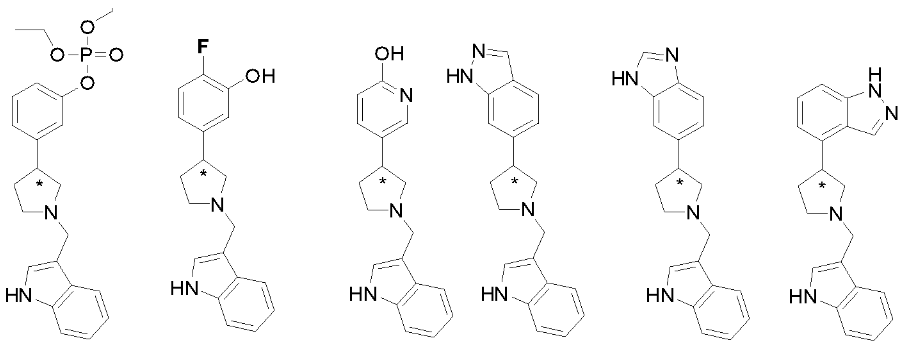

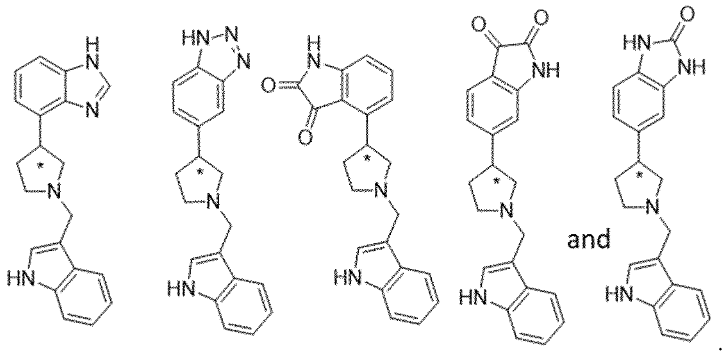

and

.

Wherein, carbon labeled with * refers to S-configuration chiral carbon, R-configuration chiral carbon or achiral carbon.

The invention also provides an S configuration or an R configuration of the following compound:

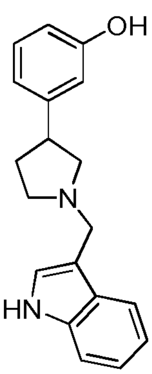

the S configuration or the R configuration is obtained by chiral HPLC resolution of the above compound. Wherein the chiral HPLC resolution method can be a conventional method and condition for chiral HPLC resolution of such compound in the art. The following methods and conditions are preferred for the present invention:

method (i) analytical HPLC; conditions are preferably: analytical column: CHIRALCE OJ-H (OJH0CE-VD046) (0.46 cm I.D. × 25 cm L) (Chiralpak AD-3R (4.6 mm × 150 mm) or CHIRALPAK AD-RH (4.6 mm × 150 mm) or Chiradex (5 μm, 4.0 × 250 mm) or Ultron ES-OVM 4.6 × 250 mm), the mobile phase is MeOH/DEA (or MeOH/ammonium formate or MeOH/ammonium acetate or MeOH/ammonia water) = 100/0.1 (V/V) (isocratic elution); the flow rate is 1.0 ml/min; the injection volume is 10.0 uL; the detection wavelength is UV 254 nm (or UV 214 nm); the column temperature is 35°C (the column temperature in the range of 30°C-40°C can be selected according to actual needs); wherein the retention time is 6.17 minutes to obtain a compound with first configuration, and the retention time is 8.74 minutes to obtain a compound with second configuration (wherein that optical rotation of the compound with first configuration is $[\alpha]_D^{20}$ = + 34 (c 0.5, $CH_3OH$), and the optical rotation of the compound with second configuration is $[\alpha]_D^{20}$ = -32 (c 0.5, $CH_3OH$));

method (ii) preparative HPLC; conditions are preferably: preparative column: CHIRALCE OJ (5.0 cm I.D. × 25 cm

L), the mobile phase is MeOH/DEA (or MeOH/ammonium formate or MeOH/ammonium acetate or MeOH/ammonia water) = 100/0.1 (V/V); the flow rate is 60.0 mL/min; the detection wavelength is UV 214 nm (or UV 254 nm); the column temperature is 35°C (the column temperature in the range of 30°C-40°C can be selected according to actual needs).

In the present invention, the S configuration or R configuration of the tetrahydropyrrole compound represented by general formula (I) can be obtained by referring to the above-mentioned HPLC resolution method.

The invention also provides a method for resolving the S configuration or R configuration of the tetrahydropyrrole compound represented by general formula (I), which comprises the following steps: the tetrahydropyrrole compound represented by general formula (I) is resolved by analytical HPLC or by preparative HPLC.

In the method using analytical HPLC and the method using preparative HPLC, the mobile phase is preferably a mixed solution of an alcohol solvent and an organic amine. The alcohol solvent is preferably methanol, and the organic amine is preferably one or more of diethanolamine, ammonium formate, ammonium acetate and ammonia water. The volume ratio of the alcohol solvent to the organic amine in the mixed solution is preferably 1000 : 1. Equal elution is preferably used in the method using analytical HPLC.

In the method using analytical HPLC, except for the mobile phase, other chromatographic conditions can be those conventional in the method using analytical HPLC in the art, and in the present invention, preferably comprise the following:

HPLC chromatograph is preferably Shimadzu LC-20AD chromatograph, CP-HPLC-05 chromatograph, Agilent 1200/1260 chromatograph or Waters E2695 chromatograph. Analytical column is preferably CHIRALCE OJ-H (OJH0CE-VD046) (0.46 cm I.D. × 25 cm L), Chiralpak AD-3R (4.6 mm × 150 mm), CHIRALPAK AD-RH (4.6 mm × 150 mm), Chiradex (4.0 × 250 mm) or Ultron ES-OVM 4.6 × 250 mm. The flow rate is preferably 0.5-1.5 ml/min, more preferably 1.0 ml/min. The injection volume is preferably 5-20 uL, more preferably 10.0 uL. The detection wavelength is UV 254 nm. The column temperature is preferably 30°C-40°C, more preferably 35°C.

In the method using preparative HPLC, except for the mobile phase, other chromatographic conditions can be those conventional in the method using preparative HPLC in the art, and in the present invention, preferably comprise the following:

Preparative column is preferably CHIRALCE OJ (5.0 cm I.D. × 25 cm L), HIRALPAK AD-RH (20 mm × 150 mm), or Ultron ES-OVM (20 × 250 mm). The flow rate is preferably from 50.0 mL/min to 100.0 mL/min, more preferably 60.0 mL/min. The detection wavelength is UV 214 nm or UV 214 nm. The column temperature is preferably 30°C-40°C, more preferably 35°C.

The preparative HPLC chromatograph is preferably Agilent 1200/1260 Infinity II preparative liquid chromatograph, Shimadzu Prominence LC-20AP chromatograph or Waters 2545 chromatograph. The injection volume is not specifically defined, and is usually selected according to the actual selected preparative column.

The invention also provides a method for preparing the tetrahydropyrrole compound represented by general formula (I).

In the tetrahydropyrrole compound represented by general formula (I), when L is a direct bond and K is

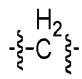

,

then the tetrahydropyrrole compound is prepared by the following method 1, which comprises the following steps: compound I-M and

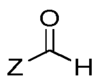

are subjected to a reductive amination reaction as shown below to prepare compound I-A;

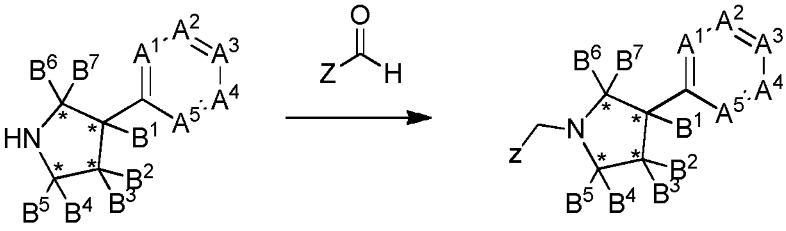

I-M I-A

wherein the definitions of $B_1$-$B_7$, $A^1$-$A^5$, Z and * are the same as described above.

For example, in the tetrahydropyrrole compound represented by general formula (I), when L is a direct bond and K is

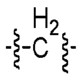,

then the tetrahydropyrrole compound is prepared using the following synthesis routes 1 and 2:

Route 1:

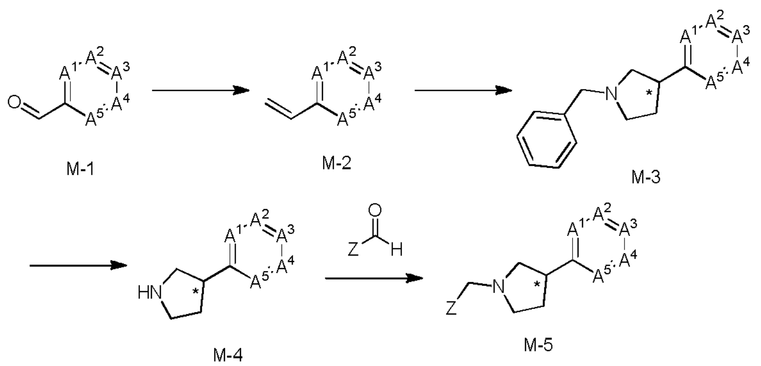

wherein the definitions of $A^1$ to $A^5$ and Z are the same as described above; the substituted aldehyde group in M-1 is subjected to Wittig reaction to obtain M-2, which then is subjected to σ-1,3 addition reaction with substituted benzylamine to construct five-membered ring M-3, which is subjected to debenzylation to obtain M-4, which is subjected to reductive amination with corresponding aryl-aldehyde to obtain the product.

Route 2:

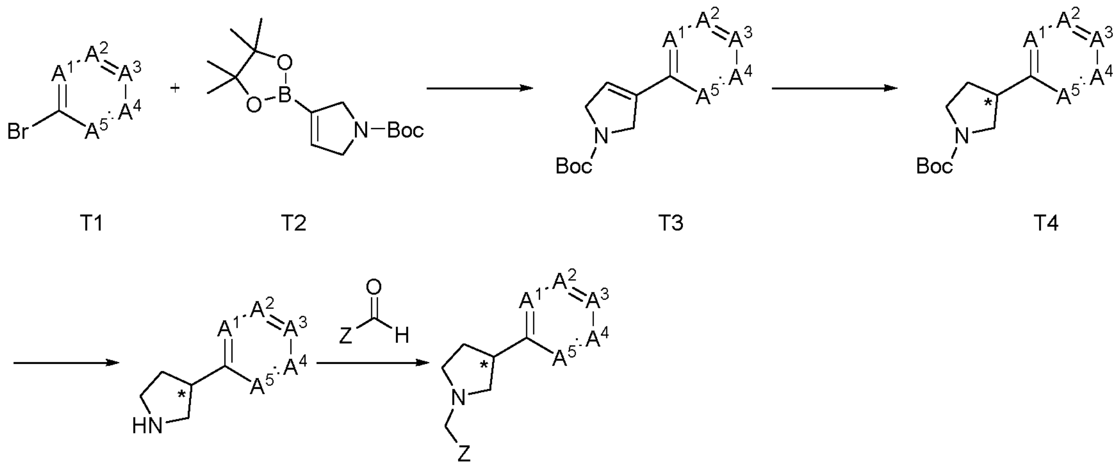

T1 T2 T3 T4

M-4 M-5

wherein the definitions of $A^1$ to $A^5$ and Z are the same as described above; substituted brominates T1 and T2 is subjected to Suzuki reaction to obtain T3, which is hydrogenated to obtain T4, which is subjected to de-Boc reaction and then reduction amination with corresponding aryl-aldehyde to obtain the product.

In the tetrahydropyrrole compound represented by general formula (I), when Z is substituted or unsubstituted $C_2$-$C_{10}$ heteroaryl containing at least one N atom, then the tetrahydropyrrole compound is prepared by the following method 2, which comprises the following steps: compound I-Ma is subjected to the following deamination reaction to remove amino protecting group so as to prepare the tetrahydropyrrole compound represented by general formula (I);

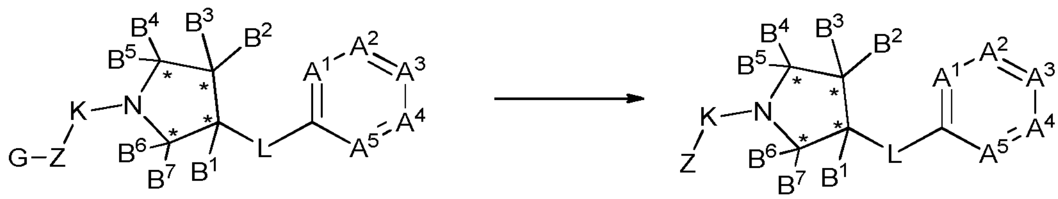

I-Ma (I)

wherein, L, Z, K, $B_1$-$B_7$, $A^1$-$A^5$, Z and * are the same as described above; in compound I-Ma, G refers to an amino protecting group, wherein G is connected to a nitrogen atom in Z.

For example, in the tetrahydropyrrole compound represented by general formula (I), the following synthetic route can be used for preparation:

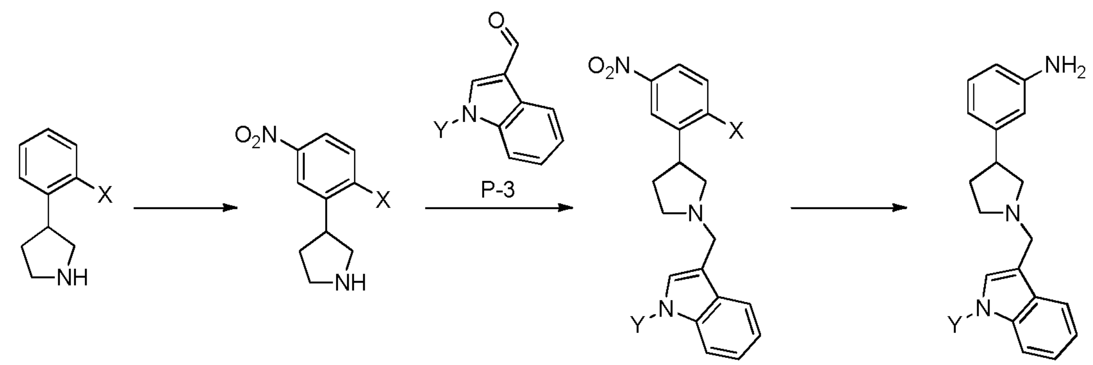

P-3

P-1 P-2 P-4 P-5

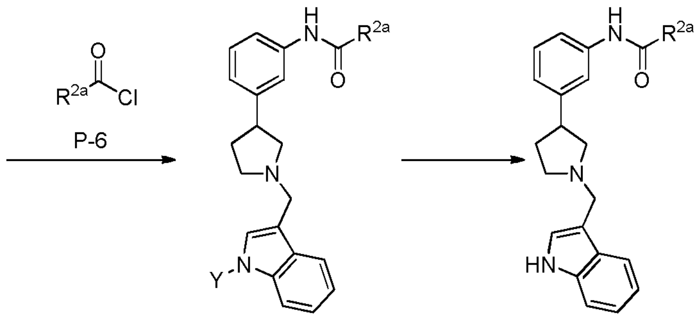


P-7 P-8

wherein $R^{2a}$ is as defined above, X is chlorine, bromine, iodine and Y is an amino protecting group.

Halogen-substituted phenyltetrahydropyrrole P-1 is subjected to nitration reaction to obtain meta-nitration product P-2, which is then subjected to reductive amination reaction with N-protected indolealdehyde P-3 to obtain P-4, which is subjected to hydrogenation reduction reaction on the nitro group and dehalogenation to obtain P-5, which is reacted with corresponding acyl chloride P-6 to obtain corresponding amide P-7, which is then subjected to deprotection reaction to remove the protective group so as to obtain the product.

The present invention also provides a pharmaceutical composition comprising the tetrahydropyrrole compound represented by general formula (I), the enantiomer, diastereomer, isotope compound, pharmaceutically acceptable prodrug, pharmaceutically acceptable ester or pharmaceutically acceptable salt thereof, and pharmaceutically acceptable excipients.

The present invention also provides a pharmaceutical composition comprising the tetrahydropyrrole compound represented by general formula (I), the enantiomer, diastereomer, isotope compound, pharmaceutically acceptable prodrug, pharmaceutically acceptable ester or pharmaceutically acceptable salt thereof, and additional therapeutic drugs. The additional therapeutic drugs include, but not limited to, drugs for treating or preventing lesions and central nervous system diseases associated with dopamine receptor and dopamine transporter dysfunction. Lesions and central nervous system diseases associated with dopamine receptor and dopamine transporter dysfunction include but not limited to schizophrenia, and positive symptoms, negative symptoms, cognitive impairment, schizoaffective disorder, bipolar disorder, mania, depression, anxiety disorder, dementia, memory impairment and other psychosis involving paranoia and/or delusion associated with schizophrenia.

The pharmaceutical composition of the present invention can be formulated in any wide range of dosage forms, such as tablets, capsules, aqueous suspensions, oily suspensions, dispersible powders, granules, lozenges, emulsions, syrup, creams, ointments, suppositories or injections.

The pharmaceutical composition of the present invention may be administered in any suitable manner, including oral, topical (including buccal and sublingual), rectal, vaginal, transdermal, parenteral, subcutaneous, intraperitoneal, intrapulmonary, intradermal, intrathecal, epidural, intranasal, and, if desired for topical treatment, intralesional administration. Parenteral infusion includes intramuscular, intravenous, intraarterial, intraperitoneal and subcutaneous administration.

The invention also provides a use of the tetrahydropyrrole compound represented by general formula (I), the enantiomer, diastereomer, isotope compound, pharmaceutically acceptable prodrug, pharmaceutically acceptable ester or pharmaceutically acceptable salt thereof in the manufacture of D2 receptor and DAT receptor inhibitors.

The invention also provides a use of the tetrahydropyrrole compound represented by general formula (I), the enantiomer, diastereomer, isotope compound, pharmaceutically acceptable prodrug, pharmaceutically acceptable ester or pharmaceutically acceptable salt thereof in the manufacture of a medicament for the treatment or prevention of schizophrenia or diseases associated with schizophrenia. The diseases associated with schizophrenia are preferably positive symptoms, negative symptoms, cognitive impairment, schizoaffective disorder, bipolar disorder, mania, depression, anxiety disorder, dementia, memory impairment and other psychosis involving paranoia and/or delusion associated with schizophrenia.

The pharmaceutically acceptable salt of the tetrahydropyrrole compound represented by general formula (I) described in the present invention is preferably hydrochloride, hydrobromide, sulfate, phosphate, nitrates, formates, acetate, hydroxyacetate, gluconate, lactate, pyruvate, oxalate, malonate, aspartate, ascorbate, glutamate, cinnamate, benzoate, phenylacetate, mandelate, trifluoroacetate, methanesulfonate, trifluoromethanesulfonate, ethanesulfonate, p-phenylmesylate, tartrate, maleate, fumarate, succinate, malate, citrate or salicylate.

The pharmaceutically acceptable salt of the tetrahydropyrrole compound represented by general formula (I) in the present invention may also be an addition salt formed by the compound of the general formula (I) and an organic or inorganic base. The organic or inorganic bases include, but not limited to, sodium, potassium, calcium, magnesium,

iron, zinc, copper, aluminium, ammonia, isopropylamine, trimethylamine, triethylamine, diethylamine, tripropylamine, diisopropylamine, diisopropylethylamine, ethanolamine, 2-diethylaminoethanol, dicyclohexylamine, lysine, arginine, ornithine, histidine, caffeine, procaine, hydrabamine, choline, betaine, glucosamine, methylglucosamine, theobromine, purine, piperazine, piperidine, N-methylpiperazine, N-ethylpiperazine, hydroxyethylpiperazine, tetrahydropyrrole or morpholine.

Enantiomers of the tetrahydropyrrole compound represented by general formula (I) in the present invention include *cis* and *trans* isomers, (-)-and (+)-enantiomers, (R)-and (S)-enantiomers.

Isotopic compounds of the tetrahydropyrrole compound represented by general formula (I) in the present invention refer to compounds in which chemical elements in the compound of general formula (I) are replaced by one or more isotopes. For example, the compounds having the structure of the present invention but substituting "deuterium" or "tritium" for hydrogen, substituting $^{18}F$ isotope for fluorine, substituting $^{11}C$, $^{13}C$, $^{14}C$ isotopes for carbon, or substituting $^{18}O$ isotope for oxygen are within the scope of the present invention. Such compounds can be used, for example, as analytical tools or probes in biological assays, or as *in vivo* diagnostic imaging tracers for diseases, or as tracers for pharmacodynamics, pharmacokinetics or receptor studies.

The compound of the present invention can be derivatized at functional groups to provide derivatives that can be converted back to the parent compound *in vivo*. Metabolically unstable derivatives capable of producing the parent compound of the present invention *in vivo* are also within the scope of the present invention, including pharmaceutically acceptable prodrugs and pharmaceutically acceptable esters.

The term "pharmaceutically acceptable form of prodrugs" refers to any nontoxic salt, ester, salt of ester or other derivative that, when administered to a recipient, is capable of providing, directly or indirectly, the compound of the present invention or active metabolites or residues thereof.

The term "pharmaceutically acceptable esters" refers to derivatives that convert carboxyl groups in the compound of the present invention into esters or convert hydroxyl groups in the compound of the present invention into esters with other inorganic or organic acids, including but not limited to: nitric acid, sulfuric acid, phosphoric acid, formic acid, acetic acid, trifluoroacetic acid, methanesulfonic acid, trifluoromethanesulfonic acid, p-toluenesulfonic acid, tartaric acid, maleic acid, fumaric acid, succinic acid, malic acid, or citric acid.

The term "excipient" includes, but is not limited to, any adjuvant, carrier, excipient, glidant, sweetener, dispersant, diluent, preservative, suspending agent, stabilizer, dye/colorant, flavoring agent, surfactant, wetting agent, isotonic agent, solvent or emulsifier approved by the National Medical Products Administration for use in human or livestock.

In the present invention, the term cycloalkyl is preferably selected from $C_3$-Cscycloalkyl. Examples of cycloalkyl include, but not limited to: cyclopropyl, cyclobutyl, cyclopentyl, cyclohexyl, cycloheptyl and cyclooctyl.

In the present invention, the term heterocyclyl refers to a $C_2$-$C_8$non-aromatic ring having 1, 2, 3 or 4 heteroatoms selected from O, N and S. Examples of heterocyclyl include but not limited to: tetrahydropyranyl, azetidinyl, 1,4-dioxanyl, piperazinyl, piperidinyl, pyrrolidinyl, morpholinyl, thiomorpholinyl, dihydrofuranyl, dihydroimidazolyl, dihydroindolyl, dihydroisoxazolyl, dihydroisothiazolyl, dihydrooxadiazolyl, dihydrooxazolyl, dihydropyrazinyl, dihydropyrazolyl, dihydropyridinyl, dihydropyrimidinyl, dihydropyrrolyl, dihydroquinolinyl, dihydrotetrazolyl, dihydrothiadiazolyl, dihydrothiazolyl, dihydrothienyl, dihydrotriazolyl, dihydroazetidinyl, methylene dioxybenzoyl, tetrahydrofuranyl, tetrahydrothienyl,

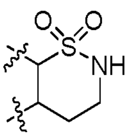 , 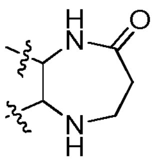 , 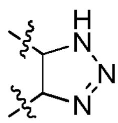 , 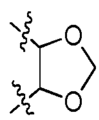 , 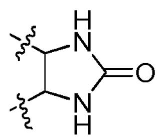 ,

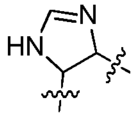 , 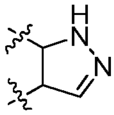 , 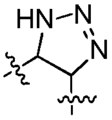 , 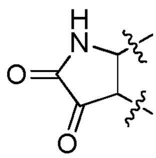 or 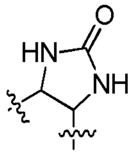 .

In the present invention, the term aryl is preferably $C_6$-$C_{14}$aryl, more preferably $C_6$-$C_{10}$aryl. Examples of aryl include, but not limited to, phenyl, naphthyl, tetrahydronaphthyl, 2,3-dihydroindene group, biphenyl, phenanthryl, anthracyl and acenaphthyl.

In the present invention, the term heteroaryl is preferably $C_2$-$C_{10}$heteroaryl having 1, 2, 3 or 4 heteroatoms selected from O, N and S, further preferably $C_2$-$C_8$heteroaryl having 1, 2, 3 or 4 heteroatoms selected from O, N and S. Examples of heteroaryl include, but not limited to pyridyl (for example

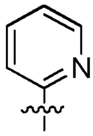 , 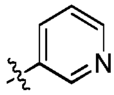

or

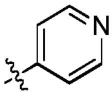

), furanyl (for example

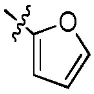

), thienyl (for example

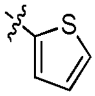

), thiazolyl (for example

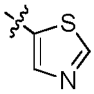 , 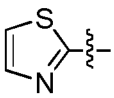 or 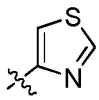

), isothiazolyl (

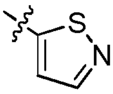 , 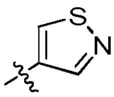

or

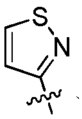

), oxazolyl (for example

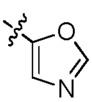 , 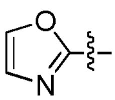 or 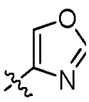

), isoxazolyl (for example

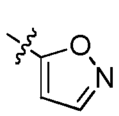, 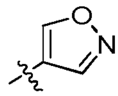 or 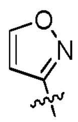

), pyrrolyl (for example

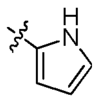

), imidazolyl (for example

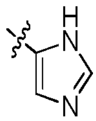

), pyrazolyl (for example

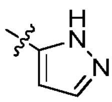, 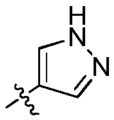 or 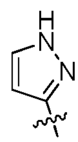

), indolyl (for example

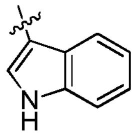, 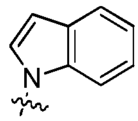, 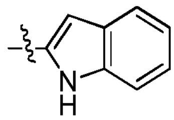, 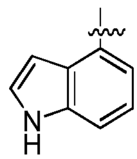, 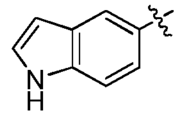,

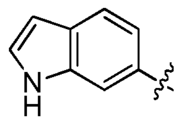 or 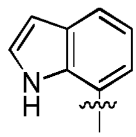

), 4-azaindolyl (for example

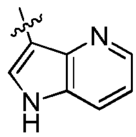

), 5-azaindolyl (for example

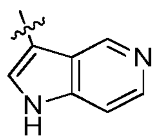

), 6-azaindolyl (for example

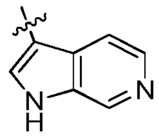

), 7-azaindolyl (for example

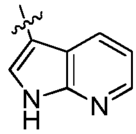

), quinolinyl, isoquinolinyl, quinazolinyl, quinoxalinyl, cinnolinyl, 1,5-naphthyridinyl, 1,6-naphthyridinyl, 1,7-naphthyridinyl, 1,8-naphthyridinyl, purinyl, indazolyl, benzimidazolyl, benzothienyl (for example

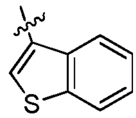

), benzofuranyl (for example

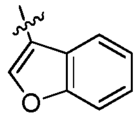

), benzotriazolyl, benzopyrazolyl (for example

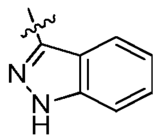

), benzoxazolyl, benzisoxazolyl (for example

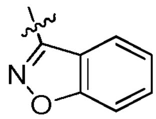

), benzothiazolyl or benzisothiazolyl.

In the present invention, the term halogen is preferably fluorine, chlorine, bromine or iodine.

In the present invention, the term alkyl is preferably methyl, ethyl, n-propyl, isopropyl, n-butyl, isobutyl or t-butyl.

In the present invention, the term alkoxy refers to a cyclic or acyclic alkyl group having specified number of carbon atoms and an oxygen bridge connection. As such, the alkoxy comprise the above definitions of alkyl and cycloalkyl. In the present invention, the alkoxy is preferably $C_1$-$C_4$alkoxy, more preferably methoxy, ethoxy, n-propoxy, isopropoxy or t-butoxy. On the basis of not departing from common knowledge in the art, the above-mentioned various preferred conditions can be combined in any manner, such that various preferred examples of the present invention are obtained.

In the present invention, room temperature refers to 10°C-30°C. Overnight refers to 8-15 hours.

Reagents and raw materials used in the present invention are all commercially available.

The positive effect of the present invention lies in that

The tetrahydropyrrole compound of the present invention has better inhibitory effects on the positive symptoms

of schizophrenia, and the potency thereof is equivalent to or slightly stronger than that of the positive drug olanzapine. In addition, the compound of the present invention has dual inhibitory effects on D2 receptors and DAT receptors, and is effective for treating schizophrenia and improving negative symptoms and cognitive functions, while also reducing vertebral side effects and prolactin secretion.

## Detailed description of the preferred embodiment

In the following examples, room temperature means 10°C-30°C. Where the specific operating temperature is not defined, the operating temperature is room temperature (e.g., 10°C-30°C). Overnight refers to 8-15 hours. The purity of a compound is determined by high performance liquid chromatography (HPLC). Min refers to minutes.

Example 1

1-(indole-3-methyl)-3-(3-hydroxyphenyl)pyrrolidine (MDC-161502-002)

Step 1

Synthesis of 1-benzyl-3-(3-methoxyphenyl)pyrrolidine

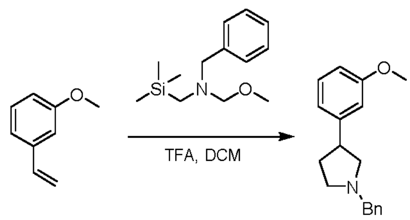


3-methoxyvinylbenzene (10 g, 74.5 mmol, 10.34 mL, 1 eq) was dissolve in dichloromethane (250 mL), trifluoroacetic acid (0.85 g, 7.45 mmol, 552 uL, 0.1 eq) was added, and N-(methoxymethyl)-N-(trimethylsilylmethyl) benzylamine (35.4 g, 149 mmol, 2 eq) was added dropwise at 0°C within 30 minutes. The temperature of the reaction was raised to room temperature and then the reaction was stirred for 48 hours, the reaction solution was diluted with dichloromethane (250 mL) and washed 3 times with water (300 mL), the organic phase was dried with anhydrous sodium sulfate, filtered and concentrated, and the residue was purified by silica gel chromatography (eluent: petroleum ether : ethyl acetate = 10 : 1 - 1 : 1) to give a yellowish solid (13 g, yield 65%). $^1H$ NMR (400 MHz, $CDCl_3$): δ 7.35-7.08 (m, 6H), 6.80-6.75 (m, 2H), 6.70-6.60 (m, 1H), 3.72 (s, 3H), 3.59 (s, 2H), 3.32-3.20 (m, 1H), 2.93 (t, *J* = 8.4 Hz, 1H), 2.80-2.70 (m, 1H), 2.65-2.55 (m, 1H), 2.43 (t, *J* = 8.4 Hz, 1H), 2.30-2.20 (m, 1H), 1.85-1.75 (m, 1H).

Step 2

Synthesis of 3-(3-methoxyphenyl)pyrrolidine

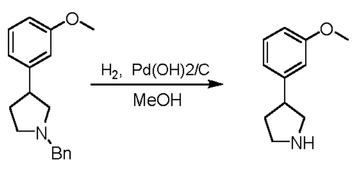


1-benzyl-3-(3-methoxyphenyl)pyrrolidine (13.0 g, 48.6 mmol, 1 eq) was dissolved in methanol (150 mL), $Pd(OH)_2$ (20%, 3.41 g, 4.86 mmol, 0.1 eq) was added, the resulting solution was subjected to nitrogen replacement three times, and stirred at room temperature for 4 hours under a hydrogen atmosphere (50 Psi). After filtration, the filtrate was evaporated to dry to give a gray solid (7.0 g, yield 81%), and the crude product was directly used in the next step.

Step 3

Synthesis of 1-(indole-3-methyl)-3-(3-methoxyphenyl)pyrrolidine (MDC-161502-001)

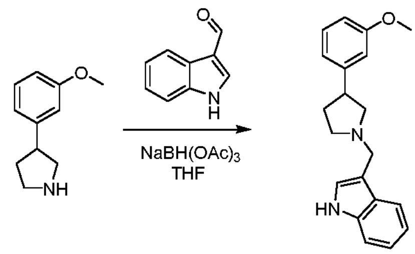


3-(3-methoxyphenyl)pyrrolidine (4.91 g, 33.8 mmol, 1 eq) and 3-indolealdehyde were dissolved in tetrahydrofuran (120 mL), and then NaBH $(OAc)_3$ (14.35 g, 67.7 mmol, 2 eq) was added and stirred at room temperature for 5 hours. The resulting mixture was quenched by adding saturated aqueous solution of ammonium chloride (50 mL) under ice bath, extracted 3 times with ethyl acetate (200 mL). The organic phase was washed with a saturated solution of sodium chloride, dried over anhydrous sodium sulfate, filtered and concentrated. The residue was purified by silica gel column chromatography (eluent: dichloromethane : methanol = 100 : 1-20 : 1) to give a yellowish solid (5.5 g, yield 51%, purity 96%). $^1H$ NMR (400 MHz, $CDCl_3$): δ 9.05 (s, 1H), 7.70-7.65 (m, 1H), 7.52 (s, 1H), 7.50-7.45 (m, 1H), 7.25-7.20 (m, 3H), 6.85-6.25 (m, 3H), 4.22 (s, 2H), 3.78 (s, 3H), 3.57-3.42 (m, 2H), 3.30-3.12 (m, 2H), 3.30-2.90 (m, 1H), 2.45-2.35 (m, 1H), 2.15-2.00 (m, 1H); $^{13}C$ NMR (200 MHz, $CDCl_3$): δ160.12, 136.17, 130.11, 128.15, 127.18, 122.68, 120.74, 119.36, 117.48, 113.18, 112.95, 112.48, 58.33, 58.30, 55.47, 49.39, 42.82; High Resolution Mass Spectrometry HRMS (ESI): $C_{20}H_{23}N_2O^+[M+H]^+$ calculated value: 307.1810, measured value: 307.1802; HPLC purity: 96.4% .

Step 4

Synthesis of 1-(indole-3-methyl)-3-(3-hydroxyphenyl)pyrrolidine (MDC-161502-002)

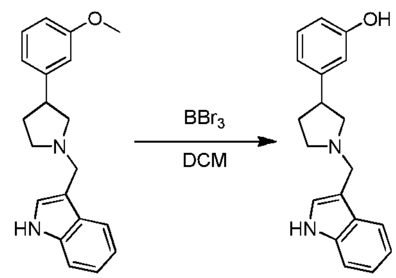


Compound 1-(indole-3-methyl)-3-(3-methoxyphenyl)pyrrolidine (700 mg, 2.28 mmol) was placed in a reaction flask, and the reaction flask was subjected to argon replacement three times. Anhydrous dichloromethane (20 mL) was added, and the resulting mixture was cooled to -20°C. Subsequently, a solution of boron tribromide in dichloromethane (boron tribromide content being 17%, 3.23 mL, 5.70 mmol) was slowly added and stirred for 30 minutes while keeping the temperature constant. The reaction solution was then warmed to room temperature and the reaction was stirred overnight. After the reaction was completed, the reaction system was cooled to -20°C. Methanol (3 mL) was slowly added dropwise to quench the reaction. The organic phase was washed with a saturated aqueous solution of sodium bicarbonate (10 mL) three times. The aqueous phase and the organic phase were all concentrated, then methanol was added for dissolution. Inorganic salts were removed by extraction filtration, and then the resulting mixture was purified on a silica gel column (dichloromethane : methanol = 19 : 1) to give a desired compound (480 mg, yield 72%). $^1H$ NMR (800 MHz, $CDCl_3$): δ 8.37 (s, 1H), 7.68 (d, *J* = 7.9 Hz, 1H), 7.34 (d, *J* = 8.1 Hz, 1H), 7.18 (dd, *J* = 10.9, 3.8 Hz, 2H), 7.11 (ddd, *J* = 28.3, 11.7, 4.3 Hz, 2H), 6.70 (d, *J* = 7.6 Hz, 1H), 6.66-6.62 (m, 1H), 6.61 (s, 1H), 4.02-3.96 (m, 2H), 3.31-3.25 (m, 1H), 3.19-3.14 (m, 1H), 2.99 (dd, *J* = 16.9, 7.5 Hz, 1H), 2.93 (dt, *J* = 14.7, 7.4 Hz, 1H), 2.70 (t, *J* = 9.2 Hz, 1H), 2.30-2.23 (m, 1H), 1.90-1.84 (m, 1H); HRMS (ESI) $C_{19}H_{21}N_2O$ $[M+H]^+$ calculated value: 293.1654, measured value:

293.1653; HPLC purity: 97.5%.

The racemate obtained above can be resolved by chiral HPLC using the following method.

Analysis method:

| Column | CHIRALCE OJ-H(OJH0CE-VD046) | |
|---|---|---|
| Column Specifications | 0.46 cm I.D. × 25 cm L | |
| Injection volume | 10.0 ul | |
| Mobile phase | MeOH/DEA = 100/0.1 (V/V) | |
| Flow rate | 1.0 ml/min | |
| Detection wavelength | UV 254 nm | |
| Column temperature | 35°C | |
| HPLC equipment | Shimadzu LC-20AD | CP-HPLC-05 |

Peak 1 (MDC-161502-010): RT = 6.17 min, $[\alpha]_D^{20}$ = + 34 (c 0.5, $CH_3OH$); Peak 2 (MDC-161502-011): RT = 8.74 min, $[\alpha]_D^{20}$ = -32 (*c* 0.5, $CH_3OH$).

Preparation conditions:

| Column | CHIRALCEL OJ |
|---|---|
| Column Specifications | 5.0 cm I.D. × 25 cm L |
| Mobile phase | MeOH/DEA = 100/0.1 (V/V) |
| Flow rate | 60 ml/min |
| Detection wavelength | UV 214 nm |
| Column temperature | 35°C |

Referring to the method described in Example 1, the compounds listed in Table 1 can be prepared using different substituted styrene as starting materials.

Table 1

| Structure | MS | Structure | MS |
|---|---|---|---|
| 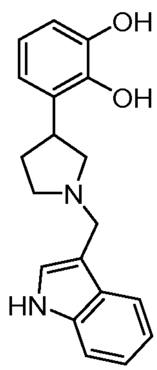 | 309.1 | 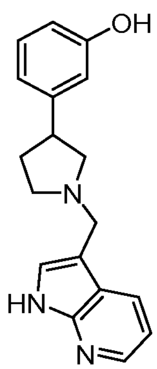 | 294.2 |

(continued)

| Structure | MS | Structure | MS |
|---|---|---|---|
| 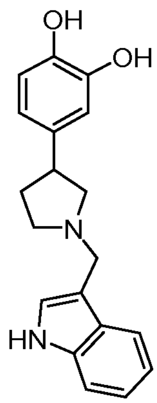 | 309.2 | 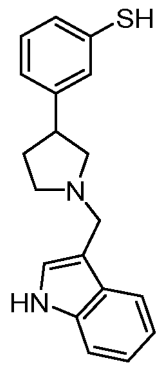 | 309.1 |
| 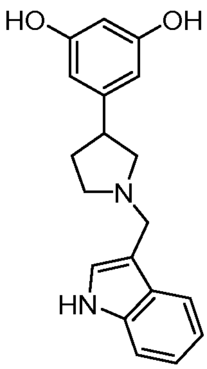 | 309.2 | 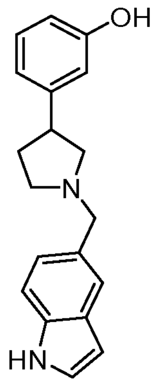 | 293.2 |
| 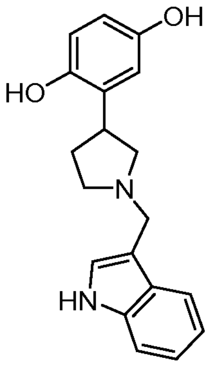 | 309.2 | 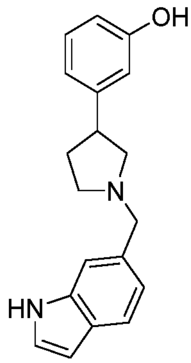 | 293.2 |
| 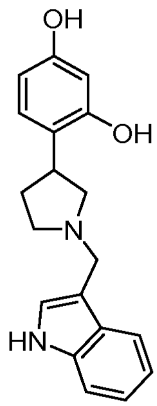 | 309.1 | 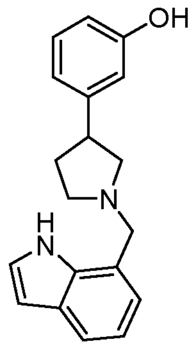 | 293.2 |

(continued)

| Structure | MS | Structure | MS |
|---|---|---|---|
| 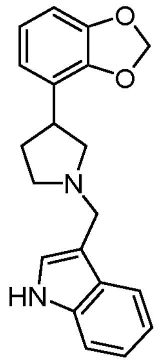 | 321.2 | 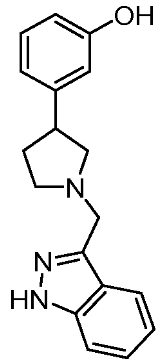 | 294.2 |
| 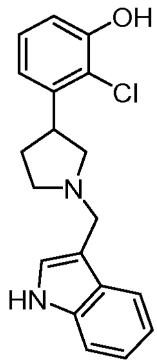 | 328.2 | 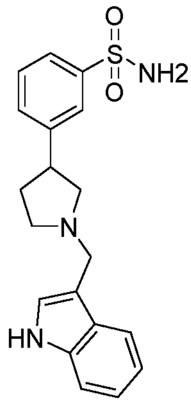 | 356.1 |
| 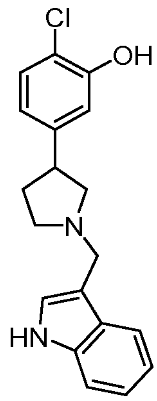 | 328.2 | 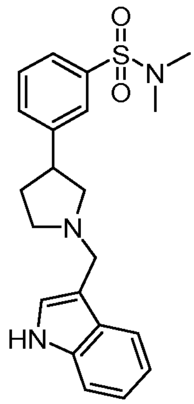 | 384.2 |
| 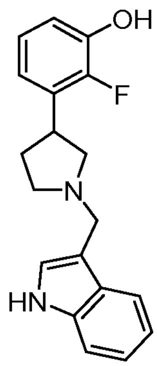 | 311.2 | 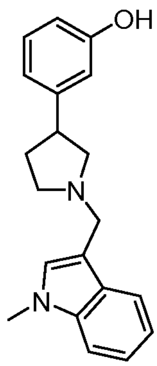 | 307.2 |

(continued)

| Structure | MS | Structure | MS |
|---|---|---|---|
| 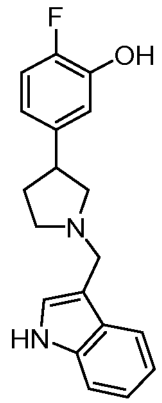 | 311.2 | 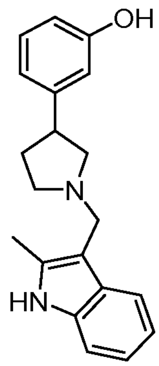 | 307.2 |
| 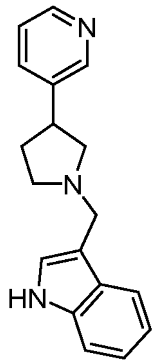 | 278.2 | 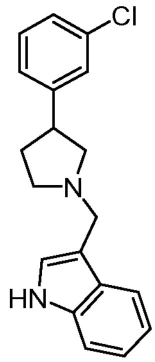 | 312.1 |
| 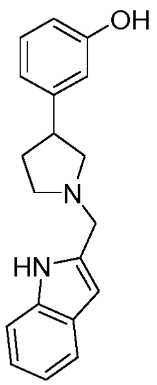 | 293.2 | 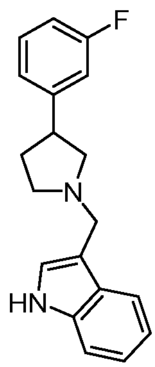 | 295.2 |
| 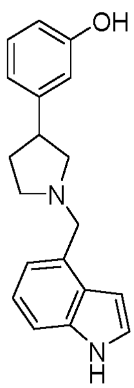 | 293.2 | 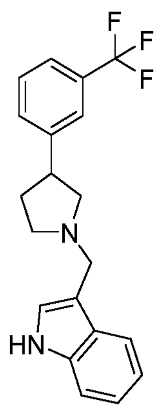 | 345.2 |

Compounds having phenolic hydroxyl substitution can be reacted with alkyl anhydrides to prepare esters, e.g.

Table 2:

Table 2

| Structure | MS | Structure | MS |
| --- | --- | --- | --- |
| 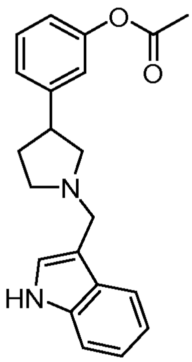 | 335.2 | 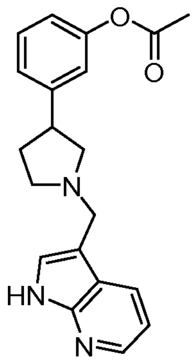 | 336.2 |
| 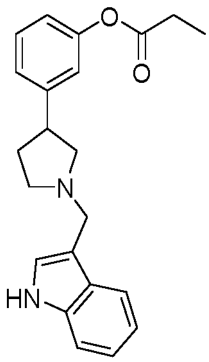 | 349.2 | 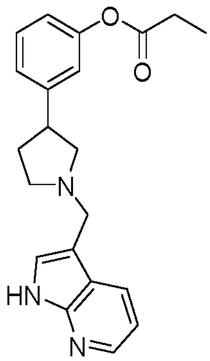 | 350.2 |
| 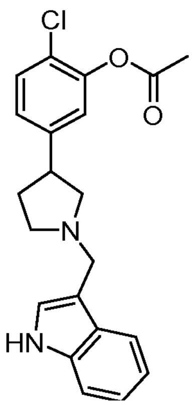 | 369.1 | 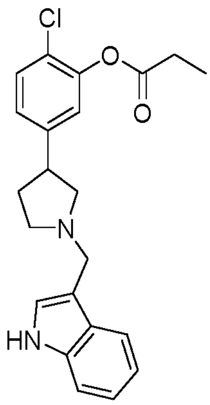 | 383.1 |

Example 2

1-(indole-3-methyl)-3-(3-acetylaminophenyl)pyrrolidine (MDC-161502-003)

Step 1

Synthesis of 3-(2-chloro-5-nitrophenyl)pyrrolidine

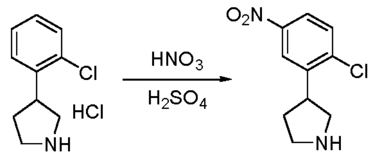


3-(2-chlorophenyl)pyrrolidine hydrochloride (900 mg, 4.12 mmol) was dissolved in concentrated sulfuric acid (15 mL). Fuming nitric acid (1.0 mL) was added dropwise at -15°C and stirred at low temperature for 1 hour. The reaction solution was added dropwise into ice water (150 mL) under an ice bath, adjusted to pH 8-9 with IN NaOH solution, extracted with ethyl acetate (200 mL) 3 times. The organic phase was washed with saturated sodium chloride solution, dried over anhydrous sodium sulfate, filtered and concentrated to give a yellow oily liquid (900 mg, crude yield 96%), and the crude product was directly used for the next step. $^1$H NMR (800 MHz, $CDCl_3$) δ 8.26 (s, 1H), 8.05 (d, *J* = 8.7 Hz, 1H), 7.56 (d, *J* = 8.7 Hz, 1H), 3.83 (p, *J* = 8.2 Hz, 1H), 3.61-3.58 (m, 1H), 3.40-3.33 (m, 1H), 3.28-3.22 (m, 1H), 3.04 (dd, *J* = 11.0, 8.1 Hz, 1H), 2.43-2.38 (m, 1H), 2.03-1.95 (m, 1H). $^{13}$C NMR (200 MHz, $CDCl_3$) δ 146.89, 142.44, 141.06, 130.56, 122.59, 122.45, 52.33, 46.58, 41.37, 32.44; HRMS(ESI) $C_{10}H_{12}ClN_2O_2^+$ $[M+H]^+$ calculated value: 227.0582, measured value: 227.0583.

Step 2

Synthesis of 1-(1-tert-butoxycarbonylindole-3-methyl)-3-(2-chloro-5-nitrophenyl)pyrrolidine

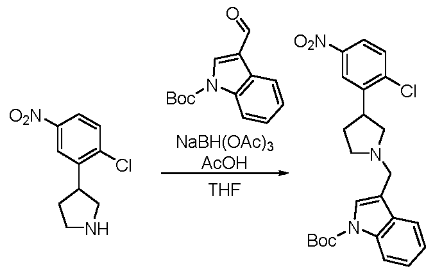


3-(2-chloro-5-nitrophenyl)pyrrolidine (900 mg, 3.97 mmol) and 1-tert-butoxycarbonyl 3-indolealdehyde (1.2 g, 4.8 mmol) were dissolved in anhydrous tetrahydrofuran (30 mL). Acetic acid (240 mg, 4 mmol) and $NaBH(OAc)_3$ (2.5 g, 12 mmol) were added at room temperature, and the resulting mixture was stirred at room temperature for 3 hours. After concentration under reduced pressure, the residue was dissolved in ethyl acetate (50 mL), washed once with saturated sodium bicarbonate solution (30 mL). The organic phase was dried over anhydrous sodium sulfate, filtered and concentrated, and the residue was purified on a silica gel column (petroleum ether : ethyl acetate = 3 : 1) to give a yellow oily liquid (1.4 g, yield 77%). HRMS (ESI) $C_{24}H_{27}ClN_3O_4^+$ $[M+H]^+$ calculated value: 456.1685, measured value: 456.1674.

Step 3

Synthesis of 1-(1-tert-butoxycarbonylindole-3-methyl)-3-(3-aminophenyl)pyrrolidine

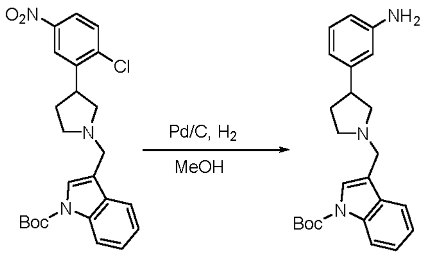

1-(1-tert-butoxycarbonylindole-3-methyl)-3-(2-chloro-5-nitrophenyl)pyrrolidine (1.4 g, 3.07 mmol) was dissolved in methanol (40 mL). Palladium carbon (1.4 g, 10%) was added, and the resulting mixture was stirred at room temperature for 2 hours under hydrogen atmosphere (20 Psi). After filtration, the filtrate was concentrated to give a brown oily liquid (1.1 g, crude yield 92%), which was directly used for the next reaction. $^1$H NMR (800 MHz, Methanol-*d*$_4$) δ 8.18 (d, *J* = 8.3 Hz, 1H), 7.97 (s, 1H), 7.81 (d, *J* = 7.8 Hz, 1H), 7.43-7.38 (m, 1H), 7.35 (t, *J* = 7.5 Hz, 1H), 7.07 (t, *J* = 7.8 Hz, 1H), 6.65 (s, 1H), 6.62 (dd, *J* = 7.8, 2.0 Hz, 2H), 4.56 (ABq, 2H), 3.73 (dd, *J* = 11.2, 8.1 Hz, 1H), 3.60-3.45 (m, 3H), 3.28 (t, *J* = 10.8 Hz, 1H), 2.47-2.41 (m, 1H), 2.20-2.14 (m, 1H), 1.70 (s, 9H). HRMS(ESI) $C_{24}H_{30}N_3O_2^+$ [M+H]$^+$calculated value: 392.2333, measured value: 392.2334.

Step 4

Synthesis of 1-(1-tert-butoxycarbonylindole-3-methyl)-3-(3-acetylaminophenyl)pyrrolidine

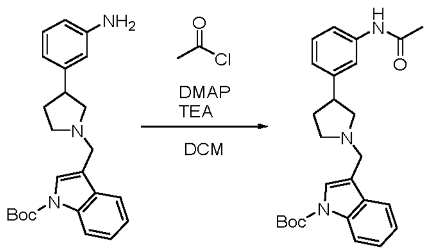


1-(1-tert-butoxycarbonylindole-3-methyl)-3-(3-aminophenyl)pyrrolidine (100 mg, 0.25 mmol), triethylamine (80 mg, 0.78 mmol) and DMAP (5 mg, 0.04 mmol) were dissolved in dichloromethane (5 mL). Acetyl chloride (30 mg, 0.38 mmol) was added dropwise, and the resulting mixture was stirred at room temperature for 16 hours. After concentration under reduced pressure, the residue was purified by silica gel column chromatography (dichloromethane/methanol = 10 : 1) to give a colorless oily liquid (90 mg, yield 80%). HRMS(ESI) $C_{26}H_{32}N_3O_3^+$ [M+H]$^+$calculated value: 434.2438, measured value: 434.2424.

Step 5

Synthesis of 1-(indole-3-methyl)-3-(3-acetylaminophenyl)pyrrolidine (MDC-161502-003)

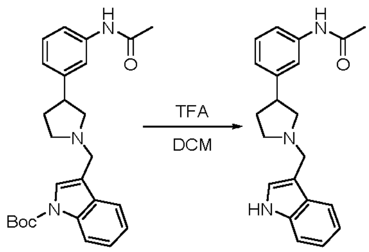


1-(1-Tert-butoxycarbonylindole-3-methyl)-3-(3-acetylaminophenyl)pyrrolidine (90 mg, 0.2 mmol) was dissolved in dichloromethane (5 mL). Trifluoroacetic acid (2.0 mL) was added dropwise, and the resulting mixture was stirred at room temperature for 4 hours. After concentration under reduced pressure, the residue was purified by preparative HPLC to give a white solid (35 mg, yield 50%). $^1$H NMR (500 MHz, Methanol-*d*$_4$) δ 7.77 (d, *J* = 8.0 Hz, 1H), 7.68 (s, 1H), 7.57 (s, 1H), 7.45 (d, *J* = 8.1 Hz, 1H), 7.32-7.25 (m, 2H), 7.22 (t, *J* = 8.1 Hz, 1H), 7.17 (t, *J* = 7.5 Hz, 1H), 7.04 (d, *J* = 6.7 Hz, 1H), 4.67 (ABq, *J* = 22.4 Hz, 6.4 Hz, 2H), 3.83 (dd, *J* = 11.5, 8.0 Hz, 1H), 3.68 - 3.60 (m, 1H), 3.59-3.51 (m, 1H), 3.42-3.34 (m, 1H), 3.21 (q, *J* = 7.3 Hz, 1H), 2.54-2.45 (m, 1H), 2.19 (t, *J* = 7.6 Hz, 1H), 2.12 (s, 3H); HRMS(ESI) $C_{21}H_{24}N_3O^+$ [M+H]$^+$ calculated value: 334.1914, measured value: 334.1925; HPLC purity: 97.7% (RT = 13.82 min, λ = 254 nm).

The purification condition for preparative liquid phase chromatography: Shim-pack GIST C18 column (250 × 20 mm, particle size 5 μM); water (containing 0.05% trifluoroacetic acid)/methanol (containing 0.05% trifluoroacetic acid) gradient elution; the flow rate was 10.0 mL/min.

Example 3

1-(indole-3-methyl)-3-(3-methanesulfonamidophenyl)pyrrolidine (MDC-161502-005)

Step 1

Synthesis of 1-(1-tert-butoxycarbonylindole-3-methyl)-3-(3-methanesulfonamidophenyl)pyrrolidine

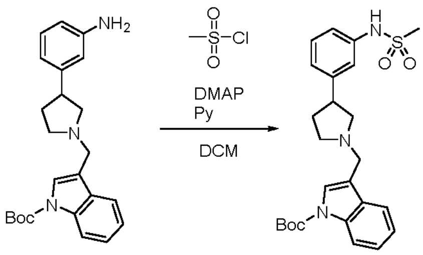


1-(1-tert-butoxycarbonylindole-3-methyl)-3-(3-aminophenyl)pyrrolidine (100 mg, 0.25 mmol), pyridine (65 mg, 0.78 mmol) and DMAP (5 mg, 0.04 mmol) were dissolved in dichloromethane (5 mL). Methanesulfonyl chloride (30 mg, 0.26 mmol) was added, and the resulting mixture was stirred at room temperature for 16 hours. After concentration under reduced pressure, the residue was purified by silica gel column chromatography (dichloromethane/methanol = 10 : 1) to give a colorless oily liquid (70 mg, yield 60%). HRMS(ESI) $C_{25}H_{32}N_3O_4S^+$ $[M+H]^+$calculated value: 470.2108, measured value: 470.2111.

Step 2

Synthesis of 1-(indole-3-methyl)-3-(3-methanesulfonamidophenyl)pyrrolidine (MDC-161502-005)

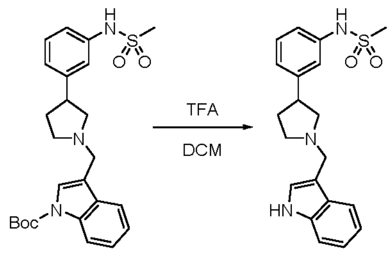


1-(1-tert-butoxycarbonylindole-3-methyl)-3-(3-methanesulfonamidophenyl)pyrrolidine (70 mg, 0.15 mmol) was dissolved in dichloromethane (5 mL). Trifluoroacetic acid (2.0 mL) was added dropwise, and the resulting mixture was stirred at room temperature for 4 hours. After concentration under reduced pressure, the residue was purified by preparative high performance liquid chromatography to give a white solid (25 mg, yield 45%). $^1H$ NMR (800 MHz, Methanol-$d_4$) $\delta$ 7.78 (d, $J$ = 8.0 Hz, 1H), 7.58 (s, 1H), 7.47 (d, $J$ = 8.2 Hz, 1H), 7.35 (t, $J$ = 7.9 Hz, 1H), 7.28-7.22 (m, 2H), 7.19 (t, $J$ = 8.0 Hz, 1H), 7.15 (d, $J$ = 8.0 Hz, 1H), 7.12 (d, $J$ = 7.7 Hz, 1H), 4.67 (ABq, $J$ = 22.4 Hz, 13.6 Hz, 2H), 3.84 (dd, $J$ = 11.6, 8.1 Hz, 1H), 3.66-3.61 (m, 2H), 3.59-3.54 (m, 1H), 3.37 (t, $J$ = 11.1 Hz, 1H), 2.96 (s, 3H), 2.54-2.48 (m, 1H), 2.23-2.16 (m, 1H); HRMS(ESI) $C_{20}H_{24}N_3O_2S^+$ $[M+H]^+$calculated value: 370.1584, measured value: 370.1590; HPLC purity: 95.7% (RT = 13.42 min, $\lambda$ = 280 nm).

The purification condition for preparative liquid phase chromatography: Shim-pack GIST C18 column (250 × 20 mm, particle size 5 μM); water (containing 0.05% trifluoroacetic acid)/methanol (containing 0.05% trifluoroacetic acid) gradient elution; the flow rate was 10.0 mL/min.

Example 4

1-(indole-3-methyl)-3-(3-trifluoroethanesulfonamidophenyl)pyrrolidine (MDC-161502-006)

Step 1

Synthesis of 1-(1-tert-butoxycarbonylindole-3-methyl)-3-(3-trifluoroethanesulfonamidophenyl)pyrrolidine

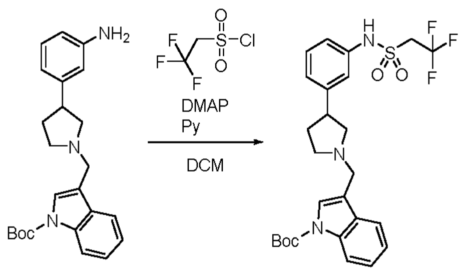


1-(1-tert-butoxycarbonylindole-3-methyl)-3-(3-aminophenyl)pyrrolidine (90 mg, 0.23 mmol), pyridine (65 mg, 0.78 mmol) and DMAP (5 mg, 0.04 mmol) were dissolved in dichloromethane (5 mL). Trifluoroethylsulfonyl chloride (45 mg, 0.25 mmol) was added, and the resulting mixture was stirred at room temperature for 16 hours. After concentration under reduced pressure, the residue was purified on a silica gel column (dichloromethane/methanol = 10 : 1) to give a colorless oily liquid (70 mg, yield 57%). HRMS(ESI) $C_{26}H_{31}F_3N_3O_4S^+$ $[M+H]^+$ calculated value: 538.1982, measured value: 538.1969.

Step 2

Synthesis of 1-(indole-3-methyl)-3-(3-trifluoroethanesulfonamidophenyl)pyrrolidine (MDC-161502-006)

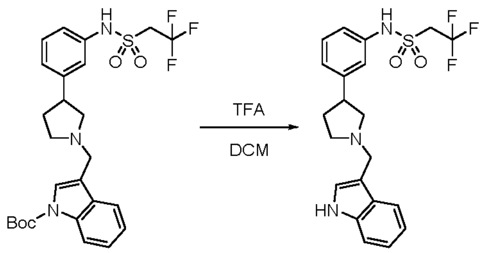


1-(1-tert-butoxycarbonylindole-3-methyl)-3-(3-trifluoroethanesulfonamidophenyl)pyrrolidine (70 mg, 0.13 mmol) was dissolved in dichloromethane (5 mL). Trifluoroacetic acid (2.0 mL) was added dropwise, and the resulting mixture was stirred at room temperature for 4 hours. After concentration under reduced pressure, the residue was purified by preparative high performance liquid chromatography to give a white solid (35 mg, yield 62%). $^1H$ NMR (500 MHz, DMSO-$d_6$) $\delta$ 10.86 (s, 1H), 7.61 (d, $J$ = 7.8 Hz, 1H), 7.31 (d, $J$ = 8.1 Hz, 1H), 7.21 (s, 1H), 7.04 (t, $J$ = 7.5 Hz, 1H), 6.96 (t, $J$ = 7.4 Hz, 1H), 6.87 (t, $J$ = 7.7 Hz, 1H), 6.70 (s, 1H), 6.67 (d, $J$ = 8.6 Hz, 1H), 6.46 (d, $J$ = 7.4 Hz, 1H), 4.10 (s, 1H), 3.73 (s, 2H), 3.46 (q, $J$ = 10.8 Hz, 2H), 3.12-3.03 (m, 1H), 2.91 (t, $J$ = 8.4 Hz, 1H), 2.71 (q, $J$ = 8.1 Hz, 1H), 2.57 (q, $J$ = 8.5 Hz, 1H), 2.36 (*t*, $J$ = 8.3 Hz, 1H), 2.18-2.08 (m, 1H), 1.75-1.65 (m, 1H); HRMS(ESI) $C_{21}H_{23}F_3N_3O_2S^+$ $[M+H]^+$ calculated value: 438.1458, measured value: 438.1454; HPLC purity: 98.6% (RT = 15.34 min, $\lambda$ = 254 nm).

The purification condition for preparative liquid phase chromatography: Shim-pack GIST C18 column (250 × 20 mm, particle size 5 $\mu$M); water (containing 0.05% trifluoroacetic acid)/methanol (containing 0.05% trifluoroacetic acid) gradient elution; the flow rate was 10.0 mL/min.

Example 5

1-(indole-3-methyl)-3-[3-(2-thiophenesulfonamido)phenyl]pyrrolidine (MDC-161502-008)

Step 1

Synthesis of 1-(1-tert-butoxycarbonylindole-3-methyl)-3-[3-(2-thiophenesulfonamido)phenyl]pyrrolidine

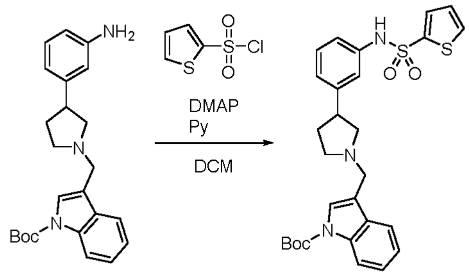


1-(1-tert-butoxycarbonylindole-3-methyl)-3-(3-aminophenyl)pyrrolidine (90 mg, 0.23 mmol), pyridine (5 mL) and DMAP (5 mg, 0.04 mmol) were dissolved in dichloromethane (5 mL). 2-thiophenesulfonyl chloride (70 mg, 0.38 mmol) was added, and the resulting mixture was stirred at room temperature for 3 hours. After concentration under reduced pressure, the residue was purified by silica gel column chromatography (dichloromethane/methanol = 10 : 1) to give a colorless oily liquid (90 mg, yield 67%). HRMS(ESI) $C_{28}H_{32}N_3O_4S_2^+$ $[M+H]^+$ calculated value: 538.1829, measured value: 538.1834.

Step 2

Synthesis of 1-(indole-3-methyl)-3-[3-(2-thiophenesulfonamido)phenyl]pyrrolidine (MDC-161502-008)

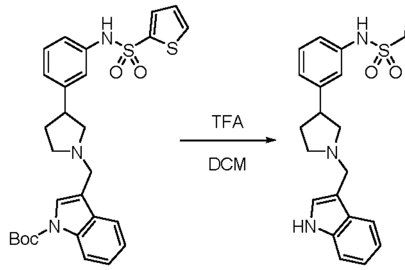


1-(1-tert-butoxycarbonylindole-3-methyl)-3-[3-(2-thiophenesulfonamido)phenyl]pyrrolidine (90 mg, 0.17 mmol) was dissolved in dichloromethane (5 mL). Trifluoroacetic acid (2.0 mL) was added dropwise, and the resulting mixture was stirred at room temperature for 4 hours. After concentration under reduced pressure, the residue was purified by preparative high performance liquid chromatography to give a white solid (45 mg, yield 60%). $^1H$ NMR (800 MHz, Methanol-$d_4$) $\delta$ 7.66 (d, $J$ = 7.9 Hz, 1H), 7.52 (d, $J$ = 5.0 Hz, 1H), 7.40-7.35 (m, 2H), 7.25 (s, 1H), 7.11 (t, $J$ = 8.1 Hz, 1H), 7.09 (t, $J$ = 7.8 Hz, 1H), 7.05 (t, $J$ = 8.0 Hz, 1H), 6.95 (s, 1H), 6.94-6.91 (m, 2H), 6.87 (d, $J$ = 7.6 Hz, 1H), 3.92 (s, 2H), 3.26 (t, $J$ = 8.4 Hz, 1H), 3.13 (t, $J$ = 9.8 Hz, 1H), 2.99-2.95 (m, 1H), 2.80-2.75 (m, 1H), 2.47 (t, $J$ = 9.4 Hz, 1H), 2.28-2.21 (m, 1H), 1.83-1.77 (m, 1H); HRMS(ESI) $C_{23}H_{24}N_3O_2S_2^+$ $[M+H]^+$ calculated value: 438.1304, measured value: 438.1309; HPLC purity: 95.3% (RT = 15.55 min, $\lambda$ = 254 nm).

The purification condition for preparative liquid phase chromatography: Shim-pack GIST C18 column (250 $\times$ 20 mm, particle size 5 $\mu$M); water (containing 0.05% trifluoroacetic acid)/methanol (containing 0.05% trifluoroacetic acid) gradient elution; the flow rate was 10.0 mL/min.

Example 6

1-(indole-3-methyl)-3-(3-ethanesulfonamidophenyl)pyrrolidine (MDC-161502-009)

Step 1

Synthesis of 1-(1-tert-butoxycarbonylindole-3-methyl)-3-(3-ethanesulfonamidophenyl)pyrrolidine

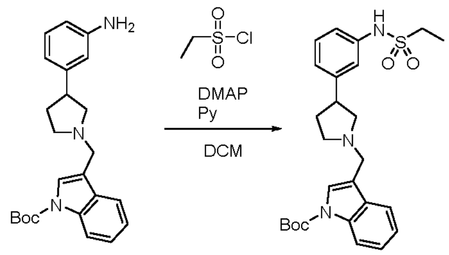


1-(1-tert-butoxycarbonylindole-3-methyl)-3-(3-aminophenyl)pyrrolidine (100 mg, 0.25 mmol), pyridine (65 mg, 0.78 mmol) and DMAP (5 mg, 0.04 mmol) were dissolved in dichloromethane (5 mL). Ethylsulfonyl chloride (50 mg, 0.38 mmol) was added, and the resulting mixture was stirred at room temperature for 16 hours. After concentration under reduced pressure, the residue was purified on a silica gel column (dichloromethane/methanol = 10 : 1) to give a colorless oily liquid (70 mg, yield 58%). HRMS(ESI) $C_{26}H_{34}N_3O_4S^+$ $[M+H]^+$ calculated value: 484.2265, measured value: 484.2263.

Step 2

Synthesis of 1-(indole-3-methyl)-3-(3-ethanesulfonamidophenyl)pyrrolidine (MDC-161502-009)

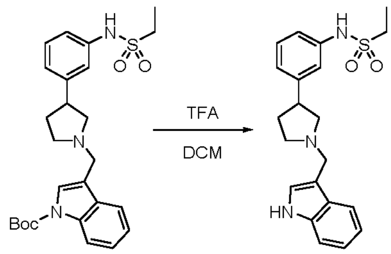


1-(1-tert-butoxycarbonylindole-3-methyl)-3-(3-ethanesulfonamidophenyl)pyrrolidine (70 mg, 0.14 mmol) was dissolved in dichloromethane (5 mL). Trifluoroacetic acid (2 mL) was added dropwise, and the resulting mixture was stirred at room temperature for 4 hours. After concentration under reduced pressure, the residue was purified by preparative high performance liquid chromatography to give a white solid (40 mg, yield 74%). $^1$H NMR (800 MHz, Methanol-$d_4$) $\delta$ 7.66 (d, $J$ = 7.9, 1H), 7.35 (d, $J$ = 8.1, 1H), 7.24 (s, 1H), 7.21 (t, $J$ = 7.8 Hz, 1H), 7.12 (s, 1H), 7.10 (t, $J$ = 8.1 Hz, 1H), 7.07 (dd, $J$ = 8.1, 2.2 Hz, 1H), 7.03 (t, $J$ = 8.0 Hz, 1H), 7.01 (d, $J$ = 7.8 Hz, 1H), 3.93 (ABq, $J$ = 16.8, 12.8 Hz, 2H), 3.37-3.33 (m, 1H), 3.18 (dd, $J$ = 9.8, 8.1 Hz, 1H), 3.03 (q, $J$ = 7.4 Hz, 2H), 2.98-2.94 (m, 1H), 2.82 (td, $J$ = 9.2, 6.3 Hz, 1H), 2.58 (t, $J$ = 8.8 Hz, 1H), 2.36-2.26 (m, 1H), 1.91-1.84 (m, 1H), 1.26 (t, $J$ = 7.4 Hz, 3H); HRMS(ESI) $C_{21}H_{26}N_3O_2S^+$ $[M+H]^+$ calculated value: 384.1740, measured value: 384.1730; HPLC purity: 95.7% (RT = 14.06 min, $\lambda$ = 280 nm).

The purification condition for preparative liquid phase chromatography: Shim-pack GIST C18 column (250 $\times$ 20 mm, particle size 5 $\mu$M); water (containing 0.05% trifluoroacetic acid)/methanol (containing 0.05% trifluoroacetic acid) gradient elution; the flow rate was 10.0 mL/min.

The compounds listed in Table 3 below can also be prepared by referring to the methods of Examples 2 to 6:

Table 3

| Structure | MS | Structure | MS |
|---|---|---|---|
| 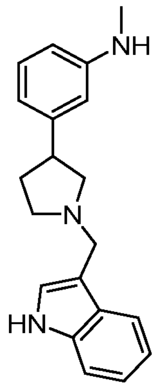 | 306.2 | 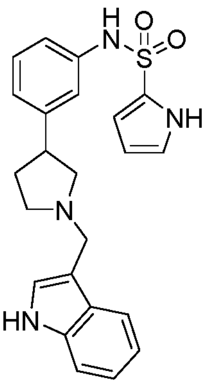 | 421.2 |
| 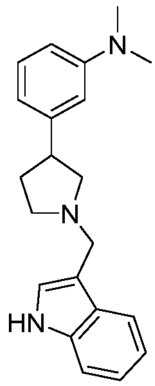 | 320.2 | 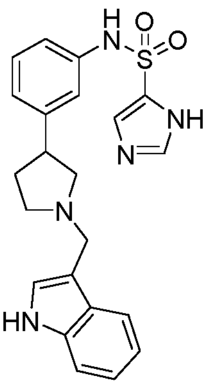 | 422.2 |
| 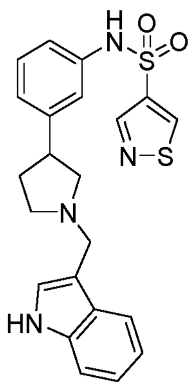 | 439.1 | 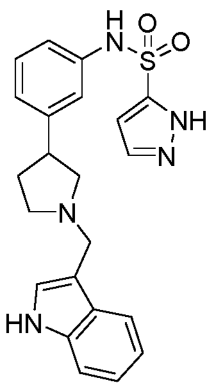 | 422.1 |
| 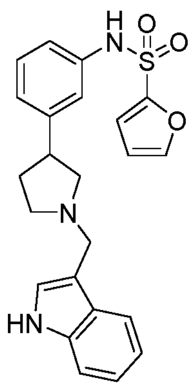 | 422.2 | 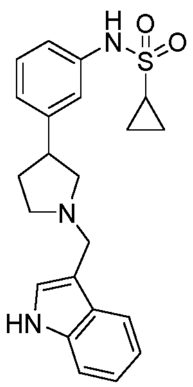 | 396.2 |

(continued)

| Structure | MS | Structure | MS |
|---|---|---|---|
| 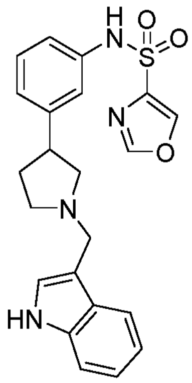 | 423.1 | 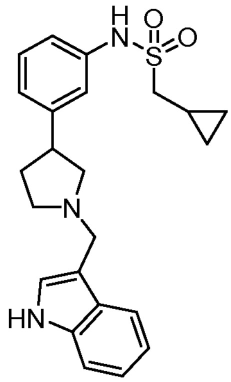 | 410.2 |
| 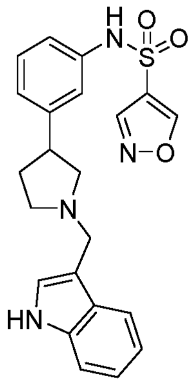 | 423.1 | 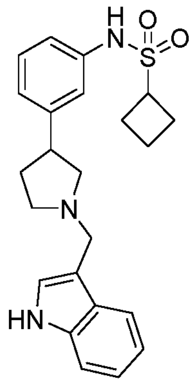 | 410.2 |
| 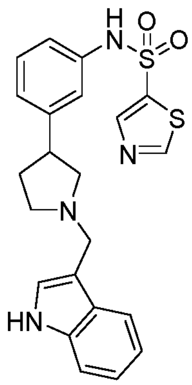 | 439.1 | 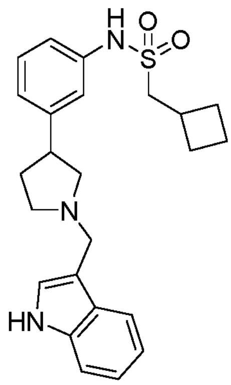 | 424.2 |
| 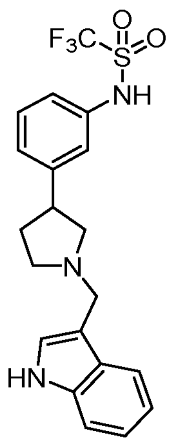 | 424.1 | / | / |

Example 7

Synthesis of 1-(indole-3-methyl)-3-(3-hydroxy-4-chlorophenyl)pyrrolidine (MDC-161502-013)

Step 1

Synthesis of 1-tert-butoxycarbonyl-3-(3-hydroxy-4-chlorophenyl)-2,5-dihydropyrrole

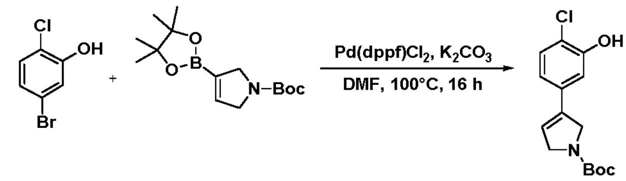


2-chloro-5-bromophenol (253 mg, 1.22 mmol, 1.2 eq), 1-tert-butoxycarbonyl-2,5-dihydro-1H-pyrrole-3-boronic acid pinacol ester (300 mg, 1.02 mmol, 1 eq), $K_2CO_3$ (564 mg, 4.08 mmol, 4 eq) and Pd(dppf)$Cl_2$ (75 mg, 0.103 mmol, 0.1 eq) were mixed in DMF (6 mL), stirred overnight at 100°C under the protection of nitrogen. After the reaction was completed, ethyl acetate (400 mL) was added, and the resulting mixture was successively washed with water (50 mL $\times$ 2) and saturated sodium chloride solution (50 mL $\times$ 3). The organic phase was dried over anhydrous sodium sulfate and concentrated. The crude product was purified by column chromatography (petroleum ether/ethyl acetate = 10/1) to give a pale yellow solid (230 mg, yield 76%). LCMS(ESI) $[M-56+H]^+$: 240.0.

Step 2

Synthesis of 1-tert-butoxycarbonyl-3-(3-hydroxy-4-chlorophenyl)-pyrrolidine

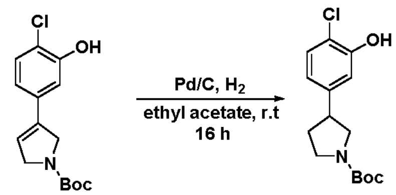


1- tert-butoxycarbonyl-3-(3-hydroxy-4-chlorophenyl)-2,5-dihydropyrrole (200 mg, 0.676 mmol, 1 eq) was dissolved in ethyl acetate (50 mL). Pd/C (40 mg, 10%) was added, and the resulting mixture was stirred overnight at room temperature under $H_2$ (20 Psi). Pd/C was removed by filtration, the filtrate was subjected to reduced pressure to remove solvent, and was purified by column chromatography (petroleum ether/ethyl acetate = 10/1-5/1) to give a yellowish solid (130 mg, yield 65%). LCMS(ESI) $[M-56+H]^+$: 242.0.

Step 3

Synthesis of 3-(3-hydroxy-4-chlorophenyl)-pyrrolidine

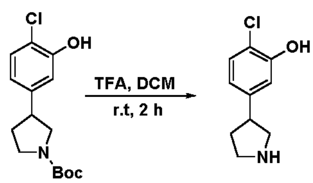

1- tert-butoxycarbonyl-3-(3-hydroxy-4-chlorophenyl)-pyrrolidine (130 mg, 0.437 mmol, 1 eq) was dissolved in dichloromethane (3 mL) and trifluoroacetic acid (3 mL), stirred at room temperature for 2 hours, and then the solvent was removed under reduced pressure to give a crude product, which was directly used in the next reaction. LCMS(ESI) $[M+H]^+$: 198.0.

Step 4

Synthesis of 1-(indole-3-methyl)-3-(3-hydroxy-4-chlorophenyl)pyrrolidine (MDC-161502-013)

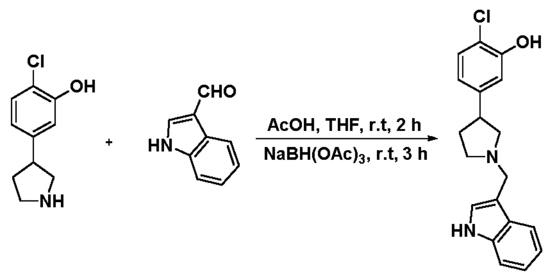


The crude product from the previous step and 3-indolealdehyde (76 mg, 0.524 mmol, 1.2 eq) were dissolved in dry tetrahydrofuran (5 mL). Acetic acid (0.2 mL) was added and stirred at room temperature for 2 h, then sodium triacetoxyborohydride (400 mg, 1.89 mmol, 4 eq) was added and stirred at room temperature for 3 hours. The reaction solution was concentrated and purified by preparative TLC (dichloromethane/methanol = 8/1), and then purified by column chromatography (dichloromethane/methanol = 20/1) to give a nearly white solid (80 mg, yield 56.0%). LCMS(ESI) $[M+H]^+$: 327.0. HNMR (400 MHz, DMSO-$d_6$) $\delta$: 11.44 (bs, 1 H), 10.17 (bs, 1 H), 7.82 (d, $J$ = 6.4 Hz, 1 H), 7.65 (s, 1 H), 7.45 (d, $J$ = 8.0 Hz, 1 H), 7.29 (d, $J$ = 8.0 Hz, 1 H), 7.07-7.21 (m, 2 H), 6.89 (s, 1 H), 6.79 (d, $J$ = 6.8 Hz, 1 H), 4.55 (s, 2 H), 3.50-3.75 (m, 2 H), 3.05-3.21 (m, 1 H), 2.27-2.42 (m, 1 H), 1.85-2.13 (m, 1 H).

Example 8

Synthesis of 1-(indole-3-methyl)-3-(2-chloro-3-hydroxyphenyl)pyrrolidine (MDC-161502-014)

Step 1

Synthesis of 1-tert-butoxycarbonyl-3-(2-chloro-3-hydroxyphenyl)-2,5-dihydropyrrole

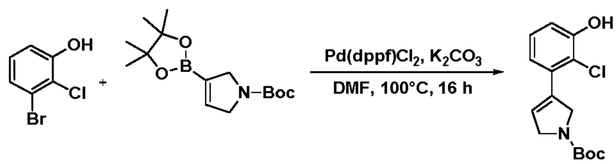


2-chloro-3-bromophenol (338 mg, 1.63 mmol, 1.2 eq), 1-tert-butoxycarbonyl-2,5-dihydro-1H-pyrrole-3-boronic acid pinacol ester (400 mg, 1.36 mmol, 1 eq), $K_2CO_3$ (770 mg, 5.57 mmol, 4 eq) and Pd(dppf)$Cl_2$ (100 mg, 0.137 mmol, 0.1 eq) were mixed in DMF (10 mL), stirred overnight at 100°C under the protection of nitrogen. After the reaction was completed, ethyl acetate (400 mL) was added, and the resulting mixture was successively washed with water (50 mL $\times$ 2) and saturated sodium chloride solution (50 mL $\times$ 3). The organic phase was dried over anhydrous sodium sulfate and concentrated. The crude product was purified by column chromatography (petroleum ether/ethyl acetate = 10/1) to give a pale yellow solid (350 mg, yield 87%). LCMS(ESI) $[M-56+H]^+$: 240.0.

Step 2

Synthesis of 1-tert-butoxycarbonyl-3-(2-chloro-3-hydroxyphenyl)-pyrrolidine

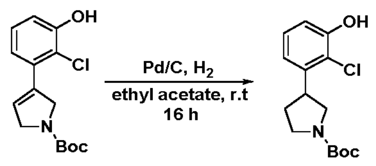


1- tert-butoxycarbonyl-3-(2-chloro-3-hydroxyphenyl)-2,5-dihydropyrrole (220 mg, 0.744 mmol, 1 eq) was dissolved in ethyl acetate (10 mL). Pd/C (50 mg, 10%) was added, and the resulting mixture was stirred overnight at room temperature under $H_2$ (20 Psi). Pd/C was removed by filtration, the filtrate was subjected to reduced pressure to remove solvent, and was purified by column chromatography (petroleum ether/ethyl acetate = 10/1-5/1) to give a yellowish solid (180 mg, yield 81%). LCMS(ESI) $[M-56+H]^+$: 242.0.

Step 3

Synthesis of 3-(2-chloro-3-hydroxyphenyl)-pyrrolidine

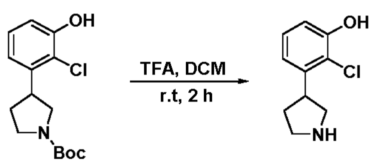


1- tert-butoxycarbonyl-3-(2-chloro-3-hydroxyphenyl)-pyrrolidine (90 mg, 0.302 mmol, 1 eq) was dissolved in dichloromethane (3 mL) and trifluoroacetic acid (3 mL), stirred at room temperature for 2 hours, and then the solvent was removed under reduced pressure to give a crude product, which was directly used in the next reaction. LCMS(ESI) $[M+H]^+$: 198.0.

Step 4

Synthesis of 1-(indole-3-methyl)-3-(2-chloro-3-hydroxyphenyl)pyrrolidine (MDC-161502-014)

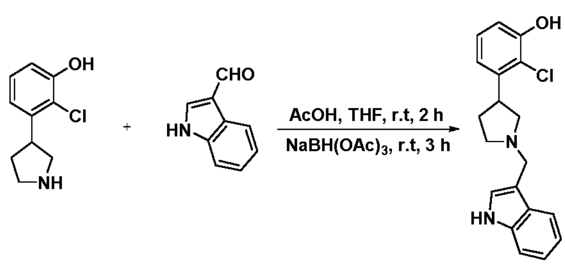


The crude product from the previous step and 3-indolealdehyde (50 mg, 0.344 mmol, 1.2 eq) were dissolved in dry tetrahydrofuran (5 mL). Acetic acid (0.1 mL) was added and stirred at room temperature for 2 h, then sodium triacetoxyborohydride (300 mg, 1.42 mmol) was added and stirred at room temperature for 3 hours. The reaction solution was concentrated and purified by preparative TLC (dichloromethane/methanol = 8/1), and then purified by column chromatography (dichloromethane/methanol = 20/1) to give a nearly white solid (45 mg, yield 46%). LCMS(ESI) $[M+H]^+$: 327.0. HNMR (400 MHz, DMSO-$d_6$) $\delta$: 11.43 (bs, 1 H), 10.23 (s, 1 H), 7.83 (d, $J$ = 7.6 Hz, 1 H), 7.64 (s, 1 H), 7.44 (d, $J$

= 8.0 Hz, 1 H), 7.07-7.19 (m, 3 H), 6.95 (d, *J* = 7.2 Hz, 1 H), 6.91 (d, *J* = 8.0 Hz, 1 H), 4.57 (s, 1 H), 3.79-4.06 (m, 1 H), 3.61-3.76 (m, 1 H), 3.38-3.57 (m, 1 H), 3.15-3.27 (m, 1 H), 2.30-2.42 (m, 1 H), 1.94-2.12 (m, 1 h).

Example 9

1-(indole-3-methyl)-3-(3-cyclopropylsulfonamidophenyl)pyrrolidine (MDC-161502-015)

Step 1

Synthesis of 3-nitrovinylbenzene

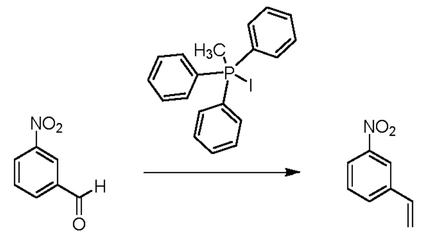

Methyltriphenylphosphine iodide (64.2 g, 158.8 mmol, 1.2 eq) was dissolved in 1,4-dioxane (500 ml). Potassium carbonate (27.4 g, 198.5 mmol, 1.5 eq) was added under nitrogen protection, and the resulting mixture was stirred at room temperature for 1 hour. 3-nitrobenzaldehyde (20 g, 132.3 mmol, 1.0 eq) was added to the reaction system. The resulting mixture was stirred at 110°C for 16 hours under nitrogen protection. The solvent was removed under reduced pressure. Ethyl acetate (200 ml) was added, and the resulting mixture was washed with water (80 ml × 3). The organic phase was dried over anhydrous sodium sulfate, filtered, concentrated, and the residue was purified by column chromatography (n-hexane) to give a yellow oily liquid (17.8 g, yield 90%). HNMR (400 MHz, $CDCl_3$) δ 8.22 (t, 1 H), 8.08 (dt, *J* = 8.0 Hz, 1 H), 7.70 (d, *J* = 7.6 Hz, 1 H), 7.48 (t, *J* = 8.0 Hz, 1 H), 6.76 (dd, $J_1$ = 17.2 Hz, $J_2$ = 10.8 Hz, 1 H), 5.88 (d, *J* = 17.6 Hz, 1 H), 5.43 (d, *J* = 10.8 Hz, 1 H).

Step 2

Synthesis of 1-benzyl-3-(3-nitrophenyl) pyrrolidine

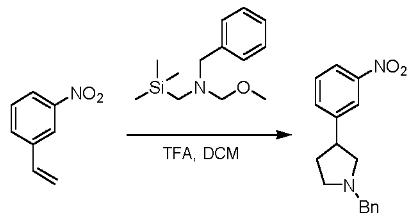


3-nitrovinylbenzene (13.2 g, 88.4 mmol, 1 eq) was dissolve in dichloromethane (90 mL). Trifluoroacetic acid (1.0 g, 8.84 mmol, 0.1 eq) was added. N-(methoxymethyl)-N-(trimethylsilylmethyl)benzylamine (45.7 g, 192.5 mmol, 2.1 eq) was added dropwise at 0°C within 30 minutes. The temperature of the reaction was raised to room temperature and then the reaction was stirred for 16 hours. The solvent was removed under reduced pressure, the residue was diluted with dichloromethane (250 mL) and washed 3 times with water (100 mL). The organic phase was dried over anhydrous sodium sulfate, filtered and concentrated, and the residue was purified by silica gel chromatography (dichloromethane : methanol = 100 : 1) to give a yellowish oily liquid (21.5 g, yield 86%). LCMS(ESI) $[M+H]^+$: 283.1.

Step 3

Synthesis of 1-benzyl-3-(3-aminophenyl)pyrrolidine

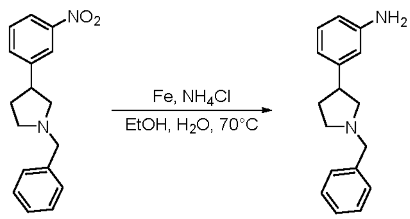


1- benzyl-3-(3-nitrophenyl)pyrrolidine (19.82 g, 70.2 mmol, 1 eq) was dissolved in water (50 mL) and ethanol (300 mL). Reduced iron powder (31.35 g, 561.6 mmol, 8 eq) and $NH_4Cl$ (30.04 g, 561.6 mmol, 8 eq) were added, and the resulting mixture was stirred at 70°C for 16 hours. The mixture was filtered. The filtrate was concentrated under reduced pressure. The residue was dissolved in ethyl acetate (300 mL) and washed once with saturated sodium bicarbonate solution (100 mL) and saturated brine (100 mL) respectively. The organic phase was dried over anhydrous sodium sulfate and concentrated. The residue was purified by silica gel chromatography (dichloromethane : methanol = 30 : 1, 10 : 1) to give a yellowish oily liquid (13.8 g, yield 78%). LCMS(ESI) $[M+H]^+$: 253.0.

Step 4

Synthesis of 1-benzyl-3-(3-aminophenyl)pyrrolidine

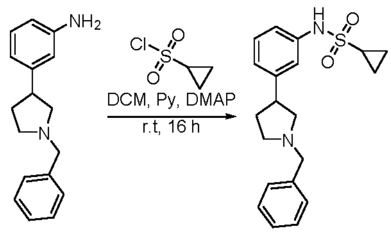


1- benzyl-3-(3-aminophenyl)pyrrolidine (190 mg, 0.79 mmol, 1 eq), DMAP (10 mg, 0.08 mmol, 0.1 eq) and pyridine (1 mL) were mixed in dichloromethane (3 mL). Cyclopropyl sulfonyl chloride (127 mg, 0.90 mmol, 1.1 eq) was added under stirring and reacted at room temperature for 16 hours. After the reaction was completed, the solvent was removed under reduced pressure and ethyl acetate (100 mL) was added. The resulting mixture was successively washed with water (20 mL × 2) and saturated sodium chloride solution (20 mL). The organic phase was dried over sodium sulfate, concentrated, and purified by column chromatography (dichloromethane/methanol = 50/1) to give a colorless oily liquid (190 mg, yield 71%).

Step 5

Synthesis of 3-(3-aminophenyl)pyrrolidine

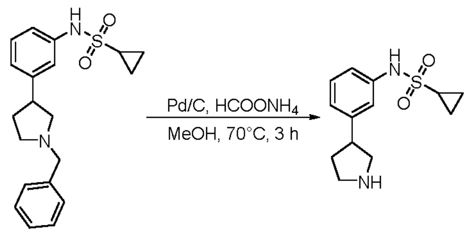


1- benzyl-3-(3-cyclopropylsulfonamidophenyl)pyrrolidine (140 mg, 0.39 mmol, 1 eq), ammonium formate (247 mg, 3.9 mmol, 10 eq), Pd/C (20 mg, 10%) were mixed in a solution of methanol (3 mL) and ammonia/methanol (3 mL, 2 mol/L), stirred at 70°C for 16 hours and filtered. The filtrate was subjected to reduced pressure to remove solvent, and the residue was purified by column chromatography (dichloromethane/methanol = 20/1, 10/1) to give a white solid (50 mg, yield 48%).

Step 6

Synthesis of 1-(indole-3-methyl)-3-(3-cyclopropylsulfonamidophenyl)pyrrolidine (MDC-161502-015)

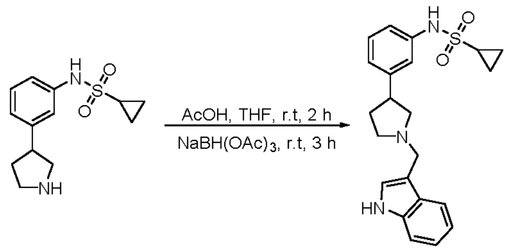


3-(3-cyclopropylsulfonamidophenyl)pyrrolidine (50 mg, 0.19 mmol, 1 eq) and 3-indolealdehyde (30 mg, 0.21 mmol, 1.1 eq) were dissolved in dry tetrahydrofuran (3 mL). Acetic acid (0.1 mL) was added and stirred at room temperature for 2 h. Sodium triacetoxyborohydride (162 mg, 0.77 mmol, 4 eq) was added and stirred at room temperature for 2 hours. The reaction solution was concentrated and purified by column chromatography (dichloromethane/methanol = 20/1) to give a white solid (25 mg, yield 34%). $^1H$ NMR (400 MHz, Methanol-$d_4$) $\delta$ 7.76 (d, $J$ = 8.0 Hz, 1H), 7.56 (s, 1H), 7.45 (d, $J$ = 8.0 Hz, 1H), 7.32-7.29 (m, 1H), 7.29 - 7.24 (m, 4H), 7.09 (d, $J$ = 8.0 Hz, 1H), 4.61 (s, 2H), 3.79 (dd, $J$ = 12, 8.0 Hz, 1H), 3.60 - 3.51 (m, 3H), 2.53-2.49 (m, 2H), 2.48-2.16 (m, 1H), 1.41-1.36 (m, 1H), 1.02-0.94 (m, 2H), 0.93-0.89 (m, 2H); LCMS(ESI) $[M+H]^+$: 396.2.

Example 10

1-(indole-3-methyl)-3-(3-cyclobutylmethylsulfonamidophenyl)pyrrolidine (MDC-161502-017)

Step 1

Synthesis of 1-benzyl-3-(3-cyclobutylmethylsulfonamidophenyl)pyrrolidine

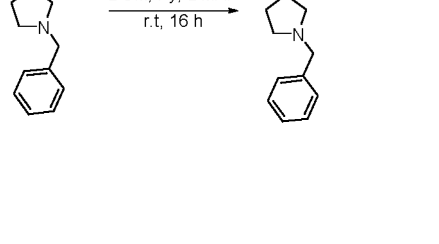

1-benzyl-3-(3-aminophenyl)pyrrolidine (200 mg, 0.793 mmol, 1 eq), DMAP (10 mg, 0.0820 mmol, 0.1 eq) and pyridine (1 mL) were mixed in dichloromethane (3 mL). Cyclobutyl methanesulfonyl chloride (150 mg, 0.890 mmol, 1.1 eq) was added under stirring and reacted overnight at room temperature. After the reaction was completed, the solvent was removed under reduced pressure and ethyl acetate (100 mL) was added. The resulting mixture was successively washed with water (20 mL × 2) and saturated sodium chloride solution (20 mL). The organic phase was dried over sodium sulfate, concentrated, and purified by column chromatography (dichloromethane/methanol = 50/1) to give a yellowish solid (160 mg, yield 53%). LCMS(ESI) $[M+H]^+$: 385.0.

Step 2

Synthesis of 3-(3-cyclobutylmethylsulfonamidophenyl)pyrrolidine

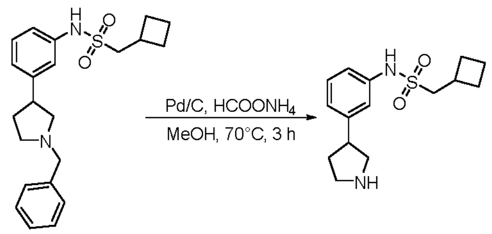


1-benzyl-3-(3-cyclobutylmethylsulfonamidophenyl)pyrrolidine (62 mg, 0.161 mmol, 1 eq), ammonium formate (82 mg, 1.30 mmol, 8 eq) and Pd/C (10 mg, 10%) were mixed in methanol (5 mL), stirred at 70°C for 3 hours and filtered. The filtrate was subjected to reduced pressure to remove solvent, and the residue was purified by column chromatography (dichloromethane/methanol = 20/1, 10/1) to give a white solid (40 mg, 85% yield). LCMS(ESI) $[M+H]^+$: 295.0.

Step 3

Synthesis of 1-(indole-3-methyl)-3-(3-cyclobutylmethylsulfonamidophenyl)pyrrolidine (MDC-161502-017)

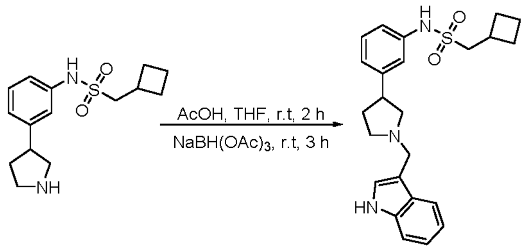


3-(3-cyclobutylmethylsulfonamidophenyl)pyrrolidine (40 mg, 0.136 mmol, 1 eq) and 3-indolealdehyde (25 mg, 0.172 mmol, 1.2 eq) were dissolved in dry tetrahydrofuran (5 mL). Acetic acid (0.1 mL) was added and stirred at room temperature for 2 h. Sodium triacetoxyborohydride (120 mg, 0.566 mmol, 4 eq) was added and stirred at room temperature for 2 hours. The reaction solution was concentrated and purified by column chromatography (dichloromethane/methanol = 20/1) to give a white solid (28 mg, yield 49%). LCMS(ESI) $[M+H]^+$: 424.0; HNMR (400 MHz, DMSO-$d_6$) δ: 11.43 (bs, 1 H), 9.75 (s, 1 H), 7.82 (d, $J$ = 5.6 Hz, 1 H), 7.64 (s, 1 H), 7.44 (d, $J$ = 8.0 Hz, 1 H), 7.30 (t, $J$ = 8.0 Hz, 1 H), 7.17 (t, $J$ = 8.0 Hz, 1 H), 7.05-7.10 (m, 4 H), 4.58 (s, 2 H), 3.37-3.81 (m, 3 H), 3.07-3.25 (m, 3 H), 2.61-2.72 (m, 1 H), 2.31-2.43 (m, 1 H), 1.94-2.11 (m, 3 h), 1.67-1.87 (m, 4 H).

Example 11

1-(3-indolylmethyl)-3-(3-diethoxyphosphoryloxyphenyl) pyrrolidine (MDC-161502-030)

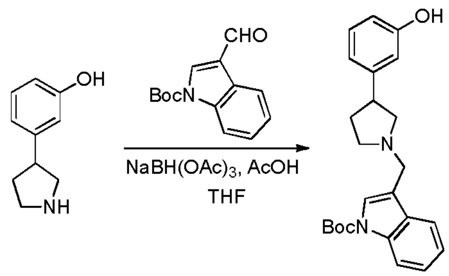


3-(3-hydroxyphenyl)pyrrolidine (1.65 g, 10.1 mmol) and 1-tert-butoxycarbonyl 3-indolealdehyde (2.75 g, 11.2 mmol) were dissolved in dry tetrahydrofuran (40 mL). Acetic acid (2 mL) was added and stirred at room temperature for 2 hours. And then sodium borohydride triacetate (8.60 g, 40.6 mmol) was added and stirred at room temperature for 16 hours. Ethyl acetate (450 mL) was added to dilute the reaction system. The system was successively washed with saturated sodium bicarbonate solution (100 mL × 3) and saturated brine (100 mL 2). The organic phase was dried over anhydrous sodium sulfate. The desiccant was removed by filtration. The filtrate was concentrated under reduced pressure to remove solvent and then purified by column chromatography (dichloromethane/methanol = 8/1) to give a pale yellow solid (3.5 g, 76% yield). LCMS: m/z = 393 $[M+H]^+$.

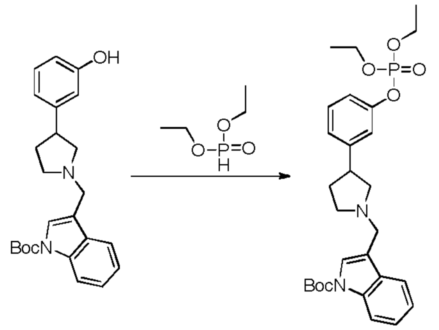

1-(1-tert-butoxycarbonylindole-3-methyl)-3-(3-hydroxyphenyl)pyrrolidine (400 mg, 1.02 mmol) was dissolved in dry acetonitrile (20 mL) and cooled to -10°C. Diisopropylethylamine (264 mg, 2.04 mmol), N,N-dimethylpyridine (13 mg, 0.106 mmol), carbon tetrachloride (786 mg, 5.11 mmol), and diethylphosphite (212 mg, 1.54 mmol) were added successively, and then the resulting mixture was stirred at room temperature for 16 hours. The system was diluted by adding ethyl acetate (300 mL), washed successively with water (80 mL × 2) and saturated brine (80 mL × 2), dried over anhydrous sodium sulfate, filtered to remove desiccant, subjected to reduced pressure to remove solvent, and purified with flash silica gel column chromatography (petroleum ether/ethyl acetate/ethanol = 12/3/1-4/3/1) to give a yellowish solid (360 mg, yield 67%). LCMS: m/z = 529 $[M+H]^+$.

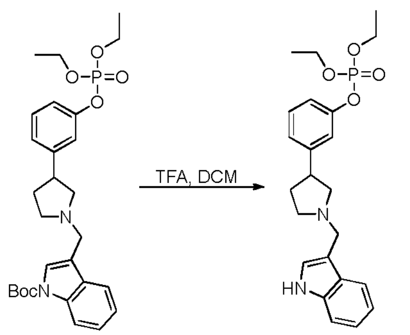


MDC-161502-030

1-(1-tert-butoxycarbonylindole-3-methyl)-3-(3-diethoxyphosphoryloxyphenyl)pyrrolidine (300 mg, 0.568 mmol) was dissolved in dry dichloromethane (10 mL). Trifluoroacetic acid (8 mL) was added and stirred at room temperature for 3 hours, and the solvent was removed under reduced pressure. Triethylamine alkalization system was added, and the solvent was removed under reduced pressure again. The resulting product was purified by preparative liquid phase chromatography (acetonitrile-water-acetic acid) to give a yellowish viscous solid (180 mg, yield 74%). LCMS: m/z = 429

[M+H]$^+$; $^1$H NMR (500 MHz, DMSO-$d_6$) δ 11.18 (s, 1 H), 7.73 (d, *J* = 8.0 Hz, 1 H), 7.44 (s, 1 H), 7.40 (d, *J* = 8.5 Hz, 1 H), 7.33 (t, *J* = 8.0 Hz, 1 H), 7.16 (d, *J* = 8.5 Hz, 1 H), 7.15 (s, 1 H), 7.12 (td, $J_1$ = 80 Hz, $J_2$ = 0.5 Hz, 1 H), 7.03-7.08 (m, 2 H), 4.09-4.23 (m, 6 H), 3.73 (m, 1 H), 3.14 (m, 1 H), 3.03 (m, 1 H), 2.88 (m, 1 H), 2.33 (m, 1 H), 1.87 (m, 1 H), 1.25 (m, 3 H), 1.13 (m, 1 H).

Example 12 (MDC-161502-031)

Step 1

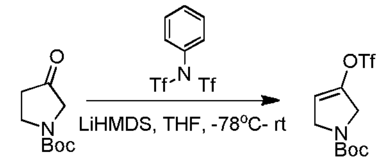


1-tert-butoxycarbonyl-3-pyrrolidone (15 g, 80.98 mmol, 1.0 eq) was placed in a 1L three-necked reaction flask and the flask was subjected to replacement with nitrogen three times. Anhydrous tetrahydrofuran (200 mL) was added, and the temperature was reduced to -78°C. Subsequently, a solution of lithium bis(trimethylsilyl)amide (121 ml, 1M) in tetrahydrofuran was slowly added and stirred for 1h while keeping the temperature constant. N-phenyl bis(trifluoromethanesulfonimide) (43.4 g, 121.5 mmol, 1.5 eq) was then dissolved in tetrahydrofuran solution (80 ml) and slowly added to the reaction system. Then the temperature of the reaction solution was raised to room temperature and the reaction was stirred overnight. Saturated sodium bicarbonate solution (100 ml) was added to the reaction solution. The resulting mixture was extracted 3 times with ethyl acetate (80 ml). The organic phases were combined and washed 3 times with saturated brine (80 ml), dried over anhydrous sodium sulfate, filtered and concentrated to give 60 g of yellow crude product. LCMS: m/z = 318[M+H]$^+$.

Step 2

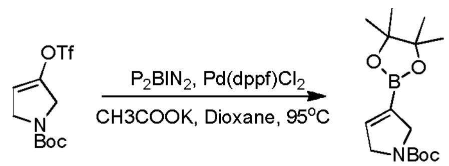


3-(trifluoromethylsulfonyloxy)-2H-pyrrole-1(5H)-carboxylic acid tert-butyl ester crude product (6 g), pinacol borate (2.45 g, 9.64 mmol, 1.2 eq), potassium acetate (1.32 g, 16.1 mmol, 2 eq) and [1,1'-bis(diphenylphosphine)ferrocene] palladium dichloride dichloromethane complex (131 mg, 0.16 mmol, 0.02 eq) were dissolved in 1,4-dioxane (60 ml). The mixture was subjected to replacement with nitrogen three times. The temperature of the mixture was raised to 95°C and the reaction was stirred for 5h while keeping the temperature constant. Water (80 ml) was added to the reaction solution. The resulting mixture was extracted 3 times with ethyl acetate (80 ml). The organic phases were combined and washed three times with saturated brine (80 ml), dried over anhydrous sodium sulfate, filtered and concentrated to give a black crude product. LCMS: m/z = 296[M+H]$^+$.

Step 3

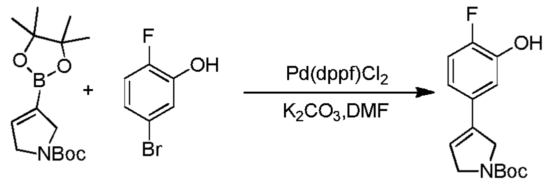


1-tert-butoxycarbonyl-2,5-dihydro-1H-pyrrole-3-boronic acid pinacol ester (1220 mg, 5.08 mmol, 1.0 eq), 5-bromo-2-fluorophenol (970 mg, 5.08 mmol, 1.0 eq), potassium carbonate (2.11 g, 15.24 mmol, 3 eq) and [1,1'-bis(diphenylphosphine)ferrocene]palladium dichloride dichloromethane complex (82 mg, 0.1 mmol, 0.02 eq) were dissolved in N,N-dimethylformamide (20 ml). The mixture was subjected to replacement with nitrogen three times. The temperature of the mixture was raised to 90°C and the reaction was stirred overnight. Water (50 ml) was added to the reaction solution. The resulting mixture was extracted 3 times with ethyl acetate (30 ml). The organic phases were combined and washed three times with saturated brine (30 ml), dried over anhydrous sodium sulfate, filtered, concentrated and purified on a silica gel column (dichloromethane : methanol = 19 : 1) to give a desired white compound (500 mg, 1.79 mmol, 35%). LCMS: m/z = 280$[M+H]^+$.

Step 4

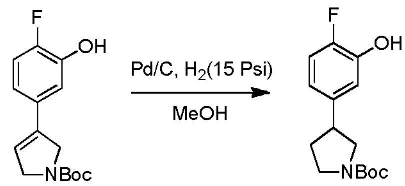


1-tert-butoxycarbonyl-3-(4-fluoro-3-hydroxyphenyl)-2,5-dihydro-1H-pyrrolidine (500 mg, 1.79 mmol) was dissolved in methanol (30 ml). Palladium carbon (200 mg) was added, and the resulting mixture was stirred at room temperature for 16 hours under hydrogen atmosphere (15 Psi). After filtration, the filtrate was concentrated to give a white solid (450 mg, 1.60 mmol, 89.3% yield). The crude product was directly used for the next step. LCMS: m/z = 282$[M+H]^+$.

Step 5

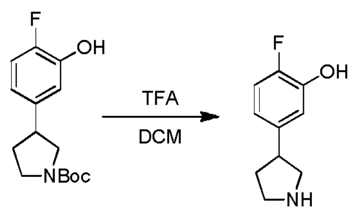


1-tert-butoxycarbonyl-3-(4-fluoro-3-hydroxyphenyl)pyrrolidine (450 mg, 1.6 mmol) was dissolved in dichloromethane (5 ml). Trifluoroacetic acid (5 ml) was added dropwise and the resulting mixture was stirred at room temperature for 2 hours and then concentrated under reduced pressure to give a white solid (280 mg, 1.54 mmol, yield: 96.6%). LCMS: m/z = 182$[M+H]^+$.

Step 6

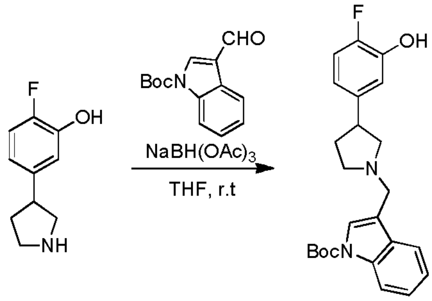


3-(4-fluoro-3-hydroxyphenyl)pyrrolidine (170 mg, 0.939 mmol) and 1-tert-butoxycarbonyl-3-indolealdehyde (230 mg, 0.94 mmol, 1.0 eq) were dissolved in anhydrous tetrahydrofuran (10 ml). Acetic acid (60 mg, 1 mmol) and $NaBH(OAc)_3$ (795 mg, 3.75 mmol) were added at room temperature, and the resulting mixture was stirred at room temperature for 3 hours. After concentration under reduced pressure, the residue was dissolved in ethyl acetate (50 ml) and the resulting mixture was washed with saturated sodium bicarbonate solution (30 ml). The organic phase was dried over anhydrous sodium sulfate, filtered and concentrated, and the residue was purified on a silica gel column (dichloromethane/methanol = 10 : 1) to give a white solid (150 mg, 0.37 mmol, yield: 38.9%). LCMS: m/z = 411$[M+H]^+$.

Step 7

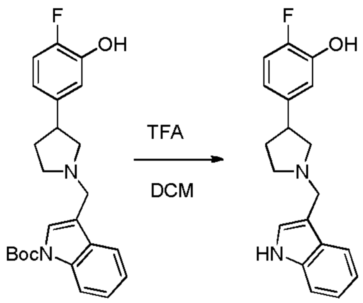


MDC-161502-031

1-(1-tert-butoxycarbonylindole-3-methyl)-3-(4-fluoro-3-hydroxyphenyl)pyrrolidine (150 mg, 0.37 mmol) was dissolved in dichloromethane (5 ml). Trifluoroacetic acid (5 ml) was added dropwise and the resulting mixture was stirred at room temperature for 2 hours. After concentration under reduced pressure, the residue was purified by prep-HPLC to give a white solid (70 mg, 0.23 mmol, yield: 62%). $^1H$ NMR (500 MHz, DMSO-$d_6$) $\delta$ 11.18 (s, 1H), 9.81 (brs, 1H), 7.72 (d, $J$ = 7.5 Hz, 1H), 7.44(s, 1H), 7.40(d, $J$ = 8.0 Hz, 1H), 7.13-7.10 (m, 1H), 7.06-7.02 (m, 2H), 6.88 (dd, $J_1$= 2.0 Hz, $J_2$= 8.5 Hz, 1H), 6.71-6.68 (m, 1H), 4.19 (s, 2H), 3.34-3.32 (m, 1H), 3.11-3.05 (m, 3H), 2.82-2.80 (m, 1H), 2.28-2.25 (m, 1H), 1.85-1.79 (m, 1H).LCMS: m/z = 311$[M+H]^+$; Prep-HPLC method: Shim-pack GIST C18 column (250 × 50 mm, particle size 5 $\mu$M); 0.1% $CH_3COOH$ in $H_2O$/0.1% $CH_3COOH$ in ACN gradient eluting system; flow rate = 40.0 mL/min.

Example 13 (MDC-161502-032)

Step 1

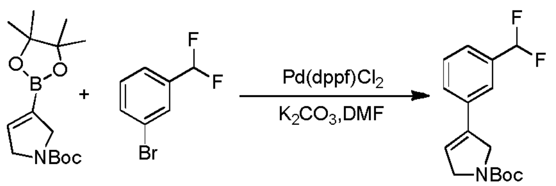


1-tert-butoxycarbonyl-2,5-dihydro-1H-pyrrole-3-boronic acid pinacol ester (584 mg, 2.43 mmol, 1.0 eq), 3-difluoromethylbromobenzene (500 mg, 2.43 mmol, 1.0 eq), potassium carbonate (1 g, 7.25 mmol, 3 eq) and [1,1'-bis(diphenylphosphine)ferrocene]palladium dichloride dichloromethane complex (40 mg, 0.05 mmol, 0.02 eq) were dissolved in N,N-dimethylformamide (20 ml). The mixture was subjected to replacement with nitrogen three times. The temperature of the mixture was raised to 90°C and the reaction was stirred overnight. Water (50 ml) was added to the reaction solution. The resulting mixture was extracted 3 times with ethyl acetate (30 ml). The organic phases were combined and washed three times with saturated brine (30 ml), dried over anhydrous sodium sulfate, filtered, concentrated and purified on a silica gel column (dichloromethane : methanol = 19 : 1) to give a desired compound (500 mg, 1.69 mmol, 69.5%). LCMS: m/z = 296[M+H]$^+$;

Step 2

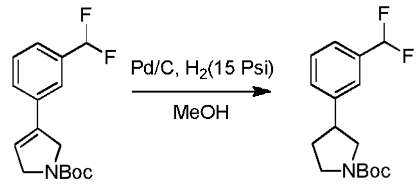


1-tert-butoxycarbonyl-3-(3-difluoromethylphenyl)-2,5-dihydro-1H-pyrrolidine (400 mg, 1.35 mmol) was dissolved in methanol (30 ml). Palladium carbon (200 mg) was added, and the resulting mixture was stirred at room temperature for 16 hours under hydrogen atmosphere (15 Psi). After filtration, the filtrate was concentrated to give a white solid (370 mg, 1.24 mmol, 91.8% yield), and the crude product was directly used for the next reaction. LCMS: m/z = 298[M+H]$^+$;

Step 3

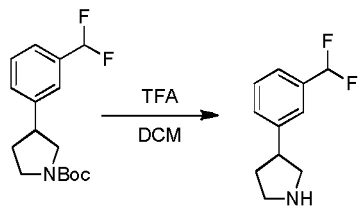


1-tert-butoxycarbonyl-3-(3-difluoromethylphenyl) pyrrolidine (370 mg, 1.25 mmol) was dissolved in dichloromethane (5 ml). Trifluoroacetic acid (5 ml) was added dropwise and the resulting mixture was stirred at room temperature for 2 hours and then concentrated under reduced pressure to give a brown oil (245 mg, 1.24 mmol, yield: 99.2%). LCMS: m/z = 198[M+H]$^+$;

Step 4

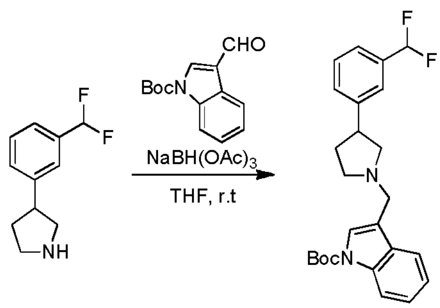

3-(3-difluoromethylphenyl) pyrrolidine (245 mg, 1.24 mmol) and 1-tert-butoxycarbonyl 3-indolealdehyde (304 mg, 1.24 mmol, 1.0 eq) were dissolved in anhydrous tetrahydrofuran (10 ml). Acetic acid (72 mg, 1.2 mmol) and NaBH(OAc)3 (1.04 g, 4.96 mmol) were added at room temperature, and the resulting mixture was stirred at room temperature for 3 hours and concentrated under reduced pressure. The residue was dissolved with ethyl acetate (50 ml), washed with saturated sodium bicarbonate solution (30 ml). The organic phase was dried over anhydrous sodium sulfate, filtered, and concentrated, and the residue was purified on a silica gel column (dichloromethane/methanol = 10 : 1) to give a white solid (280 mg, 0.66 mmol, yield: 53.2%). LCMS: m/z = 427[M+H]$^+$.

Step 5

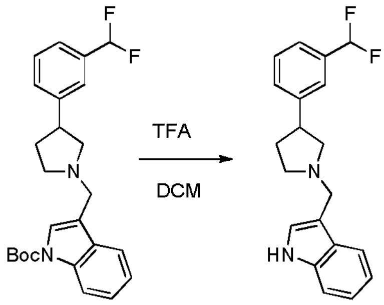


MDC-161502-032

1-(1-tert-butoxycarbonylindole-3-methyl)-3-(3-difluoromethylphenyl)pyrrolidine (280 mg, 0.66 mmol) was dissolved in dichloromethane (5 ml). Trifluoroacetic acid (5 ml) was added dropwise and the resulting mixture was stirred at room temperature for 2 hours and then concentrated under reduced pressure. The residue was purified by prep-HPLC to give a white solid (150 mg, 0.46 mmol, yield: 70%). $^1$H NMR (500 MHz, DMSO-*d$_6$)* $\delta$ 11.38 (s, 1H), 10.09 (brs, 1H),7.79 (d, *J* = 7.5 Hz, 1H), 7.57-7.43 (m, 5H), 7.15 (d, *J* = 7.5 Hz, 1H), 7.12-7.08 (m, 1H), 6.95 (d, *J* = 55.5 Hz, 1H), 4.50 (s, 2H), 3.54-3.53 (m, 3H), 3.32-3.31 (m, 2H), 2.41-2.36 (m, 1H), 2.01-2.00 (m, 1H).LCMS: m/z = 327[M+H]$^+$; Prep-HPLC method: Shim-pack GIST C18 column (250 $\times$ 50 mm, particle size 5 $\mu$M); 0.1% $CH_3COOH$ in $H_2O$/0.1% $CH_3COOH$ in ACN gradient eluting system; flow rate = 40.0 mL/min.

Example 14 (MDC-161502-033)

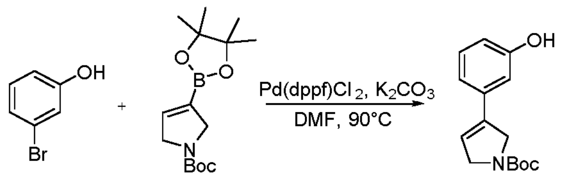


3-bromophenol (13.6 g, 78.6 mmol), 1-tert-butoxycarbonyl-2,5-dihydro-1H-pyrrole-3-boronic acid pinacol ester (16.8 g, 57.1 mmol), 1,1'-bis-diphenylphosphinoferrocene palladium dichloride (2.1 g, 2.87 mmol) and potassium carbonate (23.7 g, 172 mmol) were mixed in N,N-dimethylformamide (100 mL). The mixture was subjected to replacement with nitrogen to remove oxygen and then the mixture was raised to 95°C in temperature and reacted for 16 hours. The reaction system was diluted with ethyl acetate (300 mL), washed successively with water (300 mL $\times$ 2) and saturated brine (200 mL $\times$ 5), and dried over anhydrous sodium sulfate. The desiccant was removed by filtration. The filtrate was concentrated under reduced pressure to remove solvent and then purified by column chromatography (petroleum ether/ethyl acetate = 3/1, 2/1, ethyl acetate) to give a yellowish solid (3.1 g, yield 21%). LCMS: m/z = 262 [M+H]$^+$.

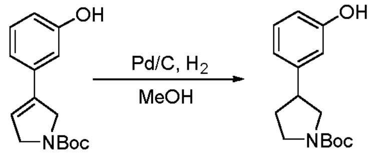


1-tert-butoxycarbonyl-3-(3-hydroxyphenyl)-2,5-dihydro-1H-pyrrolidine (400 mg, 1.53 mmol) was dissolved in methanol (40 mL). Palladium carbon (80 mg) was added, and the resulting mixture was stirred at room temperature for 3 hours under hydrogen (latm). Palladium carbon was removed by filtration, and the filtrate was concentrated under reduced pressure to give a pale yellow solid (402 mg, yield > 99%). LCMS: m/z = 208 $[M-56+H]^+$.

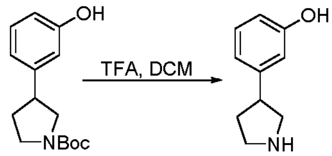


1-tert-butoxycarbonyl-3-(3-hydroxyphenyl)pyrrolidine (380 mg, 1.44 mmol) was dissolved in dry dichloromethane (15 mL). Trifluoroacetic acid (10 mL) was added, and the resulting mixture was stirred at room temperature for 3 hours, and then concentrated under reduced pressure to remove dichloromethane and excess trifluoroacetic acid to give a light brown viscous oil. LCMS: m/z = 164 $[M+H]^+$.

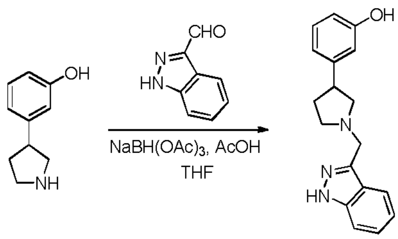


MDC-161502-033

The crude 3-(3-hydroxyphenyl)pyrrolidine (375 mg, 1.44 mmol) and 3-indazolecarbaldehyde (220 mg, 1.51 mmol) were dissolved in dry tetrahydrofuran (20 mL). Acetic acid (0.3 mL) was added and stirred at room temperature for 4 hours. And then sodium borohydride triacetate (1.50 g, 7.08 mmol) was added and stirred at room temperature for 16 hours. The reaction system was diluted with ethyl acetate (300 mL), washed with saturated sodium bicarbonate solution (50 mL × 3), and the organic phase was dried over anhydrous sodium sulfate. The desiccant was removed by filtration. The filtrate was concentrated under reduced pressure to remove solvent and purified on a preparative silica gel plate (dichloromethane/methanol = 9/1) and then by preparative liquid phase chromatography (acetonitrile-water-acetic acid) to give a white solid (80 mg, yield 19%). LCMS: m/z = 294 $[M+H]^+$; $^1H$ NMR (500 MHz, DMSO-$d_6$) $\delta$ 12.82 (bs, 1 H), 9.23 (s, 1 H), 7.87 (d, $J$ = 8.0 Hz, 1 H), 7.48 (d, $J$ = 8.5 Hz, 1 H), 7.33 (ddd, $J_1$ = 8.0 Hz, $J_2$ = 7.0 Hz, $J_3$ = 1.0 Hz, 1 H), 7.10 (t, $J$ = 7.5 Hz, 1 H), 7.04 (t, $J$ = 8.0 Hz, 1 H), 6.65-6.68 (m, 2 H), 6.55 (ddd, $J_1$ = 8.0 Hz, $J_2$ = 2.0 Hz, $J_3$ = 1.0 Hz, 1 H), 4.03 (s, 2 H), 3.22 (m, 1 H), 2.98 (m, 1 H), 2.72-2.81 (m, 2 H), 2.54 (m, 1 H), 2.21 (m, 1 H), 1.74 (m, 1 H).

Example 15 (MDC-161502-034)

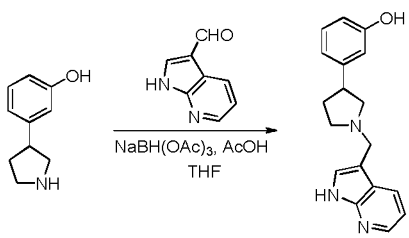


MDC-161502-034

The crude 3-(3-hydroxyphenyl)pyrrolidine (210 mg, 0.759 mmol) and 1H-pyrrolo[2,3-b]pyridin-3-carbaldehyde (115 mg, 0.787 mmol) were dissolved in dry tetrahydrofuran (10 mL). Acetic acid (0.2 mL) was added and stirred at room temperature for 4 hours. And then sodium borohydride triacetate (805 mg, 3.80 mmol) was added and stirred at room temperature for 16 hours. The reaction system was diluted with ethyl acetate (300 mL), washed with saturated sodium bicarbonate solution (50 mL × 3), and the organic phase was dried over anhydrous sodium sulfate. The desiccant was removed by filtration. The filtrate was concentrated under reduced pressure to remove solvent and purified on a preparative silica gel plate (dichloromethane/methanol = 8/1) and then by preparative liquid phase chromatography (acetonitrile-water-acetic acid) to give a white solid (60 mg, yield 27%). LCMS: m/z = 294 $[M+H]^+$; $^1H$ NMR (500 MHz, DMSO-$d_6$) δ 11.61 (s, 1 H), 9.29 (s, 1 H), 8.22 (dd, $J_1$ = 4.5 Hz, $J_2$ = 1.5 Hz, 1 H), 8.11 (d, $J$ = 7.0 Hz, 1 H), 7.50 (s, 1 H), 7.08 (dd, $J_1$ = 8.0 Hz, $J_2$ = 4.5 Hz, 1 H), 7.06 (t, $J$ = 8.0 Hz, 1 H), 6.69 (d, $J$ = 3.5 Hz, 1 H), 6.68 (d, $J$ = 1.5 Hz, 1 H), 6.59 (dd, $J_1$ = 7.0 Hz, $J_2$ = 1.5 Hz, 1 H), 4.04 (s, 2 H), 3.14 (m, 1 H), 2.92 (m, 2 H), 2.70 (m, 1 H), 2.25 (m, 1 H), 1.81 (m, 1 H).

Example 16 (MDC-161502-035)

Step 1

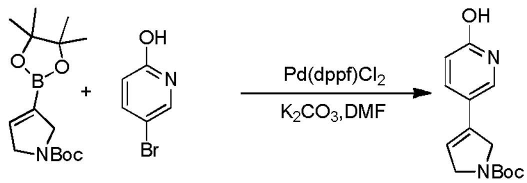


1-tert-butoxycarbonyl-2,5-dihydro-1H-pyrrole-3-boronic acid pinacol ester (584 mg, 2.43 mmol, 1.0 eq), 2-hydroxy-5-bromopyridine (500 mg, 2.43 mmol, 1.0 eq), potassium carbonate (1 g, 7.25 mmol, 3.0 eq) and [1,1'-bis(diphenylphosphine)ferrocene]palladium dichloride dichloromethane complex (40 mg, 0.05 mmol. 0.02 eq) were dissolved in N,N-dimethylformamide (20 ml). The mixture was subjected to replacement with nitrogen three times. The temperature of the mixture was raised to 90°C and the reaction was stirred overnight. Water (50 ml) was added to the reaction solution. The resulting mixture was extracted 3 times with ethyl acetate (30 ml). The organic phases were combined and washed three times with saturated brine (30 ml), dried over anhydrous sodium sulfate, filtered, concentrated and purified on a silica gel column (dichloromethane : methanol = 19 : 1) to give a desired compound (350 mg, 1.34 mmol, 54.9%). LCMS: m/z = $262[M+H]^+$.

Step 2

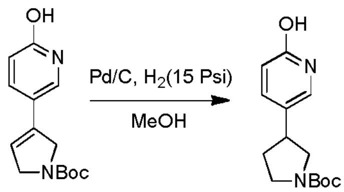


1-tert-butoxycarbonyl-3-(6-hydroxy-3-pyridinyl)-2,5-dihydro-1H-pyrrolidine (370 mg, 1.34 mmol) was dissolved in methanol (30 ml). Palladium carbon (180 mg) was added, and the resulting mixture was stirred at room temperature for 16 hours under hydrogen atmosphere (15 Psi). After filtration, the filtrate was concentrated to give a white solid (350 mg, 1.33 mmol, 98.8% yield), and the crude product was directly used for the next reaction. LCMS: m/z = $264[M+H]^+$.

Step 3

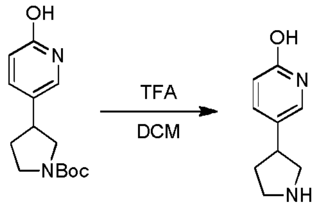


1-tert-butoxycarbonyl-3-(6-hydroxy-3-pyridinyl)pyrrolidine (350 mg, 1.33 mmol) was dissolved in dichloromethane (5 ml). Trifluoroacetic acid (5 ml) was added dropwise and the resulting mixture was stirred at room temperature for 2 hours and then concentrated under reduced pressure to give a brown oil (210 mg, 1.28 mmol, yield: 96.2%). LCMS: m/z = 164$[M+H]^+$.

Step 4

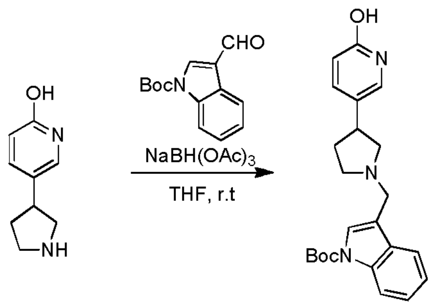


3-(6-hydroxy-3-pyridinyl) pyrrolidine (210 mg, 1.28 mmol) and 1-tert-butoxycarbonyl 3-indolealdehyde (313 mg, 1.28 mmol, 1.0 eq) were dissolved in anhydrous tetrahydrofuran (10 ml). Acetic acid (90 mg, 1.5 mmol) and $NaBH(OAc)_3$ (1.08 g, 5.12 mmol) were added at room temperature, and the resulting mixture was stirred at room temperature for 3 hours and concentrated under reduced pressure. The residue was dissolved with ethyl acetate (50 ml), washed with saturated sodium bicarbonate solution (30 ml). The organic phase was dried over anhydrous sodium sulfate, filtered, and concentrated, and the residue was purified on a silica gel column (dichloromethane/methanol = 10 : 1) to give a white solid (120 mg, 0.31 mmol, yield: 24.2%). LCMS: m/z = 394$[M+H]^+$.

Step 5

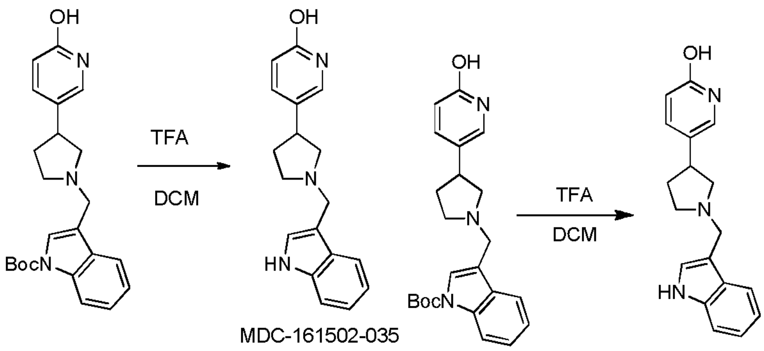


MDC-161502-035

1-(1-tert-butoxycarbonylindole-3-methyl)-3-(6-hydroxy-3-pyridinyl)pyrrolidine (120 mg, 0.31 mmol) was dissolved in dichloromethane (5 ml). Trifluoroacetic acid (5 ml) was added dropwise and the resulting mixture was stirred at room temperature for 1 hour and then concentrated under reduced pressure. The residue was purified by prep-HPLC to give a white solid (60 mg, 0.204 mmol, yield: 84.6%). $^1$HNMR (500 MHz, DMSO-$d_6$) δ 11.49-11.48 (m, 1H), 10.13-10.08 (m, 2H), 8.07-7.04 (m, 2H), 7.84-7.80 (m, 1H), 7.65-7.63 (m, 1H), 7.45 (d, $J$ = 8.0 Hz, 1H), 7.26-7.06 (m, 2H), 4.62 (s,

2H), 3.79-3.54 (m, 3H), 3.42-3.37 (m, 1H), 3.31-3.28 (m, 2H), 2.47-2.35 (m, 1H), 2.17-1.95 (m, 1H). LCMS: m/z = 294[M+H]+; Prep-HPLC method: Shim-pack GIST C18 column (250 × 50 mm, particle size 5 μM); 0.1% $CH_3COOH$ in $H_2O$/0.1% $CH_3COOH$ in ACN gradient eluting system; flow rate = 40.0 mL/min.

Example 17 (MDC-161502-036)

Step 1

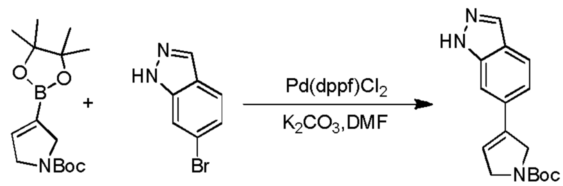


1-tert-butoxycarbonyl-2,5-dihydro-1H-pyrrole-3-boronic acid pinacol ester (610 mg, 2.54 mmol, 1.0 eq), 6-bromoindazole (500 mg, 2.54 mmol, 1.0 eq), potassium carbonate (1.05 g, 7.61 mmol, 3.0 eq) and [1,1'-bis(diphenylphosphine)ferrocene]palladium dichloride dichloromethane complex (40 mg, 0.05 mmol, 0.02 eq) were dissolved in N,N-dimethylformamide (20 ml). The mixture was subjected to replacement with nitrogen 3 times. The temperature of the mixture was raised to 90°C and the reaction was stirred overnight. Water (50 ml) was added to the reaction solution. The resulting mixture was extracted 3 times with ethyl acetate (30 ml). The organic phases were combined and washed 3 times with saturated brine (30 ml), dried over anhydrous sodium sulfate, filtered, concentrated and purified on a silica gel column (dichloromethane : methanol = 19 : 1) to give a desired compound (320 mg, 1.12 mmol, 44%). LCMS: m/z = 286[M+H]+;

Step 2

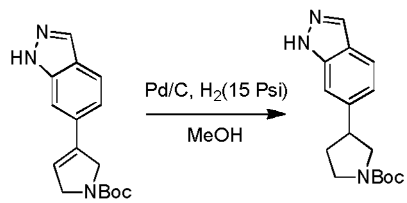


1-tert-butoxycarbonyl-3-(6-indazolyl)-2,5-dihydro-1H-pyrrolidine (320 mg, 1.12 mmol) was dissolved in methanol (30 ml). Palladium carbon (160 mg) was added, and the resulting mixture was stirred at room temperature for 16 hours under hydrogen atmosphere (15 Psi). After filtration, the filtrate was concentrated to give a white solid (260 mg, 0.91 mmol, 80.9% yield), and the crude product was directly used for the next reaction. LCMS: m/z = 288[M+H]+;

Step 3

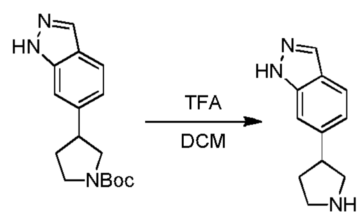


1-tert-butoxycarbonyl-3-(6-indazolyl)pyrrolidine (260 mg, 0.91 mmol) was dissolved in dichloromethane (5 ml). Trifluoroacetic acid (5 ml) was added dropwise and the resulting mixture was stirred at room temperature for 2 hours

and then concentrated under reduced pressure to give a brown oil (160 mg, 0.85 mmol, yield: 93.5%). LCMS: m/z = 188[M+H]$^+$;

Step 4

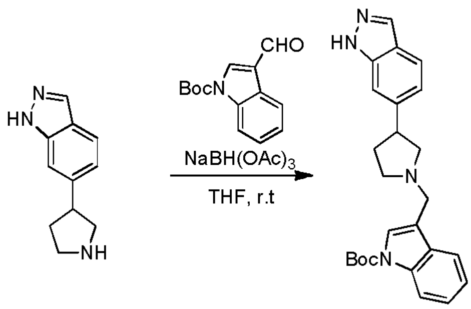


3-(6-indazolyl)pyrrolidine (160 mg, 0.85 mmol) and 1-tert-butoxycarbonyl 3-indolealdehyde (209 mg, 0.85 mmol, 1.0 eq) were dissolved in anhydrous tetrahydrofuran (10 ml). Acetic acid (60 mg, 1.0 mmol) and $NaBH(OAc)_3$ (724 mg, 3.42 mmol) were added at room temperature, and the resulting mixture was stirred at room temperature for 3 hours and concentrated under reduced pressure. The residue was dissolved with ethyl acetate (50 ml), washed with saturated sodium bicarbonate solution (30 mL). The organic phase was dried over anhydrous sodium sulfate, filtered, and concentrated, and the residue was purified on a silica gel column (dichloromethane/methanol = 9 : 1) to give a white solid (200 mg, 0.48 mmol, yield: 56.4%). LCMS: m/z = 417[M+H]$^+$;

Step 5

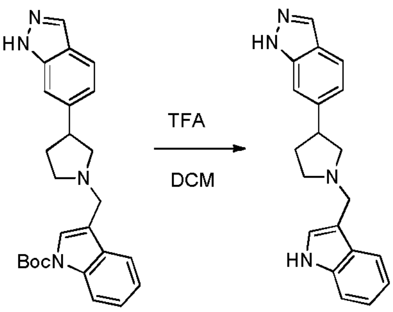


MDC-161502-036

1-(1-tert-butoxycarbonylindole-3-methyl)-3-(6-indazolyl)pyrrolidine (200 mg, 0.47 mmol) was dissolved in dichloromethane (5 ml). Trifluoroacetic acid (5 ml) was added dropwise and the resulting mixture was stirred at room temperature for 0.5 hour and then concentrated under reduced pressure. The residue was purified by prep-HPLC to give a white solid (120 mg, 0.378 mmol, yield: 80.5%). $^1$HNMR (500 MHz, DMSO-$d_6$) $\delta$ 13.05 (s, 1H), 11.45 (s, 1H), 8.03 (s, 1H), 7.82 (d, $J_1$= 7.5Hz, 1H), 7.73 (d, $J_1$= 8.5Hz, 1H), 7.63 (s, 1H), 7.45-7.44 (m, 2H), 7.18-7.09 (m, 3H), 4.62 (s, 2H), 3.78-3.55 (m, 4H), 3.34-3.33 (m, 1H), 2.50-2.48 (m, 1H), 2.18-2.03 (m, 1H). LCMS: m/z = 317[M+H]$^+$; Prep-HPLC method: Shim-pack GIST C18 column (250 × 50 mm, particle size 5 μM); 0.1% $CH_3COOH$ in $H_2O$/0.1% $CH_3COOH$ in ACN gradient eluting system; flow rate = 40.0 mL/min.

Example 18 (MDC-161502-037)

Step 1

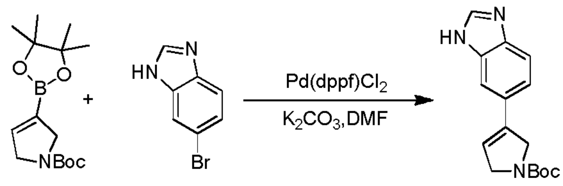


1-tert-butoxycarbonyl-2,5-dihydro-1H-pyrrole-3-boronic acid pinacol ester (610 mg, 2.54 mmol, 1.0 eq), 5-bromo-1H-benzimidazole (500 mg, 2.54 mmol, 1.0 eq), potassium carbonate (1.05 g, 7.61 mmol, 3 eq) and [1,1'-bis(diphenylphosphine)ferrocene]palladium dichloride dichloromethane complex (40 mg, 0.05 mmol. 0.02 eq) were dissolved in N,N-dimethylformamide (20 ml). The mixture was subjected to replacement with nitrogen three times. The temperature of the mixture was raised to 90°C and the reaction was stirred overnight. Water (50 ml) was added to the reaction solution. The resulting mixture was extracted 3 times with ethyl acetate (30 ml). The organic phases were combined and washed 3 times with saturated brine (30 ml), dried over anhydrous sodium sulfate, filtered, concentrated and purified on a silica gel column (dichloromethane : methanol = 19 : 1) to give a desired compound (100 mg, 0.35 mmol, 13.8%). LCMS: m/z = 286[M+H]$^+$;

Step 2

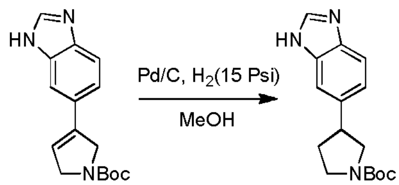


1-tert-butoxycarbonyl-3-(6-benzimidazolyl)-2,5-dihydro-1H-pyrrolidine (320 mg, 1.12 mmol) was dissolved in methanol (30 ml). Palladium carbon (160 mg) was added, and the resulting mixture was stirred at room temperature for 16 hours under hydrogen atmosphere (15 Psi). After filtration, the filtrate was concentrated to give a white solid (260 mg, 0.91 mmol, 80.9% yield), and the crude product was directly used for the next reaction. LCMS: m/z = 288[M+H]$^+$;

Step 3

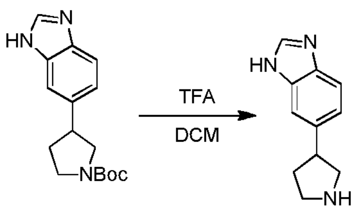


1-tert-butoxycarbonyl-3-(6-benzimidazolyl)pyrrolidine (260 mg, 0.91 mmol) was dissolved in dichloromethane (5 ml). Trifluoroacetic acid (5 ml) was added dropwise and the resulting mixture was stirred at room temperature for 2 hours and then concentrated under reduced pressure to give a brown oil (160 mg, 0.85 mmol, yield: 93.5%). LCMS: m/z = 188[M+H]$^+$;

Step 4

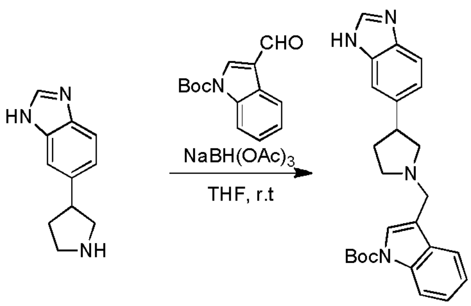


3-(6-benzimidazolyl)pyrrolidine (160 mg, 0.85 mmol) and 1-tert-butoxycarbonyl-3-indolealdehyde (209 mg, 0.85 mmol, 1.0 eq) were dissolved in anhydrous tetrahydrofuran (10 ml). Acetic acid (60 mg, 1.0 mmol) and $NaBH(OAc)_3$ (724 mg, 3.42 mmol) were added at room temperature, and the resulting mixture was stirred at room temperature for 3 hours and concentrated under reduced pressure. The residue was dissolved with ethyl acetate (50 ml), washed with saturated sodium bicarbonate solution (30 ml). The organic phase was dried over anhydrous sodium sulfate, filtered, and concentrated, and the residue was purified on a silica gel column (dichloromethane/methanol = 10 : 1) to give a white solid (200 mg, 0.48 mmol, yield: 56.4%). LCMS: m/z = 417$[M+H]^+$;

Step 5

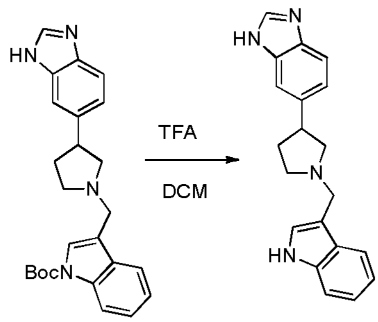


MDC-161502-037

1-(1-tert-butoxycarbonylindole-3-methyl)-3-(6-benzimidazolyl) pyrrolidine (200 mg, 0.47 mmol) was dissolved in dichloromethane (5 ml). Trifluoroacetic acid (5 ml) was added dropwise and the resulting mixture was stirred at room temperature for 1 hour and then concentrated under reduced pressure. The residue was purified by prep-HPLC to give a white solid (120 mg, 0.378 mmol, yield: 80.5%). $^1H$ NMR (500 MHz, DMSO-$d_6$) $\delta$ 12.28 (s, 1H), 10.91 (brs, 1H), 8.13 (s, 1H),7.67 (d, $J$ = 7.5 Hz, 1H), 7.49-7.48 (m, 2H), 7.34 (d, $J$ = 8.0 Hz, 1H), 7.26 (d, $J$ = 2.0 Hz, 1H), 7.12 (d, $J$ = 8.0 Hz, 1H), 7.08-7.05 (m, 1H), 7.01-6.98 (m, 1H), 3.84 (s, 2H), 3.42-3.37 (m, 1H), 3.00 (t, $J$ = 8.0 Hz, 1H), 2.78-2.76 (m, 2H), 2.57-2.54 (m, 1H), 2.32-2.25 (m, 1H), 1.85-1.79 (m, 1H).LCMS: m/z = 317$[M+H]^+$; Prep-HPLC method: Shim-pack GIST C18 column (250 × 50 mm, particle size 5 μM); 0.1% $CH_3COOH$ in $H_2O$/0.1% $CH_3COOH$ in ACN gradient eluting system; flow rate = 40.0 mL/min.

Example 19 (MDC-161502-038)

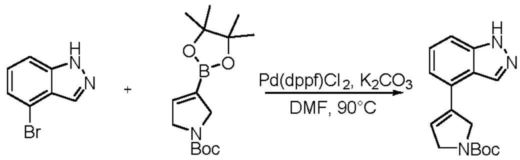


4-bromo-1H-indazole (421 g, 2.14 mmol), 1-tert-butoxycarbonyl-2,5-dihydro-1H-pyrrole-3-boronic acid pinacol ester (630 mg, 2.13 mmol), 1,1'-bis-diphenylphosphinoferrocene palladium dichloride (157 mg, 0.215 mmol) and potassium carbonate (890 mg, 6.44 mmol) were mixed in N,N-dimethylformamide (20 mL). The mixture was subjected to replacement with nitrogen to remove oxygen and then the mixture was raised to 110°C in temperature and reacted for 7 hours. The reaction system was diluted with ethyl acetate (500 mL), washed successively with water (200 mL) and saturated brine (100 mL × 5), and dried over anhydrous sodium sulfate. The desiccant was removed by filtration. The filtrate was concentrated under reduced pressure to remove solvent and then purified by flash column chromatography (petroleum ether/ethyl acetate/ethanol = 40/3/1-16/3/1-3/3/1) to give a yellowish solid (450 mg, yield 33%). LCMS: m/z = 286 $[M+H]^+$;

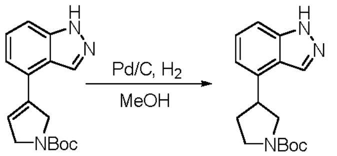


1-(1-tert-butoxycarbonyl)-3 -(4-indazolyl)-2,5 -dihydro-1H-pyrrolidine (300 mg, 1.05 mmol) was dissolved in methanol (30 mL). Palladium carbon (60 mg) was added, and the resulting mixture was stirred at room temperature for 16 hours under hydrogen (latm). Palladium carbon was removed by filtration, and the filtrate was concentrated under reduced pressure to remove solvent to give a pale yellow solid (301 mg, yield 97%). LCMS: m/z = 288 $[M+H]^+$;

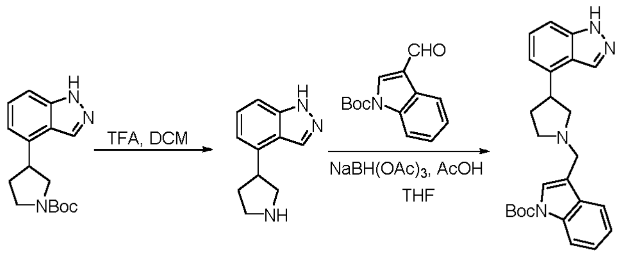


1-(1-tert-butoxycarbonyl)-3-(4-indazolyl)pyrrolidine (301 mg, 1.05 mmol) was dissolved in dry dichloromethane (10 mL). Trifluoroacetic acid (10 mL) was added and the resulting mixture was stirred for 1 hour at room temperature. The solvent was removed under reduced pressure, and the residue was dissolved in dry tetrahydrofuran (15 mL). 1-tert-butoxycarbonyl 3-indolealdehyde (260 mg, 1.06 mmol) and acetic acid (0.2 mL) were added and stirred at room temperature for 2 hours. And then sodium borohydride triacetate (1.12 mg, 5.28 mmol) was added and stirred at room temperature for 2 hours. Ethyl acetate (300 mL) was added to dilute the reaction system. The system was successively washed with saturated sodium bicarbonate solution (100 mL) and saturated brine (100 mL × 2). The organic phase was dried over anhydrous sodium sulfate. The desiccant was removed by filtration. The filtrate was concentrated under reduced pressure to remove solvent and then purified on a preparative silica gel plate (dichloromethane/methanol = 12/1) to give a pale yellow solid (180 mg, yield 41%). LCMS: m/z = 417 $[M+H]^+$.

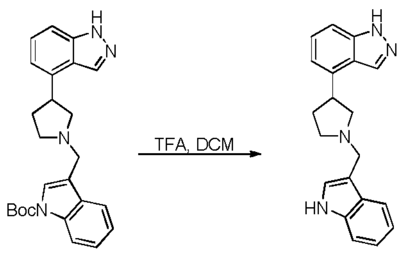


MDC-161502-038

1-(1-tert-butoxycarbonylindole-3-methyl)-3-(4-indazolyl) pyrrolidine
(150 mg, 0.360 mmol) was dissolved in dry dichloromethane (8 mL). Trifluoroacetic acid (8 mL) was added, and the resulting mixture was stirred at room temperature for 1 hour. The solvent was removed under reduced pressure. The residue was dissolved in dichloromethane. A alkalization system (a solution of ammonia in methanol) was added. The solvent was removed under reduced pressure again, and then the resulting product was purified by preparative liquid phase chromatography (acetonitrile-water-acetic acid) to give a white solid (30 mg, yield 26%). LCMS: m/z = 317 $[M+H]^+$; $^1H$ NMR (500 MHz, DMSO-$d_6$) δ 13.06 (s, 1 H), 11.09 (s, 1 H), 8.22 (s, 1 H), 7.73 (d, *J* = 7.5 Hz, 1 H), 7.40 (s, 1 H), 7.39

(s, 1 H), 7.37 (s, 1 H), 7.25 (t, *J* = 7.5 Hz, 1 H), 7.11 (t, *J* = 7.5 Hz, 1 H), 7.04 (t, *J* = 7.5 Hz, 1 H), 7.00 (d, *J* = 7.0 Hz, 1 H), 4.13 (s, 2 H), 3.80 (m, 1 H0, 3.14 (m, 1 H), 2.39 (m, 1 H), 2.03 (m, 1 H).

Example 20 (MDC-161502-039)

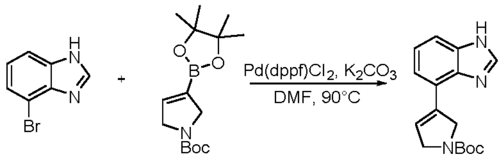


4-bromo-1H-benzimidazole (1.20 g, 6.09 mmol), 1-tert-butoxycarbonyl-2,5-dihydro-1H-pyrrole-3-boronic acid pinacol ester (1.77 g, 6.00 mmol), 1,1'-bis-diphenylphosphinoferrocene palladium dichloride (230 mg, 0.314 mmol) and potassium carbonate (2.49 g, 18.0 mmol) were mixed in N,N-dimethylformamide (30 mL). The mixture was subjected to replacement with nitrogen to remove oxygen and then the mixture was raised to 100°C in temperature and reacted for 16 hours. The reaction system was diluted with ethyl acetate (300 mL), and the organic phase was washed successively with water (100 mL) and saturated brine (100 mL × 5), and dried over anhydrous sodium sulfate. The desiccant was removed by filtration. The filtrate was concentrated under reduced pressure to remove solvent and then purified by flash column chromatography (petroleum ether/ethyl acetate/ethanol = 40/3/1-3/3/1) to give a yellowish solid (450 mg, yield 26%). LCMS: m/z = 286 $[M+H]^+$;

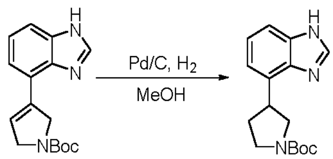


1-(1-tert-butoxycarbonyl)-3-(4-benzimidazolyl)-2,5-dihydro-lH-pyrrolidine (450 mg, 1.58 mmol) was dissolved in methanol (30 mL). Palladium carbon (90 mg) was added, and the resulting mixture was stirred at room temperature for 48 hours under hydrogen (latm). Palladium carbon was removed by filtration. The filtrate was concentrated under reduced pressure to remove solvent, and purified on a flash silica gel column (dichloromethane/methanol = 20/1) to obtain light brown solid (210 mg, yield 44%). LCMS: m/z = 288 $[M+H]^+$;

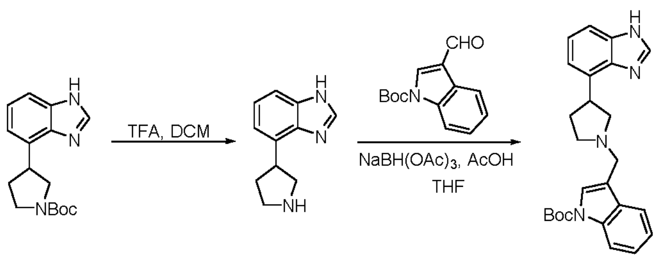


1-(1-tert-butoxycarbonyl)-3-(4-benzimidazolyl)pyrrolidine (210 mg, 0.731 mmol) was dissolved in dry dichloromethane (10 mL). Trifluoroacetic acid (8 mL) was added and the resulting mixture was stirred for 2 hours at room temperature. The solvent was removed under reduced pressure, and the residue was dissolved in dry tetrahydrofuran (10 mL). 1-tert-butoxycarbonyl 3-indolealdehyde (180 mg, 0.734 mmol) and acetic acid (0.2 mL) were added and stirred at room temperature for 3 hours. And then sodium borohydride triacetate (620 mg, 2.93 mmol) was added and stirred at room temperature for 3 hours. Ethyl acetate (300 mL) was added to dilute the reaction system. The system was successively washed with saturated sodium bicarbonate solution (100 mL× 2) and saturated brine (100 mL × 2). The organic phase was dried over anhydrous sodium sulfate. The desiccant was removed by filtration. The filtrate was concentrated under reduced pressure to remove solvent and then purified on a preparative silica gel plate (dichloromethane/methanol = 20/1) to give a pale yellow solid (47 mg, yield 15%). LCMS: m/z = 417 $[M+H]^+$.

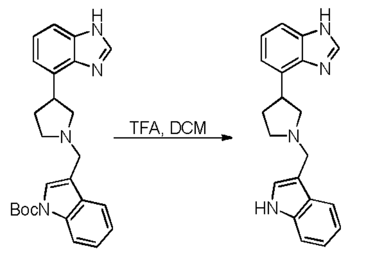


MDC-161502-039

1-(1-tert-butoxycarbonylindole-3-methyl)-3-(4-benzimidazolyl)pyrrolidine (35 mg, 0.0840 mmol) was dissolved in dry dichloromethane (10 mL). Trifluoroacetic acid (8 mL) was added, and the resulting mixture was stirred at room temperature for 2 hours. The solvent was removed under reduced pressure. The residue was dissolved in dichloromethane. A alkalization system (a solution of ammonia in methanol) was added. The solvent was removed under reduced pressure again, and then the resulting product was purified by preparative liquid phase chromatography (acetonitrile-water-acetic acid) to give a white solid (14 mg, yield 54%). LCMS: m/z = 317 $[M+H]^+$; $^1H$ NMR (500 MHz, DMSO-$d_6$) $\delta$ 11.06 (s, 1 H), 8.03 (s, 1 H), 7.77 (d, *J* = 7.5 Hz, 1 H), 7.37-7.42 (m, 3 H), 7.05-7.14 (m, 4 H), 4.16 (d, *J* = 13.0 Hz, 1 H), 4.04 (d, *J* = 13.0 Hz, 1 H), 3.81 (m, 1 H), 3.20 (m, 1 H), 2.96 (m, 1 h), 2.88 (m, 1 H), 2.39 (m, 1 H), 1.95 (m, 2 H).

Example 21 (MDC-161502-040)

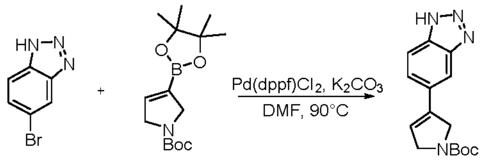


5-bromo-1H-benzotriazole (423 g, 2.14 mmol), 1-tert-butoxycarbonyl-2,5-dihydro-1H-pyrrole-3-boronic acid pinacol ester (630 mg, 2.13 mmol), 1,1'-bis-diphenylphosphinoferrocene palladium dichloride (157 mg, 0.215 mmol) and potassium carbonate (890 mg, 6.44 mmol) were mixed in N,N-dimethylformamide (20 mL). The mixture was subjected to replacement with nitrogen to remove oxygen and then the mixture was raised to 96°C in temperature and reacted for 16 hours. The reaction system was diluted with ethyl acetate (400 mL), washed successively with water (100 mL) and saturated brine (100 mL5), and dried over anhydrous sodium sulfate. The desiccant was removed by filtration. The filtrate was concentrated under reduced pressure to remove solvent and then purified by flash column chromatography (petroleum ether/ethyl acetate/ethanol = 16/3/1-3/3/1) to give a yellowish solid (100 mg, yield 11%). LCMS: m/z = 287 $[M+H]^+$;

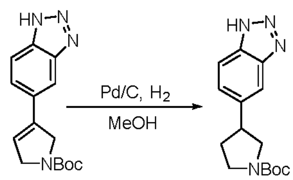


1-(1-tert-butoxycarbonyl)-3-(5-benzotriazolyl)-2,5-dihydro-lH-pyrrolidine (96 mg, 0.335 mmol) was dissolved in methanol (20 mL). Palladium carbon (20 mg) was added, and the resulting mixture was stirred at room temperature for 16 hours under hydrogen (1 atm). Palladium carbon was removed by filtration, and the filtrate was concentrated under reduced pressure to remove solvent to give a pale yellow solid (96 mg, yield 97%). LCMS: m/z = 289 $[M+H]^+$;

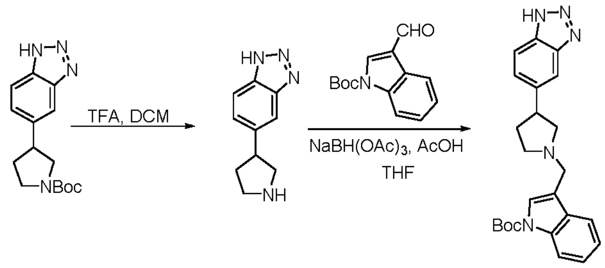


1-(1-tert-butoxycarbonyl)-3-(5-benzotriazolyl)pyrrolidine (96.0 mg, 0.335 mmol) was dissolved in dry dichloromethane (6 mL). Trifluoroacetic acid (6 mL) was added and the resulting mixture was stirred for 1 hour at room temperature. The solvent was removed under reduced pressure, and the residue was dissolved in dry tetrahydrofuran (10 mL). 1-tert-butoxycarbonyl 3-indolealdehyde (85 mg, 0.347 mmol) and acetic acid (0.1 mL) were added and stirred at room temperature for 2 hours. And then sodium borohydride triacetate (360 mg, 1.70 mmol) was added and stirred at room temperature for 2 hours. Ethyl acetate (150 mL) was added to dilute the reaction system. The system was successively washed with saturated sodium bicarbonate solution (60 mL) and saturated brine (50 mL × 2). The organic phase was dried over anhydrous sodium sulfate. The desiccant was removed by filtration. The filtrate was concentrated under reduced pressure to remove solvent and then purified on a preparative silica gel plate (dichloromethane/methanol = 12/1) to give a pale yellow solid (65 mg, yield 46%). LCMS: m/z = 418 $[M+H]^+$.

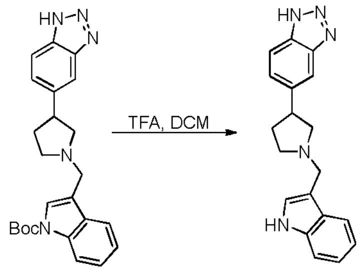


MDC-161502-040

1-(1-tert-butoxycarbonylindole-3-methyl)-3-(5-benzotriazolyl) pyrrolidine (60 mg, 0.144 mmol) was dissolved in dry dichloromethane (5 mL). Trifluoroacetic acid (5 mL) was added, and the resulting mixture was stirred at room temperature for 1 hour. The solvent was removed under reduced pressure. The residue was dissolved in dichloromethane. A alkalization system (a solution of ammonia in methanol) was added. The solvent was removed under reduced pressure again, and then the resulting product was purified by preparative liquid phase chromatography (acetonitrile-water-acetic acid) to give a white solid (18 mg, yield 39%). LCMS: m/z = 318 $[M+H]^+$; $^1H$ NMR (500 MHz, DMSO-$d_6$) δ 10.93 (s, 1 H), 7.82 (d, $J$ = 8.5 Hz, 1 H), 7.71 (s, 1 H), 7.68 (d, $J$ = 80 Hz, 1 H), 7.40 (dd, $J_1$ = 90 Hz, $J_2$ = 1.0 Hz, 1 H), 7.35 (d, $J$ = 8.0 Hz, 1 H), 7.28 (d, $J$ = 2.0 Hz, 1 H), 7.07 (td, $J_1$ = 7.5 Hz, $J_2$ = 1.0 Hz, 1 H), 7.00 (td, $J_1$ = 7.5 Hz, $J_2$ = 1.0 Hz, 1 H), 3.88 (s, 2 H), 3.50 (m, 1 H), 3.01 (m, 1 H), 2.86 (m, 1 H), 2.75 (m, 1 H), 2.63 (m, 1 H), 2.32 (m, 1 H0, 1.84 (m, 1 H).

Example 22 (MDC-161502-041)

Step 1

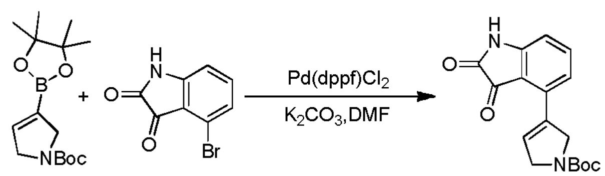


1-tert-butoxycarbonyl-2,5-dihydro-1H-pyrrole-3-boronic acid pinacol ester (452 mg, 2 mmol, 1.2 eq), 2,3-dicarbonyl-4-bromoindole (491 mg, 1.67 mmol, 1.0 eq), potassium carbonate (690 mg, 5 mmol, 3 eq) and [1,1'-bis(diphenylphosphine)ferrocene]palladium dichloride dichloromethane complex (25 mg, 0.03 mmol. 0.02 eq) were dissolved in N,N-dimethylformamide (15 ml). The mixture was subjected to replacement with nitrogen three times. The temperature of the mixture was raised to 90°C and the reaction was stirred overnight. Water (50 ml) was added to the reaction solution. The resulting mixture was extracted 3 times with ethyl acetate (30 ml). The organic phases were combined and washed 3 times with saturated brine (30 ml), dried over anhydrous sodium sulfate, filtered, concentrated and purified on a silica gel column (dichloromethane : methanol = 19 : 1) to give a desired compound (150 mg, 0.477 mmol, 28.6%). LCMS: m/z = 315[M+H]$^+$;

Step 2

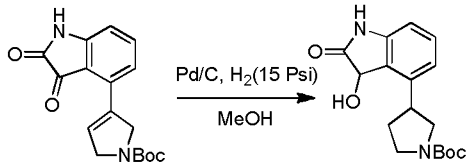


1-tert-butoxycarbonyl-3-[4-(2,3-dicarbonylindolyl)]-2,5-dihydro-1H-pyrrolidine (150 mg, 0.477 mmol) was dissolved in methanol (30 ml). Palladium carbon (80 mg) was added, and the resulting mixture was stirred at room temperature for 16 hours under hydrogen atmosphere (15 Psi). After filtration, the filtrate was concentrated to give a yellow solid (90 mg, 0.284 mmol, 59.7% yield), and the crude product was directly used for the next reaction. LCMS: m/z = 263[M-56]$^+$;

Step 3

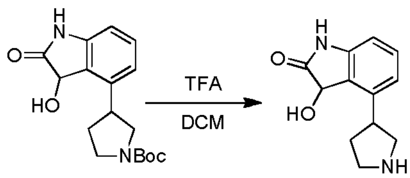


1-tert-butoxycarbonyl-3-[4-(2-carbonyl-3-hydroxyindolyl)] pyrrolidine (90 mg, 0.284 mmol) was dissolved in dichloromethane (5 ml). Trifluoroacetic acid (5 ml) was added dropwise and the resulting mixture was stirred at room temperature for 2 hours and then concentrated under reduced pressure to give a brown oil (50 mg, 0.23 mmol, yield: 80.7%). LCMS: m/z = 219[M+H]$^+$;

Step 4

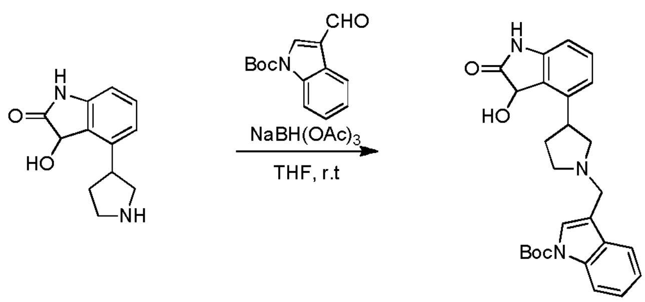


3-[4-(2-carbonyl-3-hydroxyindolyl)]pyrrolidine (50 mg, 0.23 mmol) and 1-tert-butoxycarbonyl-3-indolealdehyde (56 mg, 0.23 mmol, 1.0 eq) were dissolved in anhydrous tetrahydrofuran (10 ml). Acetic acid (20 mg, 0.3 mmol) and NaBH(OAc)3 (194 mg, 0.92 mmol) were added at room temperature, and the resulting mixture was stirred at room

temperature for 3 hours and concentrated under reduced pressure. The residue was dissolved with ethyl acetate (50 ml), washed with saturated sodium bicarbonate solution (30 ml). The organic phase was dried over anhydrous sodium sulfate, filtered, and concentrated, and the residue was purified on a silica gel column (dichloromethane/methanol = 10 : 1) to give a yellow solid (50 mg, 0.11 mmol, yield: 48.6%). LCMS: m/z = 448[M+H]$^+$;

Step 5

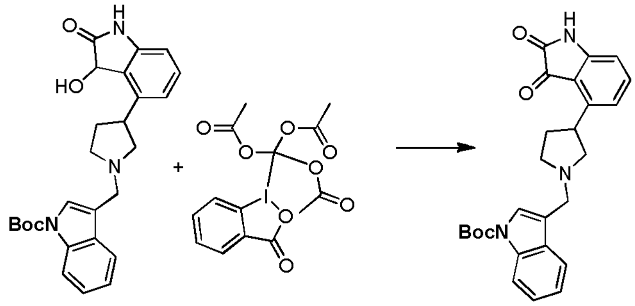

1-(1-tert-butoxycarbonylindole-3 -methyl)-3 - [4-(2-carbonyl-3 - hydroxyindolyl)]pyrrolidine (50 mg, 0.11 mmol) was dissolved in dichloromethane (8 ml). Dess-Martin reagent (73 mg, 0.167 mmol, 1.5 eq) was added, and the resulting mixture was stirred at room temperature for 2 hours. After completion of the reaction, a saturated sodium bicarbonate solution (20 ml) was added, then ethyl acetate (20 ml) was added for dissolution. The organic phase was dried over anhydrous sodium sulfate, filtered and concentrated, and the residue was purified on a TLC plate (dichloromethane/methanol = 9 : 1) to give a yellow solid (30 mg, 0.07 mmol, yield: 61.2%). LCMS: m/z = 446[M+H]$^+$;

Step 6

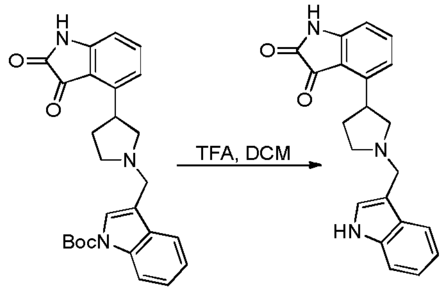


MDC-161502-041

1-(1-tert-butoxycarbonylindole-3-methyl)-3-[4-(2,3-dicarbonylindolyl)]pyrrolidine (30 mg, 0.07 mmol) was dissolved in dichloromethane (2 ml). Trifluoroacetic acid (2 ml) was added dropwise and the resulting mixture was stirred at room temperature for 0.5 hour and then concentrated under reduced pressure. The residue was purified by prep-HPLC to give a yellow solid (20 mg, 0.06 mmol, yield: 82.5%). $^1$H NMR (500 MHz, DMSO-$d_6$) $\delta$ 10.98 (brs, 1H), 10.87 (s, 1H), 7.64 (d, *J* = 80 Hz, 1H), 7.46 (t, *J* = 8.0 Hz, 1H), 7.33 (d, *J* = 8.0 Hz, 1H), 7.22 (d, *J* = 2.0 Hz, 1H), 7.10-7.04 (m, 2H), 6.99-6.96(m, 1H), 6.69-6.67 (m, 1H), 4.12-4.08 (m, 1H), 3.80-3.78 (m, 2H), 2.80-2.76 (m, 2H), 2.66-2.60 (m, 1H), 2.54-2.52 (m, 1H), 2.25-2.17 (m, 1H), 1.74-1.68 (m, 1H).LCMS: m/z = 346[M+H]$^+$; Prep-HPLC method: Shim-pack GIST C18 column (250 × 50 mm, particle size 5 $\mu$M); 0.1% $CH_3COOH$ in $H_2O$/0.1% $CH_3COOH$ in ACN gradient eluting system; flow rate = 40.0 mL/min.

Example 23 (MDC-161502-042)

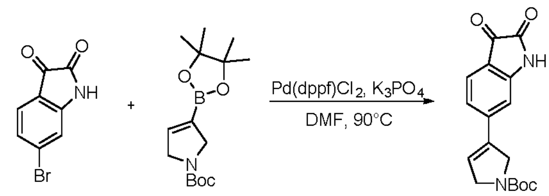


6-Bromoisatin (540 mg, 2.39 mmol), 1-tert-butoxycarbonyl-2,5-dihydro-1-pyrrole-3-boronic acid pinacol ester (700 mg, 2.37 mmol), 1,1'-bis-diphenylphosphinoferrocene palladium dichloride (180 mg, 0.246 mmol) and potassium carbonate (1.53 g, 7.21 mmol) were mixed in N,N-dimethylformamide (20 mL). The mixture was subjected to replacement with nitrogen to remove oxygen and then the mixture was raised to 93°C in temperature and reacted for 2 hours. The reaction system was diluted with ethyl acetate (500 mL), washed successively with water (200 mL) and saturated brine (100 mL × 5), and dried over anhydrous sodium sulfate. The desiccant was removed by filtration. The filtrate was concentrated under reduced pressure to remove solvent and then purified by flash column chromatography (petroleum ether/ethyl acetate = 4/1-1/1) to give a yellowish solid (106 mg, yield 14%). LCMS: m/z = 315 $[M+H]^+$;

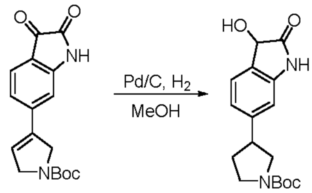


1-tert-butoxycarbonyl-3-[6-(2,3-dicarbonylindolyl)]-2,5-dihydro-1-pyrrolidine (106 mg, 0.337 mmol) was dissolved in methanol (20 mL). Palladium carbon (21 mg) was added, and the resulting mixture was stirred at room temperature for 16 hours under hydrogen (latm). Palladium carbon was removed by filtration, and the filtrate was concentrated under reduced pressure to remove solvent to give a pale yellow solid (100 mg, yield 93%). LCMS: m/z = 319 $[M+H]^+$;

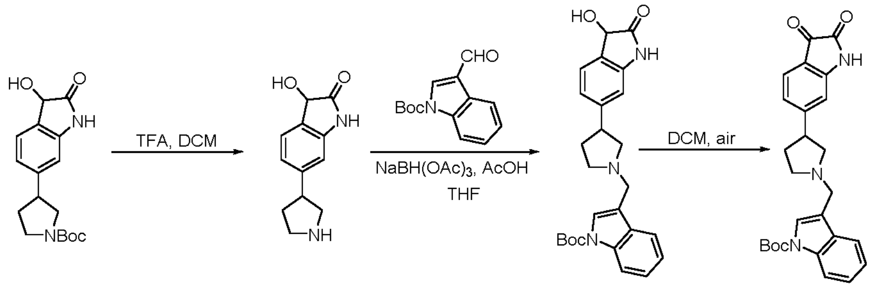


1-tert-butoxycarb onyl-3 - [6-(2-carbonyl-3 -hydroxyindolyl)]pyrrolidine (301 mg, 1.05 mmol) was dissolved in dry dichloromethane (10 mL). Trifluoroacetic acid (8 mL) was added and the resulting mixture was stirred for 1 hour at room temperature. The solvent was removed under reduced pressure, and the residue was dissolved in dry tetrahydrofuran (15 mL). 1-tert-butoxycarbonyl -3-indolealdehyde (80 mg, 0.326 mmol) and acetic acid (0.1 mL) were added and stirred at room temperature for 2 hours. And then sodium borohydride triacetate (333 mg, 1.57 mmol) was added and stirred at room temperature for 2 hours. Ethyl acetate (300 mL) was added to dilute the reaction system. The system was successively washed with saturated sodium bicarbonate solution (50 mL) and saturated brine (50 mL × 2). The organic phase was dried over anhydrous sodium sulfate. The desiccant was removed by filtration. The filtrate was concentrated under reduced pressure to remove solvent. The residue was dissolved in dry dichloromethane, stirred at room temperature for 16 hours while keeping the container open. The solvent was removed under reduced pressure and the resulting product was purified on a preparative silica gel plate (dichloromethane/methanol = 12/1) to give a pale yellow solid (50 mg, yield 36%). LCMS: m/z = 446 $[M+H]^+$.

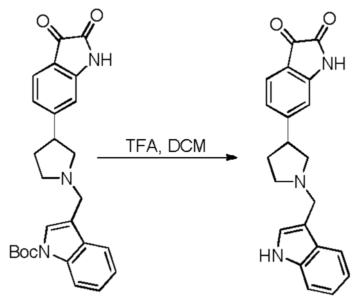


MDC-161502-042

1-(1-tert-butoxycarbonylindole-3 -methyl)-3 - [6-(2-carbonyl-3 - hydroxyindolyl)]pyrrolidine (50 mg, 0.112 mmol) was dissolved in dry dichloromethane (5 mL). Trifluoroacetic acid (3 mL) was added, and the resulting mixture was stirred at room temperature for 2 hours. The solvent was removed under reduced pressure. The residue was dissolved in dichloromethane. A alkalization system (triethylamine) was added. The solvent was removed under reduced pressure again, and then the resulting product was purified by preparative liquid phase chromatography (acetonitrile-water-acetic acid) to give a pale yellow solid (6 mg, yield 15%). LCMS: m/z = 346 [M+H]$^+$; $^1$H NMR (500 MHz, DMSO-$d_6$) δ 10.99 (bs, 1 H), 10.89 (s, 1 H), 7.64 (d, *J* = 7.5 Hz, 1 H), 7.39 (d, *J* = 8.0 Hz, 1 H), 7.33 (d, *J* = 8.0 Hz, 1 H), 7.24 (d, *J* = 2.5 Hz, 1 H), 7.06 (td, $J_1$ = 7.5 Hz, $J_2$ = 1.0 Hz, 1 H), 7.00 (td, $J_1$ = 7.5 Hz, $J_2$ = 1.0 Hz, 1 H), 6.98 (dd, $J_1$ = 7.5 Hz, $J_2$ = 1.5 Hz, 1 H), 6.85 (s, 1 H), 3.78 (m, 2 H), 3.34 (m, 1 H), 2.76-2.84 (m, 2 H), 2.59 (m, 1 h), 2.53 (m, 1 H), 2.26 (m, 1 H), 1.72 (m, 1 H).

Example 24 (MDC-161502-043)

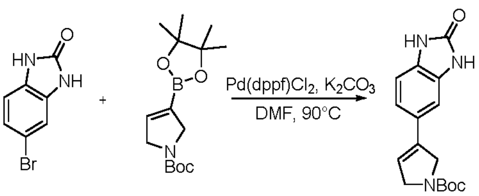


5-bromo-1,3-dihydrobenzimidazol-2-one (1.30 g, 6.10 mmol), 1-tert-butoxycarbonyl-2,5-dihydro-1H-pyrrole-3-boronic acid pinacol ester (1.77 g, 6.00 mmol), 1,1'-bis-diphenylphosphinoferrocene palladium dichloride (230 mg, 0.314 mmol) and potassium carbonate (2.49 g, 18.0 mmol) were mixed in N,N-dimethylformamide (30 mL). The mixture was subjected to replacement with nitrogen to remove oxygen and then the mixture was raised to 100°C in temperature and reacted for 16 hours. The reaction system was diluted with ethyl acetate (300 mL), and the organic phase was washed successively with water (100 mL × 2) and saturated brine (100 mL × 5), and dried over anhydrous sodium sulfate. The desiccant was removed by filtration. The filtrate was concentrated under reduced pressure to remove solvent and then purified by column chromatography (petroleum ether/ethyl acetate/ethanol = 12/3/1, 3/3/1) to give a yellowish solid (300 mg, yield 17%). LCMS: m/z = 302 [M+H]$^+$.

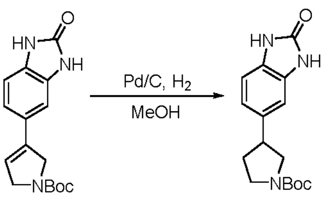


1-tert-butoxycarbonyl-3-[5-(1,3-dihydrobenzimidazol-2-one)]-2,5-dihydro-1H-pyrrolidine (300 mg, 0.996 mmol) was dissolved in methanol (30 mL). Palladium carbon (60 mg) was added, and the resulting mixture was stirred at room temperature for 16 hours under hydrogen (latm). Palladium carbon was removed by filtration, and the filtrate was concentrated under reduced pressure to give a pale yellow solid (300 mg, yield > 99%). LCMS: m/z = 304 [M+H]$^+$.

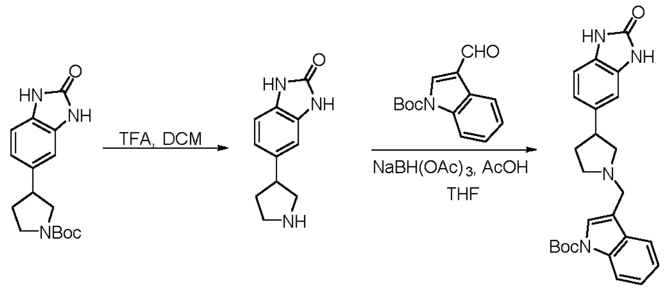


1-tert-butoxycarbonyl-3-[5-(1,3-dihydrobenzimidazol-2-one)]pyrrolidine (120 mg, 0.396 mmol) was dissolved in dry dichloromethane (10 mL). Trifluoroacetic acid (8 mL) was added and the resulting mixture was stirred for 2 hours at room temperature. The solvent was removed under reduced pressure, and the residue was dissolved in dry tetrahydrofuran (10 mL). 1-tert-butoxycarbonyl-3-indolealdehyde (100 mg, 0.408 mmol) and acetic acid (0.2 mL) were added and stirred at room temperature for 3 hours. And then sodium borohydride triacetate (420 mg, 1.98 mmol) was added and stirred at room temperature for 16 hours. Ethyl acetate (300 mL) was added to dilute the reaction system. The system was successively washed with saturated sodium bicarbonate solution (50 mL × 3), water (100 mL) and saturated brine (100 mL × 2). The organic phase was dried over anhydrous sodium sulfate. The desiccant was removed by filtration. The filtrate was concentrated under reduced pressure to remove solvent and then purified on a preparative silica gel plate (dichloromethane/methanol = 20/1) to give a pale yellow solid (60 mg, yield 35%). LCMS: m/z = 433 $[M+H]^+$.

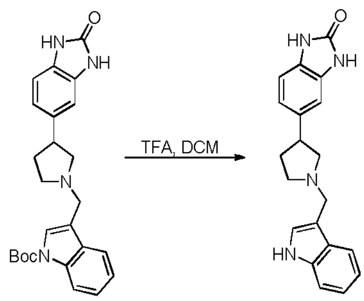


MDC-161502-043

1-(1-tert-butoxycarbonylindole-3-methyl)-3-[5-(1,3-dihydrobenzimidazol-2-one)]pyrrolidine (60 mg, 0.139 mmol) was dissolved in dry dichloromethane (5 mL). Trifluoroacetic acid (5 mL) was added, and the resulting mixture was stirred at room temperature for 2 hours. The solvent was removed under reduced pressure. The residue was dissolved in dichloromethane. A alkalization system (triethylamine) was added. The solvent was removed under reduced pressure again, and then the resulting product was purified by preparative liquid phase chromatography (acetonitrile-water-acetic acid) to give a white solid (20 mg, yield 43%). LCMS: m/z = 333 $[M+H]^+$; $^1H$ NMR (500 MHz, DMSO-$d_6$) $\delta$ 10.93 (s, 1 H), 10.49 (s, 1 H), 10.46 (s, 1 H), 7.66 (d, $J$ = 8.0 Hz, 1 H), 7.35 (d, $J$ = 8.0 Hz, 1 H), 7.27 (d, $J$ = 2.0 Hz, 1 H), 7.08 (td, $J_1$ = 7.5 Hz, $J_2$ = 1.0 Hz, 1 H), 7.00 (td, $J_1$ = 7.5 Hz, $J_2$ = 1.0 Hz, 1 H), 6.85 (s, 1 H), 6.82 (dd, $J_1$ = 8.0 Hz, $J_2$ = 1.0 Hz, 1 H), 6.79 (d, $J$ = 8.0 Hz, 1 H), 3.86 (s, 2 H), 3.28 (m, 1 H), 2.96 (m, 1 H), 2.76 (m, 2 H), 2.23 (m, 1 H), 1.74 (m, 1 H).

## Bioactivity test

I. D2 receptor affinity assay: The affinity of the compound to D2 receptor was determined by radioligand competition assay, and the cloned D2 receptor was expressed in HEK293 cell line.

II. D2 receptor function assay: The ability of the compound to antagonize the D2 receptor was determined using a calcium mobilization assay (FLIPR).

III. DAT receptor function assay: The ability of the compound to inhibit DAT was determined by neurotransmitter transporter absorption assay.

I. D2 receptor affinity assay

1. Experimental materials

1.1. Reagents and compounds

3H-7-OH-DPAT (PerkinElmer)

Tris base (Sigma, Cat: T1503-1KG), formulated as a 1M solution, pH adjusted to 7.4

PEI (Sigma-P3143)

Microscint 20 cocktail scintillation fluid (PerkinElmer-6013329)

7-OH-DPAT (Sigma)

Droperidol (Sigma)

1.2. Instruments and consumables

Unifilter-96 GF/C micropore plate (Perkin Elmer-6005174)

96-well polypropylene plate (Agilent-5042-1385)

TopSeal-A sealing film (Perkin Elmer-6005250)

MicroBeta2 microplate detector platform (PerkinElmer)

Cell counter (Perkin Elmer)

2. Experimental steps:

2.1. Preparation of buffers:

Experimental buffer: 50 mM Tris-HCl, pH 7.4, 5 mM $MgCl_2$

Washing buffer: 50 mM Tris-HCl pH 7.4, stored at 4°C ready for use.

2.2. Compound formulation:
The compound and positive control 7-OH-DPAT were serially diluted with DMSO, with the initial concentration of 2 mM and 4 times serial dilution, a total of 10 concentrations. 1 μL was transferred to the designated position of the 96-well plate.
1 μL droperidol (final concentration 10 μM) was added to the positive control well. 1 μL DMSO was added to the negative control well.

2.3. Cell membrane formulation:
Receptor cell membrane was diluted to 10 μg/well with corresponding experimental buffer, with 100 μL cell membrane/well.

2.4. Isotope formulation:
3H-7-OH-DPAT was diluted to 2 nM with corresponding experimental buffer, and the final concentration was 1 nM.

2.5. Experimental steps
After 100 μL of cell membrane and isotope were added, respectively, the reaction plate of the D2 receptor was

incubated at room temperature for 1 hour. The GF/C filter plate was soaked in 0.3% PEI solution for more than half an hour. After the reaction was completed, the cell membrane was collected on the GF/C filter plate using the cell counter, washed 4 times with cold washing buffer, and then dried in an oven at 50°C for 1 hour. The dried GF/C filter plate was sealed at the bottom, 50 μL of scintillation fluid was added to each well and the well was sealed. Reads were obtained by MicroBeta and the data was analyzed using GraphPad Prism 5.0.

3. Calculation formula for data in the project:

% inhibition rate = 100 × [1-(reading value for sample well -reading value for negative control well)/(reading value for positive control well-reading value for negative control well)]

$$Ki = IC_{50}/(1 + \text{isotope concentration}/Kd)$$

II. D2 receptor function assay

1. Experimental materials

1.1. Cell line and media

Cell line: HEK293

DMEM medium (Invitrogen)

Fetal bovine serum (FBS, Invitrogen)

L-Glutamine (Invitrogen) G418 (Invitrogen)

Blasticidin (Invitrogen)

1.2. Reagent
Fluo-4 Direct Kit (Invitrogen)

1.3. Instruments

384-well polylysine coated cell plate (Greiner)

384-well compound plate (Greiner)

FLIPR instrument (Molecular Device)

POD810 instrument (Labcyte)

Cell counter (Beckman, Vi-cell XR)

Micro reagent dispenser (Thermo)

1.4. Reference compound
Amisulpride (Sigma)

2. Experimental method

2.1. Preparation of reagent

250 mM Probenecid solution was formulated: according to the kit instructions, 1 mL of FLIPR buffer salt solution was added to 77 mg probenecid just before needed.

2X (8 μM) Fluo-4 Direct TM loading buffer was formulated: the number of tubes in Fluo-4 Direct TM needed for the experiment was melted in advance. Just before needed, 10 ml of FLIPR buffer salt solution and 0.2 mL of 250 mM Probenecid solution were added to each tube and vortexed in dark for more than 5 minutes.

2.2. Preparation of cells

a) One tube of cells was taken from the liquid nitrogen tank and quickly thawed in 37°C water bath.

b) The cell suspension was added to a centrifuge tube to which the preheated 20 ml medium was added in advance.

c) The centrifuge tube was centrifuged at 1000 rpm for 5 minutes at room temperature.

d) The supernatant was slowly decanted to avoid affecting the precipitated cells.

e) The cells were resuspended with 10-30 ml medium by pipetting the cells gently with a pipette.

f) The viability and number of cells were calculated with the cell counter.

g) The cells were diluted to $1 \times 10^5$ cells/ml with medium, and inoculated in the 384-well polylysine coated cell plate at 20 μL/well.

h) The plate was incubated in an incubator at 5% $CO_2$, 37°C for 16 to 20 hours.

2.3. FLIPR assay

a) Compound dose curve plate: The compound to be tested had an initial concentration of 2 mM and was diluted in 100% DMSO using Bravo at 4X gradient to form 10 concentration points, and then 900 nL thereof was transferred to the compound plate using Echo.

b) 30 μL of FLIPR buffer salt solution was added to the corresponding compound plate, ready for use.

c) The cell plate prepared the previous day was taken out of the incubator, 20 μL of 2X Fluo-4 Direct TM buffer was added to each well, incubated in a incubator at 37°C under 5% CO2 for 50 minutes, and left at room temperature for 10 minutes.

d) The compound plate was taken out, and run on FLIPR after tip placement in position.

e) The FLIPR instrument software was run. 10 μL of experimental buffer salt solution was added into the cell plate according to the set program, and the fluorescence signal was read. 10 μL of agonist reference compound was further added at a given concentration to the cell plate and the fluorescence signal was read. After reading, the data was derived by "Max-Min" and "Read 90 to Maximum Allowed" methods in the software and EC80 of the corresponding cell line was calculated, and the reference compound with a concentration of 6 X EC80 was prepared.

f) The FLIPR instrument software was run. 10 μL of the compound to be tested and the reference compound at a given concentration were added into the cell plate according to the set program, and the fluorescence signal was read. 10 μL of reference compound agonist was further added at a concentration of 6 X EC80 to the cell plate and the fluorescence signal was read.

g) Data Analysis

Calculation formula for data in the project:

Inhibitor% activity = 100-(signal value per well-average value for high dose control group of inhibitor)/(average value for DMSO control group-average value for high dose control group of inhibitor) * 100%.

III. DAT transporter assay

1. Experimental materials

1.1. Reagents and consumables

Neurotransmitter transporter reuptake assay kit (Molecular devices)

HEPES, Invitrogen (Cat # 15630130)

HBSS, Invitrogen (Cat # 14025126)

BSA, Sigma (Cat # 7030)

1.2. Detection buffer
HBSS IX, HEPES 20 mM.

1.3. Detection buffer containing BSA
HBSS1X, HEPES 20 mM, BSA0.1%.

1.4. Dye solution
Lyophilized fluorescent dye 1 vial, Assay buffer 10 mL.

1.5. Reference compound
BTCP - hydrochloride (Sigma)

1.6. Instruments

384-well polylysine coated cell plate (Greiner)

Vi-cell XR cell viability analyzer (Beckman Coulter)

Incubator (Thermo)

Flexstation multi-mode microplate reader (Molecular devices)

2. Experimental steps:

2.1. Preparation of cells

i) The medium was preheated in 37°C water bath in advance for more than 30 minutes for later use.

j) The preheated medium was taken out, disinfected with 75% alcohol, and put into a biosafety cabinet.

k) The cultured cells were taken out from the cabinet, and the medium was removed by a vacuum pump. 1X DPBS was added, incubated for a moment, and then removed by a vacuum pump. Appropriate amount of 0.05% trypsin-EDTA was added for cell digestion. The digestion was terminated by 10% FBS medium, and the cells were pipetted and dispersed.

l) The dispersed cells were transferred into a 50 mL centrifuge tube with a pipette. The tube was centrifuged at 800 rpm for 5 minutes, and then the cells were resuspended with medium, pipetted and dispersed, and counted.

m) The cells were diluted to 1 × 106 cells/mL with medium, and inoculated in the 384-well polylysine coated cell plate at 20 μL/well.

2.2. DAT transporter assay

a) The reference compound was diluted in a ratio of 1 : 3 to 10 concentration gradients with the detection buffer containing 0.1% BSA, with the highest concentration being 2 μM. The compound to be tested was diluted to 20 uM with the detection buffer containing 0.1% BSA.

b) A centrifuge (750 rpm, 15s) was used to spin off the culture solution in the cell culture plate, and the compound solution in step 1 was transferred to the cell culture plate (25 uL/well).

HC (High control): 25 μL of 0.1% BSA detection buffer containing 0.2% DMSO.

LC (Low control): 25 μL of solution containing 2 μM positive antagonist.

c) After centrifugation at 300 rpm for 15 seconds, the plate was incubated at 37°C for 30 minutes. After the incubation with the compound was completed, a dye solution (25 μL/well) was added and incubated at 37°C for 30 minutes. Reading was performed in Flexstation after the incubation.

The parameters are shown in Table 4:

Table 4

| | |
|---|---|
| Temperature setting | 37°C |
| Excitation wavelength (nM) | 440 |
| Emission wavelength (nM) | 520 |
| Emission cut-off (nM) | 515 |
| PMT sensitivity | medium |
| Reads/well | 3 |

d) Data Analysis

Calculation formula for data in the project:

Inhibitor% activity = 100-(signal value per well-average value for LC well)/(average value for H well-average value for LC well) * 100%.

The compounds of the present invention are dual modulators of D2 receptor and DAT, as shown in Table 5 below. Examples above in which various assays were performed reveal about 10 nM to 1 μM of Ki (D2) and 100 nM to 1 μM of Ki (DAT).

Table 5

| No. | Structure | D2 affinity assay $IC_{50}$ (nM) | Calcium mobilization assay $IC_{50}$ (nM) | Neurotransmit ter transporter (DAT) absorption assay $IC_{50}$ (nM) |
|---|---|---|---|---|
| MDC-161502-002 | 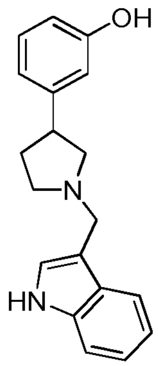 | 14 | 2.0 | 181 |
| MDC-161502-010 | - | 2.35 | 144 | 218.1 |
| MDC-161502-011 | - | 3.46 | 2.002 | 14.72 |
| MDC-161502-006 | 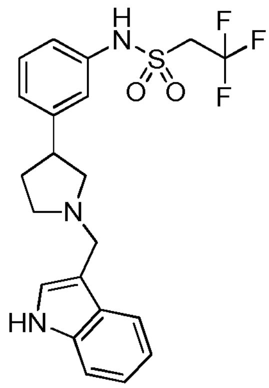 | 639 | 171 | 762 |
| MDC-161502-008 | 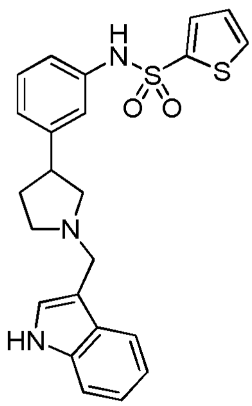 | 120 | 5.4 | 936 |

(continued)

| No. | Structure | D2 affinity assay $IC_{50}$ (nM) | Calcium mobilization assay $IC_{50}$ (nM) | Neurotransmit ter transporter (DAT) absorption assay $IC_{50}$ (nM) |
|---|---|---|---|---|
| MDC-161502-031 | 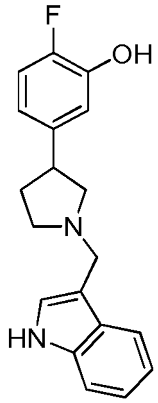 | 22.38 | 14.48 | 28.31 |
| MDC-161502-033 | 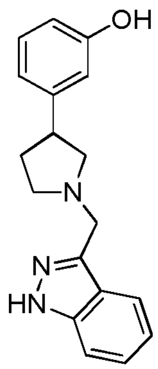 | 6.83 | 2.283 | 110.7 |
| MDC-161502-034 | 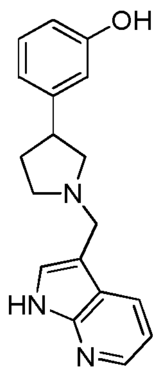 | 2.02 | 1.99 | 684.7 |

(continued)

| No. | Structure | D2 affinity assay $IC_{50}$ (nM) | Calcium mobilization assay $IC_{50}$ (nM) | Neurotransmit ter transporter (DAT) absorption assay $IC_{50}$ (nM) |
|---|---|---|---|---|
| MDC-161502-036 | 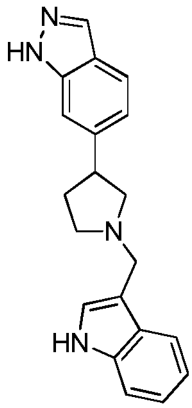 | 298.38 | 470.8 | 841.4 |
| MDC-161502-037 | 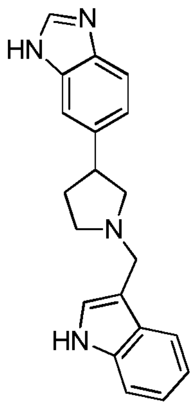 | 461.12 | 269.1 | 190.5 |
| MDC-161502-038 | 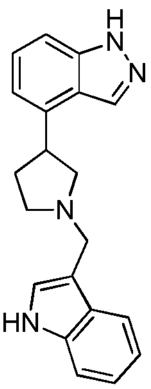 | 10.16 | 6.216 | 87.46 |

(continued)

| No. | Structure | D2 affinity assay $IC_{50}$ (nM) | Calcium mobilization assay $IC_{50}$ (nM) | Neurotransmit ter transporter (DAT) absorption assay $IC_{50}$ (nM) |
|---|---|---|---|---|
| MDC-161502-040 | 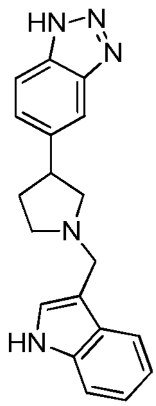 | 545.74 | 505.2 | 52.82 |
| Olanzapine | 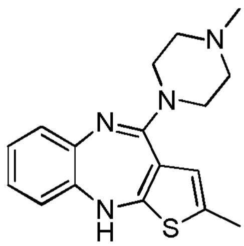 | 7.1 | 296 | > 10000 |
| Aripiprazole | 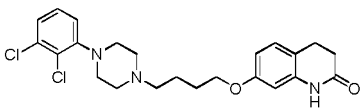 | 4.4 | 16.78 | > 10000 |

IV. Assay of therapeutic activity of the compound for "schizophreniform disorder" activity in mice

1. The effect of the compound on phencyclidine (PCP) induced high locomotor activity (LMA) in mice was detected.

The test compound MDC was milky white powder and stored in a shade and cool place (freshly formulated with 1% DMSO before use). Olanzapine, purchased from Adamas, was a yellowish powder (freshly formulated with 1% DMSO before use). Phencyclidine (PCP), purchased from Sigma Aldrich, was a grayish white powder (freshly formulated with normal saline before use). 1% DMSO was formulated by diluting pure DMSO (purity > 99.5, purchased from Sigma-Aldrich) with normal saline.

Male ICR mice having weight 22 ± 2 g were kept at 8 mice/cage with 12/12 hours light/dark cycle, temperature 23°C ± 1 °C, humidity 50% to 60%, free to eat and drink water.

The test compound and olanzapine were formulated with DMSO, and the doses of the test compound were 5 mg, 10 mg and 20 mg. The dose of olanzapine was 5 mg/kg. The administration volume was 0.1 ml/10 g body weight; PCP was formulated with normal saline at a dose of 5 mg/kg.

The test compound MDC and olanzapine were administered by gavage and PCP was administered by subcutaneous injection.

After one week of acclimation, mice were randomly divided into 6 groups according to their body weight: negative control group, model group, positive control group, and low dose group, medium dose group and high dose group for the test compound. 8-10 animals/group.

Group 1: Negative control group: Normal saline (s.c.) + 1% DMSO (10.0 ml/kg, p.o.);

Group 2: Model group: PCP (5.0 mg/kg, s.c.) + 1% DMSO (10.0 ml/kg, p.o.);

Group 3: Positive control group: PCP (5.0 mg/kg, s.c.) + olanzapine (5 mg/kg, p.o.);

Group 4: MDC low dose group: PCP (5.0 mg/kg, s.c.) + MDC (5 mg/kg, p.o.);

Group 5: MDC medium dose group: PCP (5.0 mg/kg, s.c.) + MDC (10 mg/kg, p.o.);

Group 6: MDC high dose group: PCP (5.0 mg/kg, s.c.) + MDC (20 mg/kg, p.o.).

Mice were injected subcutaneously with PCP (5.0 mg/kg, s.c.) or normal saline, and then administrated with DMSO, and low, medium or high doses of MDC, or olanzapine by oral gavage half an hour later.

After oral administration, the animals were immediately placed in a locomotor activity box and recorded by Shanghai Jiliang (上海吉量) animal video analysis system for 1 hour.

After the experiment, the activity of the animals within 1 hour was analyzed using the trajectory analysis software of Shanghai Jiliang animal video analysis system to obtain the total distance resulting from the activity.

Test results

Table 6

| Grouping (n = 8-10) | Distance resulting from the activity | |
|---|---|---|
| | First 30 min | Last 30 min |
| Negative control group | 145612 ± 16487 | 96956 ± 10529 |
| Model group | 313147 ± 17696** | 269080 ± 21931 ** |
| Positive control group | 208491 ± 24344# | 80518 ± 12716## |
| MDC-161502-002 low dose group | 241185 ± 23865# | 265712 ± 34806 |
| MDC-161502-002 medium dose group | 211543 ± 30690# | 206789 ± 26215# |
| MDC-161502-002 high dose group | 158231 ± 21482## | 110918 ± 14982## |
| MDC-161502-006 low dose group | 327586 ± 25347 | 302499 ± 26738 |
| MDC-161502-006 medium dose group | 286745 ± 28976 | 267834 ± 26876 |
| MDC-161502-006 high dose group | 248576 ± 22456# | 230867 ± 25347# |
| MDC-161502-008 low dose group | 299745 ± 21356 | 2808492 ± 23864 |
| MDC-161502-008 medium dose group | 246900 ± 21879# | 230876 ± 24875# |
| MDC-161502-008 high dose group | 220968 ± 23069## | 189765 ± 20771## |
| [** *P* < 0.01 (ANNOVA followed by Dunnett's t test, compared with the value of the negative control group during the corresponding time period);<br>## *P* < 0.01 (ANNOVA followed by Dunnett's t test, compared with the value of the model group during the corresponding time period);<br># *P* < 0.05 (ANNOVA followed by Dunnett's t test, compared with the value of the model group during the corresponding time period); | | |

Subcutaneous injection of PCP (5.0 mg/kg, s.c.) significantly increased the locomotor activity of mice (P < 0.01). Compounds MDC-161502-002, MDC-161502-006, and MDC-161502-008 (5, 10, 20 mg/kg, p.o.) all inhibited PCP-induced high locomotor activity in mice (P < 0.05-0.01), which was equivalent to the effect of the positive drug olanzapine on PCP-induced high locomotor activity in mice. Wherein, MDC-161502-002 had significant advantages over olanzapine in inhibiting PCP-induced high locomotor activity.

Conclusion: Oral administration of the compound MDC can effectively inhibit PCP-induced high locomotor activity in mice, indicating that such compound may have better inhibitory effect on positive symptoms of schizophrenia. The potency thereof was equivalent to or slightly stronger than that of positive drug olanzapine.

V. Effect of representative examples on amphetamine-induced high locomotor activity (LMA) in mice

The test compound MDC was a milky white powder and stored in a shade and cool place (formulated with 1% DMSO before use). Olanzapine, purchased from Adamas, was a yellow powder (formulated with 1% DMSO before use). Amphetamine, purchased from Sigma-Aldrich, was a white powder (formulated with normal saline before use). 1% DMSO was formulated by diluting pure DMSO (purity > 99.5, purchased from Sigma) with normal saline.

Male ICR mice having weight 22 ± 2 g were kept at 8 mice/cage with 12/12 hours light/dark cycle, temperature 23°C ± 1°C, humidity 50% to 60%, free to eat and drink water.

The test compound and olanzapine were freshly formulated with DMSO, and the test compound MDC was administrated at doses of 5 mg, 10 mg and 20 mg and administrated by gavage. Olanzapine was administrated a dose of 5 mg/kg and administrated by gavage. The administration volume was 0.1 ml/10 g body weight; Amphetamine was freshly formulated with normal saline at a dose of 1.0 mg/kg and administered subcutaneously.

After one week of acclimation, mice were randomly divided into 6 groups according to their body weight: negative control group, model group, positive control group, and low dose group, medium dose group and high dose group for the test compound. 8-10 animals/group.

Group 1: Negative control group: Normal saline (s.c.) + 1% DMSO (10.0 ml/kg, p.o.);

Group 2: Model group: Amphetamine (1.0 mg/kg, s.c.) + 1% DMSO (10.0 ml/kg, p.o.);

Group 3: Positive control group: Amphetamine (1.0 mg/kg, s.c.) + olanzapine (5 mg/kg, p.o.);

Group 4: MDC low dose group: Amphetamine (1.0 mg/kg, s.c.) + MDC (5 mg/kg, p.o.);

Group 5: MDC medium dose group: Amphetamine (1.0 mg/kg, s.c.) + MDC (10 mg/kg, p.o.);

Group 6: MDC high dose group: Amphetamine (1.0 mg/kg, s.c.) + MDC (20 mg/kg, p.o.).

Mice were injected subcutaneously with Amphetamine (1.0 mg/kg, s.c.) or normal saline, and then administrated with DMSO, and low, medium or high doses of MDC, or positive drug olanzapine by gavage half an hour later.

After administration, the animals were immediately placed in a locomotor activity box and recorded by Shanghai Jiliang animal video analysis system for 1 hour.

After the experiment, the activity of the animals within 1 hour was analyzed using the trajectory analysis software of Shanghai Jiliang animal video analysis system to obtain the total distance resulting from the activity.

The test results are shown in Table 7.

Table 7

| Grouping (n = 8-10) | Distance resulting from the activity | |
|---|---|---|
| | First 30 min | Last 30 min |
| Negative control group | 128651 ± 15767 | 118648 ± 13202 |
| Model group | 384624 ± 15879** | 307965 ± 20864** |
| Positive control group | 227653 ± 23869## | 158673 ± 15636## |
| MDC-161502-002 low dose group | 284824 ± 25868# | 257329 ± 25036# |
| MDC-161502-002 medium dose group | 250678 ± 28765## | 231296 ± 20254## |
| MDC-161502-002 high dose group | 180127 ± 22036## | 148036 ± 13897## |
| MDC-161502-006 low dose group | 340368 ± 24769 | 318973 ± 24896 |
| MDC-161502-006 medium dose group | 298763 ± 20845# | 257632 ± 23035# |
| MDC-161502-006 high dose group | 270378 ± 25867# | 231584 ± 18762## |
| MDC-161502-008 low dose group | 287643 ± 25867# | 264309 ± 22736# |
| MDC-161502-008 medium dose group | 253945 ± 28973# | 220678 ± 20419## |

(continued)

| Grouping (n = 8-10) | Distance resulting from the activity | |
|---|---|---|
| | First 30 min | Last 30 min |
| MDC-161502-008 high dose group | 209673 ± 20694## | 219876 ± 21540## |
| ** $P$ < 0.01 (ANNOVA followed by Dunnett's t test, compared with the value of the negative control group during the corresponding time period)<br>## $P$ < 0.01 (ANNOVA followed by Dunnett's t test, compared with the value of the model group during the corresponding time period)<br># $P$< 0.05 (ANNOVA followed by Dunnett's t test, compared with the value of the model group during the corresponding time period) | | |

Amphetamine (1.0 mg/kg, s.c.) significantly increased the locomotor activity of mice (P < 0.01). Compounds MDC-161502-002, MDC-161502-006 and MDC-161502-008 (5, 10, 20 mg/kg, p.o.) significantly inhibited the Amphetamine (1.0 mg/kg, s.c.)-induced high locomotor activity in mice (P < 0.05-0.01), which inhibition was equivalent to olanzapine (5.0 mg/kg, p.o.), wherein, MDC-161502-002 was better than olanzapine in inhibiting Amphetamine-induced high locomotor activity.

The test results showed that oral administration of the compound of the present invention can effectively inhibit Amphetamine-induced high locomotor activity in mice, indicating that such compound may have better inhibitory effect on positive symptoms of schizophrenia. The potency thereof was equivalent to or slightly stronger than that of positive drug olanzapine.

Although the specific embodiments of the present invention have been described above, it will be understood by those skilled in the art that these are merely illustrative, and that various alterations or modifications can be made to these embodiments without departing from the principle and essence of the present invention. Therefore, the scope of protection of the present invention is defined by the appended claims.

## Claims

1. A tetrahydropyrrole compound represented by general formula (I), an enantiomer, a diastereomer, an isotope compound, a pharmaceutically acceptable prodrug, a pharmaceutically acceptable ester or a pharmaceutically acceptable salt thereof:

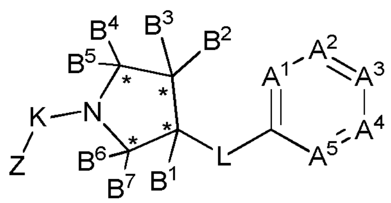

(I)

wherein:

$A^1$ is C-$R^1$ or N;
$A^2$ is C-$R^{1a}$ or N;
$A^3$ is C-$R^{1b}$ or N;
$A^4$ is C-$R^{1c}$ or N;
$A^5$ is C-$R^{1d}$ or N;
no more than 3 nitrogen atoms are present in $A^1$, $A^2$, $A^3$, $A^4$ and $A^5$;
$R^1$, $R^{1a}$, $R^{1b}$, $R^{1c}$ and $R^{1d}$ are each independently a hydrogen atom, halogen, cyano, nitro, substituted or unsubstituted $C_1$-$C_4$alkyl, substituted or unsubstituted $C_1$-$C_4$alkoxy, substituted or unsubstituted $C_3$-$C_8$cycloalkyl, substituted or unsubstituted $C_6$-$C_{14}$aryl, substituted or unsubstituted $C_2$-$C_{10}$heteroaryl,

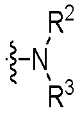, 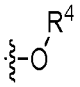, 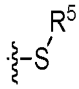, 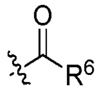,

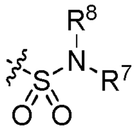, 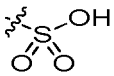, 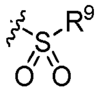 or 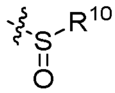,

or, adjacent $R^1$ and $R^{1a}$; or $R^{1a}$ and $R^{1b}$; or $R^{1b}$ and $R^{1c}$; or $R^{1c}$ and $R^{1d}$ and the atoms attached thereto together form substituted or unsubstituted $C_3$-$C_8$cycloalkyl, substituted or unsubstituted $C_2$-$C_8$heterocyclyl, substituted or unsubstituted $C_6$-$C_{14}$aryl, or substituted or unsubstituted $C_2$-$C_{10}$heteroaryl; the heteroatoms in the $C_2$-$C_8$heterocyclyl are selected from N, O and S, the number of the heteroatoms is 1-3, and when the number of the heteroatoms is 2 or 3, then the heteroatoms can be the same or different; the $C_2$-$C_8$heterocyclyl is a saturated $C_2$-$C_8$heterocyclyl or an unsaturated $C_2$-$C_8$heterocyclyl, the ring atoms are selected from two, three or four of C, N, O and S, and when the ring atom is C or S, then the C or S can be formed with oxygen into

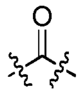, 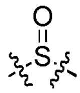 or 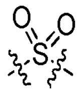;

$R^2$ and $R^3$ are each independently a hydrogen atom, hydroxyl, amino, substituted or unsubstituted $C_1$-$C_4$alkyl, substituted or unsubstituted $C_1$-$C_4$alkoxy, substituted or unsubstituted $C_3$-$C_8$cycloalkyl, substituted or unsubstituted $C_6$-$C_{14}$aryl, substituted or unsubstituted $C_2$-$C_{10}$heteroaryl,

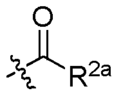, 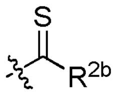 or 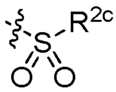;

$R^{2a}$ and $R^{2b}$ are each independently a hydrogen atom, substituted or unsubstituted $C_1$-$C_4$alkyl, substituted or unsubstituted $C_1$-$C_4$alkoxy, substituted or unsubstituted $C_3$-Cscycloalkyl, substituted or unsubstituted $C_6$-$C_{14}$aryl, substituted or unsubstituted $C_2$-$C_{10}$heteroaryl, hydroxyl or

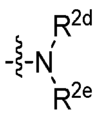;

$R^{2c}$ is substituted or unsubstituted $C_1$-$C_4$alkyl, substituted or unsubstituted $C_1$-$C_4$alkoxy, substituted or unsubstituted $C_3$-$C_8$cycloalkyl, substituted or unsubstituted $C_6$-$C_{14}$aryl, or substituted or unsubstituted $C_2$-$C_{10}$heteroaryl;

$R^{2d}$ and $R^{2e}$ are each independently a hydrogen atom, substituted or unsubstituted $C_1$-$C_4$alkyl, substituted or unsubstituted $C_1$-$C_4$alkoxy, substituted or unsubstituted $C_3$-Cscycloalkyl, substituted or unsubstituted $C_6$-$C_{14}$aryl, or substituted or unsubstituted $C_2$-$C_{10}$heteroaryl;

$R^4$ and $R^5$ are each independently a hydrogen atom, substituted or unsubstituted $C_1$-$C_4$alkyl, substituted or unsubstituted $C_1$-$C_4$alkoxy, substituted or unsubstituted $C_3$-Cscycloalkyl, substituted or unsubstituted $C_6$-$C_{14}$aryl, substituted or unsubstituted $C_2$-$C_{10}$heteroaryl,

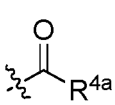 or 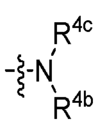;

$R^4$ can also be

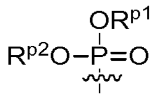,

wherein $R^{p1}$ and $R^{p2}$ are independently substituted or unsubstituted $C_1$-$C_4$alkyl;

$R^{4a}$ is a hydrogen atom, substituted or unsubstituted $C_1$-$C_4$alkyl, substituted or unsubstituted $C_2$-$C_4$alkenyl, substituted or unsubstituted $C_2$-$C_4$alkynyl, substituted or unsubstituted $C_1$-$C_4$alkoxy, substituted or unsubstituted $C_3$-$C_8$cycloalkyl, substituted or unsubstituted $C_6$-$C_{14}$aryl, substituted or unsubstituted $C_2$-$C_{10}$heteroaryl or

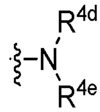;

$R^{4b}$, $R^{4c}$, $R^{4d}$ and $R^{4e}$ are each independently a hydrogen atom, substituted or unsubstituted $C_1$-$C_4$alkyl, substituted or unsubstituted $C_3$-$C_8$cycloalkyl, substituted or unsubstituted $C_6$-$C_{14}$aryl, or substituted or unsubstituted $C_2$-$C_{10}$heteroaryl;

$R^6$ is a hydrogen atom, substituted or unsubstituted $C_1$-$C_4$alkyl, substituted or unsubstituted $C_3$-$C_8$cycloalkyl, substituted or unsubstituted $C_6$-$C_{14}$aryl, substituted or unsubstituted $C_2$-$C_{10}$heteroaryl,

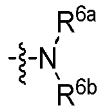, 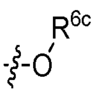 or 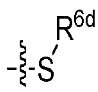;

$R^{6a}$, $R^{6b}$, $R^7$ and $R^8$ are each independently a hydrogen atom, amino, hydroxyl, substituted or unsubstituted $C_1$-$C_4$alkyl, substituted or unsubstituted $C_1$-$C_4$alkoxy, substituted or unsubstituted $C_3$-$C_8$cycloalkyl, substituted or unsubstituted $C_6$-$C_{14}$aryl, or substituted or unsubstituted $C_2$-$C_{10}$heteroaryl;

$R^{6c}$ and $R^{6d}$ are each independently a hydrogen atom, amino, substituted or unsubstituted $C_1$-$C_4$alkyl, substituted or unsubstituted $C_1$-$C_4$alkoxy, substituted or unsubstituted $C_3$-$C_8$cycloalkyl, substituted or unsubstituted $C_6$-$C_{14}$aryl, or substituted or unsubstituted $C_2$-$C_{10}$heteroaryl;

$R^9$ and $R^{10}$ are each independently substituted or unsubstituted $C_1$-$C_4$alkyl, substituted or unsubstituted $C_3$-$C_8$cycloalkyl, substituted or unsubstituted $C_6$-$C_{14}$aryl, or substituted or unsubstituted $C_2$-$C_{10}$heteroaryl;

$B^1$ is a hydrogen atom, cyano, halogen, sulfydryl, carboxyl, amino, substituted or unsubstituted $C_1$-$C_4$alkyl, or substituted or unsubstituted $C_3$-$C_8$cycloalkyl;

$B^2$, $B^3$, $B^4$, $B^5$, $B^6$ and $B^7$ are each independently a hydrogen atom, hydroxyl, substituted or unsubstituted $C_1$-$C_4$alkoxy, cyano, halogen, sulfydryl, carboxyl, amino, substituted or unsubstituted $C_1$-$C_4$alkyl, or substituted or unsubstituted $C_3$-$C_8$cycloalkyl;

L and K are each independently $C_1$-$C_4$alkylene, direct bond, $C_2$-$C_4$alkenylene,

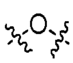, 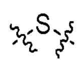, 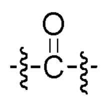, 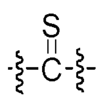 or 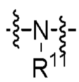;

$R^{11}$ is a hydrogen atom, hydroxyl, substituted or unsubstituted $C_1$-$C_4$alkyl, substituted or unsubstituted $C_3$-$C_8$cycloalkyl, substituted or unsubstituted $C_6$-$C_{14}$aryl, substituted or unsubstituted $C_2$-$C_{10}$heteroaryl or

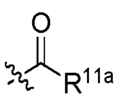;

$R^{11a}$ is a hydrogen atom, substituted or unsubstituted $C_1$-$C_4$alkyl, substituted or unsubstituted $C_3$-$C_8$cycloalkyl, substituted or unsubstituted $C_6$-$C_{14}$aryl, or substituted or unsubstituted $C_2$-$C_{10}$heteroaryl;

Z is substituted or unsubstituted $C_2$-$C_{10}$heteroaryl;

the substituents in the substituted $C_1$-$C_4$alkyl, the substituted $C_1$-$C_4$alkoxy, the substituted $C_3$-$C_8$cycloalkyl, the

$C_6$-$C_{14}$aryl, the substituted $C_2$-$C_4$alkenyl, the substituted $C_2$-$C_4$alkynyl and the substituted $C_2$-$C_8$heterocyclyl are each independently one or more of $C_1$-$C_4$alkyl, $C_1$-$C_4$alkyl substituted with halogen and/or hydroxyl, $C_3$-Cs-cycloalkyl, halogen, hydroxyl, amino, cyano, nitro, sulfydryl and carboxyl; when the substituents are plural, then the substituents can be the same or different;
the heteroatoms in the $C_2$-$C_{10}$heteroaryl are selected from O, N and S, the number of the heteroatoms is 1-3, and the heteroatoms can be the same or different;
carbon labeled with * refers to S-configuration chiral carbon, R-configuration chiral carbon or achiral carbon.

**2.** The tetrahydropyrrole compound represented by general formula (I), the enantiomer, the diastereomer, the isotope compound, the pharmaceutically acceptable prodrug, the pharmaceutically acceptable ester or the pharmaceutically acceptable salt thereof as defined in claim 1, wherein,
when the substituent in the substituted $C_1$-$C_4$alkyl, the substituted $C_1$-$C_4$alkoxy, the substituted $C_3$-$C_8$cycloalkyl, the $C_6$-$C_{14}$aryl, the substituted $C_2$-$C_4$alkenyl, the substituted $C_2$-$C_4$alkynyl and the substituted $C_2$-$C_8$heterocyclyl is $C_1$-$C_4$alkyl, then the $C_1$-$C_4$alkyl is methyl, ethyl, n-propyl, isopropyl, n-butyl, isobutyl or tert-butyl;
and/or, when the substituent in the substituted $C_1$-$C_4$alkyl, the substituted $C_1$-$C_4$alkoxy, the substituted $C_3$-$C_8$cycloalkyl, the $C_6$-$C_{14}$aryl, the substituted $C_2$-$C_4$alkenyl, the substituted $C_2$-$C_4$alkynyl and the substituted $C_2$-$C_8$heterocyclyl is $C_1$-$C_4$alkyl substituted with halogen and/or hydroxyl, then one or more hydrogens in the $C_1$-$C_4$alkyl in the $C_1$-$C_4$alkyl substituted with halogen and/or hydroxyl are substituted with halogen and/or hydroxyl;
the $C_1$-$C_4$alkyl substituted with halogen and/or hydroxyl is preferably

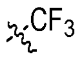, 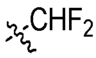 or 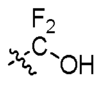;

and/or, when the substituent in the substituted $C_1$-$C_4$alkyl, the substituted $C_1$-$C_4$alkoxy, the substituted $C_3$-$C_8$cycloalkyl, the $C_6$-$C_{14}$aryl, the substituted $C_2$-$C_4$alkenyl, the substituted $C_2$-$C_4$alkynyl and the substituted $C_2$-$C_8$heterocyclyl is $C_3$-$C_8$cycloalkyl, then the $C_3$-$C_8$cycloalkyl is preferably cyclopropyl, cyclobutyl, cyclopentyl, cyclohexyl, cycloheptyl or cyclooctyl;
and/or, when the substituent in the substituted $C_1$-$C_4$alkyl, the substituted $C_1$-$C_4$alkoxy, the substituted $C_3$-$C_8$cycloalkyl, the $C_6$-$C_{14}$aryl, the substituted $C_2$-$C_4$alkenyl, the substituted $C_2$-$C_4$alkynyl and the substituted $C_2$-$C_8$heterocyclyl is halogen, then the halogen is F, Cl, Br or I.

**3.** The tetrahydropyrrole compound represented by general formula (I), the enantiomer, the diastereomer, the isotope compound, the pharmaceutically acceptable prodrug, the pharmaceutically acceptable ester or the pharmaceutically acceptable salt thereof as defined in claim 1 or 2, wherein,
the substituents in the substituted $C_1$-$C_4$alkyl, the substituted $C_1$-$C_4$alkoxy, the substituted $C_3$-$C_8$cycloalkyl, the $C_6$-$C_{14}$aryl, the $C_2$-$C_{10}$heteroaryl,the substituted $C_2$-$C_4$alkenyl, the substituted $C_2$-$C_4$alkynyl and the substituted $C_2$-$C_8$heterocyclyl are each independently one or more of $C_1$-$C_4$alkyl, $C_3$-$C_8$cycloalkyl, halogen, hydroxyl, amino, cyano and sulfydryl; the substituents in the substituted $C_1$-$C_4$alkyl are preferably one or more of halogen, hydroxyl and $C_3$-$C_8$cycloalkyl; the substituents in the substituted $C_2$-$C_{10}$heteroaryl are preferably one or more of halogen and $C_1$-$C_4$alkyl; more preferably, in $R^{2c}$, the substituents in the substituted $C_1$-$C_4$alkyl are selected from one or more of halogen and $C_3$-$C_8$cycloalkyl.

**4.** The tetrahydropyrrole compound represented by general formula (I), the enantiomer, the diastereomer, the isotope compound, the pharmaceutically acceptable prodrug, the pharmaceutically acceptable ester or the pharmaceutically acceptable salt thereof as defined in any one of claims 1-3, wherein,
in $R^1$, $R^{1a}$, $R^{1b}$, $R^{1c}$, $R^{1d}$, $B^1$, $B^2$, $B^3$, $B^4$, $B^5$, $B^6$ and $B^7$, the halogen is F, Cl, Br or I;
and/or, in $R^1$, $R^{1a}$, $R^{1b}$, $R^{1c}$, $R^{1d}$, $R^2$, $R^3$, $R^{2a}$, $R^{2b}$, $R^{2c}$, $R^{2d}$, $R^{2e}$, $R^4$, $R^5$, $R^{4a}$, $R^{4b}$, $R^{4c}$, $R^{4d}$, $R^{4e}$, $R^6$, $R^{6a}$, $R^{6b}$, $R^7$, $R^8$, $R^{6c}$, $R^{6d}$, $R^9$, $R^{10}$, $B^1$, $B^2$, $B^3$, $R^{11}$, $R^{11a}$ $R^{p1}$ and $R^{p2}$, the $C_1$-$C_4$alkyl in the substituted or unsubstituted $C_1$-$C_4$alkyl is methyl, ethyl, n-propyl, isopropyl, n-butyl, isobutyl or tert-butyl;
and/or, in $R^2$, $R^3$, $R^{2a}$, $R^{2b}$, $R^{2c}$, $R^{2d}$, $R^{2e}$, $R^4$, $R^5$, $R^{4a}$, $R^{6a}$, $R^{6b}$, $R^7$, $R^8$, $R^{6c}$, $R^{6d}$, $B^2$ and $B^3$, the $C_1$-$C_4$alkoxy in the substituted or unsubstituted $C_1$-$C_4$alkoxy is methoxy, ethoxy, n-propoxy, isopropoxy, n-butoxy, isobutoxy, or tert-butoxy;
and/or, in $R^1$, $R^{1a}$, $R^{1b}$, $R^{1c}$, $R^{1d}$, $R^2$, $R^3$, $R^{2a}$, $R^{2b}$, $R^{2c}$, $R^{2d}$, $R^{2e}$, $R^4$, $R^5$, $R^{4a}$, $R^{4b}$, $R^{4c}$, $R^{4d}$, $R^{4e}$, $R^6$, $R^{6a}$, $R^{6b}$, $R^7$, $R^8$, $R^{6c}$, $R^{6d}$, $R^9$, $R^{10}$, $B^1$, $B^2$, $B^3$, $R^{11}$ and $R^{11a}$, the $C_3$-Cscycloalkyl in the substituted or unsubstituted $C_3$-$C_8$cycloalkyl is preferably cyclopropyl, cyclobutyl, cyclopentyl, cyclohexyl, cycloheptyl or cyclooctyl;
and/or, in $R^1$, $R^{1a}$, $R^{1b}$, $R^{1c}$, $R^{1d}$, $R^2$, $R^3$, $R^{2a}$, $R^{2b}$, $R^{2c}$, $R^{2d}$, $R^{2e}$, $R^4$, $R^5$, $R^{4a}$, $R^{4b}$, $R^{4c}$, $R^{4d}$, $R^{4e}$, $R^6$, $R^{6a}$, $R^{6b}$, $R^7$, $R^8$, $R^{6c}$, $R^{6d}$, $R^9$, $R^{10}$, $R^{11}$ and $R^{11a}$, the $C_6$-$C_{14}$aryl in the substituted or unsubstituted $C_6$-$C_{14}$aryl is phenyl, naphthyl,

anthracyl or phenanthryl;

and/or, in $R^1$, $R^{1a}$, $R^{1b}$, $R^{1c}$, $R^{1d}$, $R^2$, $R^3$, $R^{2a}$, $R^{2b}$, $R^{2c}$, $R^{2d}$, $R^{2e}$, $R^4$, $R^5$, $R^{4a}$, $R^{4b}$, $R^{4c}$, $R^{4d}$, $R^{4e}$, $R^6$, $R^{6a}$, $R^{6b}$, $R^7$, $R^8$, $R^{6c}$, $R^{6d}$, $R^9$, $R^{10}$, $R^{11}$, $R^{11a}$ and Z, the $C_2$-$C_{10}$heteroaryl in the substituted or unsubstituted $C_2$-$C_{10}$heteroaryl is $C_2$-$C_8$heteroaryl; and when adjacent $R^1$ and $R^{1a}$; or $R^{1a}$ and $R^{1b}$; or $R^{1b}$ and $R^{1c}$; or $R^{1c}$ and $R^{1d}$ and the atoms attached thereto together form substituted or unsubstituted $C_2$-$C_{10}$heteroaryl,then the $C_2$-$C_{10}$heteroaryl in the substituted or unsubstituted $C_2$-$C_{10}$heteroaryl is $C_2$-$C_8$heteroaryl, the $C_2$-$C_8$heteroaryl preferably has 1-2 heteroatoms selected from O, N and S, the $C_2$-$C_{10}$heteroaryl is further preferably pyridyl, furanyl, thienyl, thiazolyl, isothiazolyl, oxazolyl, isoxazolyl, pyrrolyl, imidazolyl, pyrazolyl, indolyl, 4-azaindolyl, 5-azaindolyl, 6-azaindolyl, 7-azaindolyl, quinolinyl, isoquinolinyl, quinazolinyl, quinoxalinyl, cinnolinyl, 1,5-naphthyridinyl, 1,6-naphthyridinyl, 1,7-naphthyridinyl, 1,8-naphthyridinyl, purinyl, indazolyl, benzimidazolyl, benzothienyl, benzofuranyl, benzotriazolyl, benzopyrazolyl, benzoxazolyl, benzisoxazolyl, benzothiazolyl or benzisothiazolyl;

and/or, when adjacent $R^1$ and $R^{1a}$; or $R^{1a}$ and $R^{1b}$; or $R^{1b}$ and $R^{1c}$; or $R^{1c}$ and $R^{1d}$ and the atoms attached thereto together form substituted or unsubstituted $C_3$-Cscycloalkyl, then the $C_3$-$C_8$cycloalkyl in the substituted or unsubstituted $C_3$-Cscycloalkyl is cyclopropyl, cyclobutyl, cyclopentyl, cyclohexyl, cycloheptyl or cyclooctyl;

and/or, when adjacent $R^1$ and $R^{1a}$; or $R^{1a}$ and $R^{1b}$; or $R^{1b}$ and $R^{1c}$; or $R^{1c}$ and $R^{1d}$ and the atoms attached thereto together form substituted or unsubstituted $C_6$-$C_{14}$aryl, then the $C_6$-$C_{14}$aryl in the substituted or unsubstituted $C_6$-$C_{14}$aryl is phenyl, naphthyl, anthracyl or phenanthryl;

and/or, when adjacent $R^1$ and $R^{1a}$; or $R^{1a}$ and $R^{1b}$; or $R^{1b}$ and $R^{1c}$; or $R^{1c}$ and $R^{1d}$ and the atoms attached thereto together form substituted or unsubstituted $C_2$-$C_8$heterocyclyl, then the $C_2$-$C_8$heterocyclyl is $C_2$-$C_6$heterocyclyl; the $C_2$-$C_6$ preferably have 2-4 heteroatoms selected from N, O and S; the $C_2$-$C_8$heterocyclyl is further preferably tetrahydropyranyl, azetidinyl, 1,4-dioxanyl, piperazinyl, piperidinyl, pyrrolidinyl, morpholinyl, thiomorpholinyl, dihydrofuranyl, dihydroimidazolyl, dihydroindolyl, dihydroisoxazolyl, dihydroisothiazolyl, dihydrooxadiazolyl, dihydrooxazolyl, dihydropyrazinyl, dihydropyrazolyl, dihydropyridinyl, dihydropyrimidinyl, dihydropyrrolyl, dihydroquinolinyl, dihydrotetrazolyl, dihydrothiadiazolyl, dihydrothiazolyl, dihydrothienyl, dihydrotriazolyl, dihydroazetidinyl, methylene dioxybenzoyl, tetrahydrofuranyl, tetrahydrothienyl,

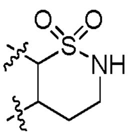, 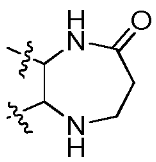, 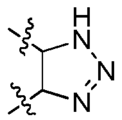, 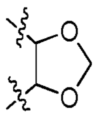, 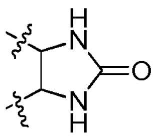, 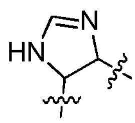, 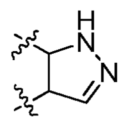,

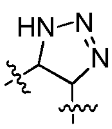, 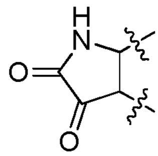 or 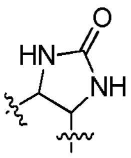,

more preferably

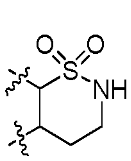, 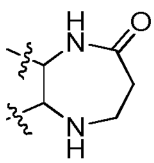,

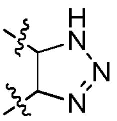, 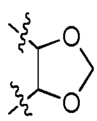, 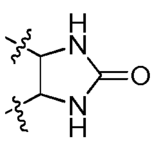, 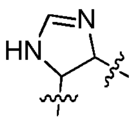, 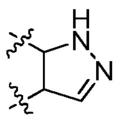, 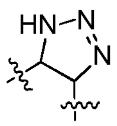, 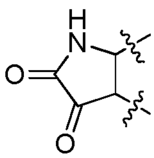

or

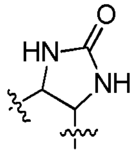.

**5.** The tetrahydropyrrole compound represented by general formula (I), the enantiomer, the diastereomer, the isotope compound, the pharmaceutically acceptable prodrug, the pharmaceutically acceptable ester or the pharmaceutically acceptable salt thereof as defined in any one of claims 1-4, wherein:

in $R^1$, $R^{1a}$, $R^{1b}$, $R^{1c}$, $R^{1d}$, $R^2$, $R^3$, $R^{2a}$, $R^{2b}$, $R^{2c}$, $R^{2d}$, $R^{2e}$, $R^4$, $R^5$, $R^{4a}$, $R^{4b}$, $R^{4c}$, $R^{4d}$, $R^{4e}$, $R^6$, $R^{6a}$, $R^{6b}$, $R^7$, $R^8$, $R^{6c}$, $R^{6d}$, $R^9$, $R^{10}$, $B^1$, $B^2$, $B^3$, $R^{11}$, $R^{11a}$, $R^{p1}$ and $R^{p2}$, the substituted $C_1$-$C_4$alkyl is

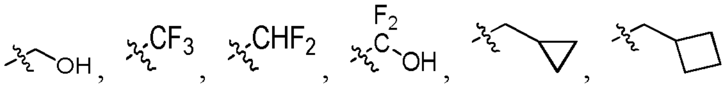

or

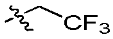;

and/or, the $C_2$-$C_{10}$heteroaryl in the substituted or unsubstituted $C_2$-$C_{10}$heteroaryl is

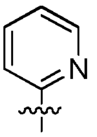, 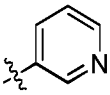, 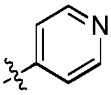, 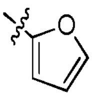, 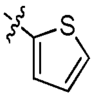, 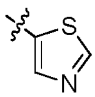, 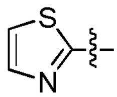, 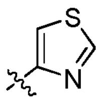,

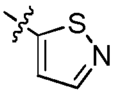, 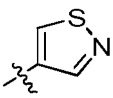, 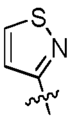, 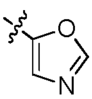, 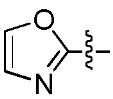, 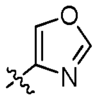, 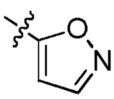, 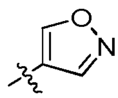,

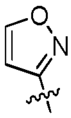, 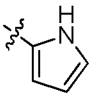, 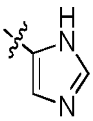, 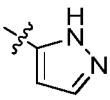, 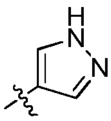, 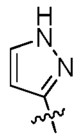, 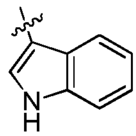, 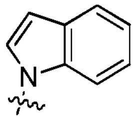,

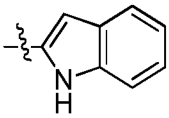, 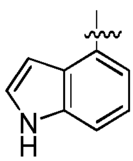, 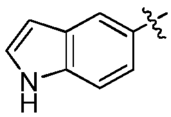, 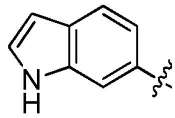, 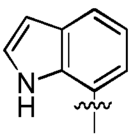, 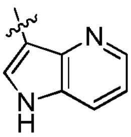,

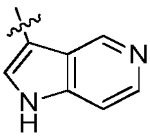, 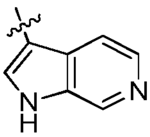, 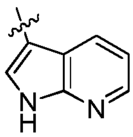, 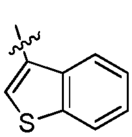, 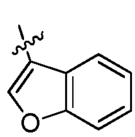, 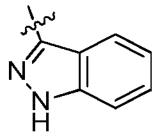,

or

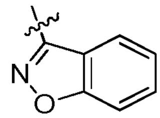;

and/or, the substituted $C_2$-$C_{10}$heteroaryl is

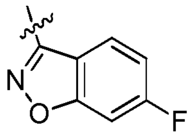, 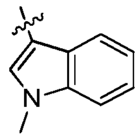

or

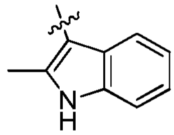;

and/or, when adjacent $R^1$ and $R^{1a}$; or $R^{1a}$ and $R^{1b}$; or $R^{1b}$ and $R^{1c}$; or $R^{1c}$ and $R^{1d}$ and the atoms attached thereto together form substituted or unsubstituted $C_2$-$C_8$heterocyclyl, then the $C_2$-$C_8$heterocyclyl is

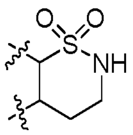, 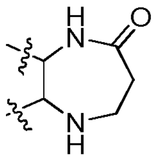, 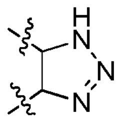,

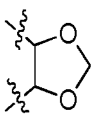, 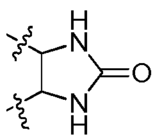, 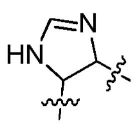, 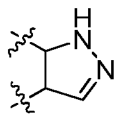, 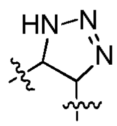, 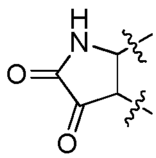 or 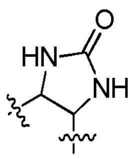.

**6.** The tetrahydropyrrole compound represented by general formula (I), the enantiomer, the diastereomer, the isotope compound, the pharmaceutically acceptable prodrug, the pharmaceutically acceptable ester or the pharmaceutically acceptable salt thereof as defined in any one of claims 1-5, wherein:

no more than 1 or 2 nitrogen atoms are present in $A^1$, $A^2$, $A^3$, $A^4$ and $A^5$;
or, $A^1$ is C-$R^1$; $A^2$ is C-$R^{1a}$; $A^3$ is C-$R^{1b}$ or N; $A^4$ is C-$R^{1c}$ or N; and $A^5$ is C-$R^{1d}$ or N;
or, $A^1$ is CH; $A^2$ is CH; $A^3$ is C-$R^{1b}$ or N; $A^4$ is C-$R^{1c}$ or N; and $A^5$ is C-$R^{1d}$ or N;
or $A^1$ is C-$R^1$; $A^2$ is C-$R^{1a}$; $A^3$ is C-$R^{1b}$; $A^4$ is C-$R^{1c}$ and $A^5$ is C-$R^{1d}$;
or $A^1$ is C-$R^1$; $A^2$ is CH; $A^3$ is CH; $A^4$ is C-$R^{1c}$ and $A^5$ is CH;
or $A^1$ is CH; $A^2$ is C-$R^{1a}$; $A^3$ is CH; $A^4$ is C-$R^{1c}$ and $A^5$ is CH;
or $A^1$ is CH; $A^2$ is CH; $A^3$ is C-$R^{1b}$; $A^4$ is C-$R^{1c}$ and $A^5$ is CH;
or $A^1$ is CH; $A^2$ is CH; $A^3$ is CH; $A^4$ is C-$R^{1c}$ and $A^5$C-$R^{1d}$; or $R^{1c}$, $R^{1d}$ and the C attached thereto together form substituted or unsubstituted $C_2$-$C_8$heterocyclyl;
or $A^1$ is CH; $A^2$ is CH; $A^3$ is CH; $A^4$ is C-$R^{1c}$ and $A^5$ is CH.

**7.** The tetrahydropyrrole compound represented by general formula (I), the enantiomer, the diastereomer, the isotope compound, the pharmaceutically acceptable prodrug, the pharmaceutically acceptable ester or the pharmaceutically acceptable salt thereof as defined in any one of claims 1-6, wherein:

$R^1$, $R^{1a}$, $R^{1b}$, $R^{1c}$ and $R^{1d}$ are each independently a hydrogen atom, halogen, cyano, nitro, substituted or unsubstituted $C_1$-$C_4$alkyl,

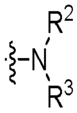 , 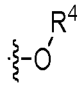 , 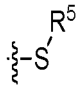 , 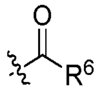 ,

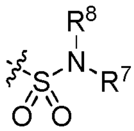 , 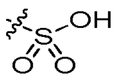 , 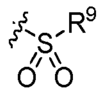 , 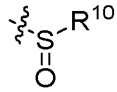 ;

or adjacent $R^1$ and $R^{1a}$; or $R^{1a}$ and $R^{1b}$; or $R^{1b}$ and $R^{1c}$; or $R^{1c}$ and $R^{1d}$ and the atoms attached thereto together form substituted or unsubstituted $C_3$-$C_8$cycloalkyl, substituted or unsubstituted $C_2$-$C_8$heterocyclyl, substituted or unsubstituted $C_6$-$C_{14}$aryl, or, substituted or unsubstituted $C_2$-$C_{10}$heteroaryl;

and/or, $R^2$ and $R^3$ are each independently a hydrogen atom, hydroxyl, amino, substituted or unsubstituted $C_1$-$C_4$alkyl, substituted or unsubstituted $C_1$-$C_4$alkoxy, substituted or unsubstituted $C_3$-$C_8$cycloalkyl,

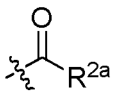 , 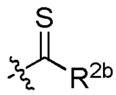 or 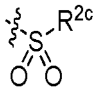 ;

and/or, $R^{2a}$ and $R^{2b}$ are each independently a hydrogen atom, substituted or unsubstituted $C_1$-$C_4$alkyl, substituted or unsubstituted $C_1$-$C_4$alkoxy, substituted or unsubstituted $C_3$-$C_8$cycloalkyl or

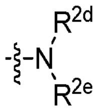 ;

and/or, $R^{2c}$ is substituted or unsubstituted $C_1$-$C_4$alkyl, substituted or unsubstituted $C_3$-$C_8$cycloalkyl, or substituted or unsubstituted $C_2$-$C_{10}$heteroaryl; preferably, $R^{2c}$ is substituted or unsubstituted $C_1$-$C_4$alkyl, $C_3$-$C_8$cycloalkyl or $C_2$-$C_{10}$heteroaryl;

and/or, $R^{2d}$ and $R^{2e}$ are independently a hydrogen atom, substituted or unsubstituted $C_1$-$C_4$alkyl, or substituted or unsubstituted $C_3$-$C_8$cycloalkyl;

and/or, $R^4$ and $R^5$ are each independently a hydrogen atom, substituted or unsubstituted $C_1$-$C_4$alkyl, substituted or unsubstituted $C_3$-$C_8$cycloalkyl,

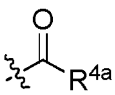

or

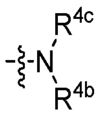 ;

$R^4$ can also be

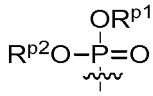 ,

wherein $R^{p1}$ and $R^{p2}$ are independently substituted or unsubstituted $C_1$-$C_4$alkyl;

and/or, $R^{4a}$ is a hydrogen atom, substituted or unsubstituted $C_1$-$C_4$alkyl, substituted or unsubstituted $C_3$-$C_8$cycloalkyl or

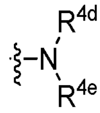 ;

and/or, $R^{4b}$, $R^{4c}$, $R^{4d}$ and $R^{4e}$ are each independently a hydrogen atom, substituted or unsubstituted $C_1$-$C_4$alkyl, or substituted or unsubstituted $C_3$-$C_8$cycloalkyl;

and/or, $R^6$ is a hydrogen atom, substituted or unsubstituted $C_3$-$C_8$cycloalkyl,

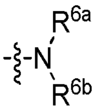 , 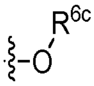 or 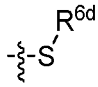 ;

and/or, $R^{6a}$ and $R^{6b}$ are each independently a hydrogen atom, amino, hydroxyl, substituted or unsubstituted $C_1$-$C_4$alkyl, substituted or unsubstituted $C_1$-$C_4$alkoxy, or substituted or unsubstituted $C_3$-$C_8$cycloalkyl;

and/or, $R^{6c}$ and $R^{6d}$ are each independently a hydrogen atom, substituted or unsubstituted $C_1$-$C_4$alkyl, or substituted or unsubstituted $C_3$-$C_8$cycloalkyl;

and/or, $R^7$ and $R^8$ are each independently a hydrogen atom, amino, hydroxyl, substituted or unsubstituted $C_1$-$C_4$alkyl, or substituted or unsubstituted $C_3$-$C_8$cycloalkyl;

and/or, $R^9$ and $R^{10}$ are each independently substituted or unsubstituted $C_1$-$C_4$alkyl, substituted or unsubstituted $C_3$-$C_8$cycloalkyl, or substituted or unsubstituted $C_2$-$C_{10}$heteroaryl; preferably $R^9$ is substituted or unsubstituted $C_1$-$C_4$alkyl; $R^{10}$ is $C_1$-$C_4$alkyl;

and/or, $B^1$ is a hydrogen atom, cyano, halogen, sulfydryl, amino, or substituted or unsubstituted $C_1$-$C_4$alkyl, preferably a hydrogen atom, cyano, halogen, or substituted or unsubstituted $C_1$-$C_4$alkyl;

and/or, $B^2$, $B^3$, $B^4$, $B^5$, $B^6$ and $B^7$ are each independently a hydrogen atom, hydroxyl, $C_1$-$C_4$alkoxy, cyano, halogen, sulfydryl, carboxyl, amino, or substituted or unsubstituted $C_1$-$C_4$alkyl;

and/or, L and K are each independently $C_1$-$C_4$alkylene, direct bond,

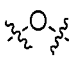 , 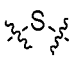 ,

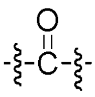 or 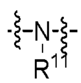 ;

and/or, $R^{11}$ is a hydrogen atom, hydroxyl, substituted or unsubstituted $C_1$-$C_4$alkyl or

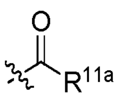 ;

and/or, $R^{11a}$ is a hydrogen atom, substituted or unsubstituted $C_1$-$C_4$alkyl, or substituted or unsubstituted $C_3$-$C_8$cycloalkyl;

and/or, Z is substituted or unsubstituted $C_2$-$C_{10}$heteroaryl containing at least one nitrogen atom.

**8.** The tetrahydropyrrole compound represented by general formula (I), the enantiomer, the diastereomer, the isotope compound, the pharmaceutically acceptable prodrug, the pharmaceutically acceptable ester or the pharmaceutically acceptable salt thereof as defined in any one of claims 1-7, wherein:

$R^1$, $R^{1a}$, $R^{1b}$, $R^{1c}$ and $R^{1d}$ are each independently a hydrogen atom, halogen, cyano, nitro, substituted or

unsubstituted $C_1$-$C_4$alkyl,

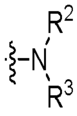, 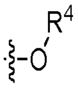, 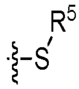, 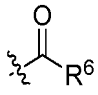,

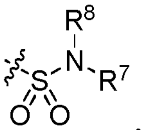, 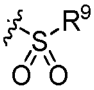, 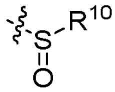;

or adjacent $R^1$ and $R^{1a}$; or $R^{1a}$ and $R^{1b}$; or $R^{1b}$ and $R^{1c}$; or $R^{1c}$ and $R^{1d}$ and the atoms attached thereto together form substituted or unsubstituted $C_3$-$C_8$cycloalkyl, substituted or unsubstituted $C_2$-$C_8$heterocyclyl, substituted or unsubstituted $C_6$-$C_{14}$aryl, or, substituted or unsubstituted $C_2$-$C_{10}$heteroaryl; preferably adjacent $R^1$ and $R^{1a}$; or $R^{1a}$ and $R^{1b}$; $R^{1c}$ and $R^{1d}$ and the atoms attached thereto together form $C_2$-$C_8$heterocyclyl;
and/or, $R^2$ and $R^3$ are each independently a hydrogen atom, hydroxyl, amino, substituted or unsubstituted $C_1$-$C_4$alkyl, $C_1$-$C_4$alkoxy, substituted or unsubstituted $C_3$-Cscycloalkyl,

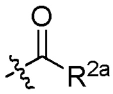 or 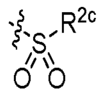;

preferably, one of $R^2$ and $R^3$ is hydrogen, the other is substituted or unsubstituted $C_1$-$C_4$alkyl,

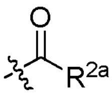 or 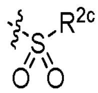;

or $R^2$ and $R^3$ are both substituted or unsubstituted $C_1$-$C_4$alkyl; most preferably, one of $R^2$ and $R^3$ is hydrogen, the other is substituted or unsubstituted $C_1$-$C_4$alkyl,

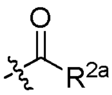 or 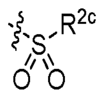,

or $R^2$ and $R^3$ are both $C_1$-$C_4$alkyl;
and/or, $R^{2a}$ and $R^{2b}$ are each independently a hydrogen atom, substituted or unsubstituted $C_1$-$C_4$alkyl, substituted or unsubstituted $C_1$-$C_4$alkoxy, substituted or unsubstituted $C_3$-$C_8$cycloalkyl or

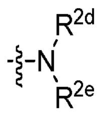;

preferably, $R^{2a}$ is a hydrogen atom, or substituted or unsubstituted $C_1$-$C_4$alkyl; most preferably, $R^{2a}$ is $C_1$-$C_4$alkyl;
and/or, $R^4$ is a hydrogen atom,

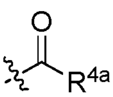 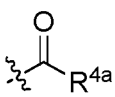 or 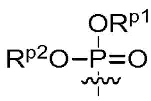;

$R^5$ is a hydrogen atom; $R^{p1}$ and $R^{p2}$ are independently $C_1$-$C_4$alkyl;

and/or, $R^{4a}$ is a hydrogen atom, substituted or unsubstituted $C_1$-$C_4$alkyl, or substituted or unsubstituted $C_3$-$C_8$cycloalkyl, preferably a hydrogen atom or $C_1$-$C_4$alkyl;
and/or, $R^6$ is

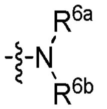 or 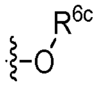 ;

and/or, $R^{6a}$ and $R^{6b}$ are each independently a hydrogen atom, amino, hydroxyl, substituted or unsubstituted $C_1$-$C_4$alkyl, or substituted or unsubstituted $C_3$-$C_8$cycloalkyl; preferably are each independently a hydrogen atom, substituted or unsubstituted $C_1$-$C_4$alkyl, or substituted or unsubstituted $C_3$-$C_8$cycloalkyl; further preferably are independently a hydrogen atom or $C_1$-$C_4$alkyl;
and/or, $R^{6c}$ and $R^{6d}$ are H;
and/or, $R^7$ and $R^8$ are each independently a hydrogen atom, substituted or unsubstituted $C_1$-$C_4$alkyl, or substituted or unsubstituted $C_3$-$C_8$cycloalkyl; preferably are independently a hydrogen atom or $C_1$-$C_4$alkyl;
and/or, $B^1$ is a hydrogen atom, cyano, halogen, or substituted or unsubstituted $C_1$-$C_4$alkyl; preferably $B^1$ is a hydrogen atom;
and/or, $B^2$, $B^3$, $B^4$, $B^5$, $B^6$ and $B^7$ are hydrogen atoms;
and/or, L is

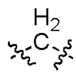,

direct bond,

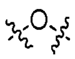, 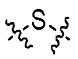, 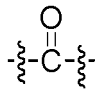 or 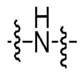;

preferably a direct bond;
and/or, K is

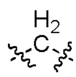;

and/or, Z is substituted or unsubstituted $C_6$-$C_8$heteroaryl containing at least one nitrogen atom, and the $C_6$-$C_8$heteroaryl is a heteroaryl with two fused rings.

**9.** The tetrahydropyrrole compound represented by general formula (I), the enantiomer, the diastereomer, the isotope compound, the pharmaceutically acceptable prodrug, the pharmaceutically acceptable ester or the pharmaceutically acceptable salt thereof as defined in any one of claims 1-8, wherein:

$R^1$, $R^{1a}$, $R^{1b}$, $R^{1c}$ and $R^{1d}$ are each independently H,

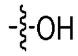, 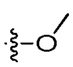, Cl, F, 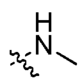,

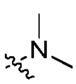, 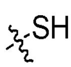, 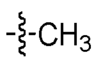, 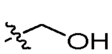, 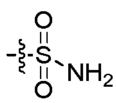, 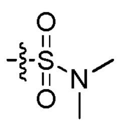, 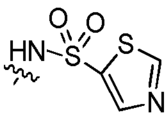, 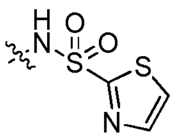,

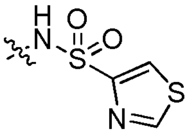, 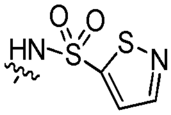, 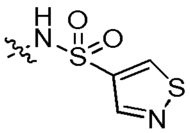, 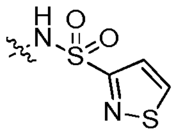, 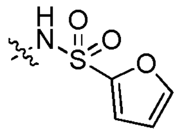,

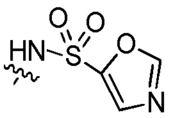, 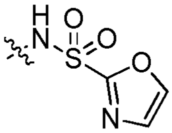, 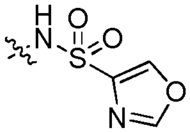, 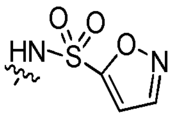, 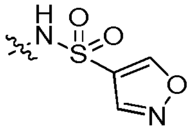,

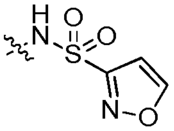, 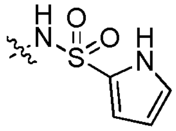, 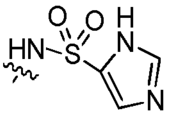, 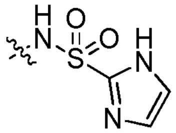, 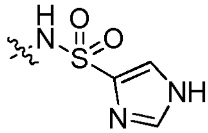,

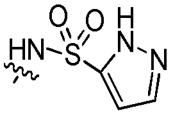, 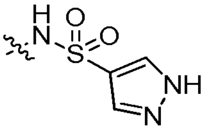, 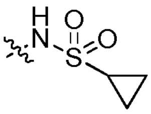, 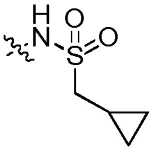, 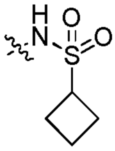, 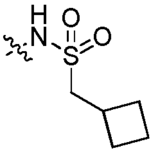,

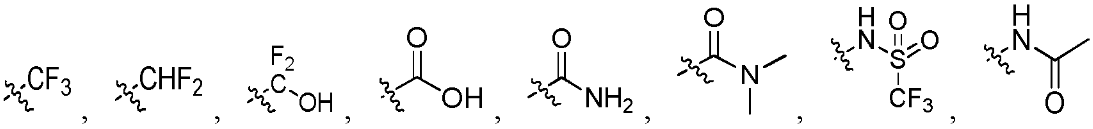
, , , , , , , ,

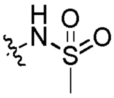, 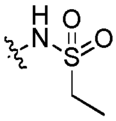, 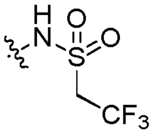, 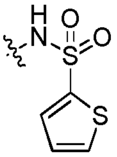, 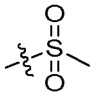, 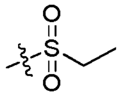, 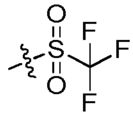,

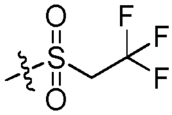, 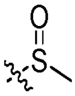, 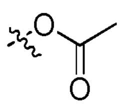, 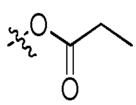 or 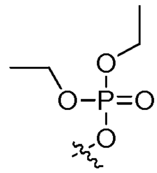;

and/or,

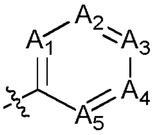 is 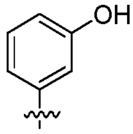, 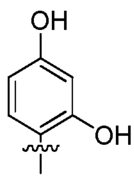, 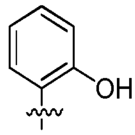, 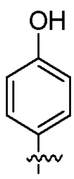, 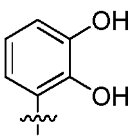,

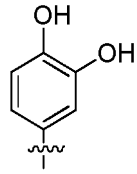 , 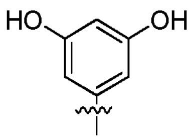 , 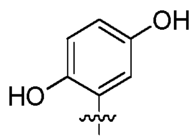 , 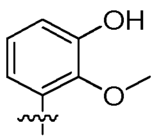 , 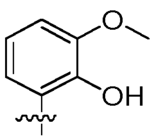 , 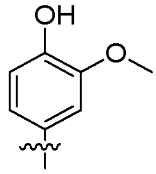 ,

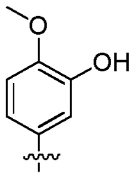 , 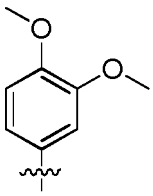 , 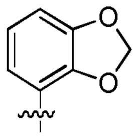 , 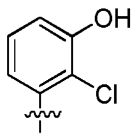 , 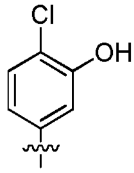 , 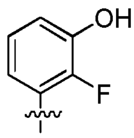 , 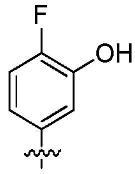 ,

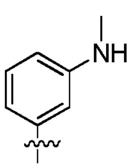 , 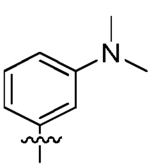 , 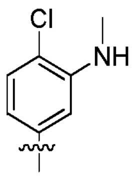 , 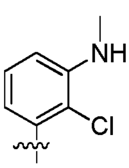 , 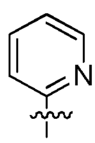 , 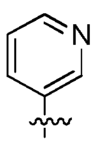 , 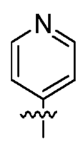 ,

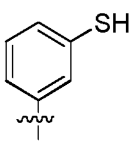 , 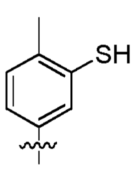 , 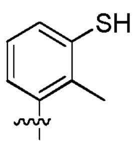 , 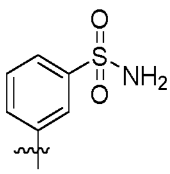 , 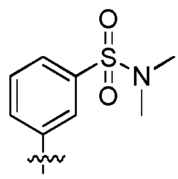 , 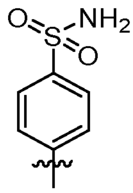 ,

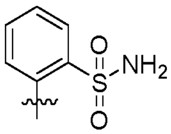 , 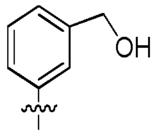 , 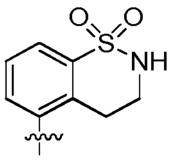 , 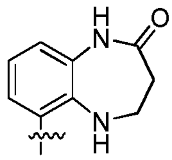 , 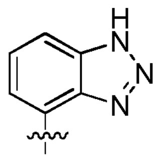 ,

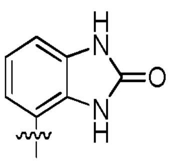 , 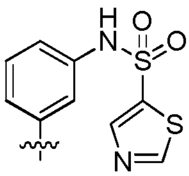 , 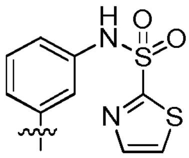 , 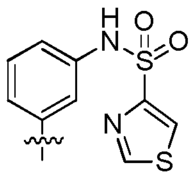 , 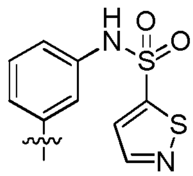 ,

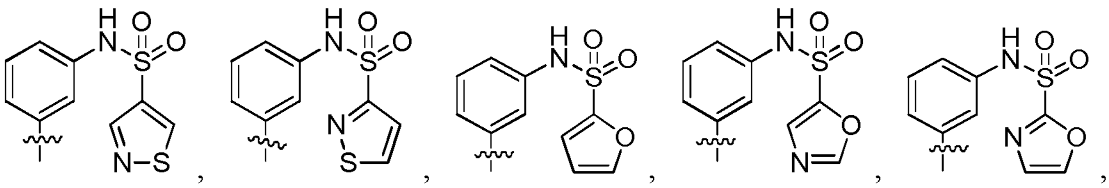 , , , , ,

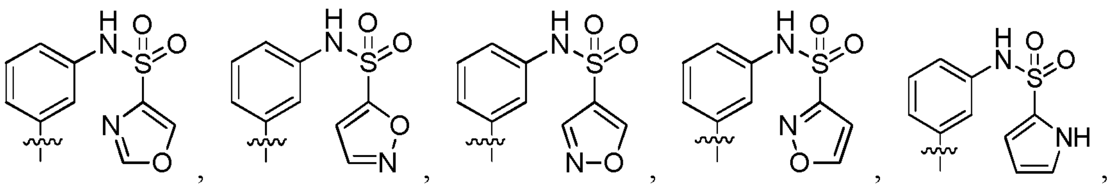 , , , , ,

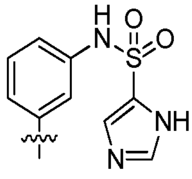, 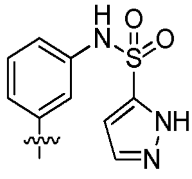, 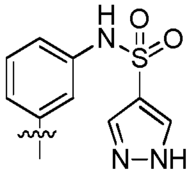, 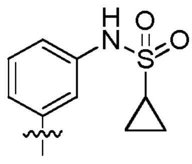, 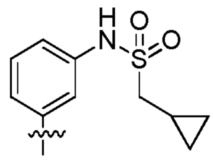,

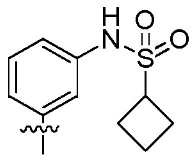, 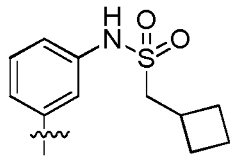, 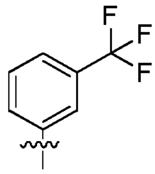, 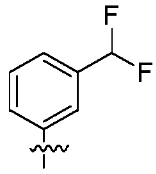, 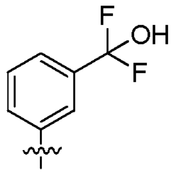,

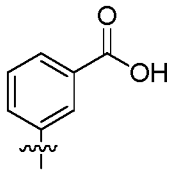, 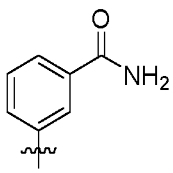, 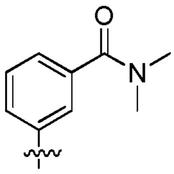, 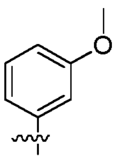, 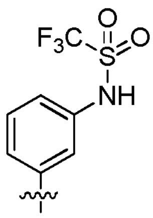, 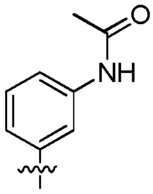,

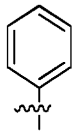, 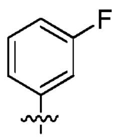, 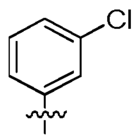, 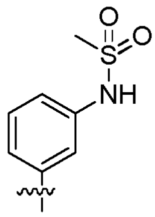, 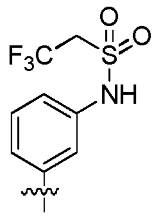, 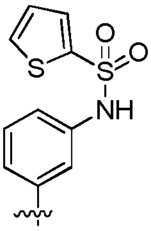, 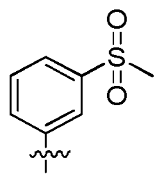,

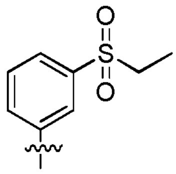, 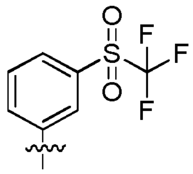, 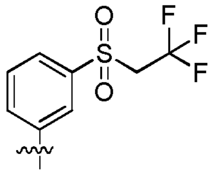, 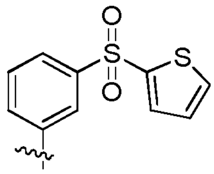, 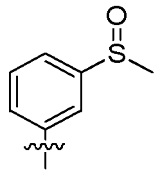,

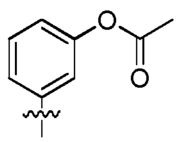, 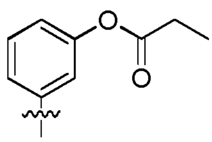, 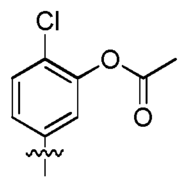, 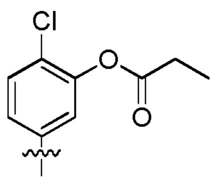, 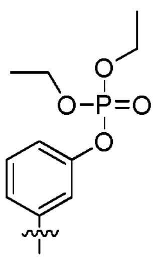,

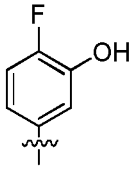, 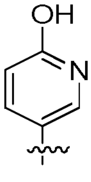, 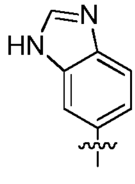, 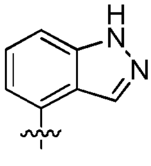, 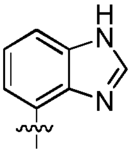, 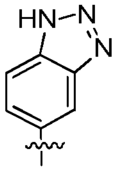, 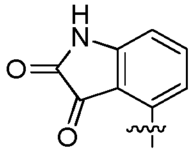,

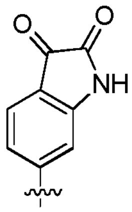 or 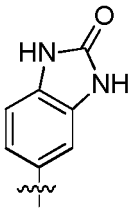 ;

and/or, Z is

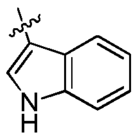 , 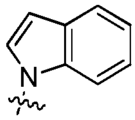 , 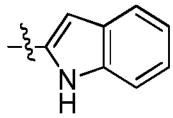 , 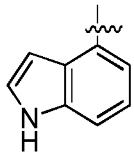 , 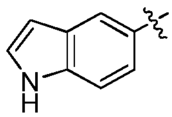 ,

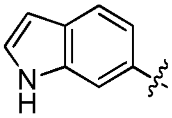 , 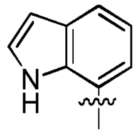 , 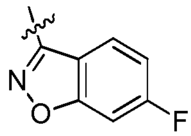 , 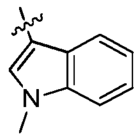 , 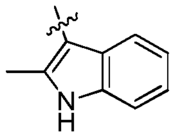 , 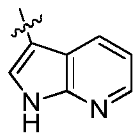 ,

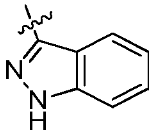 , 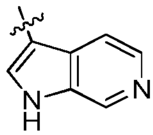 , 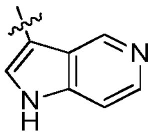 , 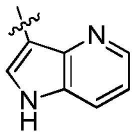 , 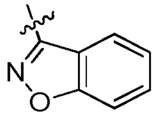 , 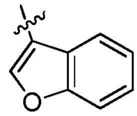

or

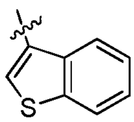 .

**10.** The tetrahydropyrrole compound represented by general formula (I), the enantiomer, the diastereomer, the isotope compound, the pharmaceutically acceptable prodrug, the pharmaceutically acceptable ester or the pharmaceutically acceptable salt thereof as defined in any one of claims 1-9, wherein:

$A^1$ is CH; $A^2$ is CH; $A^3$ is C-$R^{1b}$ or N; $A^4$ is C-$R^{1c}$ or N; and $A^5$ is C-$R^{1d}$ or N; or $A^1$ is C-$R^1$; $A^2$ is C-$R^{1a}$; $A^3$ is C-$R^{1b}$; $A^4$ is C-$R^{1c}$ and $A^5$ is C-$R^{1d}$; wherein $R^1$ and $R^{1a}$; $R^{1a}$ and $R^{1b}$; or $R^{1b}$ and $R^{1c}$; or $R^{1c}$ and $R^{1d}$ and the atoms attached thereto together form substituted or unsubstituted $C_3$-$C_8$cycloalkyl, substituted or unsubstituted $C_2$-$C_8$heterocyclyl, substituted or unsubstituted $C_6$-$C_{14}$aryl, or, substituted or unsubstituted $C_2$-$C_{10}$heteroaryl; preferably, $R^1$, $R^{1a}$, $R^{1b}$, $R^{1c}$ and $R^{1d}$ are each independently a hydrogen atom, halogen, cyano, nitro, substituted or unsubstituted $C_1$-$C_4$alkyl, $C_1$-$C_4$alkoxy,

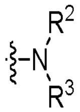 , 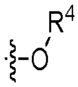 , 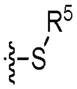 , 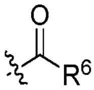 , 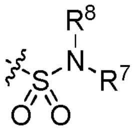 , 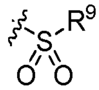 , 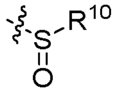 ,

or adjacent $R^1$ and $R^{1a}$; or $R^{1a}$ and $R^{1b}$; or $R^{1b}$ and $R^{1c}$; or $R^{1c}$ and $R^{1d}$ and the atoms attached thereto together form substituted or unsubstituted $C_3$-$C_8$cycloalkyl, substituted or unsubstituted $C_2$-$C_8$heterocyclyl, substituted or unsubstituted $C_6$-$C_{14}$aryl, or, substituted or unsubstituted $C_2$-$C_{10}$heteroaryl;

$R^2$ and $R^3$ are each independently a hydrogen atom, hydroxyl, amino, substituted or unsubstituted $C_1$-$C_4$alkyl,

$C_1$-$C_4$alkoxy, substituted or unsubstituted $C_3$-$C_8$cycloalkyl,

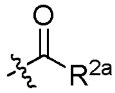 or 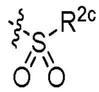 ;

$R^{2A}$ is a hydrogen atom, or substituted or unsubstituted $C_1$-$C_4$alkyl;
$R^{2c}$ is substituted or unsubstituted $C_1$-$C_4$alkyl, substituted or unsubstituted $C_3$-Cscycloalkyl, or substituted or unsubstituted $C_2$-$C_{10}$heteroaryl;
$R^4$ is a hydrogen atom or

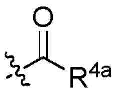 ;

$R^5$ is a hydrogen atom;
$R^6$ is a hydrogen atom, substituted or unsubstituted $C_1$-$C_4$alkyl, substituted or unsubstituted $C_3$-$C_8$cycloalkyl,

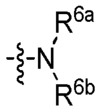, 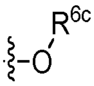 or 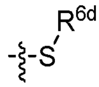 ;

$R^{6a}$ and $R^{6b}$ are each independently a hydrogen atom, amino, hydroxyl, substituted or unsubstituted $C_1$-$C_4$alkyl, or substituted or unsubstituted $C_3$-$C_8$cycloalkyl;
$R^{6c}$ and $R^{6d}$ are each independently a hydrogen atom, substituted or unsubstituted $C_1$-$C_4$alkyl, or substituted or unsubstituted $C_3$-$C_8$cycloalkyl;
$R^7$ and $R^8$ are each independently a hydrogen atom, substituted or unsubstituted $C_1$-$C_4$alkyl, or substituted or unsubstituted $C_3$-$C_8$cycloalkyl;
$R^9$ and $R^{10}$ are each independently substituted or unsubstituted $C_1$-$C_4$alkyl, substituted or unsubstituted $C_3$-$C_8$cycloalkyl or substituted or unsubstituted $C_2$-$C_{10}$heteroaryl;
$B^1$, $B^2$, $B^3$, $B^4$, $B^5$, $B^6$ and $B^7$ are hydrogen atoms;
L and K are each independently

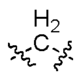,

direct bond,

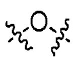, 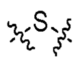, 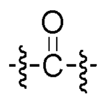

or

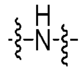;

and Z is substituted or unsubstituted $C_2$-$C_{10}$heteroaryl containing at least one nitrogen atom.

**11.** The tetrahydropyrrole compound represented by general formula (I), the enantiomer, the diastereomer, the isotope compound, the pharmaceutically acceptable prodrug, the pharmaceutically acceptable ester or the pharmaceutically acceptable salt thereof as defined in any one of claims 1-10, wherein:

$A^1$ is C-$R^1$; $A^2$ is CH; $A^3$ is CH; $A^4$ is C-$R^{1c}$ and $A^5$ is CH; or $A^1$ is CH; $A^2$ is C-$R^{1a}$; $A^3$ is CH; $A^4$ is C-$R^{1c}$ and $A^5$ is CH; or $A^1$ is CH; $A^2$ is CH; $A^3$ is C-$R^{1b}$; $A^4$ is C-$R^{1c}$ and $A^5$ is CH; or $A^1$ is CH; $A^2$ is CH; $A^3$ is CH; $A^4$ is C-$R^{1c}$ and $A^5$C-$R^{1d}$; or $R^{1c}$, $R^{1d}$ and the carbon attached thereto together form substituted or unsubstituted $C_2$-$C_8$heterocyclyl; or $A^1$ is CH; $A^2$ is CH; $A^3$ is CH; $A^4$ is C-$R^{1c}$ and $A^5$ is CH; wherein $R^1$, $R^{1a}$, $R^{1b}$, $R^{1c}$ and $R^{1d}$ are each independently a hydrogen atom, halogen, cyano, nitro, substituted or unsubstituted $C_1$-$C_4$alkyl, $C_1$-$C_4$alkoxy,

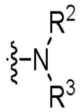, 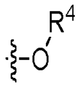, 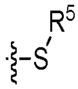, 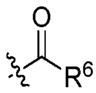,

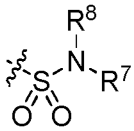, 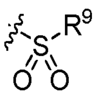, 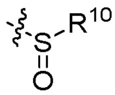;

one of $R^2$ and $R^3$ is hydrogen, the other is substituted or unsubstituted $C_1$-$C_4$alkyl,

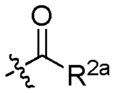 or 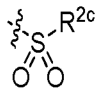;

or $R^2$ and $R^3$ are both substituted or unsubstituted $C_1$-$C_4$alkyl;
$R^{2a}$ is a hydrogen atom, or substituted or unsubstituted $C_1$-$C_4$alkyl;
$R^{2c}$ is substituted or unsubstituted $C_1$-$C_4$alkyl, substituted or unsubstituted $C_3$-Cscycloalkyl, or substituted or unsubstituted $C_2$-$C_{10}$heteroaryl;
$R^4$ and $R^5$ are hydrogen atoms;
$R^6$ is a hydrogen atom, substituted or unsubstituted $C_1$-$C_4$alkyl, substituted or unsubstituted $C_3$-$C_8$cycloalkyl,

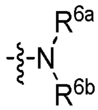, 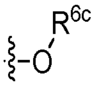 or 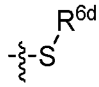;

$R^{6a}$ and $R^{6b}$ are each independently a hydrogen atom, substituted or unsubstituted $C_1$-$C_4$alkyl, or substituted or unsubstituted $C_3$-$C_8$cycloalkyl;
$R^{6c}$ and $R^{6d}$ are each independently a hydrogen atom, substituted or unsubstituted $C_1$-$C_4$alkyl, or substituted or unsubstituted $C_3$-$C_8$cycloalkyl;
$R^7$ and $R^8$ are each independently a hydrogen atom, substituted or unsubstituted $C_1$-$C_4$alkyl, or substituted or unsubstituted $C_3$-$C_8$cycloalkyl;
$R^9$ and $R^{10}$ are each independently substituted or unsubstituted $C_1$-$C_4$alkyl, substituted or unsubstituted $C_3$-$C_8$cycloalkyl or substituted or unsubstituted $C_2$-$C_{10}$heteroaryl;
$B^1$ is a hydrogen atom;
$B^2$, $B^3$, $B^4$, $B^5$, $B^6$ and $B^7$ are hydrogen atoms;
L is a direct bond;
K is

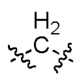;

and Z is substituted or unsubstituted $C_2$-$C_{10}$heteroaryl containing at least one nitrogen atom; the substituents in the substituted $C_2$-$C_{10}$heteroaryl are selected from one or more of halogen and $C_1$-$C_4$alkyl;
or;
$A^1$ is C-$R^1$; $A^2$ is CH; $A^3$ is CH; $A^4$ is C-$R^{1c}$ and $A^5$ is CH;

or $A^1$ is CH; $A^2$ is C-$R^{1a}$; $A^3$ is CH; $A^4$ is C-$R^{1c}$ and $A^5$ is CH;
or $A^1$ is CH; $A^2$ is CH; $A^3$ is C-$R^{1b}$; $A^4$ is C-$R^{1c}$ and $A^5$ is CH;
or $A^1$ is CH; $A^2$ is CH; $A^3$ is CH; $A^4$ is C-$R^{1c}$ and $A^5$ is C-$R^{1d}$; or $R^{1c}$, $R^{1d}$ and the C attached thereto together form substituted or unsubstituted $C_2$-$C_8$heterocyclyl;
or $A^1$ is CH; $A^2$ is CH; $A^3$ is CH; $A^4$ is C-$R^{1c}$ and $A^5$ is CH;
$R^1$, $R^{1a}$, $R^{1b}$, $R^{1c}$ and $R^{1d}$ are each independently a hydrogen atom, halogen, substituted or unsubstituted $C_1$-$C_4$alkyl,

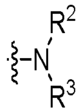, 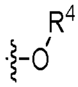, 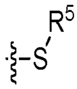, 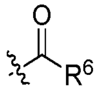, 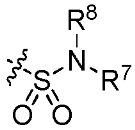,

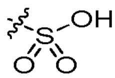, 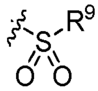 or 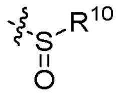,

wherein:

in $R^1$, $R^{1a}$, $R^{1b}$, $R^{1c}$ and $R^{1d}$, the substituents in the substituted $C_1$-$C_4$alkyl are selected from one or more of hydroxyl and halogen;
one of $R^2$ and $R^3$ is hydrogen, the other is substituted or unsubstituted $C_1$-$C_4$alkyl,

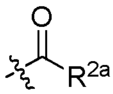 or 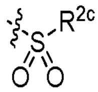,

or $R^2$ and $R^3$ are both $C_1$-$C_4$alkyl; $R^{2a}$ is $C_1$-$C_4$alkyl; $R^{2c}$ is substituted or unsubstituted $C_1$-$C_4$alkyl, $C_3$-$C_8$cycloalkyl or $C_2$-$C_{10}$heteroaryl, in $R^{2c}$, the substituents in the substituted $C_1$-$C_4$alkyl are selected from one or more of halogen and $C_3$-$C_8$cycloalkyl;
$R^4$ is a hydrogen atom,

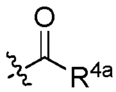 or 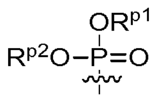;

$R^{4a}$ is a hydrogen atom or $C_1$-$C_4$alkyl; $R^{p1}$ and $R^{p2}$ are independently $C_1$-$C_4$alkyl;
$R^5$ is a hydrogen atom;
$R^6$ is

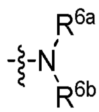 or 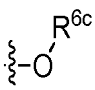;

$R^{6a}$ and $R^{6b}$ are a hydrogen atom or $C_1$-$C_4$alkyl; $R^6$ is H;
$R^7$ and $R^8$ are each independently a hydrogen atom or $C_1$-$C_4$alkyl;
$R^9$ is substituted or unsubstituted $C_1$-$C_4$alkyl; and
$R^{10}$ is $C_1$-$C_4$alkyl;
$B^1$ is a hydrogen atom;
$B^2$, $B^3$, $B^4$, $B^5$, $B^6$ and $B^7$ are hydrogen atoms;
L is a direct bond;
K is

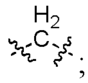

and Z is substituted or unsubstituted $C_2$-$C_{10}$heteroaryl containing at least one nitrogen atom; the substituents in the substituted $C_2$-$C_{10}$heteroaryl are selected from one or more of halogen and $C_1$-$C_4$alkyl;
or;
$A^1$ is CH; $A^2$ is C-$R^{1a}$; $A^3$ is CH; $A^4$ is C-$R^{1c}$ and $A^5$ is CH;
$R^{1a}$ is hydroxyl or

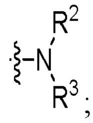

one of $R^2$ and $R^3$ is hydrogen, the other is

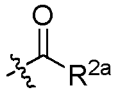 or 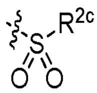 ,

$R^{2a}$ is $C_1$-$C_4$alkyl; $R^{2c}$ is substituted or unsubstituted $C_1$-$C_4$alkyl or $C_2$-$C_{10}$heteroaryl, in $R^{2c}$, the substituents in the substituted $C_1$-$C_4$alkyl are substituted with one or more of halogens;
$B^1$, $B^2$, $B^3$, $B^4$, $B^5$, $B^6$ and $B^7$ are hydrogen atoms;
L is a direct bond;
K is

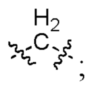

and Z is substituted or unsubstituted $C_2$-$C_{10}$heteroaryl containing at least one nitrogen atom; the substituents in the substituted $C_2$-$C_{10}$heteroaryl are selected from one or more of halogen and $C_1$-$C_4$alkyl.

**12.** The tetrahydropyrrole compound represented by general formula (I), the enantiomer, the diastereomer, the isotope compound, the pharmaceutically acceptable prodrug, the pharmaceutically acceptable ester or the pharmaceutically acceptable salt thereof as defined in any one of claims 1-11, wherein: the tetrahydropyrrole compound represented by general formula (I) is any of the following compounds:

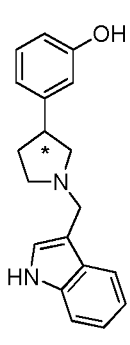 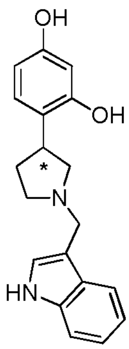 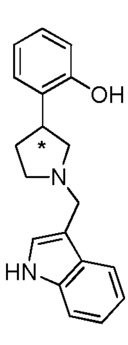 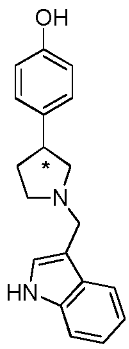 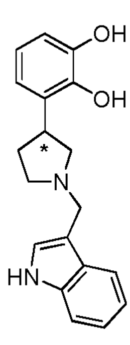 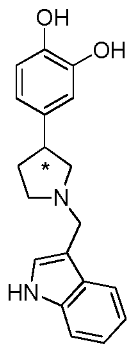

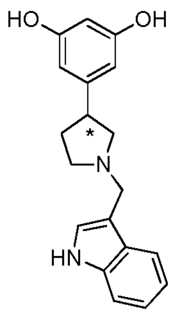 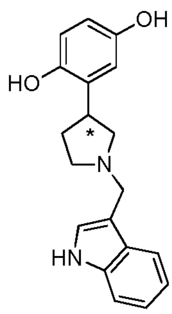 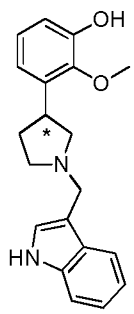 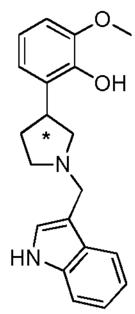 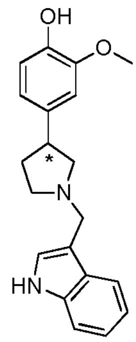 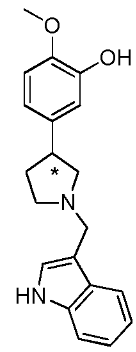

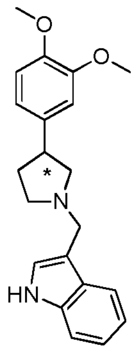 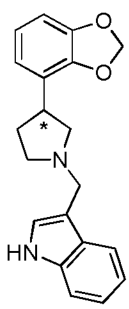 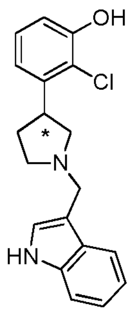 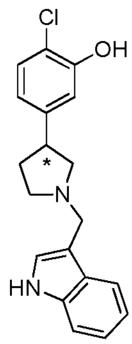 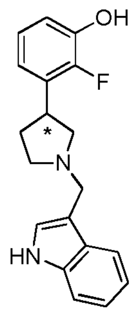 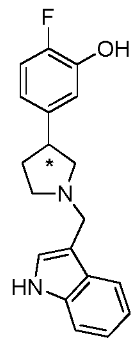

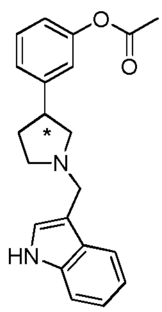 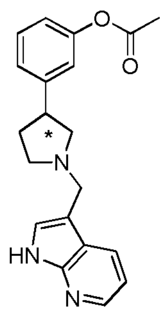 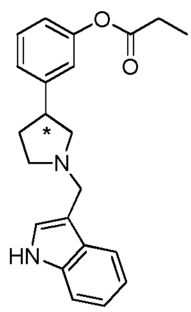 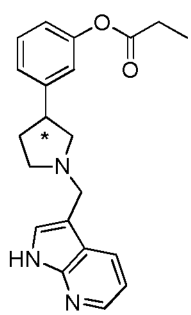 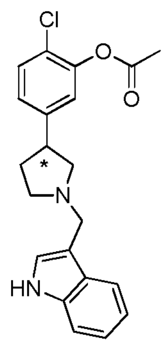 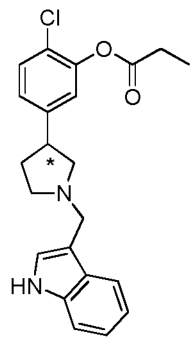

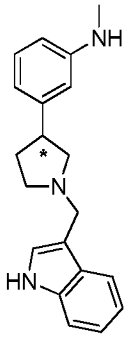 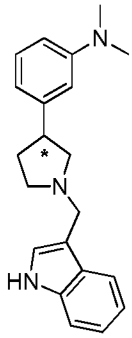 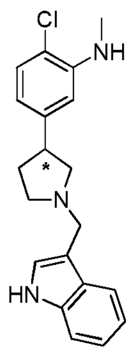 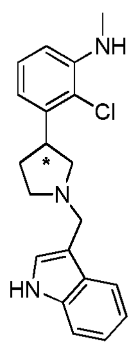 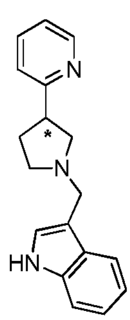 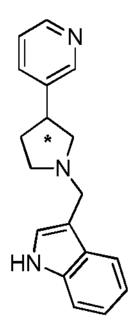

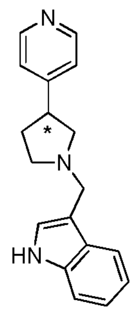
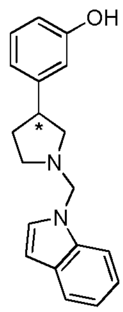
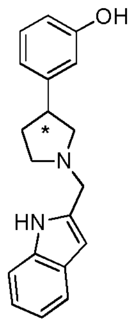
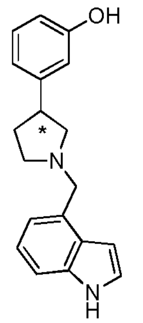
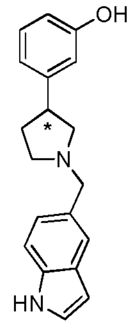
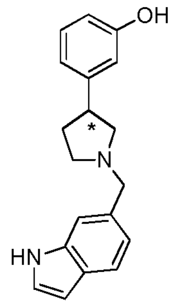

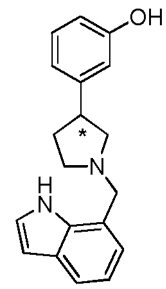
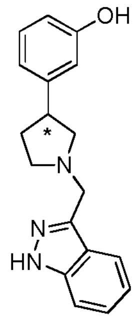
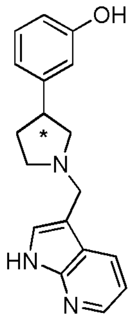
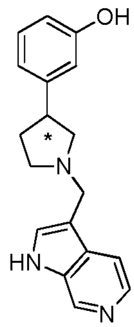
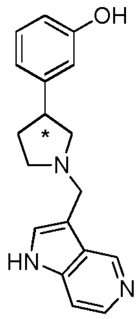
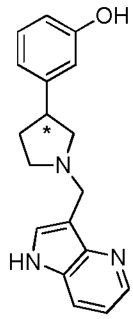

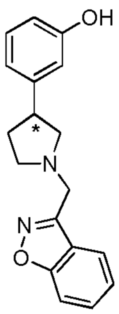
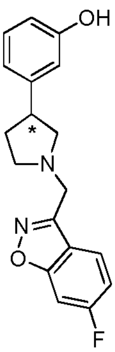
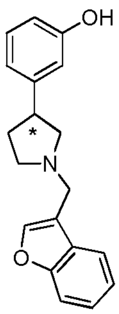
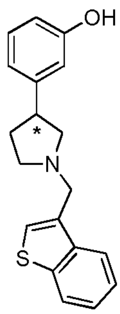
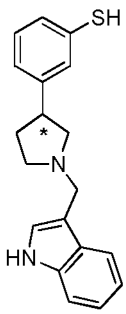
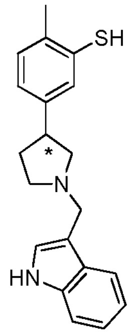

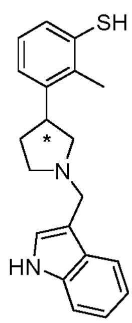
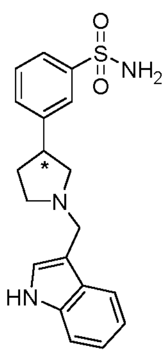
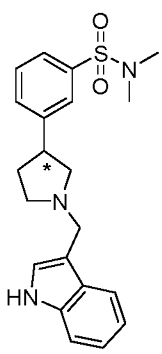
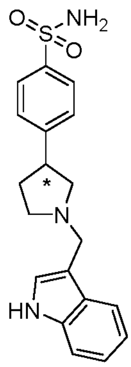
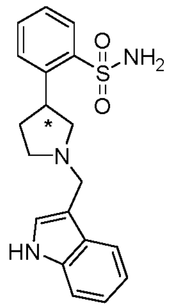
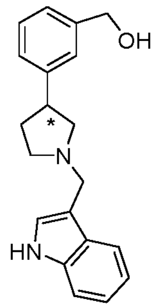

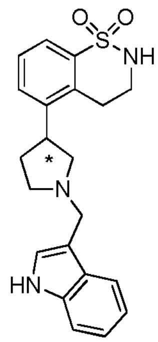 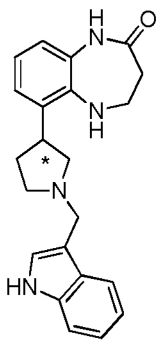 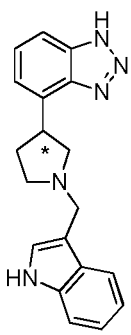 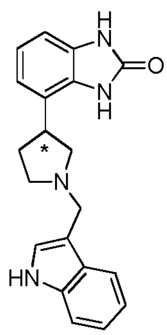 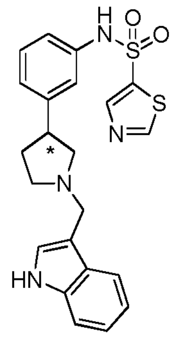 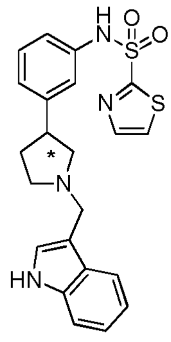

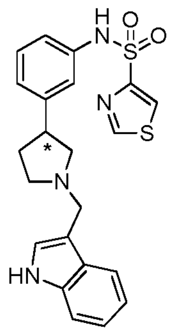 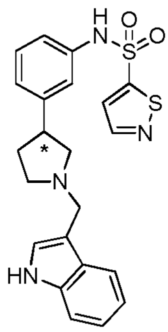 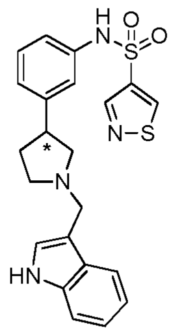 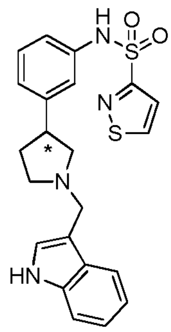 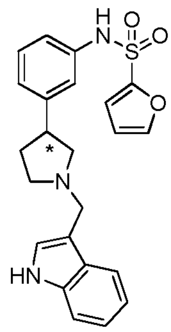 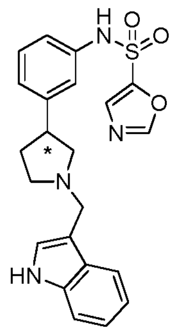

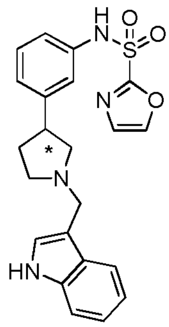 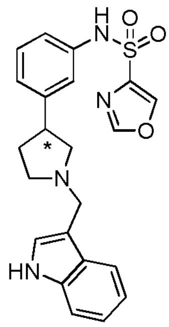 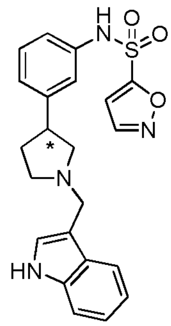 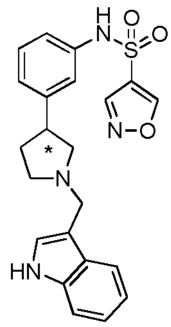 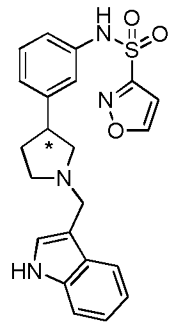 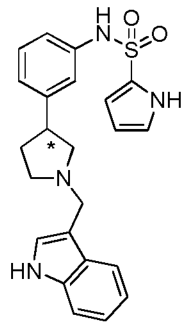

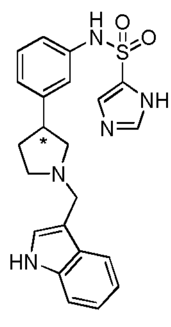 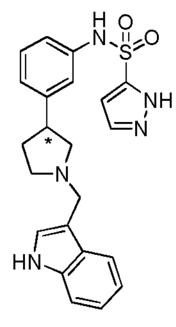 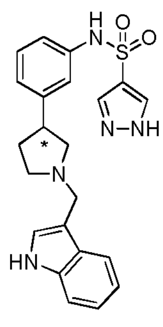 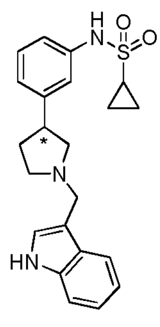 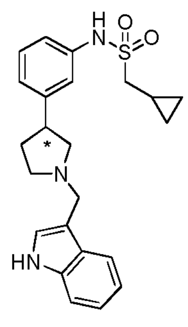 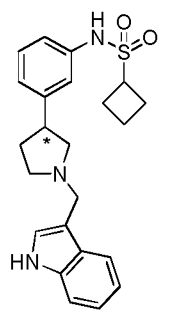

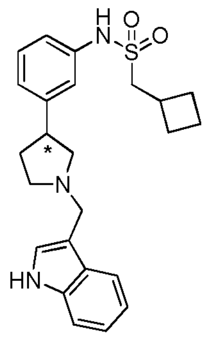
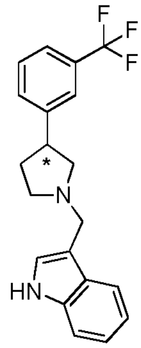
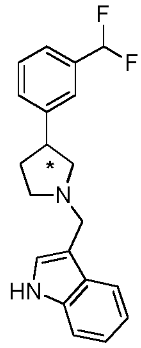
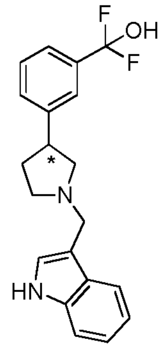
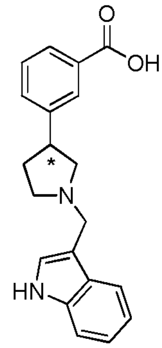
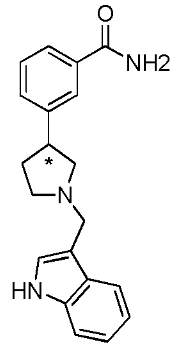

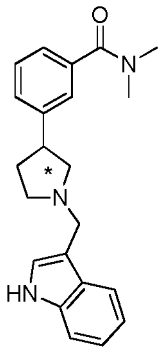
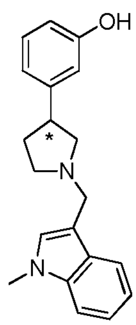
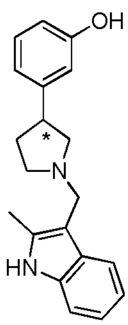
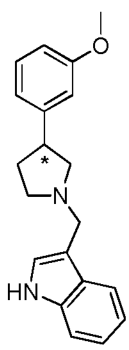
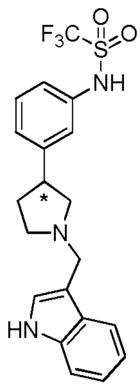
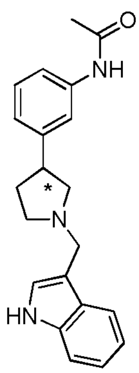

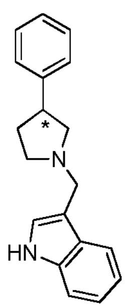
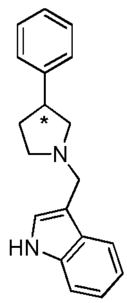
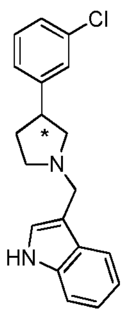
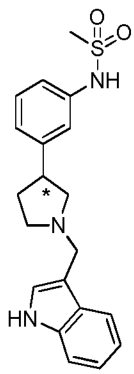
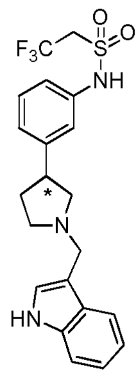
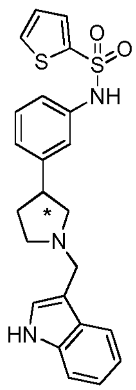

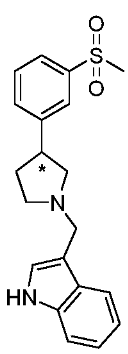
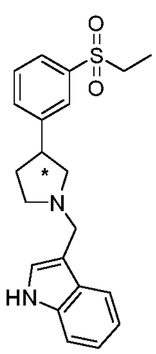
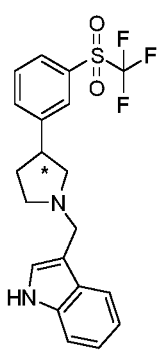
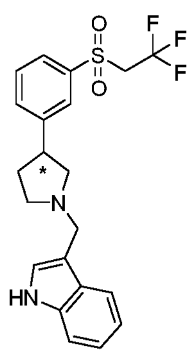
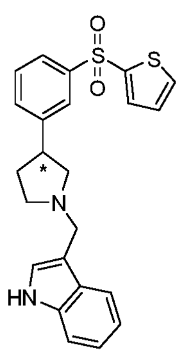
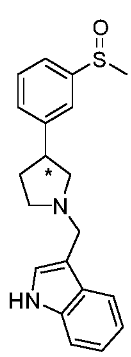

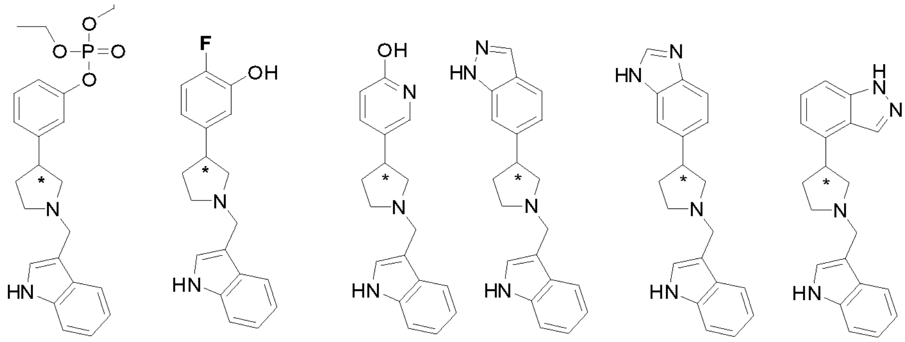

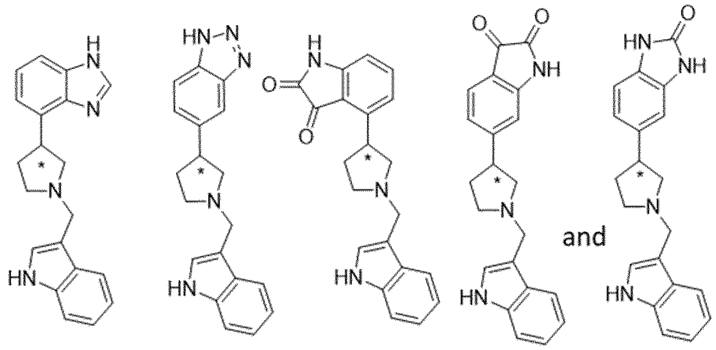

and

wherein, carbon labeled with * refers to S-configuration chiral carbon, R-configuration chiral carbon or achiral carbon.

**13.** A method for preparing the tetrahydropyrrole compound represented by general formula (I) as defined in any one of claims 1-12:

when L is a direct bond and K is

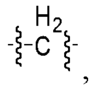

,

then the tetrahydropyrrole compound is prepared by the following method 1, which comprises the following steps: compound I-M and

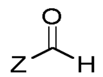

are subjected to a reductive amination reaction as shown below to prepare compound I-A;

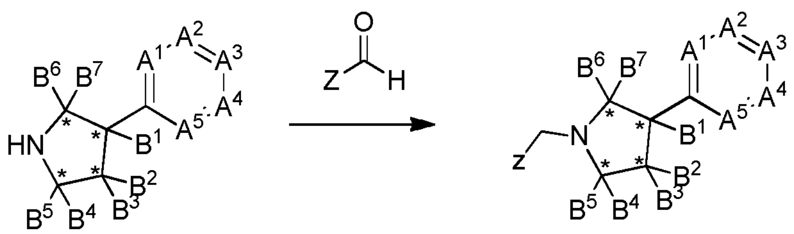

I-M I-A

wherein $B_1$-$B_7$, $A^1$-$A^5$, Z and * are defined as in any one of claims 1-12;

when Z is substituted or unsubstituted $C_2$-$C_{10}$ heteroaryl containing at least one N atom, then the tetrahydropyrrole compound is prepared by the following method 2, which comprises the following steps: compound I-Ma

is subjected to the following deamination reaction to remove amino protecting group so as to prepare the tetrahydropyrrole compound represented by general formula (I);

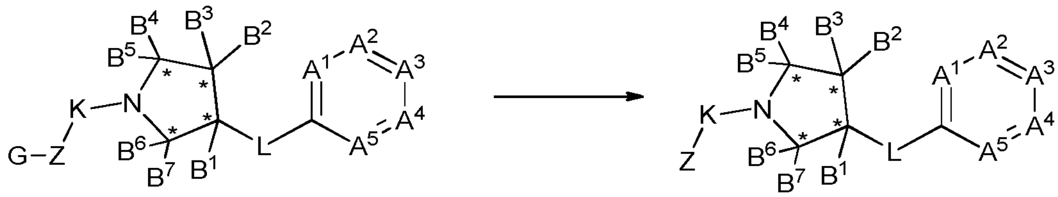

I-Ma (I)

wherein L, Z, K, $B_1$-$B_7$, $A^1$-$A^5$, Z and * are defined as in any one of claims 1-12; in compound I-Ma, G is an amino protecting group, wherein G is connected to a nitrogen atom in Z.

**14.** A pharmaceutical composition, which comprises the tetrahydropyrrole compound represented by general formula (I), the enantiomer, the diastereomer, the isotope compound, the pharmaceutically acceptable prodrug, the pharmaceutically acceptable ester or the pharmaceutically acceptable salt thereof as defined in any one of claims 1-12, and a pharmaceutically acceptable excipient.

**15.** A pharmaceutical composition, which comprises the tetrahydropyrrole compound represented by general formula (I), the enantiomer, the diastereomer, the isotope compound, the pharmaceutically acceptable prodrug, the pharmaceutically acceptable ester or the pharmaceutically acceptable salt thereof as defined in any one of claims 1-12, and additional therapeutic drugs; the additional therapeutic drugs are drugs for treating or preventing lesions and central nervous system diseases associated with dopamine receptor and dopamine transporter dysfunction; the lesions and central nervous system diseases associated with dopamine receptor and dopamine transporter dysfunction are preferably one or more of schizophrenia, and positive symptoms, negative symptoms, cognitive impairment, schizoaffective disorder, bipolar disorder, mania, depression, anxiety disorder, dementia, memory impairment and psychosis involving paranoia and/or delusion associated with schizophrenia.

**16.** Use of the tetrahydropyrrole compound represented by general formula (I), the enantiomer, the diastereomer, the isotope compound, the pharmaceutically acceptable prodrug, the pharmaceutically acceptable ester or the pharmaceutically acceptable salt thereof as defined in any one of claims 1-12 in the preparation of D2 receptor and DAT receptor inhibitors.

**17.** Use of the tetrahydropyrrole compound represented by general formula (I), the enantiomer, the diastereomer, the isotope compound, the pharmaceutically acceptable prodrug, the pharmaceutically acceptable ester or the pharmaceutically acceptable salt thereof as defined in any one of claims 1-12 in the manufacture of a medicament for the treatment or prevention of schizophrenia or diseases associated with schizophrenia; the diseases associated with schizophrenia are preferably one or more of positive symptoms, negative symptoms, cognitive impairment, schizoaffective disorder, bipolar disorder, mania, depression, anxiety disorder, dementia, memory impairment and psychosis involving paranoia and/or delusion associated with schizophrenia.

**INTERNATIONAL SEARCH REPORT**

International application No.
**PCT/CN2018/123751**

**A. CLASSIFICATION OF SUBJECT MATTER**

C07D 487/04(2006.01)i; C07D 493/04(2006.01)i; C07D 495/04(2006.01)i; C07D 498/04(2006.01)i; C07D 513/00(2006.01)i; A61P 25/18(2006.01)i

According to International Patent Classification (IPC) or to both national classification and IPC

**B. FIELDS SEARCHED**

Minimum documentation searched (classification system followed by classification symbols)

C07D487/-; C07D493/-; C07D495/-; C07D498/-; C07D513/-; A61P25/-

Documentation searched other than minimum documentation to the extent that such documents are included in the fields searched

Electronic data base consulted during the international search (name of data base and, where practicable, search terms used)

CNABS, CNKI, DWPI, SIPOABS, REG (STN), CAPLUS (STN): D2受体, DAT, 精神分裂, 精神病, 吡咯, 拮抗剂, 抑制剂, pyrrole, pyrrol, Schizophrenic disorder, mental disease, psychosis, antagonist, inhibitor, search for structural formula of the compounds based on claims 1, 11 and 12

**C. DOCUMENTS CONSIDERED TO BE RELEVANT**

| Category* | Citation of document, with indication, where appropriate, of the relevant passages | Relevant to claim No. |
|---|---|---|
| A | DONALD, M.N. et al.,. "Synthesis and Evaluation of Ligands for D2-Like Receptors: The Role of Common Pharmacophoric Groups" *Bioorganic & Medicinal Chemistry,* No. number 17, 31 December 2009 (2009-12-31), page 1717, figure 2, compounds 4 and 5 of the product and page 1718, road map 3, compound 13 | 1-17 |
| A | FELDMAN, P.L. et al.,. "Phosphodiesterase Type IV Inhibition. Structure - Activity Relationships of 1,3-Disubstituted Pyrrolidines" *Journal of Medicinal Chemistry,* Vol. 9, No. (38), 31 December 1995 (1995-12-31), entire document | 1-17 |
| A | US 2012157479 A1 (EVANS, G.B. ET AL.) 21 June 2012 (2012-06-21) see entire description | 1-17 |
| A | WO 2012074912 A1 (EINSTEIN COLL MED. ET AL.) 07 June 2012 (2012-06-07) see entire description | 1-17 |
| A | WO 2011050245 A1 (YIN, YAN ET AL.) 28 April 2011 (2011-04-28) see entire description | 1-17 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **22 March 2019** | **08 April 2019** |
| Name and mailing address of the ISA/CN<br>**State Intellectual Property Office of the P. R. China**<br>**No. 6, Xitucheng Road, Jimenqiao Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

| International application No. |
|---|
| **PCT/CN2018/123751** |

**C. DOCUMENTS CONSIDERED TO BE RELEVANT**

| Category* | Citation of document, with indication, where appropriate, of the relevant passages | Relevant to claim No. |
|---|---|---|
| A | WO 2008063670 A1 (ALANTOS PHARM. HOLDING ET AL.) 29 May 2008 (2008-05-29)<br>see entire description | 1-17 |
| A | 杨飞瀑等 (YANG, Feipu et al.). "药学学报 (Acta Pharmaceutica Sinica)"<br>*抗精神分裂症药物研究进展 (Research Progress of Antipsychotics),*<br>Vol. 12, No. (51), 12 December 2016 (2016-12-12),<br>entire document | 1-17 |

Form PCT/ISA/210 (second sheet) (January 2015)

**INTERNATIONAL SEARCH REPORT**
**Information on patent family members**

International application No.
**PCT/CN2018/123751**

| Patent document cited in search report | Publication date (day/month/year) | Patent family member(s) | Publication date (day/month/year) |
|---|---|---|---|
| US 2012157479 A1 | 21 June 2012 | ES 2687168 T3 | 24 October 2018 |
| | | US 9957272 B2 | 01 May 2018 |
| | | EP 2454263 A1 | 23 May 2012 |
| | | JP 5861243 B2 | 16 February 2016 |
| | | WO 2011008110 A1 | 20 January 2011 |
| | | JP 2012533538 A | 27 December 2012 |
| | | EP 2454263 B1 | 13 June 2018 |
| | | AU 2010271532 A1 | 09 February 2012 |
| | | US 9493465 B2 | 15 November 2016 |
| | | DK 2454263 T3 | 01 October 2018 |
| | | EP 2454263 A4 | 19 December 2012 |
| | | US 2017166573 A1 | 15 June 2017 |
| | | CA 2768291 C | 27 March 2018 |
| | | AU 2010271532 B2 | 03 March 2016 |
| | | CA 2768291 A1 | 20 January 2011 |
| WO 2012074912 A1 | 07 June 2012 | US 9290501 B2 | 22 March 2016 |
| | | US 2013274220 A1 | 17 October 2013 |
| WO 2011050245 A1 | 28 April 2011 | None | |
| WO 2008063670 A1 | 29 May 2008 | CA 2670042 A1 | 29 May 2008 |
| | | EP 2099803 A1 | 16 September 2009 |
| | | US 2008221092 A1 | 11 September 2008 |
| | | EP 2094707 A1 | 02 September 2009 |
| | | AU 2007321923 A1 | 29 May 2008 |
| | | AU 2007321922 A1 | 29 May 2008 |
| | | US 2008221083 A1 | 11 September 2008 |
| | | WO 2008063669 A1 | 29 May 2008 |
| | | CA 2670044 A1 | 29 May 2008 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 201711435683

### Non-patent literature cited in the description


- **KULAGOWSKI et al.** *J. Med. Chem.,* 1996, vol. 39, 1941-1942
- **SIKAZWE et al.** *Bioorg. Med. Chem.,* 2009, vol. 17, 1716-1723
- *J. Med. Chem.,* 1996, vol. 39, 1941-1942
- *Bioorg. Med. Chem.,* 2009, vol. 17, 1716-1723